# EUROPEAN PATENT APPLICATION

(11) **EP 2 669 371 A1**
(43) Date of publication of application: **04.12.2013**
(21) Application number: 13164698.6
(22) Date of filing: 10.11.2010
(51) Int. Cl.: C12N 9/02, C12N 15/82

(54) **HPPD variants and methods of use**

(62) Divisional of application: 10190629.5
(71) Applicant: Bayer CropScience AG, 40789 Monheim (DE)
(72) Inventor: Lange, Gudrun, 65779 Kelkheim (DE); Poree, Fabien, 65936 Frankfurt (DE); Laber, Bernd, 65510 Idstein (DE); Freigang, Jörg, 40764 Langenfeld (DE); Schulz, Arno, 65817 Eppstein (DE)
(74) Representative: BIP Patents

(57) **Abstract**

The present invention relates to an isolated nucleic acid comprising a nucleotide sequence encoding a mutated HPPD protein, wherein said mutated HPPD protein has HPPD activity, wherein in said mutated HPPD protein at least one amino acid has been replaced so that the resulting amino acid sequence comprises at least one amino acid selected from certain amino acids at specific positions important for conferring an increased HPPD inhibitor tolerance. The present invention also relates to proteins encoded by the nucleic acid of the invention, to chimeric genes, plant cells comprising the nucleic acid of the invention operably linked to a plant-expressible promoter and optionally a transcription termination and polyadenylation region, plants essentially consisting of the plant cells of the invention and methods of obtaining transgenic plants.

SEQUENCE LISTING

<110>  Bayer CropScience AG

<120>  HPPD variants and methods of use

<130>  BCS 10-1029EP04

<140>  EP10190629
<141>  10 November 2010

<160>  50

<170>  PatentIn version 3.3

<210>  1
<211>  1335
<212>  DNA
<213>  Arabidopsis thaliana

<400>  1
atgggccacc aaaacgccgc cgtttcagag aatcaaaacc atgatgacgg cgctgcgtcg      60

tcgccgggat tcaagctcgt cggattttcc aagttcgtaa gaaagaatcc aaagtctgat     120

aaattcaagg ttaagcgctt ccatcacatc gagttctggt gcggcgacgc aaccaacgtc     180

gctcgtcgct tctcctgggg tctggggatg agattctccg ccaaatccga tctttccacc     240

ggaaacatgg ttcacgcctc ttacctactc acctccggtg acctccgatt ccttttcact     300

gctccttact ctccgtctct ctccgccgga gagattaaac cgacaaccac agcttctatc     360

ccaagtttcg atcacggctc ttgtcgttcc ttcttctctt cacatggtct cggtgttaga     420

gccgttgcga ttgaagtaga agacgcagag tcagctttct ccatcagtgt agctaatggc     480

gctattcctt cgtcgcctcc tatcgtcctc aatgaagcag ttacgatcgc tgaggttaaa     540

ctatacggcg atgttgttct ccgatatgtt agttacaaag cagaagatac cgaaaaatcc     600

gaattcttgc cagggttcga gcgtgtagag gatgcgtcgt cgttcccatt
ggattatggt     660

atccggcggc ttgaccacgc cgtgggaaac gttcctgagc ttggtccggc
tttaacttat     720

gtagcggggt tcactggttt tcaccaattc gcagagttca cagcagacga
cgttggaacc     780

gccgagagcg gtttaaattc agcggtcctg gctagcaatg atgaaatggt
tcttctaccg     840

attaacgagc cagtgcacgg aacaaagagg aagagtcaga ttcagacgta
tttggaacat     900

aacgaaggcg caggctacaa acatctggct ctgatgagtg aagacatatt
caggaccctg     960

agagagatga ggaagaggag cagtattgga ggattcgact tcatgccttc
tcctccgcct    1020

acttactacc agaatctcaa gaaacgggtc ggcgacgtgc tcagcgatga
tcagatcaag    1080

gagtgtgagg aattagggat tcttgtagac agagatgatc aagggacgtt
gcttcaaatc    1140

ttcacaaaac cactaggtga caggccgacg atatttatag agataatcca
gagagtagga    1200

tgcatgatga aagatgagga agggaaggct taccagagtg gaggatgtgg
tggttttggc    1260

aaaggcaatt tctctgagct cttcaagtcc attgaagaat acgaaaagac
tcttgaagcc    1320

aaacagttag tggga
1335

<210>  2
<211>  445
<212>  PRT
<213>  Arabidopsis thaliana

<400>  2

Met Gly His Gln Asn Ala Ala Val Ser Glu Asn Gln Asn His Asp
Asp
1               5                   10                  15

Gly Ala Ala Ser Ser Pro Gly Phe Lys Leu Val Gly Phe Ser Lys
Phe
20 25 30

Val Arg Lys Asn Pro Lys Ser Asp Lys Phe Lys Val Lys Arg Phe
His
35 40 45

His Ile Glu Phe Trp Cys Gly Asp Ala Thr Asn Val Ala Arg Arg
Phe
50 55 60

Ser Trp Gly Leu Gly Met Arg Phe Ser Ala Lys Ser Asp Leu Ser
Thr
65 70 75 80

Gly Asn Met Val His Ala Ser Tyr Leu Leu Thr Ser Gly Asp Leu
Arg
85 90 95

Phe Leu Phe Thr Ala Pro Tyr Ser Pro Ser Leu Ser Ala Gly Glu
Ile
100 105 110

Lys Pro Thr Thr Thr Ala Ser Ile Pro Ser Phe Asp His Gly Ser
Cys
115 120 125

Arg Ser Phe Phe Ser Ser His Gly Leu Gly Val Arg Ala Val Ala
Ile
130 135 140

Glu Val Glu Asp Ala Glu Ser Ala Phe Ser Ile Ser Val Ala Asn
Gly
145 150 155
160

Ala Ile Pro Ser Ser Pro Pro Ile Val Leu Asn Glu Ala Val Thr
Ile
165 170 175

Ala Glu Val Lys Leu Tyr Gly Asp Val Val Leu Arg Tyr Val Ser
Tyr
            180                 185                 190

Lys Ala Glu Asp Thr Glu Lys Ser Glu Phe Leu Pro Gly Phe Glu
Arg
        195                 200                 205

Val Glu Asp Ala Ser Ser Phe Pro Leu Asp Tyr Gly Ile Arg Arg
Leu
    210                 215                 220

Asp His Ala Val Gly Asn Val Pro Glu Leu Gly Pro Ala Leu Thr
Tyr
225                 230                 235
240

Val Ala Gly Phe Thr Gly Phe His Gln Phe Ala Glu Phe Thr Ala
Asp
                245                 250                 255

Asp Val Gly Thr Ala Glu Ser Gly Leu Asn Ser Ala Val Leu Ala
Ser
            260                 265                 270

Asn Asp Glu Met Val Leu Leu Pro Ile Asn Glu Pro Val His Gly
Thr
        275                 280                 285

Lys Arg Lys Ser Gln Ile Gln Thr Tyr Leu Glu His Asn Glu Gly
Ala
    290                 295                 300

Gly Leu Gln His Leu Ala Leu Met Ser Glu Asp Ile Phe Arg Thr
Leu
305                 310                 315
320

Arg Glu Met Arg Lys Arg Ser Ser Ile Gly Gly Phe Asp Phe Met
Pro
                325                 330                 335

Ser Pro Pro Pro Thr Tyr Tyr Gln Asn Leu Lys Lys Arg Val Gly
Asp
            340                 345                 350

Val Leu Ser Asp Asp Gln Ile Lys Glu Cys Glu Glu Leu Gly Ile
Leu
        355                 360                 365

Val Asp Arg Asp Asp Gln Gly Thr Leu Leu Gln Ile Phe Thr Lys
Pro
    370                 375                 380

Leu Gly Asp Arg Pro Thr Ile Phe Ile Glu Ile Ile Gln Arg Val
Gly
385                 390                 395
400

Cys Met Met Lys Asp Glu Glu Gly Lys Ala Tyr Gln Ser Gly Gly
Cys
                405                 410                 415

Gly Gly Phe Gly Lys Gly Asn Phe Ser Glu Leu Phe Lys Ser Ile
Glu
            420                 425                 430

Glu Tyr Glu Lys Thr Leu Glu Ala Lys Gln Leu Val Gly
        435                 440                 445

<210>  3
<211>  1341
<212>  DNA
<213>  Oryza sativa

<400>  3
atgcctccca ctcccacccc caccgccacc accggcgccg tctcggccgc
tgcggcggcg      60

ggggagaacg cggggttccg cctcgtcggg caccgccgct tcgtccgcgc
caacccgcgg     120

agcgaccggt tccaggcgct cgcgttccac cacgtcgagc tctggtgcgc
cgacgccgcg     180

tccgccgcgg gccggttcgc cttcgccctg ggcgcgccgc tcgccgccag
gtccgacctc     240

tccacgggga actccgcgca cgcctccctc ctcctccgct ccgcctccgt
cgcgttcctc    300

ttcaccgccc cctacggcgg cgaccacggc gtcggcgcgg acgcggccac
caccgcctcc    360

atcccttcct tctccccagg cgccgcgcgg aggttcgccg cggaccacgg
cctcgcggtg    420

cacgccgtgg cgctgcgcgt cgccgacgcg gccgacgcct tccgcgccag
cgtcgcggcc    480

ggtgcgcgcc cggcgttcca gcccgccgac ctcggcggtg gcttcggcct
cgcggaggtg    540

gagctctacg gcgacgtcgt gctccgcttc gtcagccacc cggacggcgc
cgacgcgccc    600

ttcctcccgg gtttcgaggg cgtcagcaac ccgggcgccg tggactacgg
cctccgccgg    660

ttcgaccacg tcgtcggcaa cgtgccggag ctcgctccgg tagccgcgta
catctccggg    720

ttcaccgggt tccacgagtt cgccgagttc accgccgagg acgtgggcac
cgccgagagc    780

ggcctcaact cggtggtgct cgccaacaac gcggagaccg tgctgctgcc
gctcaacgag    840

ccggtgcacg gcaccaagcg gcggagccag atacagacgt acctggacca
ccacggcggc    900

ccgggggtgc agcacatcgc gctggccagc gacgacgtgc tcggacgct
gagggagatg    960

cgggcgcgct ccgccatggg cggcttcgag ttcttggcgc cgccgccgcc
caactactac   1020

gacggcgtgc ggcggcgcgc cggggacgtg ctctcggagg agcagatcaa
cgagtgccag   1080

gagctcgggg tgctcgtgga cagggatgac cagggggtgt tgctccagat
cttcaccaag   1140

ccagtaggag acaggccaac ctttttcttg gagatgatac aaaggattgg
gtgcatggag   1200

aaggatgaga gtgggcagga gtaccagaag ggcggctgcg gcgggtttgg
gaagggcaac   1260

ttctcggagc tgttcaagtc cattgaggag tatgagaaat cccttgaagc
caagcaagcc   1320

cctacagttc aaggatccta g
1341

<210>  4
<211>  446
<212>  PRT
<213>  Oryza sativa

<400>  4

Met Pro Pro Thr Pro Thr Pro Thr Ala Thr Thr Gly Ala Val Ser
Ala
1               5                   10                  15

Ala Ala Ala Ala Gly Glu Asn Ala Gly Phe Arg Leu Val Gly His
Arg
            20                  25                  30

Arg Phe Val Arg Ala Asn Pro Arg Ser Asp Arg Phe Gln Ala Leu
Ala
        35                  40                  45

Phe His His Val Glu Leu Trp Cys Ala Asp Ala Ala Ser Ala Ala
Gly
    50                  55                  60

Arg Phe Ala Phe Ala Leu Gly Ala Pro Leu Ala Ala Arg Ser Asp
Leu
65                  70                  75                  80

Ser Thr Gly Asn Ser Ala His Ala Ser Leu Leu Leu Arg Ser Ala
Ser
                85                  90                  95

Val Ala Phe Leu Phe Thr Ala Pro Tyr Gly Gly Asp His Gly Val
Gly
            100                 105                 110

Ala Asp Ala Ala Thr Thr Ala Ser Ile Pro Ser Phe Ser Pro Gly
Ala
        115                 120                 125

Ala Arg Arg Phe Ala Ala Asp His Gly Leu Ala Val His Ala Val
Ala
    130                 135                 140

Leu Arg Val Ala Asp Ala Ala Asp Ala Phe Arg Ala Ser Val Ala
Ala
145                 150                 155
160

Gly Ala Arg Pro Ala Phe Gln Pro Ala Asp Leu Gly Gly Gly Phe
Gly
                165                 170                 175

Leu Ala Glu Val Glu Leu Tyr Gly Asp Val Val Leu Arg Phe Val
Ser
            180                 185                 190

His Pro Asp Gly Ala Asp Ala Pro Phe Leu Pro Gly Phe Glu Gly
Val
        195                 200                 205

Ser Asn Pro Gly Ala Val Asp Tyr Gly Leu Arg Arg Phe Asp His
Val
    210                 215                 220

Val Gly Asn Val Pro Glu Leu Ala Pro Val Ala Ala Tyr Ile Ser
Gly
225                 230                 235
240

Phe Thr Gly Phe His Glu Phe Ala Glu Phe Thr Ala Glu Asp Val
Gly
                245                 250                 255

Thr Ala Glu Ser Gly Leu Asn Ser Val Val Leu Ala Asn Asn Ala
Glu
            260                 265                 270

Thr Val Leu Leu Pro Leu Asn Glu Pro Val His Gly Thr Lys Arg
Arg
        275                 280                 285

Ser Gln Ile Gln Thr Tyr Leu Asp His His Gly Gly Pro Gly Val
Gln
    290                 295                 300

His Ile Ala Leu Ala Ser Asp Asp Val Leu Gly Thr Leu Arg Glu
Met
305                 310                 315
320

Arg Ala Arg Ser Ala Met Gly Gly Phe Glu Phe Leu Ala Pro Pro
Pro
                325                 330                 335

Pro Asn Tyr Tyr Asp Gly Val Arg Arg Arg Ala Gly Asp Val Leu
Ser
            340                 345                 350

Glu Glu Gln Ile Asn Glu Cys Gln Glu Leu Gly Val Leu Val Asp
Arg
        355                 360                 365

Asp Asp Gln Gly Val Leu Leu Gln Ile Phe Thr Lys Pro Val Gly
Asp
    370                 375                 380

Arg Pro Thr Phe Phe Leu Glu Met Ile Gln Arg Ile Gly Cys Met
Glu
385                 390                 395
400

Lys Asp Glu Ser Gly Gln Glu Tyr Gln Lys Gly Gly Cys Gly Gly
Phe
                405                 410                 415

Gly Lys Gly Asn Phe Ser Glu Leu Phe Lys Ser Ile Glu Glu Tyr
Glu
            420                 425                 430

Lys Ser Leu Glu Ala Lys Gln Ala Pro Thr Val Gln Gly Ser
        435                 440                 445

<210>  5
<211>  1281
<212>  DNA
<213>  Zea mays

<400>  5
atgcccccga cccccacagc cgccgcagcc ggcgccgccg tggcggcggc
atcagcagcg      60

gagcaggcgg cgttccgcct cgtgggccac cgcaacttcg tccgcttcaa
cccgcgctcc     120

gaccgcttcc acacgctcgc gttccaccac gtggagctct ggtgcgccga
cgcggcctcc     180

gccgcgggcc gcttctcctt cggcctgggc gcgccgctcg ccgcacgctc
cgacctctcc     240

acgggcaact ccgcgcacgc gtccctgctg ctccgctccg gctccctctc
cttcctcttc     300

acggcgccct acgcgcacgg cgccgacgct gccaccgccg cgctgccctc
cttctccgcc     360

gccgccgcgc ggcgcttcgc agccgaccac ggcctcgcgg tgcgcgccgt
cgcgctccgc     420

gtcgccgacg ccgaggacgc cttccgcggc ttccgcctcg ccgaggtcga
gctctacggc     480

gacgtcgtgc tccggtacgt gagctacccg gacggcgccg cgggcgagcc
cttcctgccg     540

gggttcgagg gcgtggccag ccccgggcgg gccgactacg ggctgagcag
gttcgaccac     600

atcgtcggca acgtgccgga gctggcgccc gccgccgcct acttcgccgg
cttcacgggg     660

ttccacgagt tcgccgagtt cacgacggag gacgtgggca ccgcggagag
cggcctcaac     720

tccatggtgc tcgccaacaa ctcggagaac gtgctgctcc cgctcaacga
gccggtgcac     780

ggcaccaagc gccgcagcca gatacaaacg ttcctggacc accacggcgg
ccccggcgtg     840

cagcacatgg cgctggccag cgacgacgtg ctcaggacgc tgagggagat
gcaggcgcgc     900

tcggccatgg gcggcttcga gttcatggcg cctcccacat ccgactacta
tgacggcgtg    960

aggcggcgcg ccggggacgt gctcacggaa gcacagatta aggagtgcca
ggagctaggg    1020

gtgctggtgg acagggatga ccagggcgtg ctgctccaaa tcttcaccaa
gccagtgggg    1080

gacaggccaa cgctgttctt ggaaatcatc caaaggatcg ggtgcatgga
gaaggatgag    1140

aaggggcaag aataccaaaa gggtggctgc ggcgggttcg gcaagggaaa
cttctcgcag    1200

ctgttcaagt ccatcgagga ttatgagaag tcccttgaag ccaagcaagc
tgctgcagca    1260

gctgcagctc agggatccta g
1281



<210>  6
<211>  426
<212>  PRT
<213>  Zea mays

<400>  6

Met Pro Pro Thr Pro Thr Ala Ala Ala Ala Gly Ala Ala Val Ala
Ala
1               5                   10                  15


Ala Ser Ala Ala Glu Gln Ala Ala Phe Arg Leu Val Gly His Arg
Asn
            20                  25                  30


Phe Val Arg Phe Asn Pro Arg Ser Asp Arg Phe His Thr Leu Ala
Phe
        35                  40                  45


His His Val Glu Leu Trp Cys Ala Asp Ala Ala Ser Ala Ala Gly
Arg
    50                  55                  60


Phe Ser Phe Gly Leu Gly Ala Pro Leu Ala Ala Arg Ser Asp Leu
Ser
65                  70                  75                  80

Thr Gly Asn Ser Ala His Ala Ser Leu Leu Leu Arg Ser Gly Ser
Leu
                85                  90                  95

Ser Phe Leu Phe Thr Ala Pro Tyr Ala His Gly Ala Asp Ala Ala
Thr
            100                 105                 110

Ala Ala Leu Pro Ser Phe Ser Ala Ala Ala Ala Arg Arg Phe Ala
Ala
        115                 120                 125

Asp His Gly Leu Ala Val Arg Ala Val Ala Leu Arg Val Ala Asp
Ala
    130                 135                 140

Glu Asp Ala Phe Arg Gly Phe Arg Leu Ala Glu Val Glu Leu Tyr
Gly
145                 150                 155
160

Asp Val Val Leu Arg Tyr Val Ser Tyr Pro Asp Gly Ala Ala Gly
Glu
                165                 170                 175

Pro Phe Leu Pro Gly Phe Glu Gly Val Ala Ser Pro Gly Ala Ala
Asp
            180                 185                 190

Tyr Gly Leu Ser Arg Phe Asp His Ile Val Gly Asn Val Pro Glu
Leu
        195                 200                 205

Ala Pro Ala Ala Ala Tyr Phe Ala Gly Phe Thr Gly Phe His Glu
Phe
    210                 215                 220

Ala Glu Phe Thr Thr Glu Asp Val Gly Thr Ala Glu Ser Gly Leu
Asn
225                 230                 235
240

Ser Met Val Leu Ala Asn Asn Ser Glu Asn Val Leu Leu Pro Leu
Asn
                245                 250                 255

Glu Pro Val His Gly Thr Lys Arg Arg Ser Gln Ile Gln Thr Phe
Leu
            260                 265                 270

Asp His His Gly Gly Pro Gly Val Gln His Met Ala Leu Ala Ser
Asp
        275                 280                 285

Asp Val Leu Arg Thr Leu Arg Glu Met Gln Ala Arg Ser Ala Met
Gly
    290                 295                 300

Gly Phe Glu Phe Met Ala Pro Pro Thr Ser Asp Tyr Tyr Asp Gly
Val
305                 310                 315
320

Arg Arg Arg Ala Gly Asp Val Leu Thr Glu Ala Gln Ile Lys Glu
Cys
                325                 330                 335

Gln Glu Leu Gly Val Leu Val Asp Arg Asp Asp Gln Gly Val Leu
Leu
            340                 345                 350

Gln Ile Phe Thr Lys Pro Val Gly Asp Arg Pro Thr Leu Phe Leu
Glu
        355                 360                 365

Ile Ile Gln Arg Ile Gly Cys Met Glu Lys Asp Glu Lys Gly Gln
Glu
    370                 375                 380

Tyr Gln Lys Gly Gly Cys Gly Gly Phe Gly Lys Gly Asn Phe Ser
Gln
385                 390                 395
400

Leu Phe Lys Ser Ile Glu Asp Tyr Glu Lys Ser Leu Glu Ala Lys
Gln
                405                 410                 415

Ala Ala Ala Ala Ala Ala Ala Gln Gly Ser
            420                 425

<210>  7
<211>  1323
<212>  DNA
<213>  Avena sativa

<400>  7
atgccgccca cccccgccac cgccaccggc gccgccgcgg ccgccgtgac
tccagagcac      60

gcggcccgga gctttccccg agtggtccgc gtcaacccgc gcagcgaccg
cttccccgtg     120

ctctccttcc accacgtcga gctctggtgc gccgacgccg cctcagcggc
cggacgcttc     180

tccttcgcgc tcggcgcgcc gctcgccgcc cggtccgacc tctccacggg
gaactccgcg     240

cacgcctccc tcctgctccg ctcgggcgcc ctcgccttcc tcttcacggc
gccctacgcg     300

ccgccgccgc aggaggccgc cacggccgca gccaccgcct ccatcccctc
cttctccgcc     360

gacgccgcgc ggacgttcgc cgccgcccac ggcctcgcgg tgcgctccgt
cggggtccgc     420

gtcgctgacg ccgccgaggc cttccgcgtc agcgtagccg gcggcgctcg
cccggccttc     480

gccccagccg acctcggcca tggcttcggc ctcgccgagg tcgagctcta
cggcgacgtc     540

gtgctacgct tcgtcagcta cccggacgag acagacctgc cattcctgcc
agggttcgag     600

cgcgtgagca gccccggcgc cgtggactac ggcctcacgc ggttcgacca
cgtcgtgggc     660

aacgtcccgg agatggcccc ggtcatagac tacatgaaag gcttcttggg
gttccacgag     720

ttcgccgagt tcaccgccga ggacgtgggc acgaccgaga gcgggctcaa
ctcggtggtg    780

ctcgccaaca actccgaggc cgtgctgctg ccgctcaacg agcccgtgca
cggcacaaag    840

cgacggagcc agatacagac gtacctggag tatcacggcg ggcccggcgt
gcagcacatc    900

gcgctcgcca gcaacgacgt gctcaggacg ctcagggaga tgcgggcgcg
cacgcccatg    960

ggcggcttcg agttcatggc gccaccgcag gcgaaatact atgaaggcgt
gcggcgcatc   1020

gcaggtgacg tgctctcgga agagcagatc aaggaatgcc aggagctggg
ggtgctagtc   1080

gacagggatg atcaaggggt gttgctccaa atcttcacca agccagtagg
ggacaggcca   1140

acgtttttcc tggagatgat ccaaagaatc gggtgcatgg agaaggacga
ggtcgggcaa   1200

gagtaccaga agggtggctg cggcgggttt ggcaagggca atttctccga
gctgttcaag   1260

tccattgagg actatgagaa atcccttgag gtcaagcaat ctgttgtagc
tcagaaatcc   1320

tag
1323

<210>  8
<211>  440
<212>  PRT
<213>  Avena sativa

<400>  8

Met Pro Pro Thr Pro Ala Thr Ala Thr Gly Ala Ala Ala Ala Ala
Val
1               5                   10                  15

Thr Pro Glu His Ala Ala Arg Ser Phe Pro Arg Val Val Arg Val
Asn
            20                  25                  30

Pro Arg Ser Asp Arg Phe Pro Val Leu Ser Phe His His Val Glu
Leu
        35                  40                  45

Trp Cys Ala Asp Ala Ala Ser Ala Ala Gly Arg Phe Ser Phe Ala
Leu
    50                  55                  60

Gly Ala Pro Leu Ala Ala Arg Ser Asp Leu Ser Thr Gly Asn Ser
Ala
65                  70                  75                  80

His Ala Ser Leu Leu Leu Arg Ser Gly Ala Leu Ala Phe Leu Phe
Thr
                85                  90                  95

Ala Pro Tyr Ala Pro Pro Pro Gln Glu Ala Ala Thr Ala Ala Ala
Thr
            100                 105                 110

Ala Ser Ile Pro Ser Phe Ser Ala Asp Ala Ala Arg Thr Phe Ala
Ala
        115                 120                 125

Ala His Gly Leu Ala Val Arg Ser Val Gly Val Arg Val Ala Asp
Ala
    130                 135                 140

Ala Glu Ala Phe Arg Val Ser Val Ala Gly Gly Ala Arg Pro Ala
Phe
145                 150                 155
160

Ala Pro Ala Asp Leu Gly His Gly Phe Gly Leu Ala Glu Val Glu
Leu
                165                 170                 175

Tyr Gly Asp Val Val Leu Arg Phe Val Ser Tyr Pro Asp Glu Thr
Asp
            180                 185                 190

Leu Pro Phe Leu Pro Gly Phe Glu Arg Val Ser Ser Pro Gly Ala
Val
        195                 200                 205

Asp Tyr Gly Leu Thr Arg Phe Asp His Val Val Gly Asn Val Pro
Glu
    210                 215                 220

Met Ala Pro Val Ile Asp Tyr Met Lys Gly Phe Leu Gly Phe His
Glu
225                 230                 235
240

Phe Ala Glu Phe Thr Ala Glu Asp Val Gly Thr Thr Glu Ser Gly
Leu
                245                 250                 255

Asn Ser Val Val Leu Ala Asn Asn Ser Glu Ala Val Leu Leu Pro
Leu
            260                 265                 270

Asn Glu Pro Val His Gly Thr Lys Arg Arg Ser Gln Ile Gln Thr
Tyr
        275                 280                 285

Leu Glu Tyr His Gly Gly Pro Gly Val Gln His Ile Ala Leu Ala
Ser
    290                 295                 300

Asn Asp Val Leu Arg Thr Leu Arg Glu Met Arg Ala Arg Thr Pro
Met
305                 310                 315
320

Gly Gly Phe Glu Phe Met Ala Pro Pro Gln Ala Lys Tyr Tyr Glu
Gly
                325                 330                 335

Val Arg Arg Ile Ala Gly Asp Val Leu Ser Glu Glu Gln Ile Lys
Glu
            340                 345                 350

Cys Gln Glu Leu Gly Val Leu Val Asp Arg Asp Asp Gln Gly Val
Leu
        355                 360                 365

Leu Gln Ile Phe Thr Lys Pro Val Gly Asp Arg Pro Thr Phe Phe
Leu
    370                 375                 380

Glu Met Ile Gln Arg Ile Gly Cys Met Glu Lys Asp Glu Val Gly
Gln
385                 390                 395
400

Glu Tyr Gln Lys Gly Gly Cys Gly Gly Phe Gly Lys Gly Asn Phe
Ser
                405                 410                 415

Glu Leu Phe Lys Ser Ile Glu Asp Tyr Glu Lys Ser Leu Glu Val
Lys
            420                 425                 430

Gln Ser Val Val Ala Gln Lys Ser
        435                 440

<210>  9
<211>  1077
<212>  DNA
<213>  Pseudomonas fluorescens

<400>  9
atggcagatc tatacgaaaa cccaatgggc ctgatgggct ttgaattcat
cgaattcgcg      60

tcgccgacgc cgggtaccct ggagccgatc ttcgagatca tgggcttcac
caaagtcgcg     120

acccaccgtt ccaagaacgt gcacctgtac cgccagggcg agatcaacct
gatcctcaac     180

aacgagccca acagcatcgc ctcctacttt gcggccgaac acggcccgtc
ggtgtgcggc     240

atggcgttcc gcgtgaagga ctcgcaaaag gcctacaacc gcgccctgga
actcggcgcc     300

cagccgatcc atattgacac cgggccgatg gaattgaacc tgccggcgat
caagggcatc    360

ggcggcgcgc cgttgtacct gatcgaccgt ttcggcgaag gcagctcgat
ctacgacatc    420

gacttcgtgt acctcgaagg tgtggagcgc aatccggtcg gtgcaggtct
caaagtcatc    480

gaccacctga cccacaacgt ctatcgcggc cgcatggtct actgggccaa
cttctacgag    540

aaattgttca acttccgtga agcgcgttac ttcgatatca agggcgagta
caccggcctg    600

acttccaagg ccatgagtgc gccggacggc atgatccgca tcccgctgaa
cgaagagtcg    660

tccaagggcg cggggcagat cgaagagttc ctgatgcagt tcaacggcga
aggcatccag    720

cacgtggcgt tcctcaccga cgacctggtc aagacctggg acgcgttgaa
gaaaatcggc    780

atgcgcttca tgaccgcgcc gccagacact tattacgaaa tgctcgaagg
ccgcctgcct    840

gaccacggcg agccggtgga tcaactgcag gcacgcggta tcctgctgga
cggatcttcc    900

gtggaaggcg acaaacgcct gctgctgcag atcttctcgg aaaccctgat
gggcccggtg    960

ttcttcgaat tcatccagcg caagggcgac gatgggtttg gcgagggcaa
cttcaaggcg   1020

ctgttcgagt ccatcgaacg tgaccaggtg cgtcgtggtg tattgaccgc cgattaa
1077

<210>  10
<211>  358
<212>  PRT
<213>  Pseudomonas fluorescens

<400>  10

Met Ala Asp Leu Tyr Glu Asn Pro Met Gly Leu Met Gly Phe Glu
Phe
1               5                   10                  15

Ile Glu Phe Ala Ser Pro Thr Pro Gly Thr Leu Glu Pro Ile Phe
Glu
            20                  25                  30

Ile Met Gly Phe Thr Lys Val Ala Thr His Arg Ser Lys Asn Val
His
        35                  40                  45

Leu Tyr Arg Gln Gly Glu Ile Asn Leu Ile Leu Asn Asn Glu Pro
Asn
    50                  55                  60

Ser Ile Ala Ser Tyr Phe Ala Ala Glu His Gly Pro Ser Val Cys
Gly
65                  70                  75                  80

Met Ala Phe Arg Val Lys Asp Ser Gln Lys Ala Tyr Asn Arg Ala
Leu
                85                  90                  95

Glu Leu Gly Ala Gln Pro Ile His Ile Asp Thr Gly Pro Met Glu
Leu
            100                 105                 110

Asn Leu Pro Ala Ile Lys Gly Ile Gly Gly Ala Pro Leu Tyr Leu
Ile
        115                 120                 125

Asp Arg Phe Gly Glu Gly Ser Ser Ile Tyr Asp Ile Asp Phe Val
Tyr
    130                 135                 140

Leu Glu Gly Val Glu Arg Asn Pro Val Gly Ala Gly Leu Lys Val
Ile
145                 150                 155
160

Asp His Leu Thr His Asn Val Tyr Arg Gly Arg Met Val Tyr Trp
Ala
                165                 170                 175

Asn Phe Tyr Glu Lys Leu Phe Asn Phe Arg Glu Ala Arg Tyr Phe
Asp
            180                 185                 190

Ile Lys Gly Glu Tyr Thr Gly Leu Thr Ser Lys Ala Met Ser Ala
Pro
        195                 200                 205

Asp Gly Met Ile Arg Ile Pro Leu Asn Glu Glu Ser Ser Lys Gly
Ala
    210                 215                 220

Gly Gln Ile Glu Glu Phe Leu Met Gln Phe Asn Gly Glu Gly Ile
Gln
225                 230                 235
240

His Val Ala Phe Leu Thr Asp Asp Leu Val Lys Thr Trp Asp Ala
Leu
                245                 250                 255

Lys Lys Ile Gly Met Arg Phe Met Thr Ala Pro Pro Asp Thr Tyr
Tyr
            260                 265                 270

Glu Met Leu Glu Gly Arg Leu Pro Asp His Gly Glu Pro Val Asp
Gln
        275                 280                 285

Leu Gln Ala Arg Gly Ile Leu Leu Asp Gly Ser Ser Val Glu Gly
Asp
    290                 295                 300

Lys Arg Leu Leu Leu Gln Ile Phe Ser Glu Thr Leu Met Gly Pro
Val
305                 310                 315
320

Phe Phe Glu Phe Ile Gln Arg Lys Gly Asp Asp Gly Phe Gly Glu
Gly
                325                 330                 335

Asn Phe Lys Ala Leu Phe Glu Ser Ile Glu Arg Asp Gln Val Arg
Arg
            340                 345                 350

Gly Val Leu Thr Ala Asp
        355

<210>  11
<400>  000

<210>  12
<400>  000

<210>  13
<211>  1053
<212>  DNA
<213>  Synechococcus sp.

<400>  13
atgaacccgt ccattcgaat tgtccaaggg atccaccacc tgcacttcta
cctttgggat      60

ctgccccgtt ggcgggaaca cttttgtcgg gtttggggct tccgggtggc
aagcgacgcc     120

ggcaacaccc tggagctgga gcagggatcc ctgcgcttgc gcctgtctca
gccggcacgg     180

gcgggggacg aggtggaccg ccatttgcag cggcatgggc cgggggtggt
ggatgtggcc     240

ttggcggtgg gagagcagga gctaccggcc ttggcggagc tgttgcgggg
ccgaggcgcc     300

caactggcgt ggatcccggc agcagcggcg ctctgcctcc acacccccta
cgggatccgg     360

cattctctga tccctggccc cttggatgcc gcccctgccg aagcgggcct
gttttcccac     420

tgggatcacg tggtgttgaa cgtggagcag ggatccctgc aggcggcagc
cgactggtat     480

gggcgggtgc tgggctggcg gcggctgtac cgctacagca tcggcaccgc
cacctccggc     540

ctggaaagcg tggtggtggg ggatccggaa gcggggatcc aatgggccat
caacgagccc    600

acctgtgccg cttcccagat tcaggagttt ttgcatgccc atggcggccc
gggcattcag    660

cacgcggcgc tgcacagctc agacattgtt gccagcctgc gccggttgcg
gcagggggga    720

gtggactttt tgcaagtggc gccgcagtac tacaccagcc tggaaaggga
gctggggttg    780

gcgctccgtt ctgcccttgg gcaggccatc tcctggcaag acctggtgga
gcagcagatc    840

cttctggatg ctaccctgcc cgcttctgat ggccaggatc gccccttct
gctgcagacc    900

tttacccagc ccctctttgg tcggcccacc tttttctttg aagtcattca
acggctaggc    960

ggggccacgg gctttggcga ggccaatttt caggctttgt tcgaggccct
ggaacggcaa   1020

cagcgacagc gacaccaggc gctgacccct tag
1053



<210>  14
<211>  350
<212>  PRT
<213>  Synechococcus sp.

<400>  14

Met Asn Pro Ser Ile Arg Ile Val Gln Gly Ile His His Leu His
Phe
1               5                   10                  15


Tyr Leu Trp Asp Leu Pro Arg Trp Arg Glu His Phe Cys Arg Val
Trp
            20                  25                  30


Gly Phe Arg Val Ala Ser Asp Ala Gly Asn Thr Leu Glu Leu Glu
Gln
        35                  40                  45


Gly Ser Leu Arg Leu Arg Leu Ser Gln Pro Ala Arg Ala Gly Asp
Glu

50                  55                  60

Val Asp Arg His Leu Gln Arg His Gly Pro Gly Val Val Asp Val
Ala
65                  70                  75                  80

Leu Ala Val Gly Glu Gln Glu Leu Pro Ala Leu Ala Glu Leu Leu
Arg
                85                  90                  95

Gly Arg Gly Ala Gln Leu Ala Trp Ile Pro Ala Ala Ala Ala Leu
Cys
            100                 105                 110

Leu His Thr Pro Tyr Gly Ile Arg His Ser Leu Ile Pro Gly Pro
Leu
        115                 120                 125

Asp Ala Ala Pro Ala Glu Ala Gly Leu Phe Ser His Trp Asp His
Val
    130                 135                 140

Val Leu Asn Val Glu Gln Gly Ser Leu Gln Ala Ala Ala Asp Trp
Tyr
145                 150                 155
160

Gly Arg Val Leu Gly Trp Arg Arg Leu Tyr Arg Tyr Ser Ile Gly
Thr
                165                 170                 175

Ala Thr Ser Gly Leu Glu Ser Val Val Val Gly Asp Pro Glu Ala
Gly
            180                 185                 190

Ile Gln Trp Ala Ile Asn Glu Pro Thr Cys Ala Ala Ser Gln Ile
Gln
        195                 200                 205

Glu Phe Leu His Ala His Gly Gly Pro Gly Ile Gln His Ala Ala
Leu
    210                 215                 220

His Ser Ser Asp Ile Val Ala Ser Leu Arg Arg Leu Arg Gln Gly
Gly
225                 230                 235
240

Val Asp Phe Leu Gln Val Ala Pro Gln Tyr Tyr Thr Ser Leu Glu
Arg
                245                 250                 255

Glu Leu Gly Leu Ala Leu Arg Ser Ala Leu Gly Gln Ala Ile Ser
Trp
            260                 265                 270

Gln Asp Leu Val Glu Gln Gln Ile Leu Leu Asp Ala Thr Leu Pro
Ala
        275                 280                 285

Ser Asp Gly Gln Asp Arg Pro Leu Leu Leu Gln Thr Phe Thr Gln
Pro
    290                 295                 300

Leu Phe Gly Arg Pro Thr Phe Phe Phe Glu Val Ile Gln Arg Leu
Gly
305                 310                 315
320

Gly Ala Thr Gly Phe Gly Glu Ala Asn Phe Gln Ala Leu Phe Glu
Ala
                325                 330                 335

Leu Glu Arg Gln Gln Arg Gln Arg His Gln Ala Leu Thr Pro
            340                 345                 350

<210>  15
<211>  1149
<212>  DNA
<213>  Blepharisma japonicum

<400>  15
atgacttatt acgacaagca agaaacgcgt ccagatcttg gcgaattcta
tggtttccat      60

cacgttcgtt tttacgtctc caactcagag caagccgctt cgttctacac
atctcgcttt    120

gggttttctc cggttgccta tgaaggattg gaaacaggaa accaaaaatt
ctgtaccaat    180

gtcgtccgaa gcaaccatgt agtcatcgct tttacctcag ctctcactcc
tgaagacaat    240

gaagtgaacc gtcacgttgg caagcatagt gatggagttc aagacattgc
ctttagtgta    300

agtgacgcaa gagggatgta tgagaaagcg atagctaaag gctgtaaaag
cttccgtgag    360

ccacaggttt tacaagatca atttggatct gttataatag cgtctctcca
gacttatgga    420

gacactgttc acacattagt ccaaaatgtc gactatacag gacccttttt
gcctggcttc    480

agagcaatca caaaagatga tccattaaac tctgcctttc ctcaggtaaa
ttatgacatt    540

attgatcatg ttgtaggaaa tcagcctggt ggcgatatga ctcctacagt
agaatggtat    600

gagaaatatc tagaatttca tcgatattgg tctgctgatg agtctgtaat
ccataccgat    660

tattcagcat taaggtctgt tgtggttgct gattgggatg aagtgatcaa
aatgcctatt    720

aatgagcctg ctgatggact tagaaaaagt caaatccaag aatatgtcga
atattatggt    780

ggagcaggcg tacaacatat tgccttaaaa gtcaatgata ttatttcagt
aataagcacc    840

ttaagggcta gaggtgtgga attcttagaa gttcctccta aatattatga
tagcttaaga    900

aaaagacttg cgcattctgc ggtacaaatt gaagaagact taaaaagaat
tgaagacctt    960

catattttgg ttgactttga cgaccgtggg tatttacttc agattttcac
aaaaccagta    1020

gaagacagac ctactctgtt ttatgaaatt attcaaagac ataataacaa
tggattcgga    1080

attggaaatt ttaaagccct atttgaatca ttggaacaag agcaagaaag
aagaggtaat   1140

ttgatctaa
1149

<210>  16
<211>  382
<212>  PRT
<213>  Blepharisma japonicum

<400>  16

Met Thr Tyr Tyr Asp Lys Gln Glu Thr Arg Pro Asp Leu Gly Glu
Phe
1               5                   10                  15

Tyr Gly Phe His His Val Arg Phe Tyr Val Ser Asn Ser Glu Gln
Ala
            20                  25                  30

Ala Ser Phe Tyr Thr Ser Arg Phe Gly Phe Ser Pro Val Ala Tyr
Glu
        35                  40                  45

Gly Leu Glu Thr Gly Asn Gln Lys Phe Cys Thr Asn Val Val Arg
Ser
    50                  55                  60

Asn His Val Val Ile Ala Phe Thr Ser Ala Leu Thr Pro Glu Asp
Asn
65                  70                  75                  80

Glu Val Asn Arg His Val Gly Lys His Ser Asp Gly Val Gln Asp
Ile
                85                  90                  95

Ala Phe Ser Val Ser Asp Ala Arg Gly Met Tyr Glu Lys Ala Ile
Ala
            100                 105                 110

Lys Gly Cys Lys Ser Phe Arg Glu Pro Gln Val Leu Gln Asp Gln
Phe
        115                 120                 125

Gly Ser Val Ile Ile Ala Ser Leu Gln Thr Tyr Gly Asp Thr Val
His
    130                 135                 140

Thr Leu Val Gln Asn Val Asp Tyr Thr Gly Pro Phe Leu Pro Gly
Phe
145                 150                 155
160

Arg Ala Ile Thr Lys Asp Asp Pro Leu Asn Ser Ala Phe Pro Gln
Val
                165                 170                 175

Asn Tyr Asp Ile Ile Asp His Val Val Gly Asn Gln Pro Gly Gly
Asp
            180                 185                 190

Met Thr Pro Thr Val Glu Trp Tyr Glu Lys Tyr Leu Glu Phe His
Arg
        195                 200                 205

Tyr Trp Ser Ala Asp Glu Ser Val Ile His Thr Asp Tyr Ser Ala
Leu
    210                 215                 220

Arg Ser Val Val Val Ala Asp Trp Asp Glu Val Ile Lys Met Pro
Ile
225                 230                 235
240

Asn Glu Pro Ala Asp Gly Leu Arg Lys Ser Gln Ile Gln Glu Tyr
Val
                245                 250                 255

Glu Tyr Tyr Gly Gly Ala Gly Val Gln His Ile Ala Leu Lys Val
Asn
            260                 265                 270

Asp Ile Ile Ser Val Ile Ser Thr Leu Arg Ala Arg Gly Val Glu
Phe
        275                 280                 285

Leu Glu Val Pro Pro Lys Tyr Tyr Asp Ser Leu Arg Lys Arg Leu
Ala
    290                 295                 300

His Ser Ala Val Gln Ile Glu Glu Asp Leu Lys Arg Ile Glu Asp
Leu
305                 310                 315
320

His Ile Leu Val Asp Phe Asp Asp Arg Gly Tyr Leu Leu Gln Ile
Phe
                325                 330                 335

Thr Lys Pro Val Glu Asp Arg Pro Thr Leu Phe Tyr Glu Ile Ile
Gln
            340                 345                 350

Arg His Asn Asn Asn Gly Phe Gly Ile Gly Asn Phe Lys Ala Leu
Phe
        355                 360                 365

Glu Ser Leu Glu Gln Glu Gln Glu Arg Arg Gly Asn Leu Ile
    370                 375                 380

<210>  17
<211>  1206
<212>  DNA
<213>  Rhodococcus sp.

<400>  17
atgacgatcg agcagactct caccgacaag gaacgcctgg caggtctcga
cctcggccag      60

ctcgagcagt tggtcgggct cgtcgagtac gacggcaccc gcgacccgtt
cccggtcagc     120

ggctgggatg ccgtcgtctg ggtggtcggc aacgccaccc agaccgccca
ctacttccag     180

tccgcgttcg ggatgaccct cgtcgcctac tccggaccca ccaccggcaa
ccgcgaccac     240

cacagcttcg tcctcgaatc cggggccgtc cgcttcgtca tcaaaggcgc
cgtgaacccg     300

gacagccccc tgatcgacca ccaccgcacc cacggcgacg gcgtcgtcga
catcgccctc    360

gccgtccccg acgtcgacaa gtgcatcgcc cacgcccgcg cccagggcgc
caccgtcctc    420

gacgaacccc acgacgtgac cgacgaccac ggcaccgtcc gcctcgccgc
gatcgccacc    480

tacggcgaca cccgccacac cctcgtcgac cgcagccact acaccggccc
ctacctgccc    540

ggctacaccg cccgcacctc cggccacacc aaacgggacg gggcacccaa
gcgcctgttc    600

caggccctcg accacgtcgt cggcaacgtc gaactcggca agatggacca
ctgggtcgac    660

ttctacaacc gggtcatggg ctttacgaac atggccgagt tcgtcggcga
ggacatcgcc    720

accgactact ccgcgctgat gagcaaggtc gtctccaacg gcaaccaccg
ggtcaagttc    780

cccctcaacg aacccgccct cgccaagaaa cgctcgcaga tcgacgaata
cctcgacttc    840

taccgcggcc ccggcgccca gcacctggcc ctggccacca atgacatcct
caccgccgtc    900

gaccagctga ccgccgaggg cgtcgagttc ctggccaccc ccgactccta
ctacgaggac    960

cccgaactgc gggcccggat cggcaacgtc cgcgccccca tcgccgaact
gcagaaacgc   1020

ggcatcctcg tcgaccgcga cgaagacggc tacctgctgc agatcttcac
caaacccctc   1080

gtcgaccggc ccaccgtgtt cttcgaactc atcgaacgcc acggctccct
cggcttcggc   1140

atcggcaact tcaaagccct cttcgaggcc atcgaacgcg aacaagccgc
ccgcggaaac   1200

ttctga
1206



<210>  18
<211>  401

<212>  PRT
<213>  Rhodococcus sp.

<400>  18

Met Thr Ile Glu Gln Thr Leu Thr Asp Lys Glu Arg Leu Ala Gly
Leu
1               5                   10                  15

Asp Leu Gly Gln Leu Glu Gln Leu Val Gly Leu Val Glu Tyr Asp
Gly
            20                  25                  30

Thr Arg Asp Pro Phe Pro Val Ser Gly Trp Asp Ala Val Val Trp
Val
        35                  40                  45

Val Gly Asn Ala Thr Gln Thr Ala His Tyr Phe Gln Ser Ala Phe
Gly
    50                  55                  60

Met Thr Leu Val Ala Tyr Ser Gly Pro Thr Thr Gly Asn Arg Asp
His
65                  70                  75                  80

His Ser Phe Val Leu Glu Ser Gly Ala Val Arg Phe Val Ile Lys
Gly
                85                  90                  95

Ala Val Asn Pro Asp Ser Pro Leu Ile Asp His His Arg Thr His
Gly
            100                 105                 110

Asp Gly Val Val Asp Ile Ala Leu Ala Val Pro Asp Val Asp Lys
Cys
        115                 120                 125

Ile Ala His Ala Arg Ala Gln Gly Ala Thr Val Leu Asp Glu Pro
His
    130                 135                 140

Asp Val Thr Asp Asp His Gly Thr Val Arg Leu Ala Ala Ile Ala
Thr

145                 150                 155
160

Tyr Gly Asp Thr Arg His Thr Leu Val Asp Arg Ser His Tyr Thr
Gly
                165                 170                 175

Pro Tyr Leu Pro Gly Tyr Thr Ala Arg Thr Ser Gly His Thr Lys
Arg
            180                 185                 190

Asp Gly Ala Pro Lys Arg Leu Phe Gln Ala Leu Asp His Val Val
Gly
        195                 200                 205

Asn Val Glu Leu Gly Lys Met Asp His Trp Val Asp Phe Tyr Asn
Arg
    210                 215                 220

Val Met Gly Phe Thr Asn Met Ala Glu Phe Val Gly Glu Asp Ile
Ala
225                 230                 235
240

Thr Asp Tyr Ser Ala Leu Met Ser Lys Val Val Ser Asn Gly Asn
His
                245                 250                 255

Arg Val Lys Phe Pro Leu Asn Glu Pro Ala Leu Ala Lys Lys Arg
Ser
            260                 265                 270

Gln Ile Asp Glu Tyr Leu Asp Phe Tyr Arg Gly Pro Gly Ala Gln
His
        275                 280                 285

Leu Ala Leu Ala Thr Asn Asp Ile Leu Thr Ala Val Asp Gln Leu
Thr
    290                 295                 300

Ala Glu Gly Val Glu Phe Leu Ala Thr Pro Asp Ser Tyr Tyr Glu
Asp

305                 310                 315
320

Pro Glu Leu Arg Ala Arg Ile Gly Asn Val Arg Ala Pro Ile Ala
Glu
                325                 330                 335

Leu Gln Lys Arg Gly Ile Leu Val Asp Arg Asp Glu Asp Gly Tyr
Leu
            340                 345                 350

Leu Gln Ile Phe Thr Lys Pro Leu Val Asp Arg Pro Thr Val Phe
Phe
        355                 360                 365

Glu Leu Ile Glu Arg His Gly Ser Leu Gly Phe Gly Ile Gly Asn
Phe
    370                 375                 380

Lys Ala Leu Phe Glu Ala Ile Glu Arg Glu Gln Ala Ala Arg Gly
Asn
385                 390                 395
400

Phe

<210>  19
<211>  1107
<212>  DNA
<213>  Picrophilus torridus

<400>  19
atgtatggca aaaatttaat ctcagaacta agggaaaagg agatctttaa
acgattacat      60

cacgtggaat tttacgttag cagtgccaaa acatggtcat atttcatgaa
caggggtctt     120

ggatttaaaa cagtggcata tgccggtcca gaaaccggga taaggacaaa
gatatcctat     180

gttatgtccc agggcactgc aaggatatct tttacatcat caatgaatga
tgatagctat     240

atatcgaatc atgttaaaaa acacggggat ggcgtaaagg atatagcact
tgaggtcgat     300

gatctggacg aggcaaaaag cctgatagaa aagtatggaa caaaggtttc
aaaaataaat     360

gaaataaagg atggaaatgg aaagataaga actgcagaga taaaaacgta
cggtgaaacc     420

gttcatacat taatagaaac cggggattac aatggcgtat tcatgcccgg
ttatgaggaa     480

tctgaaataa attcaaaaaa cactgggata aaaaagatcg atcatatagt
tggaaatgtc     540

tatgagggcg agatggatag ctgggttaat ttttacatag aaaaacttgg
ctttgagcat     600

ttaataacct ttgatgataa agatataaga actgattaca gcgcattaag
atcaaaggtt     660

gtaaaataca atgacgatat cgtatttcca ataaatgagc ctgcaaaggg
cttaagaaaa     720

tcacagatag aggaatatct tgactattac aggtctgagg gcgttcagca
catagcactg     780

ttaactgatg atataataaa aactgtatcc atgatggagg aaaacggcat
agaattttta     840

aaaacaccag gatcatacta tgaatcccta tcatcaagga taggctcaat
agacgaggat     900

ttaaatgaaa tagagaaaca taacatactt gtggatcgtg atgagaacgg
atacctatta     960

cagatcttca caaagcctgt tactgacagg ccaacgttct tctttgaggt
catacagaga    1020

aagggtgcaa ggtcattcgg caacggtaac tttaaggcac tttttgaggc
gatagaaagg    1080

gagcaggcaa agagaggaaa cctatga
1107

<210>  20
<211>  368
<212>  PRT
<213>  Picrophilus torridus

<400>  20

Met Tyr Gly Lys Asn Leu Ile Ser Glu Leu Arg Glu Lys Glu Ile
Phe
1               5                   10                  15

Lys Arg Leu His His Val Glu Phe Tyr Val Ser Ser Ala Lys Thr
Trp
            20                  25                  30

Ser Tyr Phe Met Asn Arg Gly Leu Gly Phe Lys Thr Val Ala Tyr
Ala
        35                  40                  45

Gly Pro Glu Thr Gly Ile Arg Asp Lys Ile Ser Tyr Val Met Ser
Gln
    50                  55                  60

Gly Thr Ala Arg Ile Ser Phe Thr Ser Ser Met Asn Asp Asp Ser
Tyr
65                  70                  75                  80

Ile Ser Asn His Val Lys Lys His Gly Asp Gly Val Lys Asp Ile
Ala
                85                  90                  95

Leu Glu Val Asp Asp Leu Asp Glu Ala Lys Ser Leu Ile Glu Lys
Tyr
            100                 105                 110

Gly Thr Lys Val Ser Lys Ile Asn Glu Ile Lys Asp Gly Asn Gly
Lys
        115                 120                 125

Ile Arg Thr Ala Glu Ile Lys Thr Tyr Gly Glu Thr Val His Thr
Leu
    130                 135                 140

Ile Glu Thr Gly Asp Tyr Asn Gly Val Phe Met Pro Gly Tyr Glu
Glu
145                 150                 155
160

Ser Glu Ile Asn Ser Lys Asn Thr Gly Ile Lys Lys Ile Asp His
Ile
                165                 170                 175

Val Gly Asn Val Tyr Glu Gly Glu Met Asp Ser Trp Val Asn Phe
Tyr
            180                 185                 190

Ile Glu Lys Leu Gly Phe Glu His Leu Ile Thr Phe Asp Asp Lys
Asp
        195                 200                 205

Ile Arg Thr Asp Tyr Ser Ala Leu Arg Ser Lys Val Val Lys Tyr
Asn
    210                 215                 220

Asp Asp Ile Val Phe Pro Ile Asn Glu Pro Ala Lys Gly Leu Arg
Lys
225                 230                 235
240

Ser Gln Ile Glu Glu Tyr Leu Asp Tyr Tyr Arg Ser Glu Gly Val
Gln
                245                 250                 255

His Ile Ala Leu Leu Thr Asp Asp Ile Ile Lys Thr Val Ser Met
Met
            260                 265                 270

Glu Glu Asn Gly Ile Glu Phe Leu Lys Thr Pro Gly Ser Tyr Tyr
Glu
        275                 280                 285

Ser Leu Ser Ser Arg Ile Gly Ser Ile Asp Glu Asp Leu Asn Glu
Ile
    290                 295                 300

Glu Lys His Asn Ile Leu Val Asp Arg Asp Glu Asn Gly Tyr Leu
Leu
305                 310                 315
320

Gln Ile Phe Thr Lys Pro Val Thr Asp Arg Pro Thr Phe Phe Phe
Glu
                325                 330                 335

Val Ile Gln Arg Lys Gly Ala Arg Ser Phe Gly Asn Gly Asn Phe
Lys
            340                 345                 350

Ala Leu Phe Glu Ala Ile Glu Arg Glu Gln Ala Lys Arg Gly Asn
Leu
        355                 360                 365

<210>  21
<211>  1164
<212>  DNA
<213>  Kordia algicida

<400>  21
atggcagcag aaataaaaaa cttaaaagat ttacaaaata cagaatacgg
actcaaaaaa      60

ttatttgacg aagcagaaga ctttcttcca cttttaggaa cagactacgt
agaattatac     120

gtcgggaacg ccaaacaatc ggcacatttc tacaaaacgg cttttggttt
tcaatcagaa     180

gcttacgcag gattggaaac aggattaacc gacagagttt catacgtatt
aaaacaagat     240

aaaattcgct tggtcttaac aacaccatta ggaaaaggtg gcgaaatcaa
tgagcatatc     300

gatttacacg gcgatggcgt aaaagtagta gcactttggg tagaagatgc
tacaaaagcc     360

tttgaagaaa cgaccaaaag aggcgcaaaa ccgtacatgg aaccaacaaa
agaagaagat     420

gaaaacggat atgtaattcg ctcaggaatc tatacgtacg gagaaacggt
tcatgttttt     480

gtagaacgta aaaactataa cggagtcttt ttaccaggat atcaaagatg
ggaatctcac     540

tacaatccgg agccagttgg cttaaaattc atcgatcaca tggtaggaaa
tgtaggttgg     600

ggagaaatga aagaatggtg tgaattctac gcgaaagtaa tgggatttgc
gcaaattatc    660

tcctttacag atgatgatat ttctaccgat tttactgcgt tgatgagtaa
agtaatgagt    720

aatggaaatg gtagaatcaa atttccaatc aatgaacccg cagaaggaaa
aaagaaatcg    780

caaattgaag aatatctaga cttttacaat ggttcaggag tacaacatat
tgcggttgct    840

acagacaata ttattgatac ggtttcgcaa atgcgcgaac gtggagtaga
attcttatac    900

gttccagata catattatga tgacttgtta gaacgtgttg gcgacatcga
tgaagatgta    960

gaagaactca aaaaacacgg aatcttaatt gatcgtgatg aagaaggata
cttattgcag   1020

ttatttacca aaaccattgt agacagacca acaatgttct ttgaagtcat
tcagcgtaaa   1080

ggcgcacaat catttggagt aggaaacttt aaagctttat ttgaagcgat
agaaagagaa   1140

caagctgctc gcggaacatt gtaa
1164



<210>  22
<211>  387
<212>  PRT
<213>  Kordia algicida

<400>  22

Met Ala Ala Glu Ile Lys Asn Leu Lys Asp Leu Gln Asn Thr Glu
Tyr
1               5                   10                  15


Gly Leu Lys Lys Leu Phe Asp Glu Ala Glu Asp Phe Leu Pro Leu
Leu
            20                  25                  30


Gly Thr Asp Tyr Val Glu Leu Tyr Val Gly Asn Ala Lys Gln Ser
Ala
        35                  40                  45

His Phe Tyr Lys Thr Ala Phe Gly Phe Gln Ser Glu Ala Tyr Ala
Gly
    50                  55                  60

Leu Glu Thr Gly Leu Thr Asp Arg Val Ser Tyr Val Leu Lys Gln
Asp
65                  70                  75                  80

Lys Ile Arg Leu Val Leu Thr Thr Pro Leu Gly Lys Gly Gly Glu
Ile
                85                  90                  95

Asn Glu His Ile Asp Leu His Gly Asp Gly Val Lys Val Val Ala
Leu
            100                 105                 110

Trp Val Glu Asp Ala Thr Lys Ala Phe Glu Glu Thr Thr Lys Arg
Gly
        115                 120                 125

Ala Lys Pro Tyr Met Glu Pro Thr Lys Glu Glu Asp Glu Asn Gly
Tyr
    130                 135                 140

Val Ile Arg Ser Gly Ile Tyr Thr Tyr Gly Glu Thr Val His Val
Phe
145                 150                 155
160

Val Glu Arg Lys Asn Tyr Asn Gly Val Phe Leu Pro Gly Tyr Gln
Arg
                165                 170                 175

Trp Glu Ser His Tyr Asn Pro Glu Pro Val Gly Leu Lys Phe Ile
Asp
            180                 185                 190

His Met Val Gly Asn Val Gly Trp Gly Glu Met Lys Glu Trp Cys
Glu
        195                 200                 205

Phe Tyr Ala Lys Val Met Gly Phe Ala Gln Ile Ile Ser Phe Thr
Asp
    210                 215                 220

Asp Asp Ile Ser Thr Asp Phe Thr Ala Leu Met Ser Lys Val Met
Ser
225                 230                 235
240

Asn Gly Asn Gly Arg Ile Lys Phe Pro Ile Asn Glu Pro Ala Glu
Gly
                245                 250                 255

Lys Lys Lys Ser Gln Ile Glu Glu Tyr Leu Asp Phe Tyr Asn Gly
Ser
            260                 265                 270

Gly Val Gln His Ile Ala Val Ala Thr Asp Asn Ile Ile Asp Thr
Val
        275                 280                 285

Ser Gln Met Arg Glu Arg Gly Val Glu Phe Leu Tyr Val Pro Asp
Thr
    290                 295                 300

Tyr Tyr Asp Asp Leu Leu Glu Arg Val Gly Asp Ile Asp Glu Asp
Val
305                 310                 315
320

Glu Glu Leu Lys Lys His Gly Ile Leu Ile Asp Arg Asp Glu Glu
Gly
                325                 330                 335

Tyr Leu Leu Gln Leu Phe Thr Lys Thr Ile Val Asp Arg Pro Thr
Met
            340                 345                 350

Phe Phe Glu Val Ile Gln Arg Lys Gly Ala Gln Ser Phe Gly Val
Gly
        355                 360                 365

Asn Phe Lys Ala Leu Phe Glu Ala Ile Glu Arg Glu Gln Ala Ala
Arg
    370                 375                 380

Gly Thr Leu
385

<210>  23
<211>  1323
<212>  DNA
<213>  Sorghum bicolor

<400>  23
atgcccccga cccccaccac agccgccgca accggcgccg ccgtggcggc
ggcatcagcg      60

gagcaggcgg cgttccgcct cgtgggccac cgcaacttcg tccgcgtgaa
cccgcgctcc     120

gaccgcttcc acacgctcgc gttccaccac gtggagctct ggtgcgccga
cgcggcctcc     180

gccgcgggcc gcttctcctt cgggctcggc gcgccgctcg ccgcgcggtc
cgacctctcc     240

acggggaaca ccgcgcacgc gtccctgctg ctccgctcgg gcgccctcgc
gttcctcttc     300

acggcgccct acgcgcacgg cgccgacgcc gccacggcct cgctgccctc
cttctccgcc     360

gccgaggcgc ggcgcttcgc ggccgaccac ggcctcgcgg tgcgcgccgt
cgcgctccgc     420

gtggccgacg cggaggacgc cttccgcgcc agcgtcgcgg ccggcgcgcg
cccggcgttc     480

gagcccgtcg agctcggcct cggcttccgc ctcgccgaag tcgagctcta
cggcgacgtc     540

gtgctccggt acgtgagcta cccggacgac gcggacgcgt ccttcctgcc
ggggttcgtg     600

ggcgtgacca gccccggcgc ggcggactac gggctgagga ggttcgacca
catcgtcggc     660

aacgtgccgg agctggcgcc ggcggccgcc tacttcgctg gcttcacggg
gttccacgag     720

ttcgccgagt tcacggcgga ggacgtgggc accacggaga gcgggctcaa
ctcgatggtg    780

ctcgccaaca acgcggagaa cgtgctgctc ccactcaacg agccggtgca
cggcaccaag    840

cgccgcagcc agatacagac gtacttggac caccacggcg gccccggcgt
gcagcacatg    900

gcgctggcca gcgacgacgt gctcaggacg ctgagggaga tgcaagcgcg
ctcggccatg    960

ggcggcttcg agttcatggc gcctccggcg cccgaatact atgacggcgt
gaggcggcgc   1020

gccggggacg tgctcacgga ggcacagatt aaggagtgtc aggaactagg
ggtgctggtg   1080

gacagagatg accagggcgt gctgctccag atcttcacca agccagtggg
ggacaggcca   1140

acgttgttct tggagatcat tcaaaggatc gggtgcatgg agaaggatga
gaaggggcaa   1200

gaataccaga agggtggctg tggcgggttt ggcaagggaa acttctccca
gctgttcaaa   1260

tccattgagg attatgagaa gtcccttgaa gctaagcaag ctgcagcagc
tcagggatcc   1320

tag
1323

<210>  24
<211>  440
<212>  PRT
<213>  Sorghum bicolor

<400>  24

Met Pro Pro Thr Pro Thr Thr Ala Ala Ala Thr Gly Ala Ala Val
Ala
1               5                   10                  15

Ala Ala Ser Ala Glu Gln Ala Ala Phe Arg Leu Val Gly His Arg
Asn
            20                  25                  30

Phe Val Arg Val Asn Pro Arg Ser Asp Arg Phe His Thr Leu Ala
Phe
        35                  40                  45

His His Val Glu Leu Trp Cys Ala Asp Ala Ala Ser Ala Ala Gly
Arg
    50                  55                  60

Phe Ser Phe Gly Leu Gly Ala Pro Leu Ala Ala Arg Ser Asp Leu
Ser
65                  70                  75                  80

Thr Gly Asn Thr Ala His Ala Ser Leu Leu Leu Arg Ser Gly Ala
Leu
                85                  90                  95

Ala Phe Leu Phe Thr Ala Pro Tyr Ala His Gly Ala Asp Ala Ala
Thr
            100                 105                 110

Ala Ser Leu Pro Ser Phe Ser Ala Ala Glu Ala Arg Arg Phe Ala
Ala
        115                 120                 125

Asp His Gly Leu Ala Val Arg Ala Val Ala Leu Arg Val Ala Asp
Ala
    130                 135                 140

Glu Asp Ala Phe Arg Ala Ser Val Ala Ala Gly Ala Arg Pro Ala
Phe
145                 150                 155
160

Glu Pro Val Glu Leu Gly Leu Gly Phe Arg Leu Ala Glu Val Glu
Leu
                165                 170                 175

Tyr Gly Asp Val Val Leu Arg Tyr Val Ser Tyr Pro Asp Asp Ala
Asp
            180                 185                 190

Ala Ser Phe Leu Pro Gly Phe Val Gly Val Thr Ser Pro Gly Ala
Ala
        195                 200                 205

Asp Tyr Gly Leu Arg Arg Phe Asp His Ile Val Gly Asn Val Pro
Glu
    210                 215                 220

Leu Ala Pro Ala Ala Ala Tyr Phe Ala Gly Phe Thr Gly Phe His
Glu
225                 230                 235
240

Phe Ala Glu Phe Thr Ala Glu Asp Val Gly Thr Thr Glu Ser Gly
Leu
                245                 250                 255

Asn Ser Met Val Leu Ala Asn Asn Ala Glu Asn Val Leu Leu Pro
Leu
            260                 265                 270

Asn Glu Pro Val His Gly Thr Lys Arg Arg Ser Gln Ile Gln Thr
Tyr
        275                 280                 285

Leu Asp His His Gly Gly Pro Gly Val Gln His Met Ala Leu Ala
Ser
    290                 295                 300

Asp Asp Val Leu Arg Thr Leu Arg Glu Met Gln Ala Arg Ser Ala
Met
305                 310                 315
320

Gly Gly Phe Glu Phe Met Ala Pro Pro Ala Pro Glu Tyr Tyr Asp
Gly
                325                 330                 335

Val Arg Arg Arg Ala Gly Asp Val Leu Thr Glu Ala Gln Ile Lys
Glu
            340                 345                 350

Cys Gln Glu Leu Gly Val Leu Val Asp Arg Asp Asp Gln Gly Val
Leu
        355                 360                 365

Leu Gln Ile Phe Thr Lys Pro Val Gly Asp Arg Pro Thr Leu Phe
Leu
    370                 375                 380

Glu Ile Ile Gln Arg Ile Gly Cys Met Glu Lys Asp Glu Lys Gly
Gln
385                 390                 395
400

Glu Tyr Gln Lys Gly Gly Cys Gly Gly Phe Gly Lys Gly Asn Phe
Ser
                405                 410                 415

Gln Leu Phe Lys Ser Ile Glu Asp Tyr Glu Lys Ser Leu Glu Ala
Lys
            420                 425                 430

Gln Ala Ala Ala Ala Gln Gly Ser
        435                 440

<210>  25
<211>  1311
<212>  DNA
<213>  Triticum aestivum

<400>  25
atgccgccca cccccaccac ccccgcagcc accggcgccg gcgctgccgc
cgcggtgacg      60

ccggagcacg cgcggccgcg ccgaatggtc cgcttcaacc cgcgcagcga
ccgcttccac     120

acgctctcct tccaccacgt cgagttctgg tgcgcggacg ccgcctccgc
cgccggccgc     180

ttcgccttcg cgctcggcgc gccgctcgcc gccaggtccg acctctccac
ggggaactcc     240

gtgcacgcct cccagctgct ccgctcgggc aacctcgcct tcctcttcac
cgcgccctac     300

gcgaacggct gcgacgccgc caccgcctcc ctgccctcct tctccgccga
cgccgcgcgc    360

cggttctccg cggaccacgg gctcgcagtg cgctccatag cactgcgcgt
cgcagacgcc    420

gcagaggcct tccgcgccag cgtcgacgga ggcgcgcgcc cggccttcag
ccccgtggac    480

ctcggccgcg gcttcggctt cgcggaggtc gagctctacg gcgacgtcgt
gctccgcttc    540

gtcagtcacc cggatgacac ggacgtgccc ttcttgccgg ggttcgaggg
cgtgagcaac    600

ccggatgccg tggactacgg cctgacgcgg ttcgaccacg tcgtcggcaa
cgtcccggag    660

cttgcccccg ccgccgcata cgtcgccggg ttcgcggggt tccacgagtt
cgccgagttc    720

acgacggagg acgtgggcac ggccgagagc gggctcaact cgatggtgct
cgccaacaac    780

tcggagggcg tgctgctgcc gctcaacgag ccggtgcacg gcaccaagcg
ccggagccag    840

atacagacgt tcctggaaca ccacggcggc tcgggcgtgc agcacatcgc
ggtggccagc    900

agcgacgtgc tcaggacgct cagggagatg cgtgcgcgct ccgccatggg
cggcttcgac    960

ttcctgccac cccgctgccg aaagtactac gaaggcgtgc ggcgcatcgc
cggggatgtg   1020

ctctcggagg cgcagatcaa ggaatgccag gagctggggg tgctcgtcga
cagggacgac   1080

caaggggtgt tgctacaaat cttcacaaag ccagtggggg acaggccaac
gctgttcctg   1140

gagatgatcc aaaggatcgg gtgcatggag aaggacgaga gaggggaaga
gtaccagaag   1200

ggtggctgcg gcgggttcgg caaaggcaac ttctccgagc tgttcaagtc
cattgaagat   1260

tacgagaagt cccttgaagc caagcaatct gctgcagttc agggatcata g
1311

<210>  26
<211>  436
<212>  PRT
<213>  Triticum aestivum

<400>  26

Met Pro Pro Thr Pro Thr Thr Pro Ala Ala Thr Gly Ala Gly Ala
Ala
1               5                   10                  15

Ala Ala Val Thr Pro Glu His Ala Arg Pro Arg Arg Met Val Arg
Phe
            20                  25                  30

Asn Pro Arg Ser Asp Arg Phe His Thr Leu Ser Phe His His Val
Glu
        35                  40                  45

Phe Trp Cys Ala Asp Ala Ala Ser Ala Ala Gly Arg Phe Ala Phe
Ala
    50                  55                  60

Leu Gly Ala Pro Leu Ala Ala Arg Ser Asp Leu Ser Thr Gly Asn
Ser
65                  70                  75                  80

Val His Ala Ser Gln Leu Leu Arg Ser Gly Asn Leu Ala Phe Leu
Phe
                85                  90                  95

Thr Ala Pro Tyr Ala Asn Gly Cys Asp Ala Ala Thr Ala Ser Leu
Pro
            100                 105                 110

Ser Phe Ser Ala Asp Ala Ala Arg Arg Phe Ser Ala Asp His Gly
Leu
        115                 120                 125

Ala Val Arg Ser Ile Ala Leu Arg Val Ala Asp Ala Ala Glu Ala
Phe
    130                 135                 140

Arg Ala Ser Val Asp Gly Gly Ala Arg Pro Ala Phe Ser Pro Val
Asp
145                 150                 155
160

Leu Gly Arg Gly Phe Gly Phe Ala Glu Val Glu Leu Tyr Gly Asp
Val
                165                 170                 175

Val Leu Arg Phe Val Ser His Pro Asp Asp Thr Asp Val Pro Phe
Leu
            180                 185                 190

Pro Gly Phe Glu Gly Val Ser Asn Pro Asp Ala Val Asp Tyr Gly
Leu
        195                 200                 205

Thr Arg Phe Asp His Val Val Gly Asn Val Pro Glu Leu Ala Pro
Ala
    210                 215                 220

Ala Ala Tyr Val Ala Gly Phe Ala Gly Phe His Glu Phe Ala Glu
Phe
225                 230                 235
240

Thr Thr Glu Asp Val Gly Thr Ala Glu Ser Gly Leu Asn Ser Met
Val
                245                 250                 255

Leu Ala Asn Asn Ser Glu Gly Val Leu Leu Pro Leu Asn Glu Pro
Val
            260                 265                 270

His Gly Thr Lys Arg Arg Ser Gln Ile Gln Thr Phe Leu Glu His
His
        275                 280                 285

Gly Gly Ser Gly Val Gln His Ile Ala Val Ala Ser Ser Asp Val
Leu
    290                 295                 300

Arg Thr Leu Arg Glu Met Arg Ala Arg Ser Ala Met Gly Gly Phe
Asp
305                 310                 315
320

Phe Leu Pro Pro Arg Cys Arg Lys Tyr Tyr Glu Gly Val Arg Arg
Ile
                325                 330                 335

Ala Gly Asp Val Leu Ser Glu Ala Gln Ile Lys Glu Cys Gln Glu
Leu
            340                 345                 350

Gly Val Leu Val Asp Arg Asp Asp Gln Gly Val Leu Leu Gln Ile
Phe
        355                 360                 365

Thr Lys Pro Val Gly Asp Arg Pro Thr Leu Phe Leu Glu Met Ile
Gln
    370                 375                 380

Arg Ile Gly Cys Met Glu Lys Asp Glu Arg Gly Glu Glu Tyr Gln
Lys
385                 390                 395
400

Gly Gly Cys Gly Gly Phe Gly Lys Gly Asn Phe Ser Glu Leu Phe
Lys
                405                 410                 415

Ser Ile Glu Asp Tyr Glu Lys Ser Leu Glu Ala Lys Gln Ser Ala
Ala
            420                 425                 430

Val Gln Gly Ser
        435

<210>  27
<211>  1305
<212>  DNA
<213>  Hordeum vulgare

<400>  27

atgccgccca cccccaccac ccccgcggct accggcgccg ccgccgcggt
gacgccggag      60

cacgcgcgac cgcaccgaat ggtccgcttc aacccgcgca gcgaccgctt
ccacacgctc     120

tccttccacc acgtcgagtt ctggtgcgcg gacgccgcct ccgccgccgg
ccgcttcgcg     180

ttcgcgctcg gcgcgccgct cgccgccagg tccgacctct ccacggggaa
ctccgcgcac     240

gcctcccagc tgctccgctc gggctccctc gccttcctct tcaccgcgcc
ctacgccaac     300

ggctgcgacg ccgccaccgc ctccctgccc tccttctccg ccgacgccgc
gcgccggttc     360

tccgccgacc acgggatcgc ggtgcgctcc gtagcgctgc gcgtcgcaga
cgccgccgag     420

gccttccgcg ccagtcgtcg acggggcgcg cgcccggcct tcgcccccgt
ggacctcggc     480

cgcggcttcg cgttcgcgga ggtcgagctc tacggcgacg tcgtgctccg
cttcgtcagc     540

cacccggacg gcacggacgt gcccttcttg ccggggttcg agggcgtaac
caacccggac     600

gccgtggact acggcctgac gcggttcgac cacgtcgtcg gcaacgtccc
ggagcttgcc     660

cccgccgcag cctacatcgc cgggttcacg gggttccacg agttcgccga
gttcacggcg     720

gaggacgtgg gcacgaccga gagcgggctc aactcggtgg tgctcgccaa
caactcggag     780

ggcgtgctgc tgccgctcaa cgagccggtg cacggcacca agcgccggag
ccagatacag     840

acgttcctgg aacaccacgg cggcccgggc gtgcagcaca tcgcggtggc
cagcagtgac     900

gtgctcagga cgctcaggaa gatgcgtgcg cgctccgcca tgggcggctt
cgacttcctg     960

ccacccccgc tgccgaagta ctacgaaggc gtgcgacgcc ttgccgggga
tgtcctctcg    1020

gaggcgcaga tcaaggaatg ccaggagctg ggtgtgctcg tcgataggga
cgaccaaggg   1080

gtgttgctcc aaatcttcac caagccagta ggggacaggc cgaccttgtt
cctggagatg   1140

atccagagga tcgggtgcat ggagaaggac gagagagggg aagagtacca
gaagggtggc   1200

tgcggcgggt tcggcaaagg caacttctcc gagctgttca agtccattga
agattacgag   1260

aagtcccttg aagccaagca atctgctgca gttcagggat catag
1305

<210>  28
<211>  434
<212>  PRT
<213>  Hordeum vulgare

<400>  28

Met Pro Pro Thr Pro Thr Thr Pro Ala Ala Thr Gly Ala Ala Ala
Ala
1               5                   10                  15

Val Thr Pro Glu His Ala Arg Pro His Arg Met Val Arg Phe Asn
Pro
            20                  25                  30

Arg Ser Asp Arg Phe His Thr Leu Ser Phe His His Val Glu Phe
Trp
        35                  40                  45

Cys Ala Asp Ala Ala Ser Ala Ala Gly Arg Phe Ala Phe Ala Leu
Gly
    50                  55                  60

Ala Pro Leu Ala Ala Arg Ser Asp Leu Ser Thr Gly Asn Ser Ala
His
65                  70                  75                  80

Ala Ser Gln Leu Leu Arg Ser Gly Ser Leu Ala Phe Leu Phe Thr
Ala
                85                  90                  95

Pro Tyr Ala Asn Gly Cys Asp Ala Ala Thr Ala Ser Leu Pro Ser
Phe
            100                 105                 110

Ser Ala Asp Ala Ala Arg Arg Phe Ser Ala Asp His Gly Ile Ala
Val
        115                 120                 125

Arg Ser Val Ala Leu Arg Val Ala Asp Ala Ala Glu Ala Phe Arg
Ala
    130                 135                 140

Ser Arg Arg Arg Gly Ala Arg Pro Ala Phe Ala Pro Val Asp Leu
Gly
145                 150                 155
160

Arg Gly Phe Ala Phe Ala Glu Val Glu Leu Tyr Gly Asp Val Val
Leu
                165                 170                 175

Arg Phe Val Ser His Pro Asp Gly Thr Asp Val Pro Phe Leu Pro
Gly
            180                 185                 190

Phe Glu Gly Val Thr Asn Pro Asp Ala Val Asp Tyr Gly Leu Thr
Arg
        195                 200                 205

Phe Asp His Val Val Gly Asn Val Pro Glu Leu Ala Pro Ala Ala
Ala
    210                 215                 220

Tyr Ile Ala Gly Phe Thr Gly Phe His Glu Phe Ala Glu Phe Thr
Ala
225                 230                 235
240

Glu Asp Val Gly Thr Thr Glu Ser Gly Leu Asn Ser Val Val Leu
Ala
                245                 250                 255

Asn Asn Ser Glu Gly Val Leu Leu Pro Leu Asn Glu Pro Val His
Gly
            260                 265                 270

Thr Lys Arg Arg Ser Gln Ile Gln Thr Phe Leu Glu His His Gly
Gly
        275                 280                 285

Pro Gly Val Gln His Ile Ala Val Ala Ser Ser Asp Val Leu Arg
Thr
    290                 295                 300

Leu Arg Lys Met Arg Ala Arg Ser Ala Met Gly Gly Phe Asp Phe
Leu
305                 310                 315
320

Pro Pro Pro Leu Pro Lys Tyr Tyr Glu Gly Val Arg Arg Leu Ala
Gly
                325                 330                 335

Asp Val Leu Ser Glu Ala Gln Ile Lys Glu Cys Gln Glu Leu Gly
Val
            340                 345                 350

Leu Val Asp Arg Asp Asp Gln Gly Val Leu Leu Gln Ile Phe Thr
Lys
        355                 360                 365

Pro Val Gly Asp Arg Pro Thr Leu Phe Leu Glu Met Ile Gln Arg
Ile
    370                 375                 380

Gly Cys Met Glu Lys Asp Glu Arg Gly Glu Glu Tyr Gln Lys Gly
Gly
385                 390                 395
400

Cys Gly Gly Phe Gly Lys Gly Asn Phe Ser Glu Leu Phe Lys Ser
Ile
                405                 410                 415

Glu Asp Tyr Glu Lys Ser Leu Glu Ala Lys Gln Ser Ala Ala Val
Gln
            420                 425                 430

Gly Ser

<210>  29
<211>  1209
<212>  DNA
<213>  Rhodococcus sp.

<400>  29
atgactaccg ccgacattcg cctgacgccc cgcgaggtgg ccgcacatct
ggagaccgac      60

gagctccggc agttggtcgg gctcgtcgaa cacgacgacg cgtcggatcc
gtttcccgtg     120

gtcgcgatgg atgccgtggt gttcgtgtgc ggcaacgcga cgcagagcac
gcagtacttc     180

gtctccacgt ggggcatgac cctcgtcgcc tacgccgggc cggagaccgg
tcagcgctcg     240

cacaagtcct tcgtcctcga gtcggggtcg gcacggttcg tgctgcacgg
cgccgtcgat     300

ccgaagagcc cgctcgcgga ccatcaccgg gcgcacggcg acggcgtggt
ggacctggcg     360

atggaagttc tcgacgtcga ccgctgcatc gcgcatgcac gctcgcaggg
ggccaccatt     420

ctcgaggagc cgcgcgacgt cacggatcag ttcggcaccg tgcggctcgc
ggcgatcgcc     480

acgtacggca gcacccggca caccatcgtc gaccgaagcc gatacgacgg
cccctacctc     540

cccggattcg tcgcgcgctc cagcggtttc gcggcgcgac cgggtaaacc
cccgcgattg     600

ttccaggcgc tcgaccacgc cgtcggcaac gtcgagatgg gccggatgga
tcactgggtc     660

cggttctaca accgcgtcat gggcttcacg aacatggccg aattcgtcgg
cgacgacatc     720

gccacggagt actcggcgct gatgtcgaag gtcgtggcga acggcaatca
ccgggtgaag    780

ttcccgctca acgaacccgc ggtgggaaag aagaagtcgc agatcgacga
atatctcgag    840

ttctacggtg agccgggctg ccagcatctg gccctcgcga ccggagacat
cctcgcgacg    900

gtggacgcgt tgcgggccga gggtgtcgaa ttcctgaaca cacccgacgc
gtactacgag    960

gacccacagc tgcgcgcccg gatcggcagg gtgcgggtgc cggtggagga
actgcagaag   1020

cgcggaatcc tcgtcgaccg cgacgaggac ggatacctcc tgcagatctt
caccaaaccg   1080

ctcggcgacc ggccgaccgt gttcttcgag gtgatcgaac ggcacggttc
gctcgggttc   1140

ggggcgggta acttccaggc cctgttcgaa tccatcgagc gtgagcaggc
ggcgcgcggc   1200

aatctgtga
1209

<210>  30
<211>  402
<212>  PRT
<213>  Rhodococcus sp.

<400>  30

Met Thr Thr Ala Asp Ile Arg Leu Thr Pro Arg Glu Val Ala Ala
His
1               5                   10                  15

Leu Glu Thr Asp Glu Leu Arg Gln Leu Val Gly Leu Val Glu His
Asp
            20                  25                  30

Asp Ala Ser Asp Pro Phe Pro Val Val Ala Met Asp Ala Val Val
Phe
        35                  40                  45

Val Cys Gly Asn Ala Thr Gln Ser Thr Gln Tyr Phe Val Ser Thr
Trp

50                  55                  60

Gly Met Thr Leu Val Ala Tyr Ala Gly Pro Glu Thr Gly Gln Arg
Ser
65                  70                  75                  80

His Lys Ser Phe Val Leu Glu Ser Gly Ser Ala Arg Phe Val Leu
His
                85                  90                  95

Gly Ala Val Asp Pro Lys Ser Pro Leu Ala Asp His His Arg Ala
His
            100                 105                 110

Gly Asp Gly Val Val Asp Leu Ala Met Glu Val Leu Asp Val Asp
Arg
        115                 120                 125

Cys Ile Ala His Ala Arg Ser Gln Gly Ala Thr Ile Leu Glu Glu
Pro
    130                 135                 140

Arg Asp Val Thr Asp Gln Phe Gly Thr Val Arg Leu Ala Ala Ile
Ala
145                 150                 155
160

Thr Tyr Gly Ser Thr Arg His Thr Ile Val Asp Arg Ser Arg Tyr
Asp
                165                 170                 175

Gly Pro Tyr Leu Pro Gly Phe Val Ala Arg Ser Ser Gly Phe Ala
Ala
            180                 185                 190

Arg Pro Gly Lys Pro Pro Arg Leu Phe Gln Ala Leu Asp His Ala
Val
        195                 200                 205

Gly Asn Val Glu Met Gly Arg Met Asp His Trp Val Arg Phe Tyr
Asn
    210                 215                 220

Arg Val Met Gly Phe Thr Asn Met Ala Glu Phe Val Gly Asp Asp
Ile
225                 230                 235
240

Ala Thr Glu Tyr Ser Ala Leu Met Ser Lys Val Val Ala Asn Gly
Asn
            245                 250                 255

His Arg Val Lys Phe Pro Leu Asn Glu Pro Ala Val Gly Lys Lys
Lys
        260                 265                 270

Ser Gln Ile Asp Glu Tyr Leu Glu Phe Tyr Gly Glu Pro Gly Cys
Gln
    275                 280                 285

His Leu Ala Leu Ala Thr Gly Asp Ile Leu Ala Thr Val Asp Ala
Leu
    290                 295                 300

Arg Ala Glu Gly Val Glu Phe Leu Asn Thr Pro Asp Ala Tyr Tyr
Glu
305                 310                 315
320

Asp Pro Gln Leu Arg Ala Arg Ile Gly Arg Val Arg Val Pro Val
Glu
            325                 330                 335

Glu Leu Gln Lys Arg Gly Ile Leu Val Asp Arg Asp Glu Asp Gly
Tyr
        340                 345                 350

Leu Leu Gln Ile Phe Thr Lys Pro Leu Gly Asp Arg Pro Thr Val
Phe
    355                 360                 365

Phe Glu Val Ile Glu Arg His Gly Ser Leu Gly Phe Gly Ala Gly
Asn
    370                 375                 380

Phe Gln Ala Leu Phe Glu Ser Ile Glu Arg Glu Gln Ala Ala Arg
Gly
385                 390                 395
400

Asn Leu

<210>  31
<211>  1353
<212>  DNA
<213>  Artificial sequence

<220>
<223>  Nucleic acid sequence encoding Arabidopsis HPPD plus
containing
       at the 5' end a nucleic acid encoding an Ala and 6 His
amino
       acids

<220>
<221>  misc_feature
<222>  (4)..(6)
<223>  sequence coding for an Ala

<220>
<221>  misc_feature
<222>  (7)..(24)
<223>  sequence coding for an His Tag containing 6 His

<400>  31
atggctcatc accatcacca tcaccaaaac gccgccgttt cagagaatca
aaaccatgat      60

gacggcgctg cgtcgtcgcc gggattcaag ctcgtcggat tttccaagtt
cgtaagaaag     120

aatccaaagt ctgataaatt caaggttaag cgcttccatc acatcgagtt
ctggtgcggc     180

gacgcaacca acgtcgctcg tcgcttctcc tggggtctgg ggatgagatt
ctccgccaaa     240

tccgatcttt ccaccggaaa catggttcac gcctcttacc tactcacctc
cggtgacctc     300

cgattccttt tcactgctcc ttactctccg tctctctccg ccggagagat
taaaccgaca    360

accacagctt ctatcccaag tttcgatcac ggctcttgtc gttccttctt
ctcgtcacat    420

ggtctcggtg ttagagccgt tgcgattgaa gtagaagacg cagagtcagc
tttctccatc    480

agtgtagcta atggcgctat tccttcgtcg cctcctatcg tcctcaatga
agcagttacg    540

atcgctgagg ttaaactata cggcgatgtt gttctccgat atgttagtta
caaagcagaa    600

gataccgaaa aatccgaatt cttgccaggg ttcgagcgtg tagaggatgc
gtcgtcgttc    660

ccattggatt atggtatccg gcggcttgac cacgccgtgg gaaacgttcc
tgagcttggt    720

ccggctttaa cttatgtagc ggggttcact ggttttcacc aattcgcaga
gttcacagca    780

gacgacgttg gaaccgccga gagcggttta aattcagcgg tcctggctag
caatgatgaa    840

atggttcttc taccgattaa cgagccagtg cacggaacaa agaggaagag
tcagattcag    900

acgtatttgg aacataacga aggcgcaggg ctacaacatc tggctctgat
gagtgaagac    960

atattcagga ccctgagaga gatgaggaag aggagcagta ttggaggatt
cgacttcatg   1020

ccttctcctc cgcctactta ctaccagaat ctcaagaaac gggtcggcga
cgtgctcagc   1080

gatgatcaga tcaaggagtg tgaggaatta gggattcttg tagacagaga
tgatcaaggg   1140

acgttgcttc aaatcttcac aaaaccacta ggtgacaggc cgacgatatt
tatagagata   1200

atccagagag taggatgcat gatgaaagat gaggaaggga aggcttacca
gagtggagga   1260

tgtggtggtt ttggcaaagg caatttctct gagctcttca agtccattga
agaatacgaa   1320

aagactcttg aagccaaaca gttagtggga tga
1353

<210> 32
<211> 450
<212> PRT
<213> Artificial sequence

<220>
<223> encoded by SEQ ID No. 31

<220>
<221> MISC_FEATURE
<222> (2)..(2)
<223> Ala

<220>
<221> MISC_FEATURE
<222> (3)..(8)
<223> His Tag made of 6 His

<400> 32

Met Ala His His His His His His Gln Asn Ala Ala Val Ser Glu
Asn
1               5                   10                  15

Gln Asn His Asp Asp Gly Ala Ala Ser Ser Pro Gly Phe Lys Leu
Val
            20                  25                  30

Gly Phe Ser Lys Phe Val Arg Lys Asn Pro Lys Ser Asp Lys Phe
Lys
        35                  40                  45

Val Lys Arg Phe His His Ile Glu Phe Trp Cys Gly Asp Ala Thr
Asn
    50                  55                  60

Val Ala Arg Arg Phe Ser Trp Gly Leu Gly Met Arg Phe Ser Ala
Lys
65                  70                  75                  80

Ser Asp Leu Ser Thr Gly Asn Met Val His Ala Ser Tyr Leu Leu
Thr

85                  90                  95

Ser Gly Asp Leu Arg Phe Leu Phe Thr Ala Pro Tyr Ser Pro Ser
Leu
            100                 105                 110

Ser Ala Gly Glu Ile Lys Pro Thr Thr Thr Ala Ser Ile Pro Ser
Phe
        115                 120                 125

Asp His Gly Ser Cys Arg Ser Phe Phe Ser Ser His Gly Leu Gly
Val
    130                 135                 140

Arg Ala Val Ala Ile Glu Val Glu Asp Ala Glu Ser Ala Phe Ser
Ile
145                 150                 155
160

Ser Val Ala Asn Gly Ala Ile Pro Ser Ser Pro Pro Ile Val Leu
Asn
                165                 170                 175

Glu Ala Val Thr Ile Ala Glu Val Lys Leu Tyr Gly Asp Val Val
Leu
            180                 185                 190

Arg Tyr Val Ser Tyr Lys Ala Glu Asp Thr Glu Lys Ser Glu Phe
Leu
        195                 200                 205

Pro Gly Phe Glu Arg Val Glu Asp Ala Ser Ser Phe Pro Leu Asp
Tyr
    210                 215                 220

Gly Ile Arg Arg Leu Asp His Ala Val Gly Asn Val Pro Glu Leu
Gly
225                 230                 235
240

Pro Ala Leu Thr Tyr Val Ala Gly Phe Thr Gly Phe His Gln Phe
Ala

                    245                     250                     255

Glu Phe Thr Ala Asp Asp Val Gly Thr Ala Glu Ser Gly Leu Asn
Ser
                260                     265                     270

Ala Val Leu Ala Ser Asn Asp Glu Met Val Leu Leu Pro Ile Asn
Glu
            275                     280                     285

Pro Val His Gly Thr Lys Arg Lys Ser Gln Ile Gln Thr Tyr Leu
Glu
    290                     295                     300

His Asn Glu Gly Ala Gly Leu Gln His Leu Ala Leu Met Ser Glu
Asp
305                     310                     315
320

Ile Phe Arg Thr Leu Arg Glu Met Arg Lys Arg Ser Ser Ile Gly
Gly
                    325                     330                     335

Phe Asp Phe Met Pro Ser Pro Pro Pro Thr Tyr Tyr Gln Asn Leu
Lys
                340                     345                     350

Lys Arg Val Gly Asp Val Leu Ser Asp Asp Gln Ile Lys Glu Cys
Glu
            355                     360                     365

Glu Leu Gly Ile Leu Val Asp Arg Asp Asp Gln Gly Thr Leu Leu
Gln
    370                     375                     380

Ile Phe Thr Lys Pro Leu Gly Asp Arg Pro Thr Ile Phe Ile Glu
Ile
385                     390                     395
400

Ile Gln Arg Val Gly Cys Met Met Lys Asp Glu Glu Gly Lys Ala
Tyr

405                 410                 415

Gln Ser Gly Gly Cys Gly Gly Phe Gly Lys Gly Asn Phe Ser Glu
Leu
            420                 425                 430

Phe Lys Ser Ile Glu Glu Tyr Glu Lys Thr Leu Glu Ala Lys Gln
Leu
        435                 440                 445

Val Gly
    450

<210>  33
<211>  1353
<212>  DNA
<213>  Artificial sequence

<220>
<223>  nucleotide sequence encoding mutant HPPD polypeptide

<220>
<221>  misc_feature
<222>  (4)..(6)
<223>  sequence coding for Ala

<220>
<221>  misc_feature
<222>  (7)..(24)
<223>  sequence coding an His Tag containing 6 His

<400>  33
atggctcatc accatcacca tcaccaaaac gccgccgttt cagagaatca
aaaccatgat      60

gacggcgctg cgtcgtcgcc gggattcaag ctcgtcggat tttccaagtt
cgtaagaaag     120

aatccaaagt ctgataaatt caaggttaag cgcttccatc acatcgagtt
ctggtgcggc     180

gacgcaacca acgtcgctcg tcgcttctcc tggggtctgg ggatgagatt
ctccgccaaa     240

tccgatcttt ccaccggaaa catggttcac gcctcttacc tactcacctc
cggtgacctc     300

cgattccttt tcactgctcc ttactctccg tctctctccg ccggagagat
taaaccgaca    360

accacagctt ctatcccaag tttcgatcac ggctcttgtc gttccttctt
ctcgtcacat    420

ggtctcggtg ttagagccgt tgcgattgaa gtagaagacg cagagtcagc
tttctccatc    480

agtgtagcta atggcgctat tccttcgtcg cctcctatcg tcctcaatga
agcagttacg    540

atcgctgagg ttaaactata cggcgatgtt gttctccgat atgttagtta
caaagcagaa    600

gataccgaaa aatccgaatt cttgccaggg ttcgagcgtg tagaggatgc
gtcgtcgttc    660

ccattggatt atggtatccg gcggcttgac cacgccgtgg gaaacgttcc
tgagcttggt    720

ccggctttaa cttatgtagc ggggttcact ggttttcacc aattcgcaga
gttcacagca    780

gacgacgttg gaaccgccga gagcggttta aatgcggcgg tcctggctag
caatgatgaa    840

atggttcttc taccgattaa cgagccagtg cacggaacaa agaggaagag
tcagattcag    900

acgtatttgg aacataacga aggcgcaggg ctacaacatc tggctctgat
gagtgaagac    960

atattcagga ccctgagaga gatgaggaag aggagcagta ttggaggatt
cgacttcatg   1020

ccttctcctc cgcctactta ctaccagaat ctcaagaaac gggtcggcga
cgtgctcagc   1080

gatgatcaga tcaaggagtg tgaggaatta gggattcttg tagacagaga
tgatcaaggg   1140

acgttgcttc aaatcttcac aaaaccacta ggtgacaggc cgacgatatt
tatagagata   1200

atccagagag taggatgcat gatgaaagat gaggaaggga aggcttacca
gagtggagga   1260

tgtggtggtt ttggcaaagg caatttctct gagctcttca agtccattga
agaatacgaa   1320

aagactcttg aagccaaaca gttagtggga tga
1353

<210> 34
<211> 450
<212> PRT
<213> Artificial sequence

<220>
<223> encoded by SEQ ID No. 33

<220>
<221> MISC_FEATURE
<222> (2)..(2)
<223> Ala

<220>
<221> MISC_FEATURE
<222> (3)..(8)
<223> His Tag made of 6 His

<400> 34

Met Ala His His His His His His Gln Asn Ala Ala Val Ser Glu
Asn
1               5                   10                  15

Gln Asn His Asp Asp Gly Ala Ala Ser Ser Pro Gly Phe Lys Leu
Val
            20                  25                  30

Gly Phe Ser Lys Phe Val Arg Lys Asn Pro Lys Ser Asp Lys Phe
Lys
        35                  40                  45

Val Lys Arg Phe His His Ile Glu Phe Trp Cys Gly Asp Ala Thr
Asn
    50                  55                  60

Val Ala Arg Arg Phe Ser Trp Gly Leu Gly Met Arg Phe Ser Ala
Lys
65                  70                  75                  80

Ser Asp Leu Ser Thr Gly Asn Met Val His Ala Ser Tyr Leu Leu
Thr

85                  90                  95

Ser Gly Asp Leu Arg Phe Leu Phe Thr Ala Pro Tyr Ser Pro Ser
Leu
            100                 105                 110

Ser Ala Gly Glu Ile Lys Pro Thr Thr Thr Ala Ser Ile Pro Ser
Phe
        115                 120                 125

Asp His Gly Ser Cys Arg Ser Phe Phe Ser Ser His Gly Leu Gly
Val
    130                 135                 140

Arg Ala Val Ala Ile Glu Val Glu Asp Ala Glu Ser Ala Phe Ser
Ile
145                 150                 155
160

Ser Val Ala Asn Gly Ala Ile Pro Ser Ser Pro Pro Ile Val Leu
Asn
                165                 170                 175

Glu Ala Val Thr Ile Ala Glu Val Lys Leu Tyr Gly Asp Val Val
Leu
            180                 185                 190

Arg Tyr Val Ser Tyr Lys Ala Glu Asp Thr Glu Lys Ser Glu Phe
Leu
        195                 200                 205

Pro Gly Phe Glu Arg Val Glu Asp Ala Ser Ser Phe Pro Leu Asp
Tyr
    210                 215                 220

Gly Ile Arg Arg Leu Asp His Ala Val Gly Asn Val Pro Glu Leu
Gly
225                 230                 235
240

Pro Ala Leu Thr Tyr Val Ala Gly Phe Thr Gly Phe His Gln Phe
Ala

245                 250                 255

Glu Phe Thr Ala Asp Asp Val Gly Thr Ala Glu Ser Gly Leu Asn
Ala
            260                 265                 270

Ala Val Leu Ala Ser Asn Asp Glu Met Val Leu Leu Pro Ile Asn
Glu
        275                 280                 285

Pro Val His Gly Thr Lys Arg Lys Ser Gln Ile Gln Thr Tyr Leu
Glu
    290                 295                 300

His Asn Glu Gly Ala Gly Leu Gln His Leu Ala Leu Met Ser Glu
Asp
305                 310                 315
320

Ile Phe Arg Thr Leu Arg Glu Met Arg Lys Arg Ser Ser Ile Gly
Gly
                325                 330                 335

Phe Asp Phe Met Pro Ser Pro Pro Pro Thr Tyr Tyr Gln Asn Leu
Lys
            340                 345                 350

Lys Arg Val Gly Asp Val Leu Ser Asp Asp Gln Ile Lys Glu Cys
Glu
        355                 360                 365

Glu Leu Gly Ile Leu Val Asp Arg Asp Asp Gln Gly Thr Leu Leu
Gln
    370                 375                 380

Ile Phe Thr Lys Pro Leu Gly Asp Arg Pro Thr Ile Phe Ile Glu
Ile
385                 390                 395
400

Ile Gln Arg Val Gly Cys Met Met Lys Asp Glu Glu Gly Lys Ala
Tyr

405                 410                 415

Gln Ser Gly Gly Cys Gly Gly Phe Gly Lys Gly Asn Phe Ser Glu
Leu
            420                 425                 430

Phe Lys Ser Ile Glu Glu Tyr Glu Lys Thr Leu Glu Ala Lys Gln
Leu
        435                 440                 445

Val Gly
    450

<210>  35
<211>  1353
<212>  DNA
<213>  Artificial sequence

<220>
<223>  nucleotide sequence encoding mutant HPPD polypeptide

<220>
<221>  misc_feature
<222>  (4)..(6)
<223>  sequence coding for Ala

<220>
<221>  misc_feature
<222>  (7)..(24)
<223>  sequence coding for an His Tag containing 6 His

<400>  35
atggctcatc accatcacca tcaccaaaac gccgccgttt cagagaatca
aaaccatgat      60

gacggcgctg cgtcgtcgcc gggattcaag ctcgtcggat tttccaagtt
cgtaagaaag     120

aatccaaagt ctgataaatt caaggttaag cgcttccatc acatcgagtt
ctggtgcggc     180

gacgcaacca acgtcgctcg tcgcttctcc tggggtctgg ggatgagatt
ctccgccaaa     240

tccgatcttt ccaccggaaa catggttcac gcctcttacc tactcacctc
cggtgacctc     300

cgattccttt tcactgctcc ttactctccg tctctctccg ccggagagat
taaaccgaca    360

accacagctt ctatcccaag tttcgatcac ggctcttgtc gttccttctt
ctcgtcacat    420

ggtctcggtg ttagagccgt tgcgattgaa gtagaagacg cagagtcagc
tttctccatc    480

agtgtagcta atggcgctat tccttcgtcg cctcctatcg tcctcaatga
agcagttacg    540

atcgctgagg ttaaactata cggcgatgtt gttctccgat atgttagtta
caaagcagaa    600

gataccgaaa aatccgaatt cttgccaggg ttcgagcgtg tagaggatgc
gtcgtcgttc    660

ccattggatt atggtatccg gcggcttgac cacgccgtgg gaaacgttcc
tgagcttggt    720

ccggctttaa cttatgtagc ggggttcact ggttttcacc aattcgcaga
gttcacagca    780

gacgacgttg gaaccgccga gagcggttta aattcagcgg tcctggctag
caatgatgaa    840

atggttcttc taccgattgc cgagccagtg cacggaacaa agaggaagag
tcagattcag    900

acgtatttgg aacataacga aggcgcaggg ctacaacatc tggctctgat
gagtgaagac    960

atattcagga ccctgagaga gatgaggaag aggagcagta ttggaggatt
cgacttcatg   1020

ccttctcctc cgcctactta ctaccagaat ctcaagaaac gggtcggcga
cgtgctcagc   1080

gatgatcaga tcaaggagtg tgaggaatta gggattcttg tagacagaga
tgatcaaggg   1140

acgttgcttc aaatcttcac aaaaccacta ggtgacaggc cgacgatatt
tatagagata   1200

atccagagag taggatgcat gatgaaagat gaggaaggga aggcttacca
gagtggagga   1260

tgtggtggtt ttggcaaagg caatttctct gagctcttca agtccattga
agaatacgaa   1320

aagactcttg aagccaaaca gttagtggga tga
1353

<210> 36
<211> 450
<212> PRT
<213> Artificial sequence

<220>
<223> encoded by SEQ ID No. 35

<220>
<221> MISC_FEATURE
<222> (2)..(2)
<223> Ala

<220>
<221> MISC_FEATURE
<222> (3)..(8)
<223> His Tag made of 6 His

<400> 36

Met Ala His His His His His His Gln Asn Ala Ala Val Ser Glu
Asn
1               5                   10                  15

Gln Asn His Asp Asp Gly Ala Ala Ser Ser Pro Gly Phe Lys Leu
Val
            20                  25                  30

Gly Phe Ser Lys Phe Val Arg Lys Asn Pro Lys Ser Asp Lys Phe
Lys
        35                  40                  45

Val Lys Arg Phe His His Ile Glu Phe Trp Cys Gly Asp Ala Thr
Asn
    50                  55                  60

Val Ala Arg Arg Phe Ser Trp Gly Leu Gly Met Arg Phe Ser Ala
Lys
65                  70                  75                  80

Ser Asp Leu Ser Thr Gly Asn Met Val His Ala Ser Tyr Leu Leu
Thr

85                  90                  95

Ser Gly Asp Leu Arg Phe Leu Phe Thr Ala Pro Tyr Ser Pro Ser
Leu
            100                 105                 110

Ser Ala Gly Glu Ile Lys Pro Thr Thr Thr Ala Ser Ile Pro Ser
Phe
        115                 120                 125

Asp His Gly Ser Cys Arg Ser Phe Phe Ser Ser His Gly Leu Gly
Val
    130                 135                 140

Arg Ala Val Ala Ile Glu Val Glu Asp Ala Glu Ser Ala Phe Ser
Ile
145                 150                 155
160

Ser Val Ala Asn Gly Ala Ile Pro Ser Ser Pro Pro Ile Val Leu
Asn
                165                 170                 175

Glu Ala Val Thr Ile Ala Glu Val Lys Leu Tyr Gly Asp Val Val
Leu
            180                 185                 190

Arg Tyr Val Ser Tyr Lys Ala Glu Asp Thr Glu Lys Ser Glu Phe
Leu
        195                 200                 205

Pro Gly Phe Glu Arg Val Glu Asp Ala Ser Ser Phe Pro Leu Asp
Tyr
    210                 215                 220

Gly Ile Arg Arg Leu Asp His Ala Val Gly Asn Val Pro Glu Leu
Gly
225                 230                 235
240

Pro Ala Leu Thr Tyr Val Ala Gly Phe Thr Gly Phe His Gln Phe
Ala

                  245                     250                     255

Glu Phe Thr Ala Asp Asp Val Gly Thr Ala Glu Ser Gly Leu Asn
Ser
              260                     265                     270

Ala Val Leu Ala Ser Asn Asp Glu Met Val Leu Leu Pro Ile Ala
Glu
          275                     280                     285

Pro Val His Gly Thr Lys Arg Lys Ser Gln Ile Gln Thr Tyr Leu
Glu
     290                     295                     300

His Asn Glu Gly Ala Gly Leu Gln His Leu Ala Leu Met Ser Glu
Asp
305                     310                     315
320

Ile Phe Arg Thr Leu Arg Glu Met Arg Lys Arg Ser Ser Ile Gly
Gly
                  325                     330                     335

Phe Asp Phe Met Pro Ser Pro Pro Pro Thr Tyr Tyr Gln Asn Leu
Lys
              340                     345                     350

Lys Arg Val Gly Asp Val Leu Ser Asp Asp Gln Ile Lys Glu Cys
Glu
          355                     360                     365

Glu Leu Gly Ile Leu Val Asp Arg Asp Asp Gln Gly Thr Leu Leu
Gln
     370                     375                     380

Ile Phe Thr Lys Pro Leu Gly Asp Arg Pro Thr Ile Phe Ile Glu
Ile
385                     390                     395
400

Ile Gln Arg Val Gly Cys Met Met Lys Asp Glu Glu Gly Lys Ala
Tyr

405                 410                 415

Gln Ser Gly Gly Cys Gly Gly Phe Gly Lys Gly Asn Phe Ser Glu
Leu
            420                 425                 430

Phe Lys Ser Ile Glu Glu Tyr Glu Lys Thr Leu Glu Ala Lys Gln
Leu
        435                 440                 445

Val Gly
    450

<210>  37
<211>  1353
<212>  DNA
<213>  Artificial sequence

<220>
<223>  nucleotide sequence encoding mutant HPPD polypeptide

<220>
<221>  misc_feature
<222>  (4)..(6)
<223>  sequence coding for Ala

<220>
<221>  misc_feature
<222>  (7)..(24)
<223>  sequence coding for an His Tag containing 6 His

<400>  37
atggctcatc accatcacca tcaccaaaac gccgccgttt cagagaatca
aaaccatgat      60

gacggcgctg cgtcgtcgcc gggattcaag ctcgtcggat tttccaagtt
cgtaagaaag     120

aatccaaagt ctgataaatt caaggttaag cgcttccatc acatcgagtt
ctggtgcggc     180

gacgcaacca acgtcgctcg tcgcttctcc tggggtctgg ggatgagatt
ctccgccaaa     240

tccgatcttt ccaccggaaa catggttcac gcctcttacc tactcacctc
cggtgacctc     300

cgattccttt tcactgctcc ttactctccg tctctctccg ccggagagat
taaaccgaca    360

accacagctt ctatcccaag tttcgatcac ggctcttgtc gttccttctt
ctcgtcacat    420

ggtctcggtg ttagagccgt tgcgattgaa gtagaagacg cagagtcagc
tttctccatc    480

agtgtagcta atggcgctat tccttcgtcg cctcctatcg tcctcaatga
agcagttacg    540

atcgctgagg ttaaactata cggcgatgtt gttctccgat atgttagtta
caaagcagaa    600

gataccgaaa aatccgaatt cttgccaggg ttcgagcgtg tagaggatgc
gtcgtcgttc    660

ccattggatt atggtatccg gcggcttgac cacgccgtgg gaaacgttcc
tgagcttggt    720

ccggctttaa cttatgtagc ggggttcact ggttttcacc aattcgcatc
tttcacagca    780

gacgacgttg gaaccgccga gagcggttta aattcagcgg tcctggctag
caatgatgaa    840

atggttcttc taccgattaa cgagccagtg cacggaacaa agaggaagag
tcagattcag    900

acgtatttgg aacataacga aggcgcaggg ctacaacatc tggctctgat
gagtgaagac    960

atattcagga ccctgagaga gatgaggaag aggagcagta ttggaggatt
cgacttcatg   1020

ccttctcctc cgcctactta ctaccagaat ctcaagaaac gggtcggcga
cgtgctcagc   1080

gatgatcaga tcaaggagtg tgaggaatta gggattcttg tagacagaga
tgatcaaggg   1140

acgttgcttc aaatcttcac aaaaccacta ggtgacaggc cgacgatatt
tatagagata   1200

atccagagag taggatgcat gatgaaagat gaggaaggga aggcttacca
gagtggagga   1260

tgtggtggtt ttggcaaagg caatttctct gagctcttca agtccattga
agaatacgaa   1320

aagactcttg aagccaaaca gttagtggga tga
1353

<210> 38
<211> 450
<212> PRT
<213> Artificial sequence

<220>
<223> encoded by SEQ ID No. 37

<220>
<221> MISC_FEATURE
<222> (2)..(2)
<223> Ala

<220>
<221> MISC_FEATURE
<222> (3)..(8)
<223> His Tag made of 6 His

<400> 38

Met Ala His His His His His His Gln Asn Ala Ala Val Ser Glu
Asn
1               5                   10                  15

Gln Asn His Asp Asp Gly Ala Ala Ser Ser Pro Gly Phe Lys Leu
Val
            20                  25                  30

Gly Phe Ser Lys Phe Val Arg Lys Asn Pro Lys Ser Asp Lys Phe
Lys
        35                  40                  45

Val Lys Arg Phe His His Ile Glu Phe Trp Cys Gly Asp Ala Thr
Asn
    50                  55                  60

Val Ala Arg Arg Phe Ser Trp Gly Leu Gly Met Arg Phe Ser Ala
Lys
65                  70                  75                  80

Ser Asp Leu Ser Thr Gly Asn Met Val His Ala Ser Tyr Leu Leu
Thr

85                  90                  95

Ser Gly Asp Leu Arg Phe Leu Phe Thr Ala Pro Tyr Ser Pro Ser
Leu
            100                 105                 110

Ser Ala Gly Glu Ile Lys Pro Thr Thr Thr Ala Ser Ile Pro Ser
Phe
        115                 120                 125

Asp His Gly Ser Cys Arg Ser Phe Phe Ser Ser His Gly Leu Gly
Val
    130                 135                 140

Arg Ala Val Ala Ile Glu Val Glu Asp Ala Glu Ser Ala Phe Ser
Ile
145                 150                 155
160

Ser Val Ala Asn Gly Ala Ile Pro Ser Ser Pro Pro Ile Val Leu
Asn
                165                 170                 175

Glu Ala Val Thr Ile Ala Glu Val Lys Leu Tyr Gly Asp Val Val
Leu
            180                 185                 190

Arg Tyr Val Ser Tyr Lys Ala Glu Asp Thr Glu Lys Ser Glu Phe
Leu
        195                 200                 205

Pro Gly Phe Glu Arg Val Glu Asp Ala Ser Ser Phe Pro Leu Asp
Tyr
    210                 215                 220

Gly Ile Arg Arg Leu Asp His Ala Val Gly Asn Val Pro Glu Leu
Gly
225                 230                 235
240

Pro Ala Leu Thr Tyr Val Ala Gly Phe Thr Gly Phe His Gln Phe
Ala

                    245                                     250                                     255

Ser Phe Thr Ala Asp Asp Val Gly Thr Ala Glu Ser Gly Leu Asn
Ser
                260                                     265                                     270

Ala Val Leu Ala Ser Asn Asp Glu Met Val Leu Leu Pro Ile Asn
Glu
        275                                     280                                     285

Pro Val His Gly Thr Lys Arg Lys Ser Gln Ile Gln Thr Tyr Leu
Glu
    290                                     295                                     300

His Asn Glu Gly Ala Gly Leu Gln His Leu Ala Leu Met Ser Glu
Asp
305                                     310                                     315
320

Ile Phe Arg Thr Leu Arg Glu Met Arg Lys Arg Ser Ser Ile Gly
Gly
                                325                                     330                                     335

Phe Asp Phe Met Pro Ser Pro Pro Pro Thr Tyr Tyr Gln Asn Leu
Lys
                340                                     345                                     350

Lys Arg Val Gly Asp Val Leu Ser Asp Asp Gln Ile Lys Glu Cys
Glu
        355                                     360                                     365

Glu Leu Gly Ile Leu Val Asp Arg Asp Asp Gln Gly Thr Leu Leu
Gln
    370                                     375                                     380

Ile Phe Thr Lys Pro Leu Gly Asp Arg Pro Thr Ile Phe Ile Glu
Ile
385                                     390                                     395
400

Ile Gln Arg Val Gly Cys Met Met Lys Asp Glu Glu Gly Lys Ala
Tyr

405                 410                 415

Gln Ser Gly Gly Cys Gly Gly Phe Gly Lys Gly Asn Phe Ser Glu
Leu
            420                 425                 430

Phe Lys Ser Ile Glu Glu Tyr Glu Lys Thr Leu Glu Ala Lys Gln
Leu
        435                 440                 445

Val Gly
    450

<210>  39
<211>  1353
<212>  DNA
<213>  Artificial sequence

<220>
<223>  nucleotide sequence encoding mutant HPPD polypeptide

<220>
<221>  misc_feature
<222>  (4)..(6)
<223>  sequence coding for Ala

<220>
<221>  misc_feature
<222>  (7)..(24)
<223>  sequence coding for an His Tag containing 6 His

<400>  39
atggctcatc accatcacca tcaccaaaac gccgccgttt cagagaatca
aaaccatgat      60

gacggcgctg cgtcgtcgcc gggattcaag ctcgtcggat tttccaagtt
cgtaagaaag     120

aatccaaagt ctgataaatt caaggttaag cgcttccatc acatcgagtt
ctggtgcggc     180

gacgcaacca acgtcgctcg tcgcttctcc tggggtctgg ggatgagatt
ctccgccaaa     240

tccgatcttt ccaccggaaa catggttcac gcctcttacc tactcacctc
cggtgacctc     300

cgattccttt tcactgctcc ttactctccg tctctctccg ccggagagat
taaaccgaca    360

accacagctt ctatcccaag tttcgatcac ggctcttgtc gttccttctt
ctcgtcacat    420

ggtctcggtg ttagagccgt tgcgattgaa gtagaagacg cagagtcagc
tttctccatc    480

agtgtagcta atggcgctat tccttcgtcg cctcctatcg tcctcaatga
agcagttacg    540

atcgctgagg ttaaactata cggcgatgtt gttctccgat atgttagtta
caaagcagaa    600

gataccgaaa aatccgaatt cttgccaggg ttcgagcgtg tagaggatgc
gtcgtcgttc    660

ccattggatt atggtatccg gcggcttgac cacgccgtgg gaaacgttcc
tgagcttggt    720

ccggctttaa cttatgtagc ggggttcact ggttttcacc aattcgcaga
gttcacagca    780

gacgacgttg gaaccgccga gagcggttta aattcagcgg cgctggctag
caatgatgaa    840

atggttcttc taccgattaa cgagccagtg cacggaacaa agaggaagag
tcagattcag    900

acgtatttgg aacataacga aggcgcaggg ctacaacatc tggctctgat
gagtgaagac    960

atattcagga ccctgagaga gatgaggaag aggagcagta ttggaggatt
cgacttcatg   1020

ccttctcctc cgcctactta ctaccagaat ctcaagaaac gggtcggcga
cgtgctcagc   1080

gatgatcaga tcaaggagtg tgaggaatta gggattcttg tagacagaga
tgatcaaggg   1140

acgttgcttc aaatcttcac aaaaccacta ggtgacaggc cgacgatatt
tatagagata   1200

atccagagag taggatgcat gatgaaagat gaggaaggga aggcttacca
gagtggagga   1260

tgtggtggtt ttggcaaagg caatttctct gagctcttca agtccattga
agaatacgaa   1320

aagactcttg aagccaaaca gttagtggga tga
1353

<210> 40
<211> 450
<212> PRT
<213> Artificial sequence

<220>
<223> encoded by SEQ ID No. 39

<220>
<221> MISC_FEATURE
<222> (2)..(2)
<223> Ala

<220>
<221> MISC_FEATURE
<222> (3)..(8)
<223> His Tag made of 6 His

<400> 40

Met Ala His His His His His His Gln Asn Ala Ala Val Ser Glu
Asn
1               5                   10                  15

Gln Asn His Asp Asp Gly Ala Ala Ser Ser Pro Gly Phe Lys Leu
Val
            20                  25                  30

Gly Phe Ser Lys Phe Val Arg Lys Asn Pro Lys Ser Asp Lys Phe
Lys
        35                  40                  45

Val Lys Arg Phe His His Ile Glu Phe Trp Cys Gly Asp Ala Thr
Asn
    50                  55                  60

Val Ala Arg Arg Phe Ser Trp Gly Leu Gly Met Arg Phe Ser Ala
Lys
65                  70                  75                  80

Ser Asp Leu Ser Thr Gly Asn Met Val His Ala Ser Tyr Leu Leu
Thr

85                  90                  95

Ser Gly Asp Leu Arg Phe Leu Phe Thr Ala Pro Tyr Ser Pro Ser
Leu
            100                 105                 110

Ser Ala Gly Glu Ile Lys Pro Thr Thr Thr Ala Ser Ile Pro Ser
Phe
        115                 120                 125

Asp His Gly Ser Cys Arg Ser Phe Phe Ser Ser His Gly Leu Gly
Val
    130                 135                 140

Arg Ala Val Ala Ile Glu Val Glu Asp Ala Glu Ser Ala Phe Ser
Ile
145                 150                 155
160

Ser Val Ala Asn Gly Ala Ile Pro Ser Ser Pro Pro Ile Val Leu
Asn
                165                 170                 175

Glu Ala Val Thr Ile Ala Glu Val Lys Leu Tyr Gly Asp Val Val
Leu
            180                 185                 190

Arg Tyr Val Ser Tyr Lys Ala Glu Asp Thr Glu Lys Ser Glu Phe
Leu
        195                 200                 205

Pro Gly Phe Glu Arg Val Glu Asp Ala Ser Ser Phe Pro Leu Asp
Tyr
    210                 215                 220

Gly Ile Arg Arg Leu Asp His Ala Val Gly Asn Val Pro Glu Leu
Gly
225                 230                 235
240

Pro Ala Leu Thr Tyr Val Ala Gly Phe Thr Gly Phe His Gln Phe
Ala

245 250 255

Glu Phe Thr Ala Asp Asp Val Gly Thr Ala Glu Ser Gly Leu Asn
Ser
260 265 270

Ala Ala Leu Ala Ser Asn Asp Glu Met Val Leu Leu Pro Ile Asn
Glu
275 280 285

Pro Val His Gly Thr Lys Arg Lys Ser Gln Ile Gln Thr Tyr Leu
Glu
290 295 300

His Asn Glu Gly Ala Gly Leu Gln His Leu Ala Leu Met Ser Glu
Asp
305 310 315
320

Ile Phe Arg Thr Leu Arg Glu Met Arg Lys Arg Ser Ser Ile Gly
Gly
325 330 335

Phe Asp Phe Met Pro Ser Pro Pro Pro Thr Tyr Tyr Gln Asn Leu
Lys
340 345 350

Lys Arg Val Gly Asp Val Leu Ser Asp Asp Gln Ile Lys Glu Cys
Glu
355 360 365

Glu Leu Gly Ile Leu Val Asp Arg Asp Asp Gln Gly Thr Leu Leu
Gln
370 375 380

Ile Phe Thr Lys Pro Leu Gly Asp Arg Pro Thr Ile Phe Ile Glu
Ile
385 390 395
400

Ile Gln Arg Val Gly Cys Met Met Lys Asp Glu Glu Gly Lys Ala
Tyr

405                 410                 415

Gln Ser Gly Gly Cys Gly Gly Phe Gly Lys Gly Asn Phe Ser Glu
Leu
            420                 425                 430

Phe Lys Ser Ile Glu Glu Tyr Glu Lys Thr Leu Glu Ala Lys Gln
Leu
        435                 440                 445

Val Gly
    450

<210>  41
<211>  1353
<212>  DNA
<213>  Artificial sequence

<220>
<223>  nucleotide sequebc encoding mutant HPPD polypeptide

<220>
<221>  misc_feature
<222>  (4)..(6)
<223>  sequence coding for Ala

<220>
<221>  misc_feature
<222>  (7)..(24)
<223>  sequence coding for an His Tag containing 6 His

<400>  41
atggctcatc accatcacca tcaccaaaac gccgccgttt cagagaatca
aaaccatgat      60

gacggcgctg cgtcgtcgcc gggattcaag ctcgtcggat tttccaagtt
cgtaagaaag     120

aatccaaagt ctgataaatt caaggttaag cgcttccatc acatcgagtt
ctggtgcggc     180

gacgcaacca acgtcgctcg tcgcttctcc tggggtctgg ggatgagatt
ctccgccaaa     240

tccgatcttt ccaccggaaa catggttcac gcctcttacc tactcacctc
cggtgacctc     300

cgattccttt tcactgctcc ttactctccg tctctctccg ccggagagat
taaaccgaca    360

accacagctt ctatcccaag tttcgatcac ggctcttgtc gttccttctt
ctcgtcacat    420

ggtctcggtg ttagagccgt tgcgattgaa gtagaagacg cagagtcagc
tttctccatc    480

agtgtagcta atggcgctat tccttcgtcg cctcctatcg tcctcaatga
agcagttacg    540

atcgctgagg ttaaactata cggcgatgtt gttctccgat atgttagtta
caaagcagaa    600

gataccgaaa aatccgaatt cttgccaggg ttcgagcgtg tagaggatgc
gtcgtcgttc    660

ccattggatt atggtatccg gcggcttgac cacgccgtgg gaaacgttcc
tgagcttggt    720

ccggctttaa cttatgtagc ggggttcact ggttttcacc aattcgcaga
gttcacagca    780

gacgacgttg gaaccgccga gagcggttta aattcagcgg tcctggctag
caatgatgaa    840

atggttcttc tagcgattaa cgagccagtg cacggaacaa agaggaagag
tcagattcag    900

acgtatttgg aacataacga aggcgcaggg ctacaacatc tggctctgat
gagtgaagac    960

atattcagga ccctgagaga gatgaggaag aggagcagta ttggaggatt
cgacttcatg   1020

ccttctcctc cgcctactta ctaccagaat ctcaagaaac gggtcggcga
cgtgctcagc   1080

gatgatcaga tcaaggagtg tgaggaatta gggattcttg tagacagaga
tgatcaaggg   1140

acgttgcttc aaatcttcac aaaaccacta ggtgacaggc cgacgatatt
tatagagata   1200

atccagagag taggatgcat gatgaaagat gaggaaggga aggcttacca
gagtggagga   1260

tgtggtggtt ttggcaaagg caatttctct gagctcttca agtccattga
agaatacgaa   1320

aagactcttg aagccaaaca gttagtggga tga
1353

<210> 42
<211> 450
<212> PRT
<213> Artificial sequence

<220>
<223> encoded by SEQ ID No. 41

<220>
<221> MISC_FEATURE
<222> (2)..(2)
<223> Ala

<220>
<221> MISC_FEATURE
<222> (3)..(8)
<223> His Tag made of 6 His

<400> 42

Met Ala His His His His His His Gln Asn Ala Ala Val Ser Glu
Asn
1               5                   10                  15

Gln Asn His Asp Asp Gly Ala Ala Ser Ser Pro Gly Phe Lys Leu
Val
            20                  25                  30

Gly Phe Ser Lys Phe Val Arg Lys Asn Pro Lys Ser Asp Lys Phe
Lys
        35                  40                  45

Val Lys Arg Phe His His Ile Glu Phe Trp Cys Gly Asp Ala Thr
Asn
    50                  55                  60

Val Ala Arg Arg Phe Ser Trp Gly Leu Gly Met Arg Phe Ser Ala
Lys
65                  70                  75                  80

Ser Asp Leu Ser Thr Gly Asn Met Val His Ala Ser Tyr Leu Leu
Thr

85                  90                  95

Ser Gly Asp Leu Arg Phe Leu Phe Thr Ala Pro Tyr Ser Pro Ser
Leu
            100                 105                 110

Ser Ala Gly Glu Ile Lys Pro Thr Thr Thr Ala Ser Ile Pro Ser
Phe
        115                 120                 125

Asp His Gly Ser Cys Arg Ser Phe Phe Ser Ser His Gly Leu Gly
Val
    130                 135                 140

Arg Ala Val Ala Ile Glu Val Glu Asp Ala Glu Ser Ala Phe Ser
Ile
145                 150                 155
160

Ser Val Ala Asn Gly Ala Ile Pro Ser Ser Pro Pro Ile Val Leu
Asn
                165                 170                 175

Glu Ala Val Thr Ile Ala Glu Val Lys Leu Tyr Gly Asp Val Val
Leu
            180                 185                 190

Arg Tyr Val Ser Tyr Lys Ala Glu Asp Thr Glu Lys Ser Glu Phe
Leu
        195                 200                 205

Pro Gly Phe Glu Arg Val Glu Asp Ala Ser Ser Phe Pro Leu Asp
Tyr
    210                 215                 220

Gly Ile Arg Arg Leu Asp His Ala Val Gly Asn Val Pro Glu Leu
Gly
225                 230                 235
240

Pro Ala Leu Thr Tyr Val Ala Gly Phe Thr Gly Phe His Gln Phe
Ala

245 250 255

Glu Phe Thr Ala Asp Asp Val Gly Thr Ala Glu Ser Gly Leu Asn
Ser
260 265 270

Ala Val Leu Ala Ser Asn Asp Glu Met Val Leu Leu Ala Ile Asn
Glu
275 280 285

Pro Val His Gly Thr Lys Arg Lys Ser Gln Ile Gln Thr Tyr Leu
Glu
290 295 300

His Asn Glu Gly Ala Gly Leu Gln His Leu Ala Leu Met Ser Glu
Asp
305 310 315
320

Ile Phe Arg Thr Leu Arg Glu Met Arg Lys Arg Ser Ser Ile Gly
Gly
325 330 335

Phe Asp Phe Met Pro Ser Pro Pro Pro Thr Tyr Tyr Gln Asn Leu
Lys
340 345 350

Lys Arg Val Gly Asp Val Leu Ser Asp Asp Gln Ile Lys Glu Cys
Glu
355 360 365

Glu Leu Gly Ile Leu Val Asp Arg Asp Asp Gln Gly Thr Leu Leu
Gln
370 375 380

Ile Phe Thr Lys Pro Leu Gly Asp Arg Pro Thr Ile Phe Ile Glu
Ile
385 390 395
400

Ile Gln Arg Val Gly Cys Met Met Lys Asp Glu Glu Gly Lys Ala
Tyr

405                 410                 415

Gln Ser Gly Gly Cys Gly Gly Phe Gly Lys Gly Asn Phe Ser Glu
Leu
            420                 425                 430

Phe Lys Ser Ile Glu Glu Tyr Glu Lys Thr Leu Glu Ala Lys Gln
Leu
        435                 440                 445

Val Gly
    450

<210>  43
<211>  1353
<212>  DNA
<213>  Artificial sequence

<220>
<223>  nucleotide sequence encoding mutant HPPD polypeptide

<220>
<221>  misc_feature
<222>  (4)..(6)
<223>  sequence coding for Ala

<220>
<221>  misc_feature
<222>  (7)..(24)
<223>  sequence coding for an His Tag containing 6 His

<400>  43
atggctcatc accatcacca tcaccaaaac gccgccgttt cagagaatca
aaaccatgat      60

gacggcgctg cgtcgtcgcc gggattcaag ctcgtcggat tttccaagtt
cgtaagaaag     120

aatccaaagt ctgataaatt caaggttaag cgcttccatc acatcgagtt
ctggtgcggc     180

gacgcaacca acgtcgctcg tcgcttctcc tggggtctgg ggatgagatt
ctccgccaaa     240

tccgatcttt ccaccggaaa catggttcac gcctcttacc tactcacctc
cggtgacctc     300

cgattccttt tcactgctcc ttactctccg tctctctccg ccggagagat
taaaccgaca    360

accacagctt ctatcccaag tttcgatcac ggctcttgtc gttccttctt
ctcgtcacat    420

ggtctcggtg ttagagccgt tgcgattgaa gtagaagacg cagagtcagc
tttctccatc    480

agtgtagcta atggcgctat tccttcgtcg cctcctatcg tcctcaatga
agcagttacg    540

atcgctgagg ttaaactata cggcgatgtt gttctccgat atgttagtta
caaagcagaa    600

gataccgaaa aatccgaatt cttgccaggg ttcgagcgtg tagaggatgc
gtcgtcgttc    660

ccattggatt atggtatccg gcggcttgac cacgccgtgg gaaacgttcc
tgagcttggt    720

ccggctttaa cttatgtagc ggggttcact ggttttcacc aattcgcaga
gttcacagca    780

gacgacgttg gaaccgccga gagcggttta aattcagcgg tcctggctag
caatgatgaa    840

atggttcttc taccgattaa cgagccagtg cacggaacaa agaggaagag
tcagattcag    900

acgtatttgg aacataacga aggcgcaggg ctacaacatc tggctctgat
gagtgaagac    960

atattcagga ccctgagaga gatgaggaag aggagcagta ttggaggatt
cgacttcatg   1020

ccttctcctc cgcctactta ctaccagaat ctcaagaaac gggtcggcga
cgtgctcagc   1080

gatgatcaga tcaaggagtg tgaggaatta gggattatgg tagacagaga
tgatcaaggg   1140

acgttgcttc aaatcttcac aaaaccacta ggtgacaggc cgacgatatt
tatagagata   1200

atccagagag taggatgcat gatgaaagat gaggaaggga aggcttacca
gagtggagga   1260

tgtggtggtt ttggcaaagg caatttctct gagctcttca agtccattga
agaatacgaa   1320

aagactcttg aagccaaaca gttagtggga tga
1353

<210> 44
<211> 450
<212> PRT
<213> Artificial sequence

<220>
<223> encoded by SEQ ID No. 43

<220>
<221> MISC_FEATURE
<222> (2)..(2)
<223> Ala

<220>
<221> MISC_FEATURE
<222> (3)..(8)
<223> His Tag made of 6 His

<400> 44

Met Ala His His His His His His Gln Asn Ala Ala Val Ser Glu
Asn
1               5                   10                  15

Gln Asn His Asp Asp Gly Ala Ala Ser Ser Pro Gly Phe Lys Leu
Val
            20                  25                  30

Gly Phe Ser Lys Phe Val Arg Lys Asn Pro Lys Ser Asp Lys Phe
Lys
        35                  40                  45

Val Lys Arg Phe His His Ile Glu Phe Trp Cys Gly Asp Ala Thr
Asn
    50                  55                  60

Val Ala Arg Arg Phe Ser Trp Gly Leu Gly Met Arg Phe Ser Ala
Lys
65                  70                  75                  80

Ser Asp Leu Ser Thr Gly Asn Met Val His Ala Ser Tyr Leu Leu
Thr

85                  90                  95

Ser Gly Asp Leu Arg Phe Leu Phe Thr Ala Pro Tyr Ser Pro Ser
Leu
            100                 105                 110

Ser Ala Gly Glu Ile Lys Pro Thr Thr Thr Ala Ser Ile Pro Ser
Phe
        115                 120                 125

Asp His Gly Ser Cys Arg Ser Phe Phe Ser Ser His Gly Leu Gly
Val
    130                 135                 140

Arg Ala Val Ala Ile Glu Val Glu Asp Ala Glu Ser Ala Phe Ser
Ile
145                 150                 155
160

Ser Val Ala Asn Gly Ala Ile Pro Ser Ser Pro Pro Ile Val Leu
Asn
                165                 170                 175

Glu Ala Val Thr Ile Ala Glu Val Lys Leu Tyr Gly Asp Val Val
Leu
            180                 185                 190

Arg Tyr Val Ser Tyr Lys Ala Glu Asp Thr Glu Lys Ser Glu Phe
Leu
        195                 200                 205

Pro Gly Phe Glu Arg Val Glu Asp Ala Ser Ser Phe Pro Leu Asp
Tyr
    210                 215                 220

Gly Ile Arg Arg Leu Asp His Ala Val Gly Asn Val Pro Glu Leu
Gly
225                 230                 235
240

Pro Ala Leu Thr Tyr Val Ala Gly Phe Thr Gly Phe His Gln Phe
Ala

                    245                     250                     255

Glu Phe Thr Ala Asp Asp Val Gly Thr Ala Glu Ser Gly Leu Asn
Ser
                260                     265                     270

Ala Val Leu Ala Ser Asn Asp Glu Met Val Leu Leu Pro Ile Asn
Glu
            275                     280                     285

Pro Val His Gly Thr Lys Arg Lys Ser Gln Ile Gln Thr Tyr Leu
Glu
    290                     295                     300

His Asn Glu Gly Ala Gly Leu Gln His Leu Ala Leu Met Ser Glu
Asp
305                     310                     315
320

Ile Phe Arg Thr Leu Arg Glu Met Arg Lys Arg Ser Ser Ile Gly
Gly
                    325                     330                     335

Phe Asp Phe Met Pro Ser Pro Pro Pro Thr Tyr Tyr Gln Asn Leu
Lys
                340                     345                     350

Lys Arg Val Gly Asp Val Leu Ser Asp Asp Gln Ile Lys Glu Cys
Glu
            355                     360                     365

Glu Leu Gly Ile Met Val Asp Arg Asp Asp Gln Gly Thr Leu Leu
Gln
    370                     375                     380

Ile Phe Thr Lys Pro Leu Gly Asp Arg Pro Thr Ile Phe Ile Glu
Ile
385                     390                     395
400

Ile Gln Arg Val Gly Cys Met Met Lys Asp Glu Glu Gly Lys Ala
Tyr

405                 410                 415

Gln Ser Gly Gly Cys Gly Gly Phe Gly Lys Gly Asn Phe Ser Glu
Leu
            420                 425                 430

Phe Lys Ser Ile Glu Glu Tyr Glu Lys Thr Leu Glu Ala Lys Gln
Leu
        435                 440                 445

Val Gly
    450

<210>  45
<211>  1353
<212>  DNA
<213>  Artificial sequence

<220>
<223>  nucleotide sequence encoding mutant HPPD polypeptide

<220>
<221>  misc_feature
<222>  (4)..(6)
<223>  sequence coding for Ala

<220>
<221>  misc_feature
<222>  (7)..(24)
<223>  sequence coding for an His Tag containing 6 His

<400>  45
atggctcatc accatcacca tcaccaaaac gccgccgttt cagagaatca
aaaccatgat      60

gacggcgctg cgtcgtcgcc gggattcaag ctcgtcggat tttccaagtt
cgtaagaaag     120

aatccaaagt ctgataaatt caaggttaag cgcttccatc acatcgagtt
ctggtgcggc     180

gacgcaacca acgtcgctcg tcgcttctcc tggggtctgg ggatgagatt
ctccgccaaa     240

tccgatcttt ccaccggaaa catggttcac gcctcttacc tactcacctc
cggtgacctc     300

cgattccttt tcactgctcc ttactctccg tctctctccg ccggagagat
taaaccgaca    360

accacagctt ctatcccaag tttcgatcac ggctcttgtc gttccttctt
ctcgtcacat    420

ggtctcggtg ttagagccgt tgcgattgaa gtagaagacg cagagtcagc
tttctccatc    480

agtgtagcta atggcgctat tccttcgtcg cctcctatcg tcctcaatga
agcagttacg    540

atcgctgagg ttaaactata cggcgatgtt gttctccgat atgttagtta
caaagcagaa    600

gataccgaaa aatccgaatt cttgccaggg ttcgagcgtg tagaggatgc
gtcgtcgttc    660

ccattggatt atggtatccg gcggcttgac cacgccgtgg gaaacgttcc
tgagcttggt    720

ccggctttaa cttatgtagc ggggttcact ggttttcacc aattcgcaga
gttcacagca    780

gacgacgttg gaaccgccga gagcggttta aattcagcgg tcctggctag
caatgatgaa    840

atggttcttc taccgattaa cgagccagtg cacggaacaa agaggaagag
tcagattcag    900

acgtatttgg aacataacga aggcgcaggg ctacaacatc tggctctgat
gagtgaagac    960

atattcagga ccctgagaga gatgaggaag aggagcagta ttggaggatt
cgacttcatg   1020

ccttctcctc cgcctactta ctaccagaat ctcaagaaac gggtcggcga
cgtgctcagc   1080

gatgatcaga tcaaggagtg tgaggaatta gggattcttg tagacagaga
tgatcaaggg   1140

acgttgcttc aaatcttcac aaaaccacta ggtgacaggc cgacgatatt
tatagagata   1200

atccagagag taggatgcat gatgaaagat gaggaaggga aggcttacca
gagtggagga   1260

tgtggtggtt ttggcattgg caatttctct gagctcttca agtccattga
agaatacgaa   1320

aagactcttg aagccaaaca gttagtggga tga
1353

<210> 46
<211> 450
<212> PRT
<213> Artificial sequence

<220>
<223> encoded by SEQ ID No. 45

<220>
<221> MISC_FEATURE
<222> (2)..(2)
<223> Ala

<220>
<221> MISC_FEATURE
<222> (3)..(8)
<223> His Tag made of 6 His

<400> 46

Met Ala His His His His His His Gln Asn Ala Ala Val Ser Glu
Asn
1               5                   10                  15

Gln Asn His Asp Asp Gly Ala Ala Ser Ser Pro Gly Phe Lys Leu
Val
            20                  25                  30

Gly Phe Ser Lys Phe Val Arg Lys Asn Pro Lys Ser Asp Lys Phe
Lys
        35                  40                  45

Val Lys Arg Phe His His Ile Glu Phe Trp Cys Gly Asp Ala Thr
Asn
    50                  55                  60

Val Ala Arg Arg Phe Ser Trp Gly Leu Gly Met Arg Phe Ser Ala
Lys
65                  70                  75                  80

Ser Asp Leu Ser Thr Gly Asn Met Val His Ala Ser Tyr Leu Leu
Thr

85                  90                  95

Ser Gly Asp Leu Arg Phe Leu Phe Thr Ala Pro Tyr Ser Pro Ser
Leu
            100                 105                 110

Ser Ala Gly Glu Ile Lys Pro Thr Thr Thr Ala Ser Ile Pro Ser
Phe
        115                 120                 125

Asp His Gly Ser Cys Arg Ser Phe Phe Ser Ser His Gly Leu Gly
Val
    130                 135                 140

Arg Ala Val Ala Ile Glu Val Glu Asp Ala Glu Ser Ala Phe Ser
Ile
145                 150                 155
160

Ser Val Ala Asn Gly Ala Ile Pro Ser Ser Pro Pro Ile Val Leu
Asn
                165                 170                 175

Glu Ala Val Thr Ile Ala Glu Val Lys Leu Tyr Gly Asp Val Val
Leu
            180                 185                 190

Arg Tyr Val Ser Tyr Lys Ala Glu Asp Thr Glu Lys Ser Glu Phe
Leu
        195                 200                 205

Pro Gly Phe Glu Arg Val Glu Asp Ala Ser Ser Phe Pro Leu Asp
Tyr
    210                 215                 220

Gly Ile Arg Arg Leu Asp His Ala Val Gly Asn Val Pro Glu Leu
Gly
225                 230                 235
240

Pro Ala Leu Thr Tyr Val Ala Gly Phe Thr Gly Phe His Gln Phe
Ala

                    245                     250                     255

Glu Phe Thr Ala Asp Asp Val Gly Thr Ala Glu Ser Gly Leu Asn
Ser
                260                     265                     270

Ala Val Leu Ala Ser Asn Asp Glu Met Val Leu Leu Pro Ile Asn
Glu
            275                     280                     285

Pro Val His Gly Thr Lys Arg Lys Ser Gln Ile Gln Thr Tyr Leu
Glu
    290                     295                     300

His Asn Glu Gly Ala Gly Leu Gln His Leu Ala Leu Met Ser Glu
Asp
305                     310                     315
320

Ile Phe Arg Thr Leu Arg Glu Met Arg Lys Arg Ser Ser Ile Gly
Gly
                    325                     330                     335

Phe Asp Phe Met Pro Ser Pro Pro Pro Thr Tyr Tyr Gln Asn Leu
Lys
                340                     345                     350

Lys Arg Val Gly Asp Val Leu Ser Asp Asp Gln Ile Lys Glu Cys
Glu
            355                     360                     365

Glu Leu Gly Ile Leu Val Asp Arg Asp Asp Gln Gly Thr Leu Leu
Gln
    370                     375                     380

Ile Phe Thr Lys Pro Leu Gly Asp Arg Pro Thr Ile Phe Ile Glu
Ile
385                     390                     395
400

Ile Gln Arg Val Gly Cys Met Met Lys Asp Glu Glu Gly Lys Ala
Tyr

405                 410                 415

Gln Ser Gly Gly Cys Gly Gly Phe Gly Ile Gly Asn Phe Ser Glu
Leu
            420                 425                 430

Phe Lys Ser Ile Glu Glu Tyr Glu Lys Thr Leu Glu Ala Lys Gln
Leu
        435                 440                 445

Val Gly
    450

<210>  47
<211>  1353
<212>  DNA
<213>  Artificial sequence

<220>
<223>  nucleotide sequence encoding mutant HPPD polypeptide

<220>
<221>  misc_feature
<222>  (4)..(6)
<223>  sequence coding for Ala

<220>
<221>  misc_feature
<222>  (7)..(24)
<223>  sequence coding for an His Tag containing 6 His

<400>  47
atggctcatc accatcacca tcaccaaaac gccgccgttt cagagaatca
aaaccatgat      60

gacggcgctg cgtcgtcgcc gggattcaag ctcgtcggat tttccaagtt
cgtaagaaag     120

aatccaaagt ctgataaatt caaggttaag cgcttccatc acatcgagtt
ctggtgcggc     180

gacgcaacca acgtcgctcg tcgcttctcc tggggtctgg ggatgagatt
ctccgccaaa     240

tccgatcttt ccaccggaaa catggttcac gcctcttacc tactcacctc
cggtgacctc     300

cgattccttt tcactgctcc ttactctccg tctctctccg ccggagagat
taaaccgaca    360

accacagctt ctatcccaag tttcgatcac ggctcttgtc gttccttctt
ctcgtcacat    420

ggtctcggtg ttagagccgt tgcgattgaa gtagaagacg cagagtcagc
tttctccatc    480

agtgtagcta atggcgctat tccttcgtcg cctcctatcg tcctcaatga
agcagttacg    540

atcgctgagg ttaaactata cggcgatgtt gttctccgat atgttagtta
caaagcagaa    600

gataccgaaa aatccgaatt cttgccaggg ttcgagcgtg tagaggatgc
gtcgtcgttc    660

ccattggatt atggtatccg gcggcttgac cacgccgtgg gaaacgttcc
tgagcttggt    720

ccggctttaa cttatgtagc ggggttcact ggttttcacc aattcgcaga
gttcacagca    780

gacgacgttg gaaccgccga gagcggttta aattcagcgg tcctggctag
caatgatgaa    840

atggttcttc taccgattaa cgagccagtg cacggaacaa agaggaagag
tcagattcag    900

acgtatttgg aacataacga aggcgcaggg ctacaacatc tggctctgat
gagtgaagac    960

atattcagga ccctgagaga gatgaggaag aggagcagta ttggaggatt
cgacttcatg   1020

ccttctcctc cgcctactta ctaccagaat ctcaagaaac gggtcggcga
cgtgctcagc   1080

gatgatcaga tcaaggagtg tgaggaatta gggattcttg tagacagaga
tgatcaaggg   1140

acgttgcttc aaatcttcac aaaaccacta ggtgacaggc cgacgatatt
tatagagata   1200

atccagagag taggatgcat gatgaaagat gaggaaggga aggcttacca
gagtggagga   1260

tgtggtggtt ttggccaggg caatttctct gagctcttca agtccattga
agaatacgaa   1320

aagactcttg aagccaaaca gttagtggga tga
1353

<210> 48
<211> 450
<212> PRT
<213> Artificial sequence

<220>
<223> encoded by SEQ ID No. 47

<220>
<221> MISC_FEATURE
<222> (2)..(2)
<223> Ala

<220>
<221> MISC_FEATURE
<222> (3)..(8)
<223> His Tag made of 6 His

<400> 48

Met Ala His His His His His His Gln Asn Ala Ala Val Ser Glu
Asn
1               5                   10                  15

Gln Asn His Asp Asp Gly Ala Ala Ser Ser Pro Gly Phe Lys Leu
Val
            20                  25                  30

Gly Phe Ser Lys Phe Val Arg Lys Asn Pro Lys Ser Asp Lys Phe
Lys
        35                  40                  45

Val Lys Arg Phe His His Ile Glu Phe Trp Cys Gly Asp Ala Thr
Asn
    50                  55                  60

Val Ala Arg Arg Phe Ser Trp Gly Leu Gly Met Arg Phe Ser Ala
Lys
65                  70                  75                  80

Ser Asp Leu Ser Thr Gly Asn Met Val His Ala Ser Tyr Leu Leu
Thr

85                  90                  95

Ser Gly Asp Leu Arg Phe Leu Phe Thr Ala Pro Tyr Ser Pro Ser
Leu
            100                 105                 110

Ser Ala Gly Glu Ile Lys Pro Thr Thr Thr Ala Ser Ile Pro Ser
Phe
        115                 120                 125

Asp His Gly Ser Cys Arg Ser Phe Phe Ser Ser His Gly Leu Gly
Val
    130                 135                 140

Arg Ala Val Ala Ile Glu Val Glu Asp Ala Glu Ser Ala Phe Ser
Ile
145                 150                 155
160

Ser Val Ala Asn Gly Ala Ile Pro Ser Ser Pro Pro Ile Val Leu
Asn
                165                 170                 175

Glu Ala Val Thr Ile Ala Glu Val Lys Leu Tyr Gly Asp Val Val
Leu
            180                 185                 190

Arg Tyr Val Ser Tyr Lys Ala Glu Asp Thr Glu Lys Ser Glu Phe
Leu
        195                 200                 205

Pro Gly Phe Glu Arg Val Glu Asp Ala Ser Ser Phe Pro Leu Asp
Tyr
    210                 215                 220

Gly Ile Arg Arg Leu Asp His Ala Val Gly Asn Val Pro Glu Leu
Gly
225                 230                 235
240

Pro Ala Leu Thr Tyr Val Ala Gly Phe Thr Gly Phe His Gln Phe
Ala

                    245                     250                     255

Glu Phe Thr Ala Asp Asp Val Gly Thr Ala Glu Ser Gly Leu Asn
Ser
                260                     265                     270

Ala Val Leu Ala Ser Asn Asp Glu Met Val Leu Leu Pro Ile Asn
Glu
            275                     280                     285

Pro Val His Gly Thr Lys Arg Lys Ser Gln Ile Gln Thr Tyr Leu
Glu
    290                     295                     300

His Asn Glu Gly Ala Gly Leu Gln His Leu Ala Leu Met Ser Glu
Asp
305                     310                     315
320

Ile Phe Arg Thr Leu Arg Glu Met Arg Lys Arg Ser Ser Ile Gly
Gly
                    325                     330                     335

Phe Asp Phe Met Pro Ser Pro Pro Pro Thr Tyr Tyr Gln Asn Leu
Lys
                340                     345                     350

Lys Arg Val Gly Asp Val Leu Ser Asp Asp Gln Ile Lys Glu Cys
Glu
            355                     360                     365

Glu Leu Gly Ile Leu Val Asp Arg Asp Asp Gln Gly Thr Leu Leu
Gln
    370                     375                     380

Ile Phe Thr Lys Pro Leu Gly Asp Arg Pro Thr Ile Phe Ile Glu
Ile
385                     390                     395
400

Ile Gln Arg Val Gly Cys Met Met Lys Asp Glu Glu Gly Lys Ala
Tyr

405                 410                 415

Gln Ser Gly Gly Cys Gly Gly Phe Gly Gln Gly Asn Phe Ser Glu
Leu
            420                 425                 430

Phe Lys Ser Ile Glu Glu Tyr Glu Lys Thr Leu Glu Ala Lys Gln
Leu
        435                 440                 445

Val Gly
    450

<210>  49
<211>  1353
<212>  DNA
<213>  Artificial sequence

<220>
<223>  nucleotide sequence encoding mutant HPPD polypeptide

<220>
<221>  misc_feature
<222>  (4)..(6)
<223>  sequence coding for Ala

<220>
<221>  misc_feature
<222>  (7)..(24)
<223>  sequence coding for an His Tag containing 6 His

<400>  49
atggctcatc accatcacca tcaccaaaac gccgccgttt cagagaatca
aaaccatgat      60

gacggcgctg cgtcgtcgcc gggattcaag ctcgtcggat tttccaagtt
cgtaagaaag     120

aatccaaagt ctgataaatt caaggttaag cgcttccatc acatcgagtt
ctggtgcggc     180

gacgcaacca acgtcgctcg tcgcttctcc tggggtctgg ggatgagatt
ctccgccaaa     240

tccgatcttt ccaccggaaa catggttcac gcctcttacc tactcacctc
cggtgacctc     300

cgattccttt tcactgctcc ttactctccg tctctctccg ccggagagat
taaaccgaca    360

accacagctt ctatcccaag tttcgatcac ggctcttgtc gttccttctt
ctcgtcacat    420

ggtctcggtg ttagagccgt tgcgattgaa gtagaagacg cagagtcagc
tttctccatc    480

agtgtagcta atggcgctat tccttcgtcg cctcctatcg tcctcaatga
agcagttacg    540

atcgctgagg ttaaactata cggcgatgtt gttctccgat atgttagtta
caaagcagaa    600

gataccgaaa aatccgaatt cttgccaggg ttcgagcgtg tagaggatgc
gtcgtcgttc    660

ccattggatt atggtatccg gcggcttgac cacgccgtgg gaaacgttcc
tgagcttggt    720

ccggctttaa cttatgtagc ggggttcact ggttttcacc aattcgcaga
gttcacagca    780

gacgacgttg gaaccgccga gagcggttta aattcagcgg tcctggctag
caatgatgaa    840

atggttcttc taccgattaa cgagccagtg cacggaacaa agaggaagag
tcagattcag    900

acgtatttgg aacataacga aggcgcaggg ctacaacatc tggctctgat
gagtgaagac    960

atattcagga ccctgagaga gatgaggaag aggagcagta ttggaggatt
cgacttcatg   1020

ccttctcctc cgcctactta ctaccagaat ctcaagaaac gggtcggcga
cgtgctcagc   1080

gatgatcaga tcaaggagtg tgaggaatta gggattcttg tagacagaga
tgatcaaggg   1140

acgttgcttc aaatcttcac aaaaccacta ggtgacaggc cgacgatatt
tatagagata   1200

atccagagag taggatgcat gatgaaagat gaggaaggga aggcttacca
gagtggagga   1260

tgtggtggtt ttggcgtggg caatttctct gagctcttca agtccattga
agaatacgaa   1320

aagactcttg aagccaaaca gttagtggga tga
1353

<210> 50
<211> 450
<212> PRT
<213> Artificial sequence

<220>
<223> encoded by SEQ ID No. 49

<220>
<221> MISC_FEATURE
<222> (2)..(2)
<223> Ala

<220>
<221> MISC_FEATURE
<222> (3)..(8)
<223> His Tag made of 6 His

<400> 50

Met Ala His His His His His His Gln Asn Ala Ala Val Ser Glu
Asn
1               5                   10                  15

Gln Asn His Asp Asp Gly Ala Ala Ser Ser Pro Gly Phe Lys Leu
Val
            20                  25                  30

Gly Phe Ser Lys Phe Val Arg Lys Asn Pro Lys Ser Asp Lys Phe
Lys
        35                  40                  45

Val Lys Arg Phe His His Ile Glu Phe Trp Cys Gly Asp Ala Thr
Asn
    50                  55                  60

Val Ala Arg Arg Phe Ser Trp Gly Leu Gly Met Arg Phe Ser Ala
Lys
65                  70                  75                  80

Ser Asp Leu Ser Thr Gly Asn Met Val His Ala Ser Tyr Leu Leu
Thr

85                  90                  95

Ser Gly Asp Leu Arg Phe Leu Phe Thr Ala Pro Tyr Ser Pro Ser
Leu
            100                 105                 110

Ser Ala Gly Glu Ile Lys Pro Thr Thr Thr Ala Ser Ile Pro Ser
Phe
        115                 120                 125

Asp His Gly Ser Cys Arg Ser Phe Phe Ser Ser His Gly Leu Gly
Val
    130                 135                 140

Arg Ala Val Ala Ile Glu Val Glu Asp Ala Glu Ser Ala Phe Ser
Ile
145                 150                 155
160

Ser Val Ala Asn Gly Ala Ile Pro Ser Ser Pro Pro Ile Val Leu
Asn
                165                 170                 175

Glu Ala Val Thr Ile Ala Glu Val Lys Leu Tyr Gly Asp Val Val
Leu
            180                 185                 190

Arg Tyr Val Ser Tyr Lys Ala Glu Asp Thr Glu Lys Ser Glu Phe
Leu
        195                 200                 205

Pro Gly Phe Glu Arg Val Glu Asp Ala Ser Ser Phe Pro Leu Asp
Tyr
    210                 215                 220

Gly Ile Arg Arg Leu Asp His Ala Val Gly Asn Val Pro Glu Leu
Gly
225                 230                 235
240

Pro Ala Leu Thr Tyr Val Ala Gly Phe Thr Gly Phe His Gln Phe
Ala

                    245                     250                     255

Glu Phe Thr Ala Asp Asp Val Gly Thr Ala Glu Ser Gly Leu Asn
Ser
                260                     265                     270

Ala Val Leu Ala Ser Asn Asp Glu Met Val Leu Leu Pro Ile Asn
Glu
            275                     280                     285

Pro Val His Gly Thr Lys Arg Lys Ser Gln Ile Gln Thr Tyr Leu
Glu
    290                     295                     300

His Asn Glu Gly Ala Gly Leu Gln His Leu Ala Leu Met Ser Glu
Asp
305                     310                     315
320

Ile Phe Arg Thr Leu Arg Glu Met Arg Lys Arg Ser Ser Ile Gly
Gly
                    325                     330                     335

Phe Asp Phe Met Pro Ser Pro Pro Pro Thr Tyr Tyr Gln Asn Leu
Lys
                340                     345                     350

Lys Arg Val Gly Asp Val Leu Ser Asp Asp Gln Ile Lys Glu Cys
Glu
            355                     360                     365

Glu Leu Gly Ile Leu Val Asp Arg Asp Asp Gln Gly Thr Leu Leu
Gln
    370                     375                     380

Ile Phe Thr Lys Pro Leu Gly Asp Arg Pro Thr Ile Phe Ile Glu
Ile
385                     390                     395
400

Ile Gln Arg Val Gly Cys Met Met Lys Asp Glu Glu Gly Lys Ala
Tyr

405                 410                 415

Gln Ser Gly Gly Cys Gly Gly Phe Gly Val Gly Asn Phe Ser Glu
Leu
            420                 425                 430

Phe Lys Ser Ile Glu Glu Tyr Glu Lys Thr Leu Glu Ala Lys Gln
Leu
        435                 440                 445

Val Gly
    450

1

## Description

The present invention relates to an isolated nucleic acid comprising a nucleotide sequence encoding a mutated HPPD protein, wherein said mutated HPPD protein has HPPD activity, wherein in said mutated HPPD protein at least one amino acid has been replaced so that the resulting amino acid sequence comprises at least one amino acid selected from certain amino acids at specific positions important for conferring an increased HPPD inhibitor tolerance. The present invention also relates to proteins encoded by the nucleic acid of the invention, to chimeric genes, plant cells comprising the nucleic acid of the invention operably linked to a plant-expressible promoter and optionally a transcription termination and polyadenylation region, plants essentially consisting of the plant cells of the invention and methods of obtaining transgenic plants.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

HPPD (hydroxyphenylpyruvate dioxygenase) proteins are enzymes which catalyse the reaction in which para-hydroxyphenylpyruvate (abbreviated herein as HPP), a tyrosine degradation product, is transformed into homogentisate (abbreviated herein as HG), the precursor in plants of tocopherol and plastoquinone (Crouch N.P. et al. (1997) Tetrahedron, 53, 20, 6993-7010, Fritze et al., (2004), Plant Physiology 134:1388-1400). Tocopherol acts as a membrane-associated antioxidant. Plastoquinone, firstly acts as an electron carrier between PSII and the cytochrome b6/f complex and secondly, is a redox cofactor for phytoene desaturase, which is involved in the biosynthesis of carotenoids.

Up to now, more than 700 nucleic acid sequences from various organisms present in NCBI database were annotated as coding for a putative protein having an HPPD domain. But for most of these sequences, it has not been proven that the protein would have an HPPD enzymatic activity either in an in vitro assay or an in in planta approach, nor that such HPPD protein can confer herbicide tolerance to HPPD inhibitor herbicides when expressed in a plant. Several HPPD proteins and their primary sequences have been described in the state of the art, in particular the HPPDs of bacteria such as Pseudomonas (Rüetschi et al., Eur. J. Biochem., 205, 459-466, 1992, WO 96/38567), of plants such as Arabidopsis (WO 96/38567, Genebank AF047834), carrot (WO 96/38567, Genebank 87257), Avena sativa (WO 02/046387), wheat (WO 02/046387), Brachiaria platyphylla (WO 02/046387), Cenchrus echinatus (WO 02/046387), Lolium rigidum (WO 02/046387), Festuca arundinacea (WO 02/046387), Setaria faberi (WO 02/046387), Eleusine indica (WO 02/046387), Sorghum (WO 02/046387), Coccicoides (Genebank COITRP),of Coptis japonica (WO 06/132270), Chlamydomonas reinhardtii (ES 2275365), or of mammals such as mouse or pig.

Most plants synthesize tyrosine via arrogenate (Abou-Zeid et al. (1995), Applied Env Microb 41: 1298-1302; Bonner et al., (1995), Plant Cells Physiol. 36, 1013-1022; Byng et al., (1981), Phytochemistry 6: 1289-1292; Connely and Conn (1986), Z. Naturforsch 41 c: 69-78; Gaines et al., (1982), Plants 156: 233-240). In these plants, the HPP is derived only from the degradation of tyrosine. On the other hand, in organisms such as the yeast Sacharomyces cerevisiae or the bacterium Escherichia coli, HPP is a tyrosine precursor, and it is synthesized by the action of an enzyme, prephenate dehydrogenase (hereinafter referred to as PDH), which converts prephenate to HPP (Lingens et al., (1967) European J. Biochem 1: 363-374; Sampathkumar and Morrisson (1982), Bioch Biophys Acta 701: 204-211). In these organisms, the production of HPP is therefore directly connected to the aromatic amino acid biosynthetic pathway (shikimate pathway), and not to the tyrosine degradation pathway.

Inhibition of HPPD leads to uncoupling of photosynthesis, deficiency in accessory light-harvesting pigments and, most importantly, to destruction of chlorophyll by UV-radiation and reactive oxygen species (bleaching) due to the lack of photo protection normally provided by carotenoids (Norris et al. (1995), Plant Cell 7: 2139-2149). Bleaching of photosynthetically active tissues leads to growth inhibition and plant death.

At present, most commercially available HPPD inhibitor herbicides belong to one of these four chemical families:
1) the triketones, e.g. sulcotrione [i.e. 2-[2-chloro-4-(methylsulfonyl)benzoyl]-1,3-cyclohexanedione], mesotrione [i.e.2-[4-(methylsulfonyl)-2-nitrobenzoyl]-1,3-cyclohexanedione]; tembotrione [i.e.2-[2-chloro-4-(methylsulfonyl)-3-[(2,2,2,-tri-fluoroethoxy)methyl] benzoyl]-1,3-cyclo-hexanedione]; tefuryltrione [i.e. 2-[2-chloro-4-(methylsulfonyl)-3-[[(tetrahydro-2-furanyl)methoxy]methyl]benzoyl]-1,3 cyclohexanedione]]; bicyclopyrone [i.e. 4-hydroxy-3-[[2-[(2-methoxyethoxy)methyl]-6-(trifluoromethyl)-3-pyridinyl]carbonyl]bicyclo[3.2.1]oct-3-en-2-one] ; Benzobicyclon [i.e. 3-(2-chloro-4-mesylbenzoyl)-2-phenylthiobicyclo[3.2.1]oct-2-en-4-one]
2) The diketonitriles, e.g. 2-cyano-3-cyclopropyl-1-(2-methylsulphonyl-4-trifluoromethylphenyl)-propane-1,3-dione and 2-cyano-1-[4-(methylsulphonyl)-2-trifluoromethylphenyl]-3-(1-methylcyclopropyl)propane-1,3-dione;
3) the isoxazoles, e.g. isoxaflutole [i.e.(5-cyclopropyl-4-isoxazolyl)[2-(methylsulfonyl)-4-(trifluoromethyl)phenyl]methanone]. In plants, the isoxaflutole is rapidly metabolized in DKN, a diketonitrile compound which exhibits the HPPD inhibitor property; and
4) the pyrazolinates, e.g. topramezone [i.e.[3-(4,5-dihydro-3-isoxazolyl)-2-methyl-4-(methylsulfonyl) phenyl](5-hydroxy-1-methyl-1H-pyrazol-4-yl)methanone], and pyrasulfotole [(5-hydroxy-1,3-dimethylpyrazol-4-yl(2-mesyl-4-trifluaromethylphenyl)methanone]; pyrazofen [2-[4-(2,4-dichlorobenzoyl)-1,3-dimethylpyrazol-5-yloxy]acetophenone].

These HPPD-inhibiting herbicides can be used against grass and/or broad leaf weeds in crop plants that display metabolic tolerance, such as maize (Zea mays) in which they are rapidly degraded (Schulz et al., (1993). FEBS letters, 318, 162-166; Mitchell et al., (2001) Pest Management Science, Vol 57, 120-128; Garcia et al., (2000) Biochem., 39, 7501-7507; Pallett et al., (2001) Pest Management Science, Vol 57, 133-142). In order to extend the scope of these HPPD-inhibiting herbicides, several efforts have been developed in order to confer to plants, particularly plants without or with an underperforming metabolic tolerance, a tolerance level acceptable under agronomic field conditions.

Besides the attempt of by-passing HPPD-mediated production of homogentisate (US 6,812,010), overexpressing the sensitive enzyme so as to produce quantities of the target enzyme in the plant which are sufficient in relation to the herbicide has been performed (WO96/38567). Overexpression of HPPD resulted in better pre-emergence tolerance to the diketonitrile derivative (DKN) of isoxaflutole (IFT), but tolerance was not sufficient for tolerance to post-emergence treatment (Matringe et al., (2005), Pest Management Science 61: 269-276).

In WO 04/024928, the inventors have sought to increase the prenylquinone biosynthesis (e.g., synthesis of plastoquinones, tocopherols) in the cells of plants by increasing the flux of the HPP precursor into the cells of these plants. This has been done by connecting the synthesis of said precursor to the "shikimate" pathway by overexpression of a PDH enzyme. They have also noted that the transformation of plants with a gene encoding a PDH enzyme makes it possible to increase the tolerance of said plants to HPPD inhibitors.

Another strategy was to mutate the HPPD in order to obtain a target enzyme which, while retaining its properties of catalysing the transformation of HPP into homogentisate, is less sensitive to HPPD inhibitors than is the native HPPD before mutation.

This strategy has been successfully applied for the production of plants tolerant to 2-cyano-3-cyclopropyl-1-(2-methylsulphonyl-4-trifluoromethylphenyl)-propane-1,3-dione and to 2-cyano-1-[4-(methylsulphonyl)-2-trifluoromethylphenyl]-3-(1-methylcyclopropyl)propane-1,3-dione (EP496630), two HPPD-inhibiting herbicides belonging to the diketonitriles family (WO 99/24585). Pro215Leu, Gly336Glu, Gly336Ile, and more particularly Gly336Trp (positions of the mutated amino acid are indicated with reference to the Pseudomonas HPPD) were identified as mutations which are responsible for an increased tolerance to pre-emergence treatment with these diketonitrile herbicides without causing an alteration of the activity of the enzyme.

More recently, introduction of a Pseudomonas HPPD gene into the plastid genome of tobacco and soybean has shown to be more effective than nuclear transformation, conferring even tolerance to post-emergence application of isoxaflutole (Dufourmantel et al., 2007, Plant Biotechnol J.5(1):118-33).

In the patent application WO 2009/144079, a nucleic acid sequence encoding a mutated hydroxyphenylpyruvate dioxygenase (HPPD) at position 336 of the Pseudomonas fluorescens HPPD protein and its use for obtaining plants which are tolerant to HPPD inhibitor herbicides is disclosed.

In WO 2002/046387, several domains of HPPD proteins originated from plants have been identified that may be relevant to confer tolerance to various HPPD inhibitor herbicides but no in planta nor biochemical data have been shown to confirm the impact of the as described domain functions.

In WO 2008/150473, the combination of two distinct tolerance mechanisms - a modified Avena sativa gene coding for a mutant HPPD enzyme and a CYP450

Maize monooxygenase (nsf1 gene) - was exemplified in order to obtain an improved tolerance to HPPD inhibitor herbicides, but no data have been disclosed demonstrating the synergistic effects based on the combination of both proteins.

US 2010/0197503 suggests a number of mutations at different positions within or close to the active site of the HPPD taken from Avena sativa and examined some of them for their inhibition by certain HPPD inhibitors such as sulcotrione in vitro and in planta.

Despite these successes obtained for the development of plants showing tolerance to several HPPD inhibitors herbicides described above, it is still necessary to develop and/or improve the tolerance of plants to newer or to several different HPPD inhibitors, particularly HPPD inhibitors belonging to the classes of the triketones (e.g.sulcotrione, mesotrione, tembotrione, benzobicyclon and bicyclopyrone) and the pyrazolinates (e.g., topramezone and pyrasulfotole).

Accordingly, the present invention relates to an isolated nucleic acid comprising a nucleotide sequence encoding a mutated HPPD protein, wherein said mutated HPPD protein has HPPD activity,
wherein in said mutated HPPD protein at least one amino acid has been replaced so that the resulting amino acid sequence comprises at least one amino acid selected from:
a) Ala, Asp, Glu, Phe, Gly, Lys, Gln, Arg, Ser, Thr, Val, Tyr, Ile, Leu or Met at a position in an HPPD protein, said position corresponding to position 250 of the amino acid sequence of SEQ ID No. 2;
b) Ala, Asp, Glu, Ile, Lys, Leu, Asn, Pro, Arg,Ser Gly, His or Tyr" at a position in an HPPD protein, said position corresponding to position 251 of the amino acid sequence of SEQ ID No. 2;
c) Glu, Phe, Gly, His, Ile, Leu, Met, Asn, Gln, Arg, Ser, Val, Thr or Tyr at a position in an HPPD protein, said position corresponding to position 252 of the amino acid sequence of SEQ ID No.;
d) Ala, Phe, His, Gln, Val, Trp, Ile, Leu or Met at a position in an HPPD protein, said position corresponding to position 253 of the amino acid sequence of SEQ ID No. 2;
e) Leu, Val, or Met at a position in an HPPD protein, said position corresponding to position 265 of the amino acid sequence of SEQ ID No. 2;
f) Leu, Gln, Arg, Val, Tyr, Ala, Ile, Lys or Met at a position in an HPPD protein, said position corresponding to position 268 of the amino acid sequence of SEQ ID No. 2;
g) Ala, Thr or Val at a position in an HPPD protein, said position corresponding to position 269 of the amino acid sequence of SEQ ID No. 2;
h) Ala, Ile, Asn, Pro, Thr or Val at a position in an HPPD protein, said position corresponding to position 280 of the amino acid sequence of SEQ ID No. 2;
i) Leu, Gln, Val, Ala, Phe, Gly, Met, Arg or Ser at a position in an HPPD protein, said position corresponding to position 293 of the amino acid sequence of SEQ ID No. 2;
j) Ile, Met, Ala, Pro, Ser, Thr or Val at a position in an HPPD protein, said position corresponding to position 294 of the amino acid sequence of SEQ ID No. 2;
k) Gln, His or Asn at a position in an HPPD protein, said position corresponding to position 307 of the amino acid sequence of SEQ ID No. 2;
l) Ile, Met, Asn or Leu at a position in an HPPD protein, said position corresponding to position 335 of the amino acid sequence of SEQ ID No. 2;
m) Leu or Met at a position in an HPPD protein, said position corresponding to position 368 of the amino acid sequence of SEQ ID No. 2;
n) Phe or Tyr at a position in an HPPD protein, said position corresponding to position 381 of the amino acid sequence of SEQ ID No. 2;
o) Phe or Ser at a position in an HPPD protein, said position corresponding to position 392 of the amino acid sequence of SEQ ID No. 2;
p) Phe or Tyr at a position in an HPPD protein, said position corresponding to position 419 of the amino acid sequence of SEQ ID No. 2;
q) Asp, Lys, Asn, Ala, Cys, Glu, Gly, His, Ile, Leu, Met, Gln, Ser, Thr or Val at a position in an HPPD protein, said position corresponding to position 421 of the amino acid sequence of SEQ ID No. 2;
r) Ala, Gly, Met, Pro or Thr at a position in an HPPD protein, said position corresponding to position 422 of the amino acid sequence of SEQ ID No. 2;
s) Ala, Phe, Ile or Val at a position in an HPPD protein, said position corresponding to position 424 of the amino acid sequence of SEQ ID No. 2;
t) Ile, Pro, Arg, Ser Ala, Gly, Lys, Asn or Gln at a position in an HPPD protein, said position corresponding to position 425 of the amino acid sequence of SEQ ID No. 2; u) Glu, Phe, Thr, Val Ala, Gln or Ser at a position in an HPPD protein, said position corresponding to position 426 of the amino acid sequence of SEQ ID No. 2; v) Ile, Met, Gln, Val Phe or Leu at a position in an HPPD protein, said position corresponding to position 431 of the amino acid sequence of SEQ ID No. 2; and w) at least one amino acid deletion or replacement at any one of positions 228, 248, 270, 271, 379 and/or 427.

Unless indicated otherwise, the specific definitions or specific features of certain embodiments can be introduced into any other embodiment of the present invention.

According to the present invention, a "nucleic acid" is understood as being a nucleotide sequence which can be of the DNA or RNA type, preferably of the DNA type, and in particular double-stranded, whether it be of natural or synthetic origin, in particular a DNA sequence in which the codons which encode the HPPD according to the invention have been optimized in accordance with the host organism in which it is to be expressed (e.g., by replacing codons with those codons more preferred or most preferred in codon usage tables of such host organism or the group to which such host organism belongs, compared to the original or source organism).

An "isolated nucleic acid/DNA/protein", as used in the present application, refers to a nucleic acid/DNA/protein which is not naturally-occurring (such as an artificial or synthetic DNA with a different nucleotide sequence than the naturally-occurring DNA, or a modified protein) or which is no longer in the natural environment wherein it was originally present, e.g., a DNA coding sequence associated with a heterologous regulatory element (such as a bacterial coding sequence operably-linked to a plant-expressible promoter) in a chimeric gene, a DNA transferred into another host cell, such as a transgenic plant cell.

The terminology relating to nucleic acid or protein "comprising" a certain nucleotide sequence or amino acid sequence, as used throughout the text, refers to a nucleic acid or protein including or containing at least the described sequence, so that other nucleotide or amino acid sequences can be included at the 5' (or N-terminal) and/or 3' (or C-terminal) end, e.g. (the nucleotide sequence of) a selectable marker protein, (the nucleotide sequence of) a transit peptide, and/or a 5' leader sequence or a 3' trailer sequence. Similarly, use of the term "comprise", "comprising" or "comprises" throughout the text and the claims of this application should be understood to imply the inclusion of a stated integer or step or group of integers or steps but not the exclusion of any other integer or step or group of integers or steps. The term "comprising" also includes the term "consisting of".

In accordance with the present invention, the term "mutated HPPD protein" which is interchangeably used with the term "mutant HPPD protein" denotes an HPPD protein having an amino acid sequence which does not occur in nature. As opposed to the term "isolated" referred to above, the term "mutated" cannot refer to the environment of the (amino acid or protein) sequence in question, such as it being isolated from its natural environment or being coupled to a heterologous (amino acid or protein) sequence, but only refers to the amino acid sequence defining said mutated HPPD protein which cannot be found anywhere in nature but arose from a unmutated or wild-type starting amino acid sequence. In other words, in arriving at the nucleic acid of the present invention encoding a mutated HPPD protein, a starting amino acid sequence of a naturally existing protein has to be taken and to be modified by man by replacing at least one amino acid as defined in the present application.

The sequence which encodes an original unmutated HPPD which will be mutated according to the invention can be of any origin. In particular, it can be of bacterial, plant or animal origin. Advantageous examples which may be cited are bacteria of the Pseudomonas sp. type, for example Pseudomonas fluorescens, or otherwise cyanobacteria of the Synechocystis genus. The sequence can also be of plant origin, in particular derived from dicotyledonous plants, umbelliferous plants, or otherwise monocotyledonous plants. Advantageous examples which may be cited are plants such as tobacco, Arabidopsis, Daucus carotta, Zea mays (corn), wheat, barley, Avena sativa, wheat, Brachiaria platyphylla, Cenchrus echinatus, Lolium rigidum, Festuca arundinacea, Setaria faberi, Eleusine indica, and Sorghum. The coding sequences, and the way of isolating and cloning them, are described in the previously cited references. In a particular embodiment of the invention, the HPPD is from a bacterial origin, particularly from Pseudomonas sp., more particularly from Pseudomonas fluorescens, Rhodococcus sp., Blepharisma japonicum, Synechococcus sp., Picrophilus torridus, Kordia algicida or from a plant origin, particularly from Arabidopsis thaliana or Avena sativa. The HPPD to make the mutation (s) in for the purpose of the invention, can be any naturally-occurring HPPD, or any active fragment thereof or any variant thereof wherein some amino acids (1 to 10 amino acids) have been replaced, added or deleted for cloning purposes, to make a transit peptide fusion, and the like, which retains HPPD activity, i.e. the property of catalysing the conversion of para-hydroxyphenylpyruvate to homogentisate.

The mutated HPPD protein according to the present invention has HPPD activity, i. e., as described above, catalyses the reaction in which para-hydroxyphenylpyruvate is transformed into homogentisate. Preferentially, the catalytic activity of the isolated mutated HPPD of the present invention, when tested in vitro, does not differ from that of the unmutated reference HPPD protein by more than 70%, preferably more than 50%, more preferably more than 30%, even more preferably more than 20% when assayed under identical conditions and in the absence of the HPPD inhibitor herbicides described above. The catalytic activity of an HPPD enzyme may be defined by various methods well-known in the art. WO 2009/144079 describes various suitable screening methods.

Initial screens may be performed with the nucleic acid encoding the mutated HPPD protein of the invention being expressed in bacteria.

Colorimetric Screening Test for Active HPPD Enzymes:
A YT-broth-type culture medium with 1% agarose, 5mM L-Tyrosine and 42mM Succinate, which contains the selection agent for the vector pSE420 (Invitrogen, Karlsruhe, Germany) is poured into deep well plates. E.coli culture in the exponentional growth phase which contains the vector pSE420-HPPDx (HPPDx means any gene coding for a putative HPPD enzyme/protein) is applied to each well.
After 16 hours at 37°C, the wells which do not contain the culture medium, those which have been seeded with an E. coil culture containing the empty vector pSE420 are transparent, or those which have been seeded with an E. coli culture containing a vector pSE420-HPPDx containing a gene coding for an inactive HPPD are transparent, while the weels seeded with an E. coli culture containing the vector pSE420-HPPDx coding for an active HPPD are brown. It has been previously demonstrated that this test refelects the HPPD activity, whatever the orgin of this activity is, and allows the identification of HPPD activities (US 6,768,044), i.e. at a qualitative level.

Further and more elaborate screens may be carried out in plant cells or plants expressing the mutated HPPD protein of the invention.

The same screenings may also be used when examining of whether a mutated HPPD protein is capable of modulating, such as decreasing or increasing, the tolerance of a plant to at least one HPPD herbicide inhibitor which will be referred to further below, with the difference that at least one of such an HPPD inhibitor is added. Examples of HPPD inhibitors to be used in those screenings include tembotrione, mesotrione, pyrasulfotole, bicyclopyrone, topramezone and sulcotrione. A screening method which is simple to implement is to determine the dose of HPPD inhibitor which fully inhibits the original unmutated HPPD, and which is lethal for the cells which express this unmutated HPPD, and to subject the mutated cells to this predetermined dose, and thereafter to isolate the mutated cells which have withstood this lethal dose, and then to isolate and to clone the gene which encodes the mutated HPPD.

Alternatively, at the quantitative level data like pI₅₀ (pI₅₀-value means the log value of the concentration of inhibitor necessary to inhibit 50% of the enzyme activity in molar concentration) can be obtained by employing the isolated and purified HPPD polypeptide, i.e. the mutated vs. the unmutated HPPD polypeptide and in presence or absence of the any respective HPPD inhibitor herbicide.

The terms "tolerance", "tolerant" or "less sensitive" denotes the lack of susceptibility of a plant expressing the mutated HPPD protein of the present invention to substances, particularly herbicides, which inhibit HPPD proteins, optionally in comparison with the plant's own HPPD protein or with any known HPPD protein. More specifically, said terms mean the relative levels of inherent tolerance of the HPPD screened according to a visible indicator phenotype of the strain or plant transformed with a nucleic acid comprising the gene coding for the respective HPPD protein in the presence of different concentrations of the various HPPD inhibitors. Dose responses and relative shifts in dose responses associated with these indicator phenotypes (formation of brown colour, growth inhibition, bleaching, herbicidal effect etc) are conveniently expressed in terms, for example, of GR50 (concentration for 50% reduction of growth) or MIC (minimum inhibitory concentration) values where increases in values correspond to increases in inherent tolerance of the expressed HPPD, in the normal manner based upon plant damage, meristematic bleaching symptoms etc. at a range of different concentrations of herbicides. These data can be expressed in terms of, for example, GR50 values derived from dose/response curves having "dose" plotted on the x-axis and "percentage kill", "herbicidal effect", "numbers of emerging green plants" etc. plotted on the y-axis where increased GR50 values correspond to increased levels of inherent tolerance of the expressed HPPD. Herbicides can suitably be applied pre-emergence or post emergence.

Likewise, tolerance level of the nucleic acid or gene encoding an HPPD protein according to the invention, or the mutated HPPD protein of the invention is screened via transgenesis, regeneration, breeding and spray testing of a test plant such as tobacco, or a crop plant such as soybean or cotton. In line with the results obtained by such screening, such plants are at least 2-4 times more tolerant to HPPD inhibitors like tembotrione, mesotrione, diketonitrile, and/or bicyclopyrone, pyrasulfotole, than plants that do not contain any exogenous gene encoding an HPPD protein, or than plants that contain a gene comprising an Arabidopsis thaliana HPPD-encoding DNA, under control of the same promoter as the nucleic acid encoding the mutated HPPD protein of the invention. Accordingly, the term "capable of increasing the tolerance of a plant to at least one herbicide acting on HPPD" denotes a tolerance increased in a plant by at least the factor of 2, alternatively at least the factor of 3 or 4 or even 5 or 6 as compared to a plant only expressing it's endogenous HPPD or a plant expressing an Arabidopsis thaliana HPPD. In this regard, the term "herbicide acting on HPPD" is not limited to substances which are known and/or used as herbicides but to any substances which inhibits the catalytic activity of HPPD proteins.

In an alternative embodiment of the nucleic acid encoding a mutated HPPD polypeptide comprising at least one of the mutations as defined above, the HPPD protein comprises
a) a His at a position in an HPPD protein, said position corresponding to position 226 of the amino acid sequence of SEQ ID No. 2;
b) a Ser at a position in an HPPD protein, said position corresponding to position 267 of the amino acid sequence of SEQ ID No. 2;
c) an Asn at a position in an HPPD protein, said position corresponding to position 282 of the amino acid sequence of SEQ ID No. 2;
d) a His at a position in an HPPD protein, said position corresponding to position 308 of the amino acid sequence of SEQ ID No. 2;
e) a Tyr at a position in an HPPD protein, said position corresponding to position 342 of the amino acid sequence of SEQ ID No. 2;
f) a Glu at a position in an HPPD protein, said position corresponding to position 394 of the amino acid sequence of SEQ ID No. 2;
g) a Gly at a position in an HPPD protein, said position corresponding to position 420 of the amino acid sequence of SEQ ID No. 2; and
h) an Asn at a position in an HPPD protein, said position corresponding to position 423 of the amino acid sequence of SEQ ID No. 2

In the mutated HPPD protein encoded by the nucleic acid of the invention at least one amino acid has been deleted or replaced as defined above.

The replacement or deletion can be effected in the nucleic acid sequence which encodes the original unmutated, i. e. naturally occurring HPPD as defined above by any means which is appropriate for replacing, in the said sequence, the codon which encodes the amino acid to be replaced with the codon which corresponds to the amino acid which is to replace it, or by deleting a codon, with the said codons being widely described in the literature and well known to the skilled person. Several molecular biological methods can be used to achieve this replacement or deletion. A preferred method for preparing a mutated nucleic acid sequence according to the invention and the corresponding protein comprises carrying out site-directed mutagenesis on codons encoding one or more amino acids which are selected in advance. The methods for obtaining these site-directed mutations are well known to the skilled person and widely described in the literature (in particular: Directed Mutagenesis: A Practical Approach, 1991, Edited by M.J. McPHERSON, IRL PRESS), or are methods for which it is possible to employ commercial kits (for example the U. S. E. mutagenesis kit from PHARMACIA). After the site-directed mutagenesis, it is useful to select the cells which contain a mutated HPPD which is less sensitive to an HPPD inhibitor by using an appropriate screening aid. Appropriate screening methods to achieve this have been described above.

In accordance with the present invention, the term "said position corresponding to position X", X being any number to be found in the respective context in the present application, does not only include the respective position in the SEQ ID No. referred to afterwards but also includes any sequence encoding an HPPD protein, where, after alignment with the reference SEQ ID No., the respective position might have a different number but corresponds to that indicated for the reference SEQ ID No. Whereas HPPD sequences may be very diverse and may only show a low sequence identity of about 30%, HPPD proteins are characterized by a common three dimensional consensus structure which is achieved despite a low sequence identity. Due to specific positions being conserved within HPPD proteins, alignment of HPPD proteins can be effected by by applying various alignment tools in a senseful manner. Methods of aligning nucleic acid or amino acid sequences and, accordingly, determining the sequence identity of two or more sequences, are well-known in the art. They include performing mathematical algorithms such as the algorithm of Myers and Miller (1988) CABIOS 4:11-17 or the local alignment algorithm of Smith and Waterman (1981) Adv. Appl. Math. 2:482-489; the global alignment algorithm of Needleman and Wunsch (1970) J. Mol. Biol. 48:443-453; the algorithm of Karlin and Altschul (1990) Proc. Natl. Acad. Sci USA 872264 and that of Brutlag et al. (Comp. App. Biosci. 6:237-245 (1990)).

Such algorithms can be implemented in computer programs including but not limited to CLUSTALX, ALIGN, GAP, BESTFIT, BLAST, FASTDB and FASTA. For example, when using BESTFIT (Wisconsin Sequence Analysis Package, Version 8 for Unix, Genetics Computer Group, University Research Park, 575 Science Drive, Madison, WI 53711)or any other sequence alignment program to determine whether a particular sequence is, for instance, 95% identical to a reference sequence, the parameters are set, of course, such that the percentage of identity is calculated over the full length of the reference nucleotide sequence and that gaps in homology of up to 5% of the total number of nucleotides in the reference sequence are allowed.

The identity between a first sequence and a second sequence, also referred to as a global sequence alignment, is determined using the FASTDB computer program based on the algorithm of Brutlag and colleagues (Comp. App. Biosci. 6:237-245 (1990)). In a sequence alignment the query and subject sequences are both DNA sequences. The result of said global sequence alignment is in percent identity. Preferred parameters used in a FASTDB alignment of DNA sequences to calculate percent identity are: Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the length of the subject nucleotide sequence, whichever is shorter.

The present invention is based on the results of a combination of a comparison of the amino acid sequences of HPPD proteins from various organisms and the analysis of the substrate binding and inhibitor binding site of selected HPPD proteins using X-ray crystallography. Using this combined approach, it was possible to determine key positions in HPPD proteins, where an amino acid can be replaced with one of a defined set of other amino acids in order to modulate HPPD catalytic activity and the affinity to at least one HPPD herbicide inhibitor to a plant expressing the mutated HPPD protein.

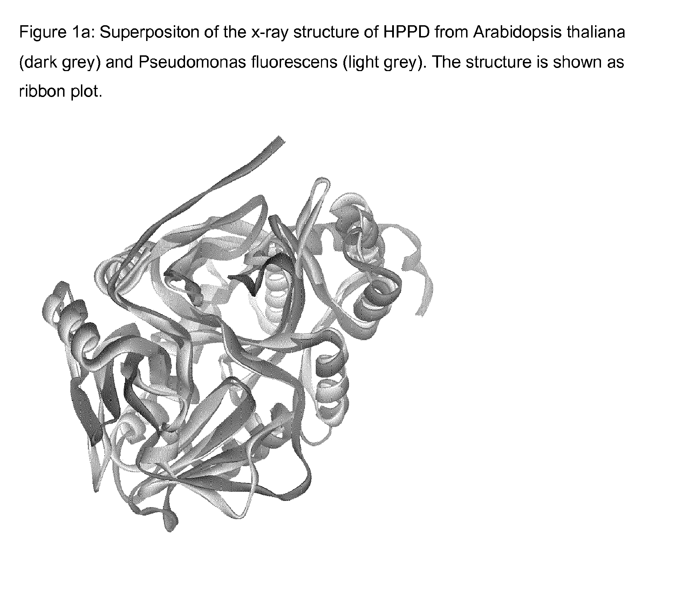

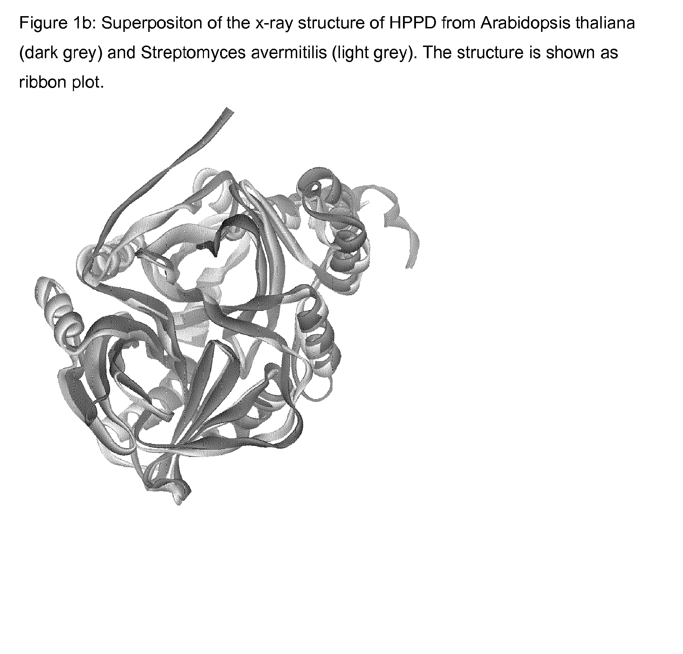

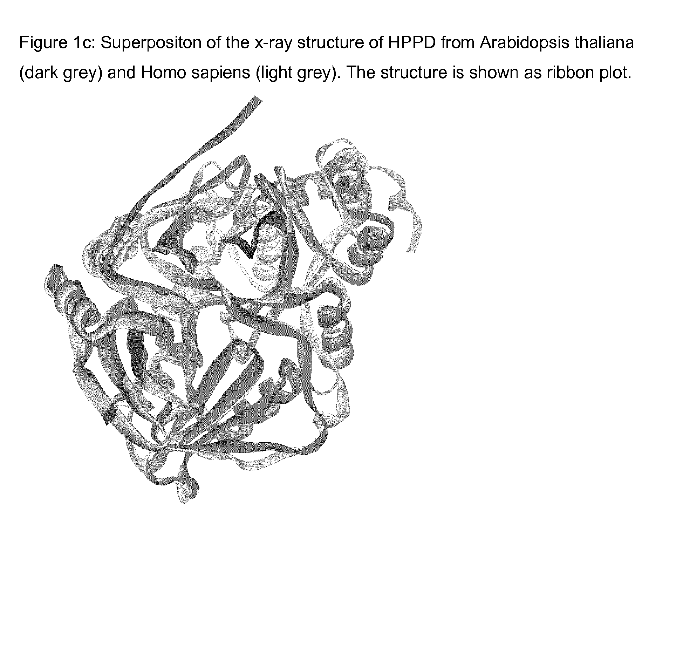

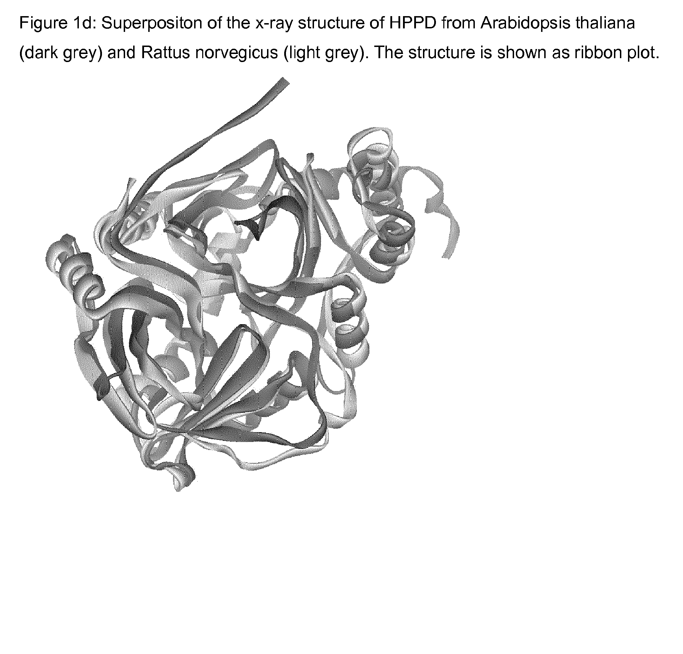

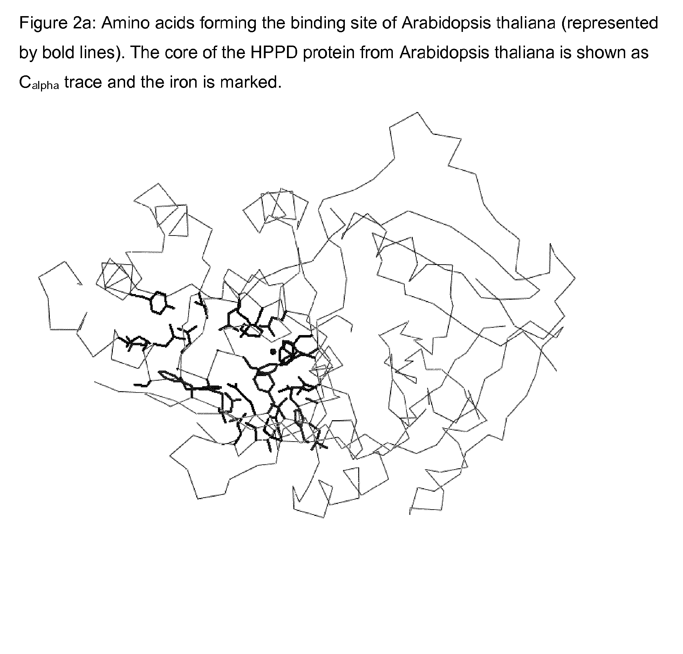

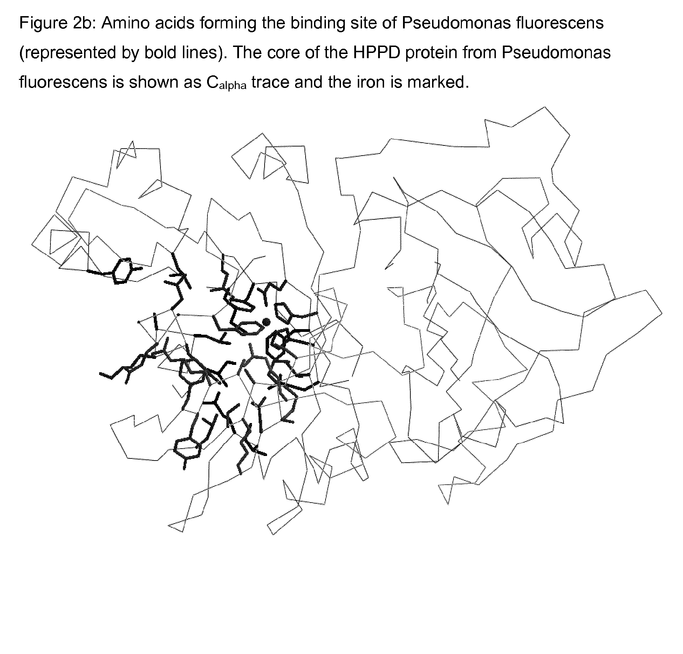

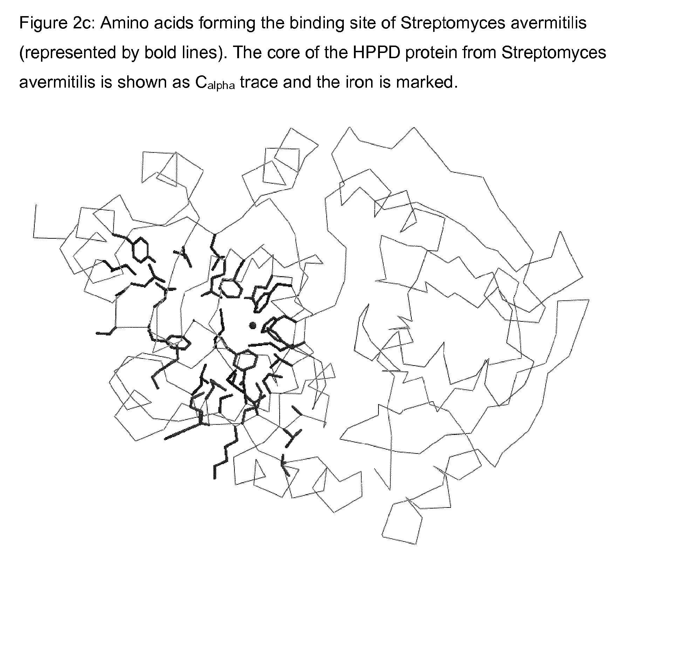

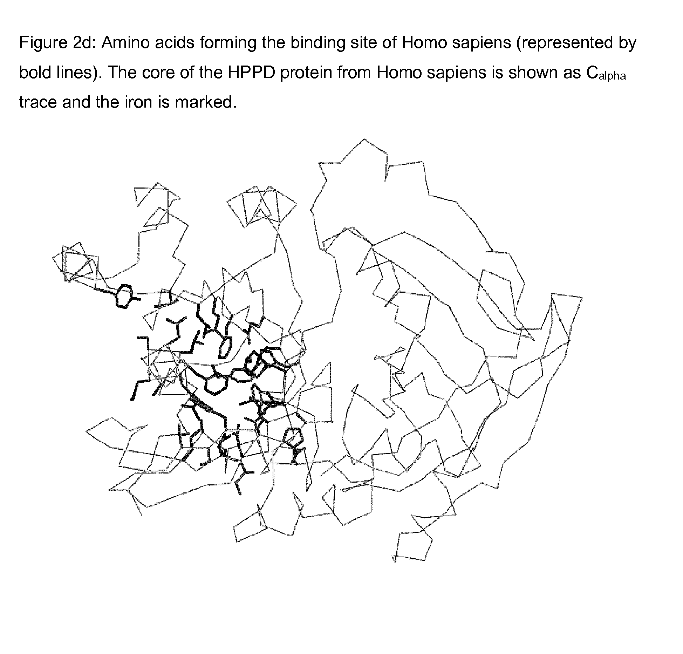

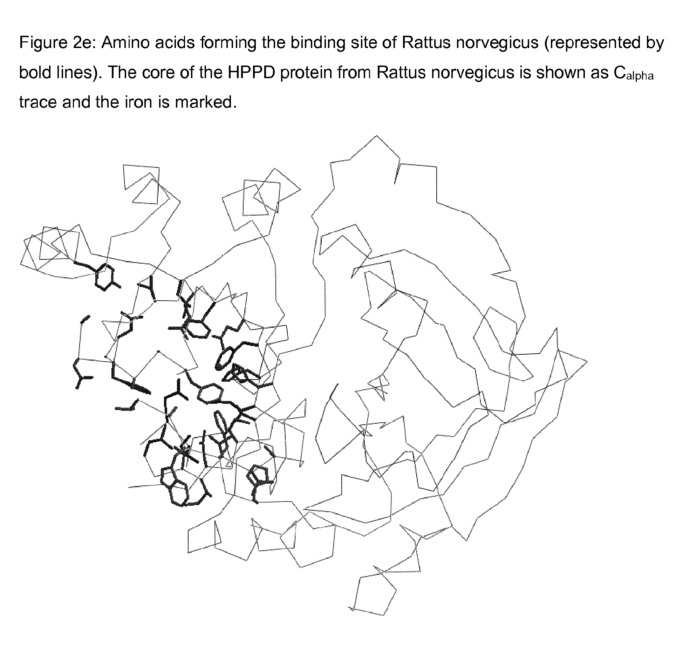

Superposition of the 3D structure of HPPD from Arabidopsis thaliana (1TFZ) (Yang et al., 2004, Biochemistry 43, 10414-10423) with the 3D structures of HPPD from other species such as Pseudomonas fluorescens (1CJX) (Serre et al., 1999, Structure Fold Des. 7, 977-988), Streptomyces avermitilis (1T47) (Brownlee et al., 2004, Biochemistry 43, 6370-6377), Homo sapiens (3ISQ) (PDB ID: 3isq Pilka et al., Structural Genomics Consortium (SGC). Crystal structure of human 4-Hydroxyphenylpyruvate dioxygenase), Rattus norvegicus (1SQI) (Yang et al., 2004, Biochemistry 43, 10414-10423) shows that they have the same folding and corresponding amino acids are at equivalent position in the 3D structure of the protein. Since the species with known 3D structures are very diverse in their amino acid sequence, it can be assumed that all HPPD sequences have the same basic folding even though the overall sequence identity is low. The sequence and the 3D structure of Arabidopsis thaliana has been used as reference structure in the present invention. Figure 1 shows the superposition of the structure of A.thaliana HPPD with the structure of (a) Pseudomonas fluorescens, (b) Streptomyces avermitilis, (c) Homo sapiens and (d) Rattus norvegicus. In order to define the binding site of the substrate and/or inhibitors, amino acids were selected which play a role in catalysis or inhibitor binding. This includes amino acids in the active site and amino acids from the C-terminal helix. The 3D arrangement is demonstrated in Figure 2 which displays the amino acids defined as binding site in case of (a) Arabidopsis thaliana, (b) Pseudomonas fluorescens, (c) Streptomyces avermitilis, (d) Homo sapiens and (e) Rattus norvegicus. The amino acid numbering of the Pseudomonas Fluorescens structure (1 cjx) was changed into the numbering according to SEQ ID No. 10. The 36 amino acids defining the binding site including their position are listed in Table 1 for (a) Arabidopsis thaliana, (b) Pseudomonas fluorescens, (c) Streptomyces avermitilis, (d) Homo sapiens and (e) Rattus norvegicus.

**Table 1: Amino acids forming the binding site in A.thaliana, P.fluorescens, S.avermitilis, H.sapiens, R. norvegicus**

| Arabidopsis thaliana | | Pseudomonas fluorescens | | Streptomyces avermitilis | | Homo sapiens | | Rattus norvegicus | |
|---|---|---|---|---|---|---|---|---|---|
| Position | Amino Acid | Position | Amino Acid | Position | Amino Acid | Position | Amino Acid | Position | Amino Acid |
| 226 | H | 162 | H | 187 | H | 183 | H | 183 | H |
| 228 | V | 164 | T | 189 | V | 185 | V | 185 | V |
| 248 | H | 186 | R | 211 | T | 207 | H | 207 | H |
| 250 | F | 188 | A | 213 | M | 209 | F | 209 | F |
| 251 | A | 189 | R | 214 | K | 210 | W | 210 | W |
| 252 | E | 190 | Y | 215 | E | 211 | S | 211 | S |
| 253 | F | 191 | F | 216 | F | 212 | V | 212 | V |
| 265 | L | 200 | L | 228 | L | 224 | L | 224 | L |
| 267 | S | 202 | S | 230 | S | 226 | S | 226 | S |
| 268 | A | 203 | K | 231 | K | 227 | I | 227 | I |
| 269 | V | 204 | A | 232 | V | 228 | V | 228 | V |
| 270 | L | 205 | M | 233 | V | 229 | V | 229 | V |
| 271 | A | 206 | S | 234 | A | 230 | A | 230 | A |
| 280 | P | 215 | P | 243 | P | 239 | P | 239 | P |
| 282 | N | 217 | N | 245 | N | 241 | N | 241 | N |
| 293 | Q | 226 | Q | 255 | Q | 251 | Q | 251 | Q |
| 294 | I | 227 | I | 256 | I | 252 | I | 252 | I |
| 307 | Q | 240 | Q | 269 | Q | 265 | Q | 265 | Q |
| 308 | H | 241 | H | 270 | H | 266 | H | 266 | H |
| 335 | M | 264 | M | 293 | L | 289 | L | 289 | L |
| 342 | Y | 271 | Y | 299 | Y | 295 | Y | 295 | Y |
| 368 | L | 295 | L | 323 | L | 323 | L | 323 | L |
| 379 | Q | 310 | Q | 334 | Q | 334 | Q | 334 | Q |
| 381 | F | 312 | F | 336 | F | 336 | F | 336 | F |
| 392 | F | 321 | F | 347 | F | 347 | F | 347 | F |
| 394 | E | 323 | E | 349 | E | 349 | E | 349 | E |
| 419 | F | 333 | F | 359 | F | 359 | F | 359 | F |
| 420 | G | 334 | G | 360 | G | 360 | G | 360 | G |
| 421 | K | 335 | E | 361 | K | 361 | A | 361 | A |
| 422 | G | 336 | G | 362 | G | 362 | G | 362 | G |
| 423 | N | 337 | N | 363 | N | 363 | N | 363 | N |
| 424 | F | 338 | F | 364 | F | 364 | F | 364 | F |
| 425 | S | 339 | K | 365 | K | 365 | N | 365 | N |
| 426 | E | 340 | A | 366 | A | 366 | S | 366 | S |
| 427 | L | 341 | L | 367 | L | 367 | L | 367 | L |
| 431 | I | 345 | I | 371 | I | 371 | F | 371 | F |

An exemplary alignment of HPPD proteins is given in Table 2a for the HPPD proteins with known 3D structures. Table 2a gives the numbering of the amino acids of the Arabidopsis sequence and also the amino acids which are common within these HPPD sequences, with these amino acids being designated by an asterisk. On the basis of such an alignment and from the definition of the Arabidopsis amino acid by its position and its nature, it is easy to identify the position of the corresponding amino acid in another HPPD sequence. Figure 2 shows that this can be done with the alignment of sequences of different plant, mammalian and bacterial origin, demonstrating that this method of alignment, which is well known to a skilled person, can be generalized to any other sequence. An alignment of different HPPD sequences is also described in Patent Application WO 97/49816.

**Table 2a: Alignment of HPPD sequences with known crystal structures i.e. A.thaliana, P.fluorescens, S.avermitilis, H.sapiens, R. norvegicus**

| Pos. | 1TFZ | Pos. | 1CJX | Pos. | 1T47 | Pos. | 3ISQ | Pos. | 1SQI | Common |
|---|---|---|---|---|---|---|---|---|---|---|
| | A. thaliana | P. fluorescens | | S. avermitilis | | H. Sapiens | | R. norvegicus | | amino acid |
| 1 | M | | - | | - | | - | | | |
| 2 | G | | - | | - | | - | | | |
| 3 | H | | - | | - | | - | | | |
| 4 | Q | | - | | - | | - | | | |
| 5 | N | | - | | - | | - | | | |
| 6 | A | | - | | - | | - | | | |
| 7 | A | | - | | - | | - | | | |
| 8 | V | | - | | - | | - | | | |
| 9 | S | | - | | - | | - | | | |
| 10 | E | | - | | - | | - | | | |
| 11 | N | | - | | - | | - | | | |
| 12 | Q | | - | | - | | - | | | |
| 13 | N | | - | | - | | - | | | |
| 14 | H | | - | 1 | M | | - | | | |
| 15 | D | | - | 2 | T | | - | | | |
| 16 | D | | - | 3 | Q | | - | | | |
| 17 | G | | - | 4 | T | | - | | | |
| 18 | A | | - | 5 | T | | - | | | |
| 19 | A | | - | 6 | H | 8 | G | 8 | G | |
| 20 | S | | - | 7 | H | 9 | A | 9 | P | |
| 21 | S | | - | 8 | T | 10 | K | 10 | K | |
| 22 | P | | - | 9 | P | 11 | P | 11 | P | |
| 23 | G | | - | | - | | - | | | |
| 24 | F | | - | | - | | - | | | |
| 25 | K | | - | | - | | - | | | |
| 26 | L | | - | | - | | - | | | |
| 27 | V | | - | | - | | - | | | |
| 28 | G | | - | | - | | - | | | |
| 29 | F | | - | | - | | - | | | |
| 30 | S | | - | | - | | - | | | |
| 31 | K | | - | | - | | - | | | |
| 32 | F | | - | | - | | - | | | |
| 33 | V | | - | | - | | - | | | |
| 34 | R | | - | 10 | D | 12 | E | 12 | E | |
| 35 | K | 2 | A | 11 | T | 13 | R | 13 | R | |
| 36 | N | 3 | D | 12 | A | 14 | G | 14 | G | |
| 37 | P | 4 | L | 13 | R | 15 | R | 15 | R | |
| 38 | K | 5 | Y | 14 | Q | 16 | F | 16 | F | |
| 39 | S | 6 | E | 15 | A | 17 | L | 17 | L | |
| 40 | D | 7 | N | 16 | D | 18 | H | 18 | H | |
| 41 | K | 8 | P | 17 | P | | - | | | |
| 42 | F | 9 | M | 18 | F | | - | | | |
| 43 | K | 10 | G | 19 | P | | - | | | |
| 44 | V | 11 | L | 20 | V | | - | | | |
| 45 | K | 12 | M | 21 | K | | - | | | |
| 46 | R | 13 | G | 22 | G | | - | | | |
| 47 | F | 14 | F | 23 | M | 19 | F | 19 | F | |
| 48 | H | 15 | E | 24 | D | 20 | H | 20 | H | |
| 49 | H | 16 | F | 25 | A | 21 | S | 21 | S | |
| 50 | I | 17 | I | 26 | V | 22 | V | 22 | V | |
| 51 | E | 18 | E | 27 | V | 23 | T | 23 | T | |
| 52 | F | 19 | F | 28 | F | 24 | F | 24 | F | X |
| 53 | W | 20 | A | 29 | A | 25 | W | 25 | W | |
| 54 | C | 21 | S | 30 | V | 26 | V | 26 | V | |
| 55 | G | 22 | P | 31 | G | 27 | G | 27 | G | |
| 56 | D | 23 | T | 32 | N | 28 | N | 28 | N | |
| 57 | A | 24 | P | 33 | A | 29 | A | 29 | A | |
| 58 | T | 25 | G | 34 | K | 30 | K | 30 | K | |
| 59 | N | 26 | T | 35 | Q | 31 | Q | 31 | Q | |
| 60 | V | 27 | L | 36 | A | 32 | A | 32 | A | |
| 61 | A | 28 | E | 37 | A | 33 | A | 33 | A | |
| 62 | R | 29 | P | 38 | H | 34 | S | 34 | S | |
| 63 | R | 30 | I | 39 | Y | 35 | F | 35 | F | |
| 64 | F | 31 | F | 40 | Y | 36 | Y | 36 | Y | |
| 65 | S | 32 | E | 41 | S | 37 | C | 37 | C | |
| 66 | W | 33 | I | 42 | T | 38 | S | 38 | N | |
| 67 | G | 34 | M | 43 | A | 39 | K | 39 | K | |
| 68 | L | 35 | G | 44 | F | 40 | M | 40 | M | |
| 69 | G | 36 | F | 45 | G | 41 | G | 41 | G | |
| 70 | M | 37 | T | 46 | M | 42 | F | 42 | F | |
| 71 | R | 38 | K | 47 | Q | 43 | E | 43 | E | |
| 72 | F | 39 | V | 48 | L | 44 | P | 44 | P | |
| 73 | S | 40 | A | 49 | V | 45 | L | 45 | L | |
| 74 | A | 41 | T | 50 | A | 46 | A | 46 | A | |
| 75 | K | 42 | H | 51 | Y | 47 | Y | 47 | Y | |
| 76 | S | 43 | R | 52 | S | 48 | R | 48 | K | |
| 77 | D | 44 | S | 53 | G | 49 | G | 49 | G | |
| 78 | L | 45 | K | 54 | P | 50 | L | 50 | L | |
| 79 | S | 46 | N | 55 | E | 51 | E | 51 | E | |
| 80 | T | | - | 56 | N | 52 | T | 52 | T | |
| 81 | G | | - | 57 | G | 53 | G | 53 | G | |
| 82 | N | | - | 58 | S | 54 | S | 54 | S | |
| 83 | M | | - | 59 | R | 55 | R | 55 | R | |
| 84 | V | | - | 60 | E | 56 | E | 56 | E | |
| 85 | H | | - | 61 | T | 57 | V | 57 | V | |
| 86 | A | | - | 62 | A | 58 | V | 58 | V | |
| 87 | S | 47 | V | 63 | S | 59 | S | 59 | S | |
| 88 | Y | 48 | H | 64 | Y | 60 | H | 60 | H | |
| 89 | L | 49 | L | 65 | V | 61 | V | 61 | V | |
| 90 | L | 50 | Y | 66 | L | 62 | I | 62 | I | |
| 91 | T | 51 | R | 67 | T | 63 | K | 63 | K | |
| 92 | S | 52 | Q | 68 | N | 64 | Q | 64 | Q | |
| 93 | G | 53 | G | 69 | G | 65 | G | 65 | G | X |
| 94 | D | 54 | E | 70 | S | 66 | K | 66 | K | |
| 95 | L | 55 | I | 71 | A | 67 | I | 67 | I | |
| 96 | R | 56 | N | 72 | R | 68 | V | 68 | V | |
| 97 | F | 57 | L | 73 | F | 69 | F | 69 | F | |
| 98 | L | 58 | I | 74 | V | 70 | V | 70 | V | |
| 99 | F | 59 | L | 75 | L | 71 | L | 71 | L | |
| 100 | T | 60 | N | 76 | T | 72 | S | 72 | C | |
| 101 | A | 61 | N | 77 | S | 73 | S | 73 | S | |
| 102 | P | 62 | E | 78 | V | 74 | A | 74 | A | |
| 103 | Y | 63 | P | 79 | I | 75 | L | 75 | L | |
| 104 | S | 64 | N | 80 | K | 76 | N | 76 | N | |
| 105 | P | 65 | S | 81 | P | | - | | | |
| 106 | S | 66 | I | 82 | A | | - | | | |
| 107 | L | 67 | A | 83 | T | | - | | | |
| 108 | S | 68 | S | 84 | P | 77 | P | 77 | P | |
| 109 | A | | - | 85 | W | 78 | W | 78 | W | |
| 110 | G | | - | 86 | G | 79 | N | 79 | N | |
| 111 | E | | - | 87 | H | 80 | K | 80 | K | |
| 112 | I | | - | 88 | F | 81 | E | 81 | E | |
| 113 | K | | - | 89 | L | 82 | M | 82 | M | |
| 114 | P | | - | 90 | A | 83 | G | 83 | G | |
| 115 | T | | - | | - | | - | | | |
| 116 | T | | - | | - | | - | | | |
| 117 | T | | - | | - | | - | | | |
| 118 | A | | - | | - | | - | | | |
| 119 | S | | - | | - | | - | | | |
| 120 | I | | - | | - | | - | | | |
| 121 | P | | - | | - | | - | | | |
| 122 | S | | - | | - | | - | | | |
| 123 | F | | - | | - | | - | | | |
| 124 | D | | - | | - | | - | | | |
| 125 | H | | - | | - | | - | | | |
| 126 | G | | - | | - | | - | | | |
| 127 | S | | - | | - | | - | | | |
| 128 | C | | - | | - | | - | | | |
| 129 | R | | - | | - | | - | | | |
| 130 | S | 69 | Y | 91 | D | 84 | D | 84 | D | |
| 131 | F | 70 | F | 92 | H | 85 | H | 85 | H | |
| 132 | F | 71 | A | 93 | V | 86 | L | 86 | L | |
| 133 | S | 72 | A | 94 | A | 87 | V | 87 | V | |
| 134 | S | 73 | E | 95 | E | 88 | K | 88 | K | |
| 135 | H | 74 | H | 96 | H | 89 | H | 89 | H | X |
| 136 | G | 75 | G | 97 | G | 90 | G | 90 | G | X |
| 137 | L | 76 | P | 98 | D | 91 | D | 91 | D | |
| 138 | G | 77 | S | 99 | G | 92 | G | 92 | G | |
| 139 | V | 78 | V | 100 | V | 93 | V | 93 | V | X |
| 140 | R | 79 | C | 101 | V | 94 | K | 94 | K | |
| 141 | A | 80 | G | 102 | D | 95 | D | 95 | D | |
| 142 | V | 81 | M | 103 | L | 96 | I | 96 | I | |
| 143 | A | 82 | A | 104 | A | 97 | A | 97 | A | X |
| 144 | I | 83 | F | 105 | I | 98 | F | 98 | F | |
| 145 | E | 84 | R | 106 | E | 99 | E | 99 | E | |
| 146 | V | 85 | V | 107 | V | 100 | V | 100 | V | X |
| 147 | E | 86 | K | 108 | P | 101 | E | 101 | E | |
| 148 | D | 87 | D | 109 | D | 102 | D | 102 | D | X |
| 149 | A | 88 | S | 110 | A | 103 | C | 103 | C | |
| 150 | E | 89 | Q | 111 | R | 104 | D | 104 | E | |
| 151 | S | 90 | K | 112 | A | 105 | Y | 105 | H | |
| 152 | A | 91 | A | 113 | A | 106 | I | 106 | I | |
| 153 | F | 92 | Y | 114 | H | 107 | V | 107 | V | |
| 154 | S | 93 | N | 115 | A | 108 | Q | 108 | Q | |
| 155 | I | 94 | R | 116 | Y | 109 | K | 109 | K | |
| 156 | S | 95 | A | 117 | A | 110 | A | 110 | A | |
| 157 | V | 96 | L | 118 | I | 111 | R | 111 | R | |
| 158 | A | 97 | E | 119 | E | 112 | E | 112 | E | |
| 159 | N | 98 | L | 120 | H | 113 | R | 113 | R | |
| 160 | G | 99 | G | 121 | G | 114 | G | 114 | G | X |
| 161 | A | 100 | A | 122 | A | 115 | A | 115 | A | |
| 162 | I | 101 | Q | 123 | R | 116 | K | 116 | K | |
| 163 | P | | - | 124 | S | 117 | I | 117 | I | |
| 164 | S | | - | 125 | V | 118 | M | 118 | V | |
| 165 | S | | - | 126 | A | 119 | R | 119 | R | |
| 166 | P | | - | 127 | E | 120 | E | 120 | E | |
| 167 | P | 102 | P | 128 | P | 121 | P | 121 | P | X |
| 168 | I | 103 | I | 129 | Y | 122 | W | 122 | W | |
| 169 | V | 104 | H | 130 | E | 123 | V | 123 | V | |
| 170 | L | 105 | I | 131 | L | 124 | E | 124 | E | |
| 171 | N | 106 | D | 132 | K | 125 | Q | 125 | E | |
| 172 | E | 107 | T | 133 | D | 126 | D | 126 | D | |
| 173 | A | 108 | G | 134 | E | 127 | K | 127 | K | |
| 174 | V | 109 | P | 135 | H | 128 | F | 128 | F | |
| 175 | T | 110 | M | 136 | G | 129 | G | 129 | G | |
| 176 | I | 111 | E | 137 | T | 130 | K | 130 | K | |
| 177 | A | 112 | L | 138 | V | 131 | V | 131 | V | |
| 178 | E | 113 | N | 139 | V | 132 | K | 132 | K | |
| 179 | V | 114 | L | 140 | L | 133 | F | 133 | F | |
| 180 | K | 115 | P | 141 | A | 134 | A | 134 | A | |
| 181 | L | 116 | A | 142 | A | 135 | V | 135 | V | |
| 182 | Y | 117 | I | 143 | I | 136 | L | 136 | L | |
| 183 | G | 118 | K | 144 | A | 137 | Q | 137 | Q | |
| 184 | D | 119 | G | 145 | T | 138 | T | 138 | T | |
| 185 | V | 120 | I | 146 | Y | 139 | Y | 139 | Y | |
| 186 | V | 121 | G | 147 | G | 140 | G | 140 | G | |
| 187 | L | 122 | G | 148 | K | 141 | D | 141 | D | |
| 188 | R | 123 | A | 149 | T | 142 | T | 142 | T | |
| 189 | Y | 124 | P | 150 | R | 143 | T | 143 | T | |
| 190 | V | 125 | L | 151 | H | 144 | H | 144 | H | |
| 191 | S | 126 | Y | 152 | T | 145 | T | 145 | T | |
| 192 | Y | 127 | L | 153 | L | 146 | L | 146 | L | |
| 193 | K | 128 | I | 154 | V | 147 | V | 147 | V | |
| 194 | A | 129 | D | 155 | D | 148 | E | 148 | E | |
| 195 | E | 130 | R | 156 | R | 149 | K | 149 | K | |
| 196 | D | 131 | F | 157 | T | 150 | M | 150 | I | |
| 197 | T | 132 | G | 158 | G | 151 | N | 151 | N | |
| 198 | E | 133 | E | 159 | Y | 152 | Y | 152 | Y | |
| | - | 134 | G | | - | | - | | | |
| 199 | K | 135 | S | 160 | D | 153 | I | 153 | T | |
| 200 | S | 136 | S | 161 | G | 154 | G | 154 | G | |
| 201 | E | 137 | I | 162 | P | 155 | Q | 155 | R | |
| 202 | F | 138 | Y | 163 | Y | 156 | F | 156 | F | |
| 203 | L | 139 | D | 164 | L | 157 | L | 157 | L | |
| 204 | P | 140 | I | 165 | P | 158 | P | 158 | P | |
| 205 | G | 141 | D | 166 | G | 159 | G | 159 | G | |
| 206 | F | 142 | F | 167 | Y | 160 | Y | 160 | F | |
| 207 | E | 143 | V | 168 | V | 161 | E | 161 | E | |
| 208 | R | 144 | Y | 169 | A | 162 | A | 162 | A | |
| 209 | V | 145 | L | 170 | A | 163 | P | 163 | P | |
| 210 | E | 146 | E | 171 | A | 164 | A | 164 | T | |
| 211 | D | 147 | G | | - | 165 | F | 165 | Y | |
| | - | | - | | - | 166 | M | 166 | K | |
| | - | | - | | - | 167 | D | 167 | D | |
| | - | | - | 172 | P | 168 | P | 168 | T | |
| 212 | A | 148 | V | 173 | I | 169 | L | 169 | L | |
| 213 | S | 149 | E | 174 | V | 170 | L | 170 | L | |
| 214 | S | 150 | R | 175 | E | 171 | P | 171 | P | |
| 215 | F | 151 | N | 176 | P | 172 | K | 172 | K | |
| 216 | P | 152 | P | 177 | P | 173 | L | 173 | L | |
| 217 | L | 153 | V | 178 | A | 174 | P | 174 | P | |
| 218 | D | 154 | G | 179 | H | 175 | K | 175 | S | |
| 219 | Y | 155 | A | 180 | R | 176 | C | 176 | C | |
| 220 | G | 156 | G | 181 | T | 177 | S | 177 | N | |
| 221 | I | 157 | L | 182 | F | 178 | L | 178 | L | |
| 222 | R | 158 | K | 183 | Q | 179 | E | 179 | E | |
| 223 | R | 159 | V | 184 | A | 180 | M | 180 | I | |
| 224 | L | 160 | I | 185 | I | 181 | I | 181 | I | |
| 225 | D | 161 | D | 186 | D | 182 | D | 182 | D | X |
| 226 | H | 162 | H | 187 | H | 183 | H | 183 | H | X |
| 227 | A | 163 | L | 188 | C | 184 | I | 184 | I | |
| 228 | V | 164 | T | 189 | V | 185 | V | 185 | V | |
| 229 | G | 165 | H | 190 | G | 186 | G | 186 | G | |
| 230 | N | 166 | N | 191 | N | 187 | N | 187 | N | X |
| 231 | V | 167 | V | 192 | V | 188 | Q | 188 | Q | |
| 232 | P | 168 | Y | 193 | E | 189 | P | 189 | P | |
| | - | 169 | R | 194 | L | 190 | D | 190 | D | |
| | - | 170 | G | 195 | G | 191 | Q | 191 | Q | |
| 233 | E | 171 | R | 196 | R | 192 | E | 192 | E | |
| 234 | L | 172 | M | 197 | M | 193 | M | 193 | M | |
| 235 | G | 173 | V | 198 | N | 194 | V | 194 | E | |
| 236 | P | 174 | Y | 199 | E | 195 | S | 195 | S | |
| 237 | A | 175 | W | 200 | W | 196 | A | 196 | A | |
| 238 | L | 176 | A | 201 | V | 197 | S | 197 | S | |
| 239 | T | 177 | N | 202 | G | 198 | E | 198 | E | |
| 240 | Y | 178 | F | 203 | F | 199 | W | 199 | W | |
| 241 | V | 179 | Y | 204 | Y | 200 | Y | 200 | Y | |
| 242 | A | 180 | E | 205 | N | 201 | L | 201 | L | |
| 243 | G | 181 | K | 206 | K | 202 | K | 202 | K | |
| 244 | F | 182 | L | 207 | V | 203 | N | 203 | N | |
| 245 | T | 183 | F | 208 | M | 204 | L | 204 | L | |
| 246 | G | 184 | N | 209 | G | 205 | Q | 205 | Q | |
| 247 | F | 185 | F | 210 | F | 206 | F | 206 | F | X |
| 248 | H | 186 | R | 211 | T | 207 | H | 207 | H | |
| 249 | Q | 187 | E | 212 | N | 208 | R | 208 | R | |
| 250 | F | 188 | A | 213 | M | 209 | F | 209 | F | |
| 251 | A | 189 | R | 214 | K | 210 | W | 210 | W | |
| 252 | E | 190 | Y | 215 | E | 211 | S | 211 | S | |
| 253 | F | 191 | F | 216 | F | 212 | V | 212 | V | |
| 254 | T | 192 | D | 217 | V | 213 | D | 213 | D | |
| 255 | A | 193 | I | 218 | G | 214 | D | 214 | D | |
| 256 | D | 194 | K | 219 | D | 215 | T | 215 | T | |
| 257 | D | 195 | G | 220 | D | 216 | Q | 216 | Q | |
| 258 | V | 196 | E | 221 | I | 217 | V | 217 | V | |
| 259 | G | | - | 222 | A | 218 | H | 218 | H | |
| 260 | T | | - | 223 | T | 219 | T | 219 | T | |
| 261 | A | | - | 224 | E | 220 | E | 220 | E | |
| 262 | E | 197 | Y | 225 | Y | 221 | Y | 221 | Y | |
| 263 | S | 198 | T | 226 | S | 222 | S | 222 | S | |
| 264 | G | 199 | G | 227 | A | 223 | S | 223 | S | |
| 265 | L | 200 | L | 228 | L | 224 | L | 224 | L | X |
| 266 | N | 201 | T | 229 | M | 225 | R | 225 | R | |
| 267 | S | 202 | S | 230 | S | 226 | S | 226 | S | X |
| 268 | A | 203 | K | 231 | K | 227 | I | 227 | I | |
| 269 | V | 204 | A | 232 | V | 228 | V | 228 | V | |
| 270 | L | 205 | M | 233 | V | 229 | V | 229 | V | |
| 271 | A | 206 | S | 234 | A | 230 | A | 230 | A | X |
| 272 | S | 207 | A | 235 | D | 231 | N | 231 | N | |
| 273 | N | 208 | P | 236 | G | 232 | Y | 232 | Y | |
| 274 | D | 209 | D | 237 | T | 233 | E | 233 | E | |
| 275 | E | 210 | G | 238 | L | 234 | E | 234 | E | |
| 276 | M | 211 | M | 239 | K | 235 | S | 235 | S | |
| 277 | V | 212 | I | 240 | V | 236 | I | 236 | I | |
| 278 | L | 213 | R | 241 | K | 237 | K | 237 | K | |
| 279 | L | 214 | I | 242 | F | 238 | M | 238 | M | |
| 280 | P | 215 | P | 243 | P | 239 | P | 239 | P | X |
| 281 | I | 216 | L | 244 | I | 240 | I | 240 | I | |
| 282 | N | 217 | N | 245 | N | 241 | N | 241 | N | X |
| 283 | E | 218 | E | 246 | E | 242 | E | 242 | E | X |
| 284 | P | 219 | E | 247 | P | 243 | P | 243 | P | |
| 285 | V | 220 | S | 248 | A | 244 | A | 244 | A | |
| 286 | H | | - | 249 | L | 245 | P | 245 | P | |
| 287 | G | | - | 250 | A | 246 | G | 246 | G | |
| 288 | T | 221 | S | 251 | K | 247 | K | 247 | R | |
| 289 | K | 222 | K | 252 | K | 248 | K | 248 | K | X |
| 290 | R | 223 | G | | - | | - | | | |
| 291 | K | 224 | A | 253 | K | 249 | K | 249 | K | |
| 292 | S | 225 | G | 254 | S | 250 | S | 250 | S | |
| 293 | Q | 226 | Q | 255 | Q | 251 | Q | 251 | Q | X |
| 294 | I | 227 | I | 256 | I | 252 | I | 252 | I | X |
| 295 | Q | 228 | E | 257 | D | 253 | Q | 253 | Q | |
| 296 | T | 229 | E | 258 | E | 254 | E | 254 | E | |
| 297 | Y | 230 | F | 259 | Y | 255 | Y | 255 | Y | |
| 298 | L | 231 | L | 260 | L | 256 | V | 256 | V | |
| 299 | E | 232 | M | 261 | E | 257 | D | 257 | D | |
| 300 | H | 233 | Q | 262 | F | 258 | Y | 258 | Y | |
| 301 | N | 234 | F | 263 | Y | 259 | N | 259 | N | |
| 302 | E | 235 | N | 264 | G | 260 | G | 260 | G | |
| 303 | G | 236 | G | 265 | G | 261 | G | 261 | G | X |
| 304 | A | 237 | E | 266 | A | 262 | A | 262 | A | |
| 305 | G | 238 | G | 267 | G | 263 | G | 263 | G | X |
| 306 | L | 239 | I | 268 | V | 264 | V | 264 | V | |
| 307 | Q | 240 | Q | 269 | Q | 265 | Q | 265 | Q | X |
| 308 | H | 241 | H | 270 | H | 266 | H | 266 | H | X |
| 309 | L | 242 | V | 271 | I | 267 | I | 267 | I | |
| 310 | A | 243 | A | 272 | A | 268 | A | 268 | A | X |
| 311 | L | 244 | F | 273 | L | 269 | L | 269 | L | |
| 312 | M | 245 | L | 274 | N | 270 | K | 270 | R | |
| 313 | S | 246 | T | 275 | T | 271 | T | 271 | T | |
| 314 | E | 247 | D | 276 | G | 272 | E | 272 | E | |
| 315 | D | 248 | D | 277 | D | 273 | D | 273 | D | X |
| 316 | I | 249 | L | 278 | I | 274 | I | 274 | I | |
| 317 | F | 250 | V | 279 | V | 275 | I | 275 | I | |
| 318 | R | 251 | K | 280 | E | 276 | T | 276 | T | |
| 319 | T | 252 | T | 281 | T | 277 | A | 277 | T | |
| 320 | L | 253 | W | 282 | V | 278 | I | 278 | I | |
| 321 | R | 254 | D | 283 | R | 279 | R | 279 | R | |
| 322 | E | 255 | A | 284 | T | 280 | H | 280 | H | |
| 323 | M | 256 | L | 285 | M | 281 | L | 281 | L | |
| 324 | R | 257 | K | 286 | R | 282 | R | 282 | R | |
| 325 | K | 258 | K | 287 | A | 283 | E | 283 | E | |
| 326 | R | 259 | I | 288 | A | 284 | R | 284 | R | |
| 327 | S | | - | | - | | - | | | |
| 328 | S | | - | | - | | - | | | |
| 329 | I | | - | | - | | - | | | |
| 330 | G | | - | | - | | - | | | |
| 331 | G | 260 | G | 289 | G | 285 | G | 285 | G | X |
| 332 | F | 261 | M | 290 | V | 286 | L | 286 | M | |
| 333 | D | 262 | R | 291 | Q | 287 | E | 287 | E | |
| 334 | F | 263 | F | 292 | F | 288 | F | 288 | F | X |
| 335 | M | 264 | M | 293 | L | 289 | L | 289 | L | |
| 336 | P | 265 | T | 294 | D | 290 | S | 290 | A | |
| 337 | S | 266 | A | 295 | T | 291 | V | 291 | V | |
| 338 | P | 267 | P | 296 | P | 292 | P | 292 | P | X |
| 339 | P | 268 | P | | - | | - | | | |
| 340 | P | 269 | D | 297 | D | 293 | S | 293 | S | |
| 341 | T | 270 | T | 298 | S | 294 | T | 294 | S | |
| 342 | Y | 271 | Y | 299 | Y | 295 | Y | 295 | Y | X |
| 343 | Y | 272 | Y | 300 | Y | 296 | Y | 296 | Y | X |
| 344 | Q | 273 | E | 301 | D | 297 | K | 297 | R | |
| 345 | N | 274 | M | 302 | T | 298 | Q | 298 | L | |
| 346 | L | 275 | L | 303 | L | 299 | L | 299 | L | X |
| 347 | K | 276 | E | 304 | G | 300 | R | 300 | R | |
| 348 | K | 277 | G | 305 | E | 301 | E | 301 | E | |
| 349 | R | 278 | R | 306 | W | 302 | K | 302 | N | |
| 350 | V | 279 | L | 307 | V | 303 | L | 303 | L | |
| 351 | G | 280 | P | 308 | G | 304 | K | 304 | K | |
| 352 | D | 281 | D | 309 | D | 305 | T | 305 | T | |
| 353 | V | 282 | H | 310 | T | 306 | A | 306 | S | |
| 354 | L | 283 | G | 311 | R | 307 | K | 307 | K | |
| 355 | S | 284 | E | 312 | V | 308 | I | 308 | I | |
| 356 | D | 285 | P | 313 | P | 309 | K | 309 | Q | |
| 357 | D | 286 | V | 314 | V | 310 | V | 310 | V | |
| 358 | Q | 287 | D | | - | 311 | K | 311 | K | |
| | - | 288 | Q | | - | 312 | E | 312 | E | |
| | - | 289 | L | | - | 313 | N | 313 | N | |
| 359 | I | 290 | Q | | - | 314 | I | 314 | M | |
| 360 | K | 291 | A | 315 | D | 315 | D | 315 | D | |
| 361 | E | 292 | R | 316 | T | 316 | A | 316 | V | |
| 362 | C | 293 | G | 317 | L | 317 | L | 317 | L | |
| 363 | E | 294 | I | 318 | R | 318 | E | 318 | E | |
| 364 | E | 295 | L | 319 | E | 319 | E | 319 | E | |
| 365 | L | 296 | L | 320 | L | 320 | L | 320 | L | X |
| 366 | G | 297 | D | 321 | K | 321 | K | 321 | K | |
| 367 | I | 298 | G | 322 | I | 322 | I | 322 | I | |
| 368 | L | 299 | S | 323 | L | 323 | L | 323 | L | |
| 369 | V | 300 | S | 324 | A | 324 | V | 324 | V | |
| 370 | D | 301 | V | 325 | D | 325 | D | 325 | D | |
| 371 | R | 302 | E | 326 | R | 326 | Y | 326 | Y | |
| 372 | D | 303 | G | 327 | D | 327 | D | 327 | D | |
| 373 | D | 304 | D | 328 | E | 328 | E | 328 | E | |
| 374 | Q | 305 | K | 329 | D | 329 | K | 329 | K | |
| 375 | G | 306 | R | 330 | G | 330 | G | 330 | G | |
| 376 | T | 307 | L | 331 | Y | 331 | Y | 331 | Y | |
| 377 | L | 308 | L | 332 | L | 332 | L | 332 | L | X |
| 378 | L | 309 | L | 333 | L | 333 | L | 333 | L | X |
| 379 | Q | 310 | Q | 334 | Q | 334 | Q | 334 | Q | X |
| 380 | I | 311 | I | 335 | I | 335 | I | 335 | I | X |
| 381 | F | 312 | F | 336 | F | 336 | F | 336 | F | X |
| 382 | T | 313 | S | 337 | T | 337 | T | 337 | T | |
| 383 | K | 314 | E | 338 | K | 338 | K | 338 | K | |
| 384 | P | 315 | T | 339 | P | 339 | P | 339 | P | |
| 385 | L | 316 | L | 340 | V | 340 | V | 340 | M | |
| 386 | G | 317 | M | 341 | Q | 341 | Q | 341 | Q | |
| 387 | D | 318 | G | 342 | D | 342 | D | 342 | D | |
| 388 | R | | - | 343 | R | 343 | R | 343 | R | |
| 389 | P | | - | 344 | P | 344 | P | 344 | P | |
| 390 | T | 319 | P | 345 | T | 345 | T | 345 | T | |
| 391 | I | 320 | V | 346 | V | 346 | L | 346 | L | |
| 392 | F | 321 | F | 347 | F | 347 | F | 347 | F | X |
| 393 | I | 322 | F | 348 | F | 348 | L | 348 | L | |
| 394 | E | 323 | E | 349 | E | 349 | E | 349 | E | X |
| 395 | I | 324 | F | 350 | I | 350 | V | 350 | V | |
| 396 | I | 325 | I | 351 | I | 351 | I | 351 | I | X |
| 397 | Q | 326 | Q | 352 | E | 352 | Q | 352 | Q | |
| 398 | R | 327 | R | 353 | R | 353 | R | 353 | R | X |
| 399 | V | 328 | K | 354 | H | 354 | H | 354 | H | |
| 400 | G | | - | | - | | - | | | |
| 401 | C | | - | | - | | - | | | |
| 402 | M | | - | | - | | - | | | |
| 403 | M | | - | | - | | - | | | |
| 404 | K | | - | | - | | - | | | |
| 405 | D | | - | | - | | - | | | |
| 406 | E | | - | | - | | - | | | |
| 407 | E | | - | | - | | - | | | |
| 408 | G | | - | | - | | - | | | |
| 409 | K | | - | | - | | - | | | |
| 410 | A | | - | | - | | - | | | |
| 411 | Y | | - | | - | | - | | | |
| 412 | Q | | - | | - | | - | | | |
| 413 | S | | - | | - | | - | | | |
| 414 | G | | - | | - | | | | | |
| 415 | G | 329 | G | 355 | G | 355 | N | 355 | N | |
| 416 | C | 330 | D | 356 | S | 356 | H | 356 | H | |
| 417 | G | 331 | D | 357 | M | 357 | Q | 357 | Q | |
| 418 | G | 332 | G | 358 | G | 358 | G | 358 | G | X |
| 419 | F | 333 | F | 359 | F | 359 | F | 359 | F | X |
| 420 | G | 334 | G | 360 | G | 360 | G | 360 | G | X |
| 421 | K | 335 | E | 361 | K | 361 | A | 361 | A | |
| 422 | G | 336 | G | 362 | G | 362 | G | 362 | G | X |
| 423 | N | 337 | N | 363 | N | 363 | N | 363 | N | X |
| 424 | F | 338 | F | 364 | F | 364 | F | 364 | F | X |
| 425 | S | 339 | K | 365 | K | 365 | N | 365 | N | |
| 426 | E | 340 | A | 366 | A | 366 | S | 366 | S | |
| 427 | L | 341 | L | 367 | L | 367 | L | 367 | L | X |
| 428 | F | 342 | F | 368 | F | 368 | F | 368 | F | X |
| 429 | K | 343 | E | 369 | E | 369 | K | 369 | K | |
| 430 | S | 344 | S | 370 | A | 370 | A | 370 | A | |
| 431 | I | 345 | I | 371 | I | 371 | F | 371 | F | |
| 432 | E | 346 | E | 372 | E | 372 | E | 372 | E | X |
| 433 | E | 347 | R | 373 | R | 373 | E | 373 | E | |
| 434 | Y | 348 | D | 374 | E | 374 | E | 374 | E | |
| 435 | E | 349 | Q | 375 | Q | 375 | Q | 375 | Q | |
| 436 | K | 350 | V | 376 | E | 376 | N | 376 | A | |
| 437 | T | 351 | R | 377 | K | 377 | L | 377 | L | |
| 438 | L | 352 | R | 378 | R | 378 | R | 378 | R | |
| 439 | E | 353 | G | 379 | G | 379 | G | 379 | G | |
| 440 | A | 354 | V | 380 | N | 380 | N | 380 | N | |
| 441 | K | 355 | L | 381 | L | 381 | L | 381 | L | |
| 442 | Q | 356 | A | | | 382 | T | 382 | T | |
| 443 | L | 357 | T | | | 383 | N | 383 | D | |
| 444 | V | 358 | D | | | 384 | M | 384 | L | |
| 445 | G | | | | | 385 | E | 385 | E | |
| | | | | | | 386 | T | 386 | T | |
| | | | | | | 387 | N | 387 | N | |
| | | | | | | 388 | G | 388 | G | |
| | | | | | | 389 | V | 389 | V | |
| | | | | | | 390 | V | 390 | R | |
| | | | | | | 391 | P | 391 | S | |
| | | | | | | 392 | G | 392 | G | |
| | | | | | | 393 | M | 393 | M | |
| | | | | | | 394 | A | 394 | | |
| | | | | | | 395 | E | 395 | | |
| | | | | | | 396 | N | 396 | | |
| | | | | | | 397 | L | 397 | | |
| | | | | | | 398 | Y | 398 | | |
| | | | | | | 399 | F | 399 | | |
| | | | | | | 400 | Q | 400 | | |

A sequence analysis of more than 700 HPPD sequences from public data bases including sequences of HPPD proteins and predicted HPPD proteins such as from plants, mammals, fungi and bacteria was performed using ClustaIX. The alignment was corrected using the information of the available 3D structures. Identical amino acid sequences with different identifiers were included only once and some sequences with obvious sequence errors were excluded. The alignment also includes incomplete sequences. Table 2b shows the sequence alignment for a representative set of HPPD proteins and includes sequences from plants, bacteria, mammals.

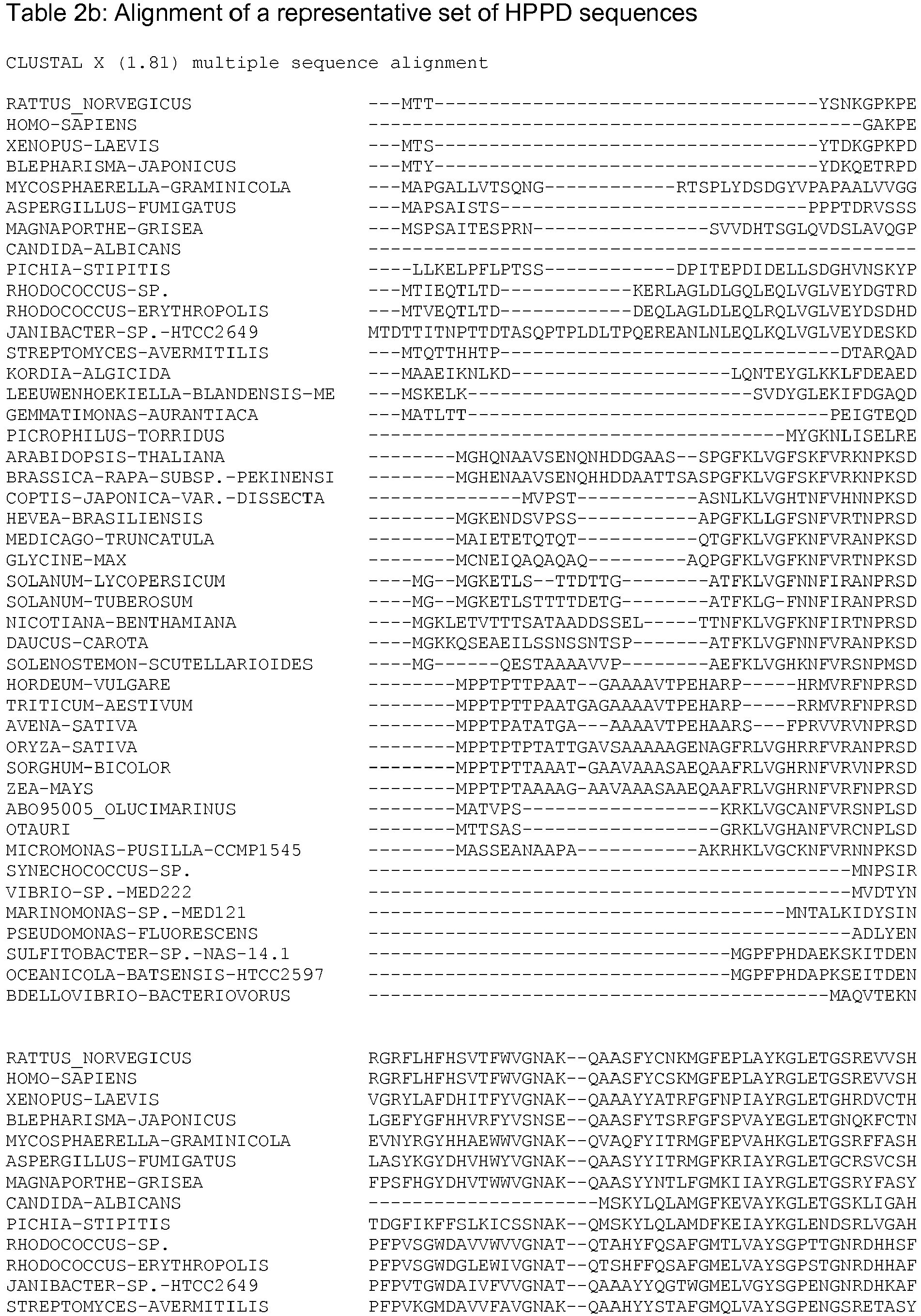

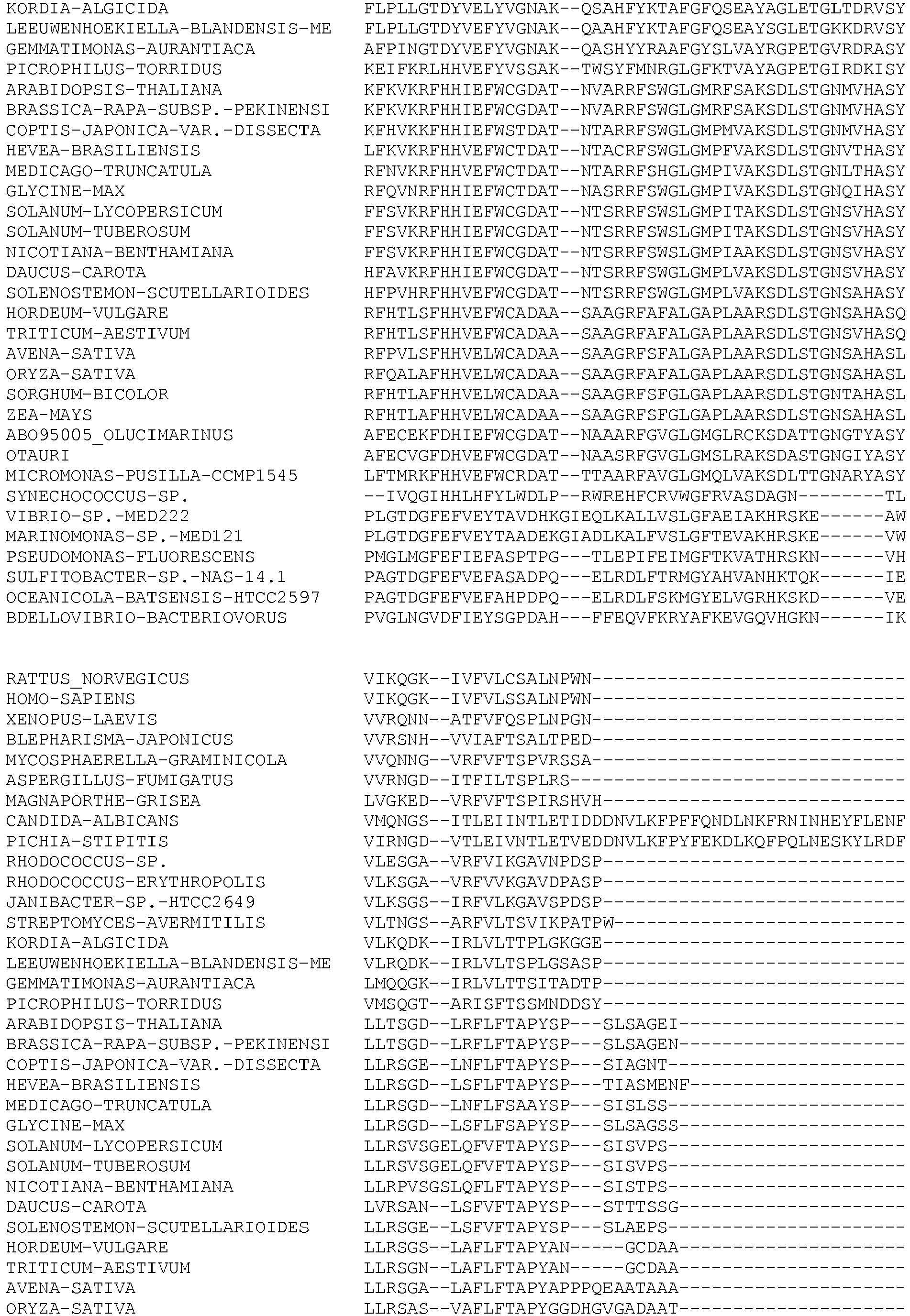

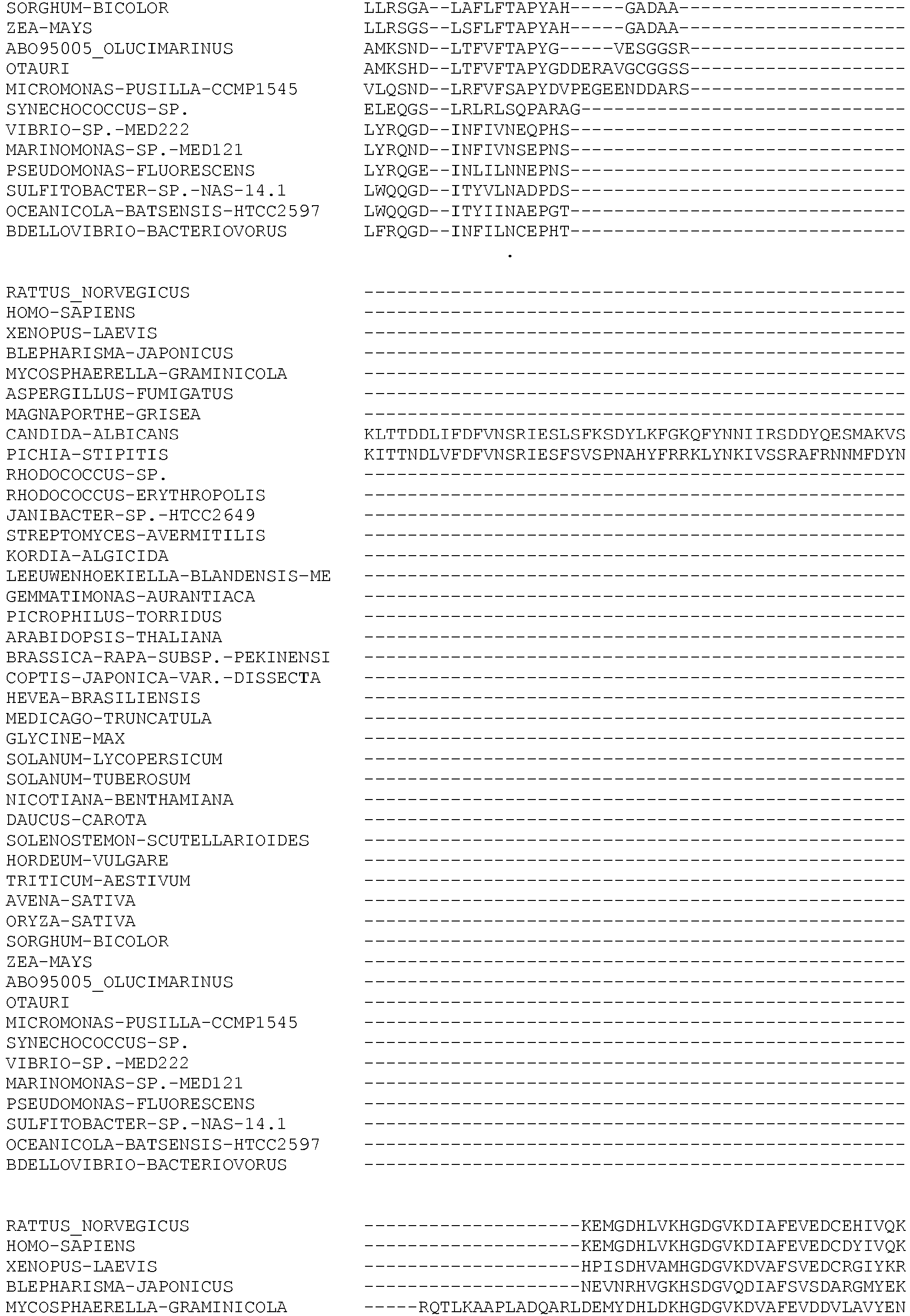

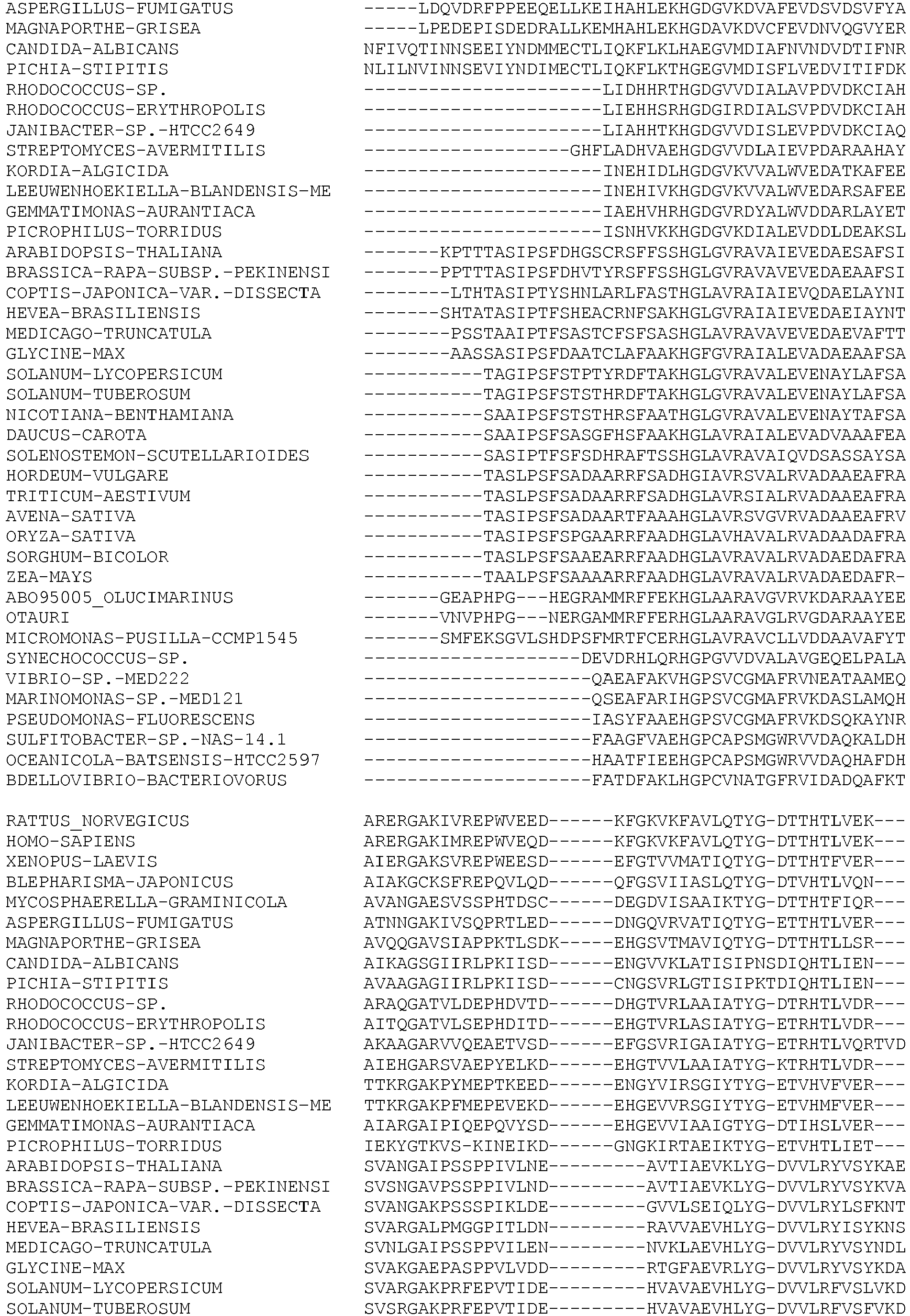

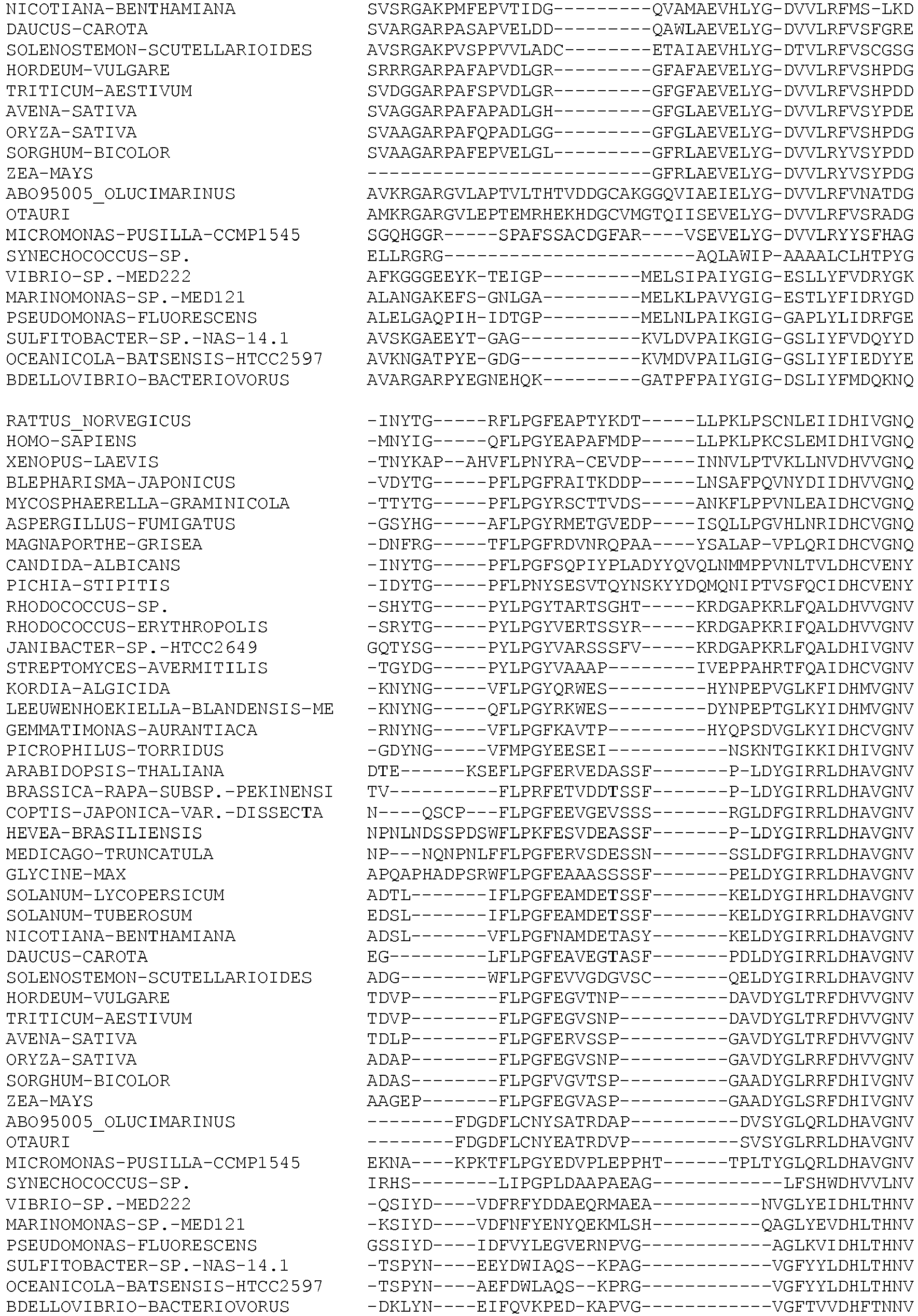

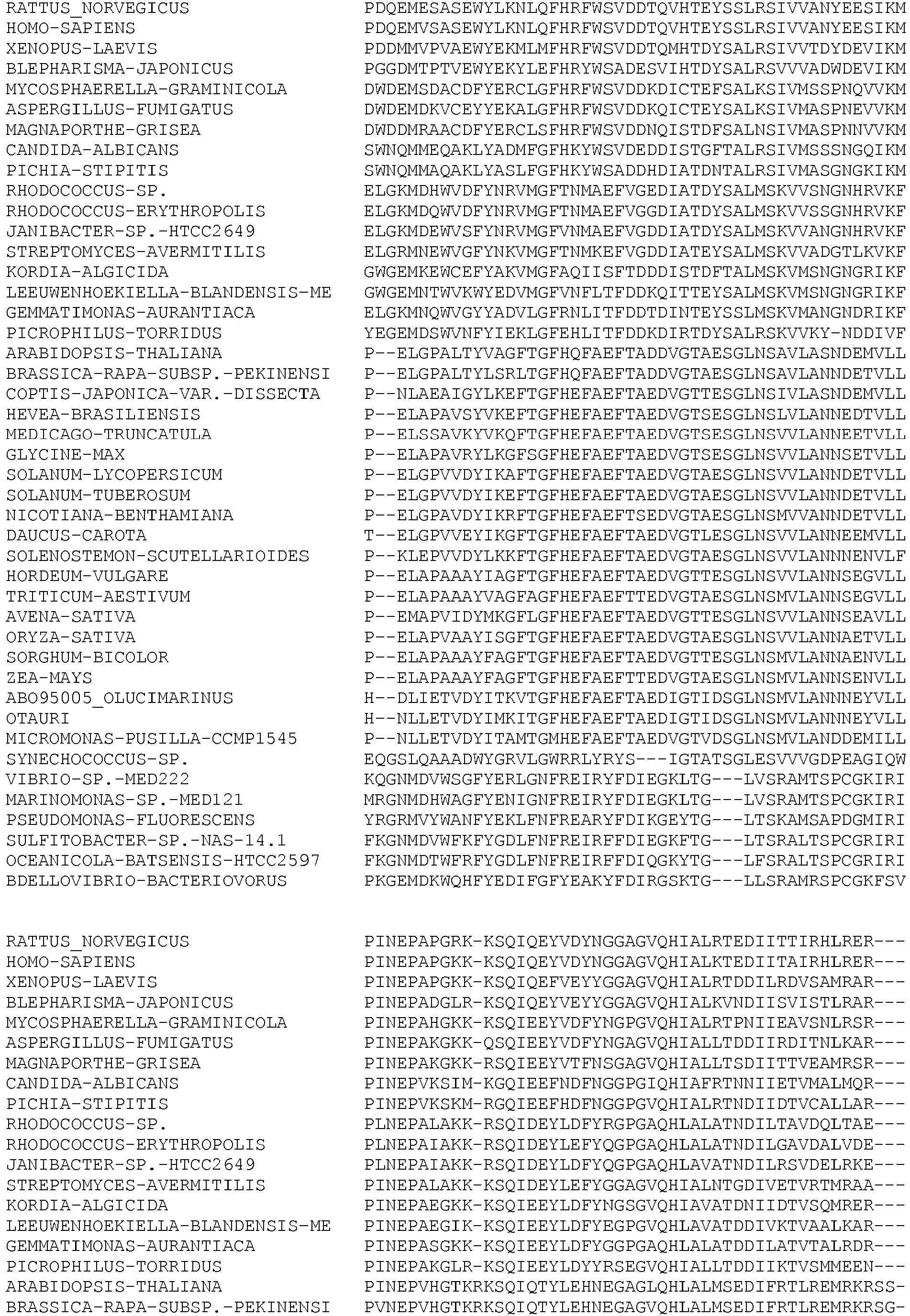

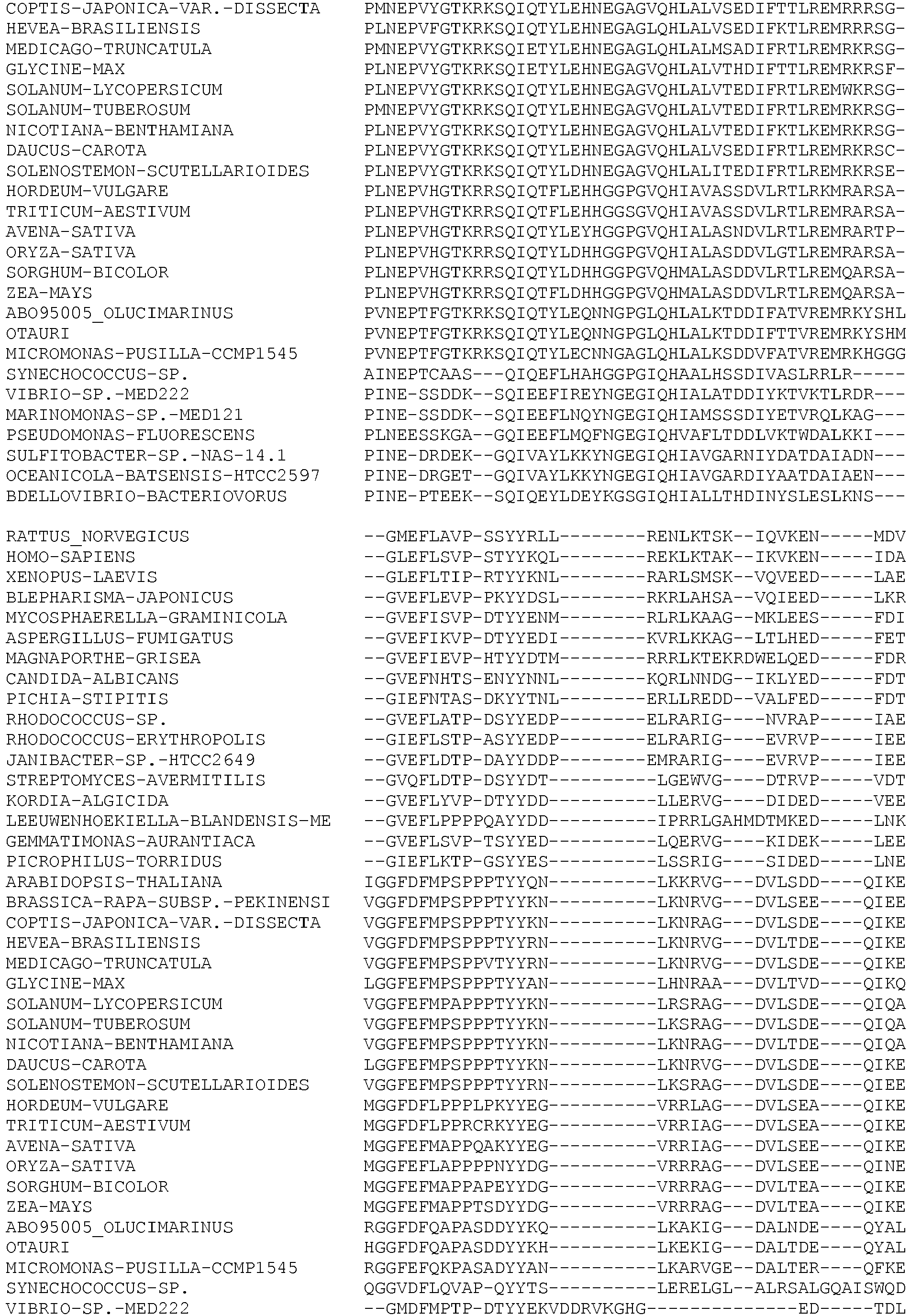

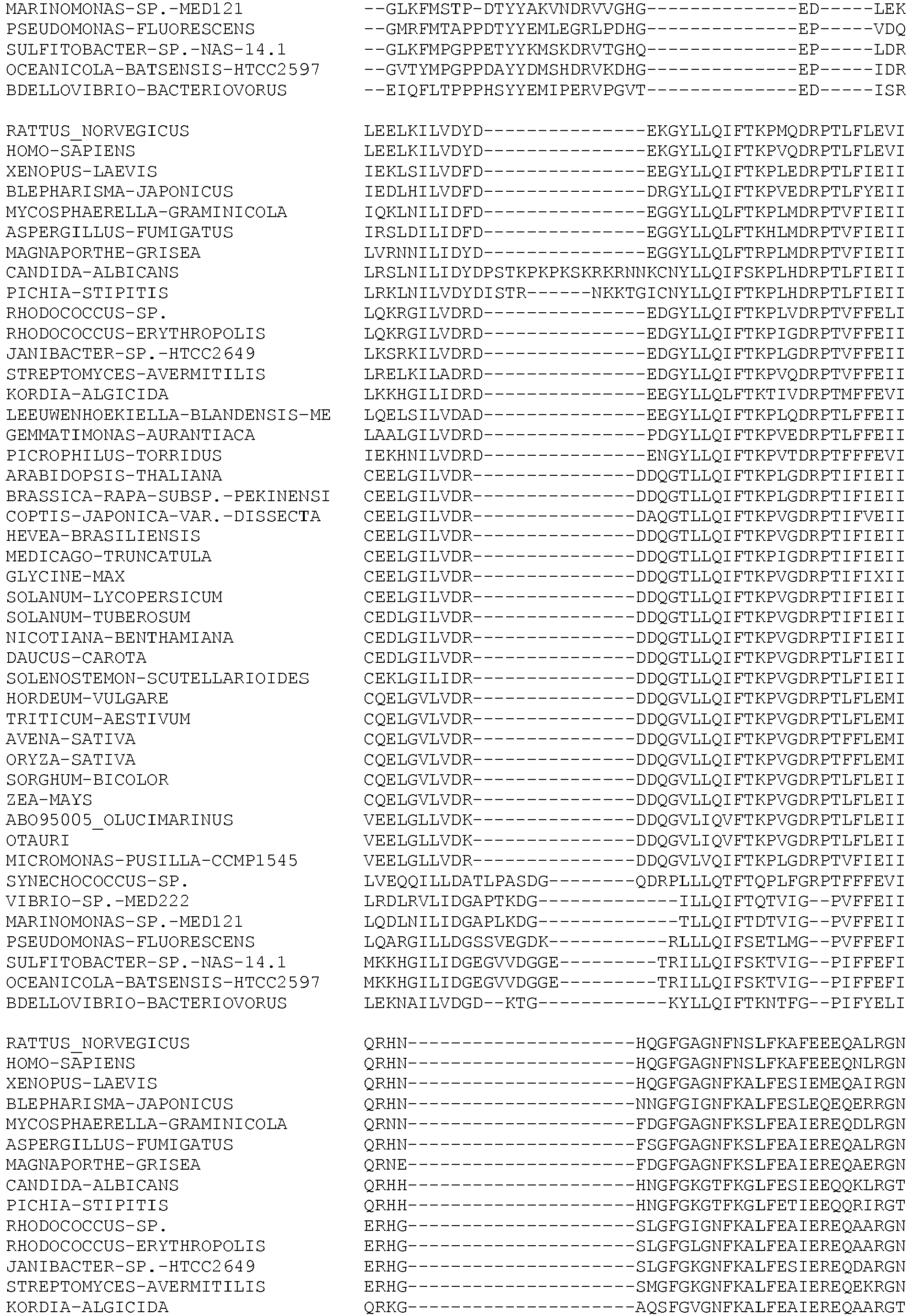

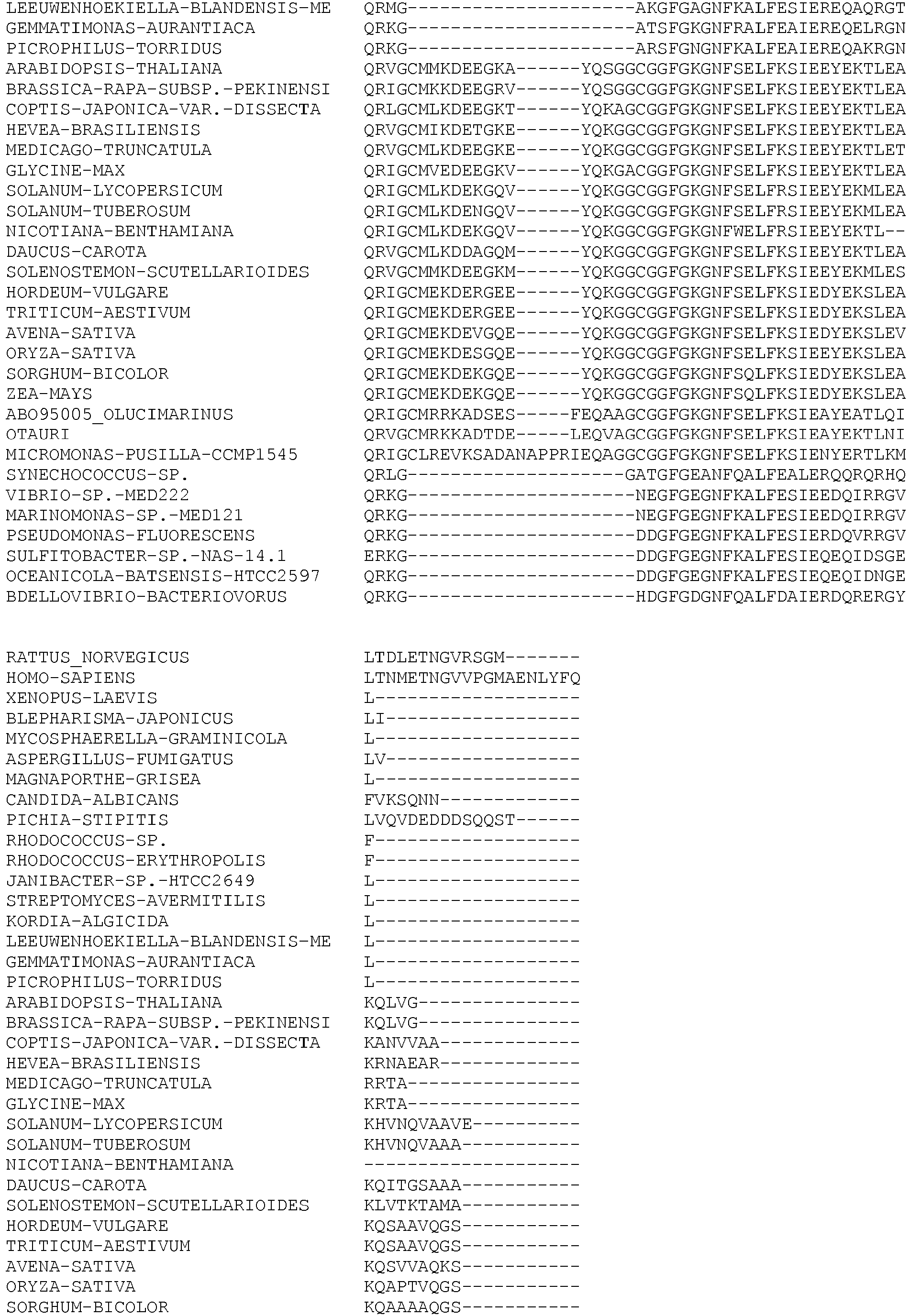

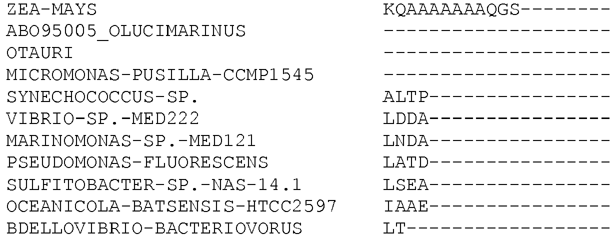

The overall sequence identity between individual full length HPPD sequences is in general quite low and is shown for the representative HHPD proteins in Table 3. Table 4a shows the sequence alignment of the binding pocket. In contrast, the sequence identity of the 36 amino acids forming the binding site is significantly higher which is shown for the representative HPPD proteins in Table 4b. In particular, the amino acids at 8 positions are strictly conserved in all species and illustrate that these amino acids have a key role (e.g. His226, His308, Glu394 binding the iron required for catalysis). These positions, with reference to the HPPD from Arabidopsis (SEQ ID No. 2) are His226, Ser267, Asn282, His308, Tyr342, Glu394, Gly420, Asn423 (Table 5a). A mutation of any of the amino acids at either of these positions will most likely lead to an inactive protein. The variability at other positions within the binding site is higher. Table 5b shows the 28 variable positions in the binding site and the amino acids which were identified at these positions using the sequence alignment. Some positions have only limited variability which reflects their role in the 3D environment. An example for this represents the position 269. All HPPD proteins have at this position either a Val, Ala or Thr. Looking at the 3D structures it seems that at this position a small apolar amino acid is required and a mutation to a polar amino acid such as Arg, His or Lys will disturb the protein structure locally. Another example represents position 379. Most sequences have a glutamine at position 379. However, there are also some bacterial sequences which have a histidine at this position. Looking at the 3D structure, it seems that only few amino acids are tolerated at this position. Gln379 in A.thaliana stabilizes via its H-bond donor the side chain conformation of the strictly conserved Glu394 which in turn interacts with the catalytic iron. In addition, with its H-bond acceptors Gln379 stabilizes the side chain conformation of the strictly conserved Asn423 which in turn interacts with the strictly conserved Tyr342. Only glutamine, asparagine and histidine have an H-bond donor and acceptor required for the stabilization of this particular 3D arrangement which very likely play a key role in the interaction of the C-terminal helix with the core of the HPPD protein. Limited variability is also seen at position 381 with either a phenylalanine or a tyrosine in all HPPD sequences. The aromatic ring stabilizes the binding of HPPD inhibitors and very likely also the binding of the substrate to the HPPD binding. However, the presence of the additional hydroxyl group in tyrosine compared to phenylalanine does not disturb the catalytic activity. The third category of positions includes those positions which display a very high natural variability. These positions may not be crucial for substrate binding and catalysis but influence inhibitor binding. These positions include adjacent positions in strand 248 to 255 and positions in the C-terminal helix 419-427. It can be assumed that the interaction of this particular strand and the C-terminal helix with the core of the protein plays a crucial role in inhibitor binding. Table 5b includes for each variable position those amino acids which have been identified in the sequence alignments using all known HPPD sequences.

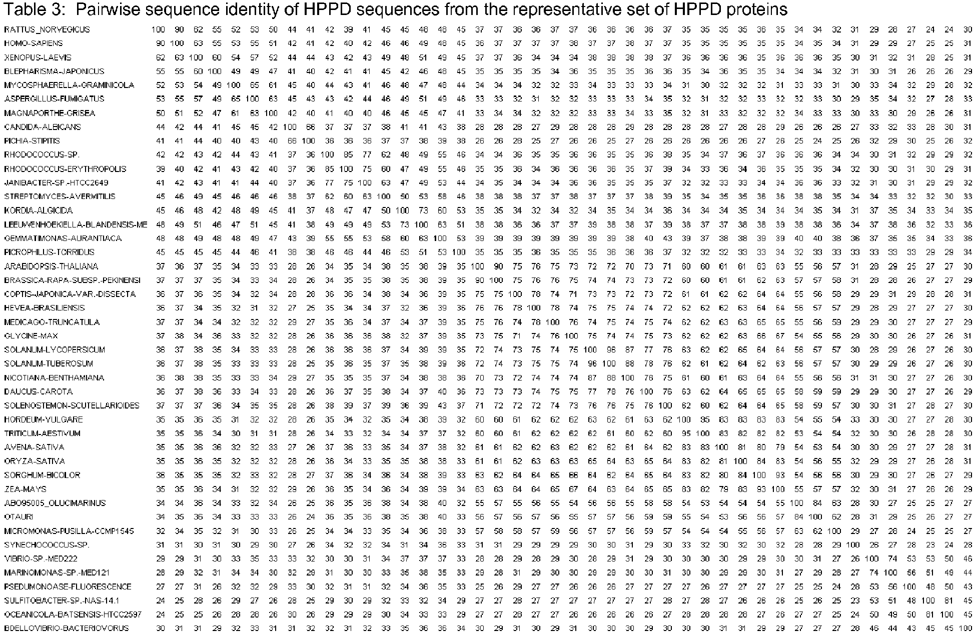

**Table 4a: Sequence alignment of amino acids forming the binding pocket in the representative set of HPPD sequences**

| | |
|---|---|
| HOMO-SAPIENS | HVHFWSVLSIVVAPNQIQHLYLQFFEFGAGNFNSLF |
| RATTUS_NORVEGICUS | HVHFWSVLSIVVAPNQIQHLYLQFFEFGAGNFNSLF |
| XENOPUS-LAEVIS | HVHFWSVLSIVVTPNQIQHLYLQFFEFGAGNFKALI |
| ASPERGILLUS-FUMIGATUS | HVHFWSVLSIVMAPNQIQHIYLQFFEFGAGNFKSLI |
| MAGNAPORTHE-GRISEA | HVHFWSVLSIVMAPNQIQHIYLQFFEFGAGNFKSLI |
| MYCOSPHAERELLA-GRAMINICOLA | HVHFWSVLSIVMSPNQIQHIYLQFFEFGAGNFKSLI |
| CANDIDA-ALBICANS | HVHYWSVLSIVMSPNQIQHNYLQFFEFGKGTFKGLI |
| PICHIA-STIPITIS | HVHYWSALSIVMAPNQIQHNYLQFFEFGKGTFKGLI |
| ARABIDOPSIS-THALIANA | HVHFAEFLSAVLAPNQIQHMYLQFFEFGKGNFSELI |
| BRASSICA-RAPA-SUBSP.-PEKINENSI | HVHFAEFLSAVLAPNQIQHMYLQFFEFGKGNFSELI |
| HEVEA-BRASILIENSIS | HVHFAEFLSLVLAPNQIQHMYLQFFEFGKGNFSELI |
| COPTIS-JAPONICA-VAR.-DISSECTA | HVHFAEFLSIVLAPNQIQHMYLQFFEFGKGNFSELI |
| SOLENOSTEMON-SCUTELLARIOIDES | HVHFAEFLSVVLAPNQIQHMYLQFFEFGKGNFSELI |
| AVENA-SATIVA | HVHFAEFLSVVLAPNQIQHMYLQFFEFGKGNFSELI |
| DAUCUS-CAROTA | HVHFAEFLSVVLAPNQIQHMYLQFFEFGKGNFSELI |
| SOLANUM-TUBEROSUM | HVHFAEFLSVVLAPNQIQHMYLQFFEFGKGNFSELI |
| SOLANUM-LYCOPERSICUM | HVHFAEFLSVVLAPNQIQHMYLQFFEFGKGNFSELI |
| MEDICAGO-TRUNCATULA | HVHFAEFLSVVLAPNQIQHMYLQFFEFGKGNFSELI |
| GLYCINE-MAX | HVHFAEFLSVVLAPNQIQHMYLQFFXFGKGNFSELI |
| HORDEUM-VULGARE | HVHFAEFLSVVLAPNQIQHLYLQFFEFGKGNFSELI |
| ORYZA-SATIVA | HVHFAEFLSVVLAPNQIQHLYLQFFEFGKGNFSELI |
| SORGHUM-BICOLOR | HVHFAEFLSMVLAPNQIQHMYLQFFEFGKGNFSQLI |
| ZEA-MAYS | HVHFAEFLSMVLAPNQIQHMYLQFFEFGKGNFSQLI |
| AB095005_OLUCIMARINUS | HVHFAEFLSMVLAPNQIQHQYLQFFEFGKGNFSELI |
| OTAURI | HVHFAEFLSMVLAPNQIQHQYLQFFEFGKGNFSELI |
| MICROMONAS-PUSILLA-CCMP1545 | HVHFAEFLSMVLAPNQIQHQYLQFFEFGKGNFSELI |
| TRITICUM-AESTIVUM | HVHFAEFLSMVLAPNQIQHLYLQFFEFGKGNFSELI |
| NICOTIANA-BENTHAMIANA | HVHFAEFLSMVVAPNQIQHMYLQFFEFGKGNFWELI |
| RHODOCOCCUS-SP. | HVTMAEFLSKVVSPNQIQHLYLQFFEFGIGNFKALI |
| RHODOCOCCUS-ERYTHROPOLIS | HVTMAEFLSKVVSPNQIQHLYLQFFEFGLGNFKALI |
| STREPTOMYCES-AVERMITILIS | HVTMKEFLSKVVAPNQIQHLYLQFFEFGKGNFKALI |
| JANIBACTER-SP.-HTCC2649 | HVVMAEFLSKVVAPNQIQHLYLQFFEFGKGNFKALI |
| KORDIA-ALGICIDA | HVAIISFLSKVMSPNQIQHLYLQFFEFGVGNFKALI |
| LEEUWENHOEKIELLA-BLANDENSIS-ME | HVVFLTFLSKVMSPNQIQHLYLQFFEFGAGNFKALI |
| PICROPHILUS-TORRIDUS | HVELITFLSKVVKPNQIQHLYLQFFEFGNGNFKALI |
| GEMMATIMONAS-AURANTIACA | HVRLITFLSKVMAPNQIQHLYLQFFEFGKGNFRALI |
| VIBRIO-SP.-MED222 | HTRIRYFLSRAMTPNQIQHMYLQFFEFGEGNFKALI |
| MARINOMONAS-SP.-MED121 | HTRIRYFLSRAMTPNQIQHMYLQFFEFGEGNFKALI |
| SULFITOBACTER-SP.-NAS-14.1 | HTRIRFFLSRALTPNQIQHMYLQFFEFGEGNFKALI |
| OCEANICOLA-BATSENSIS-HTCC2597 | HTRIRFFLSRALTPNQIQHMYLQFFEFGEGNFKALI |
| PSEUDOMONAS-FLUORESCENS | HTRARYFLSKAMSPNQIQHMYLQFFEFGEGNFKALI |
| BDELLOVIBRIO-BACTERIOVORUS | HTYAKYFLSRAMRPNQIQHLYLQFFEFGDGNFQALI |
| BLEPHARISMA-JAPONICUS | HVHYWSALSVVVAPNQIQHLYLQFFEFGIGNFKALL |
| SYNECHOCOCCUS-SP. | HVRLYRYLSVVVGANQIQHLYLQFFEFGEANFQALL |

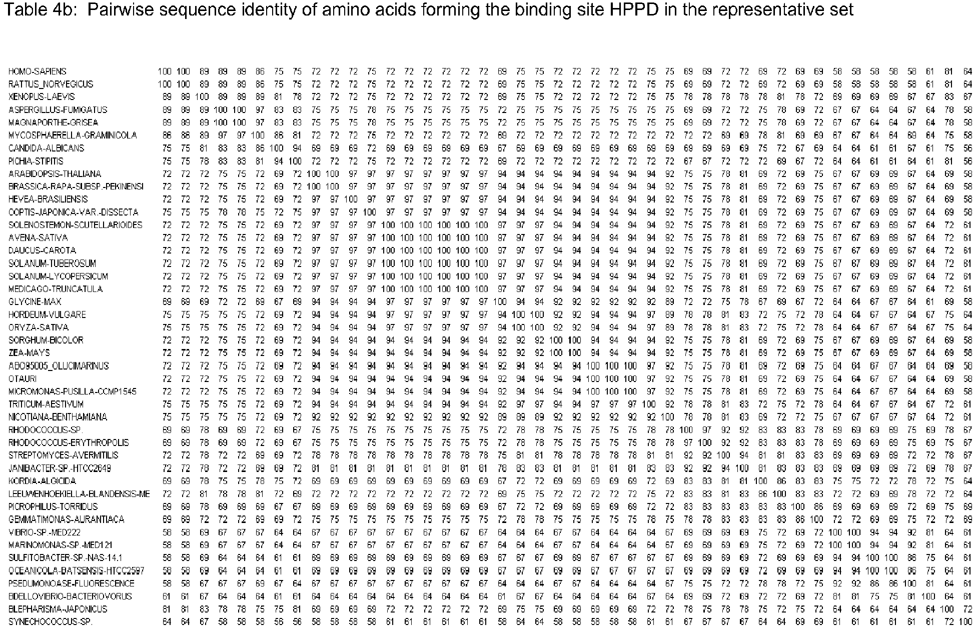

**Table 5a: Amino acid positions which are strictly conserved shown for the crystal structures**

| Arabidopsis thaliana | | Pseudomonas fluorescens | | Streptomyces avermitilis | | Homos apiens | | Rattus norvegicus | |
|---|---|---|---|---|---|---|---|---|---|
| Position | Amino Acid | Position | Amino Acid | Position | Amino Acid | Position | Amino Acid | Position | Amino Acid |
| 226 | H | 162 | H | 187 | H | 183 | H | 183 | H |
| 267 | S | 202 | S | 230 | S | 226 | S | 226 | S |
| 282 | N | 217 | N | 245 | N | 241 | N | 241 | N |
| 308 | H | 241 | H | 270 | H | 266 | H | 266 | H |
| 342 | Y | 271 | Y | 299 | Y | 295 | Y | 295 | Y |
| 394 | E | 323 | E | 349 | E | 349 | E | 349 | E |
| 420 | G | 334 | G | 360 | G | 360 | G | 360 | G |
| 423 | N | 337 | N | 363 | N | 363 | N | 363 | N |

**Table 5b: Amino acid position with the amino acids at this positions in the known crystal structures and the amino acids a these position within all HPPD sequences**

| Arabidopsis thaliana | | Pseudomonas fluorescens | | Streptomyces avermitilis | | Homo sapiens | | Rattus norvegicus | | Natural Variabaility |
|---|---|---|---|---|---|---|---|---|---|---|
| Pos. | Amino Acid | Pos. | Amino Acid | Pos. | Amino Acid | Pos. | Amino Acid | Pos. | Amino Acid | Amino Acid |
| 228 | V | 164 | T | 189 | V | 185 | V | 185 | V | VTCAG |
| 248 | H | 186 | R | 211 | T | 207 | H | 207 | H | HRQTKEYLAGSN |
| 250 | F | 188 | A | 213 | M | 209 | F | 209 | F | FYILVAQEDGTSMRK |
| 251 | A | 189 | R | 214 | K | 210 | W | 210 | W | AWILSRKHDEPGNY |
| 252 | E | 190 | Y | 215 | E | 211 | S | 211 | S | ESTYFHQNGLMVILIR |
| 253 | F | 191 | F | 216 | F | 212 | V | 212 | V | FVIALWMQHY |
| 265 | L | 200 | L | 228 | L | 224 | L | 224 | L | LMVIA |
| 268 | A | 203 | K | 231 | K | 227 | I | 227 | I | AVLMIKRQY |
| 269 | V | 204 | A | 232 | V | 228 | V | 228 | V | VAT |
| 270 | L | 205 | M | 233 | V | 229 | V | 229 | V | LVMIA |
| 271 | A | 206 | S | 234 | A | 230 | A | 230 | A | ASTVRKELIMHG |
| 280 | P | 215 | P | 243 | P | 239 | P | 239 | P | PAVTNI |
| 293 | Q | 226 | Q | 255 | Q | 251 | Q | 251 | Q | QLAVRSGVFM |
| 294 | I | 227 | I | 256 | I | 252 | I | 252 | I | IMVTASP |
| 307 | Q | 240 | Q | 269 | Q | 265 | Q | 265 | Q | QHN |
| 335 | M | 264 | M | 293 | L | 289 | L | 289 | L | MLIN |
| 368 | L | 295 | L | 323 | L | 323 | L | 323 | L | LM |
| 379 | Q | 310 | Q | 334 | Q | 334 | Q | 334 | Q | QH |
| 381 | F | 312 | F | 336 | F | 336 | F | 336 | F | FY |
| 392 | F | 321 | F | 347 | F | 347 | F | 347 | F | FS |
| 419 | F | 333 | F | 359 | F | 359 | F | 359 | F | FY |
| 421 | K | 335 | E | 361 | K | 361 | A | 361 | A | KQAILVNDEGS |
| 422 | G | 336 | G | 362 | G | 362 | G | 362 | G | GAPVTM |
| 424 | F | 338 | F | 364 | F | 364 | F | 364 | F | FAVIL |
| 425 | S | 339 | K | 365 | K | 365 | N | 365 | N | SNKGRAPSIKQR |
| 426 | E | 340 | A | 366 | A | 366 | S | 366 | S | EQSAIVFT |
| 427 | L | 341 | L | 367 | L | 367 | L | 367 | L | LR |
| 431 | I | 345 | I | 371 | I | 371 | F | 371 | F | IFVMLQR |

**Table 5c: Amino acid position with the amino acids at this positions in the known crystal structures and most common amino acids a these position within all HPPD sequences**

| Arabidopsis thaliana | | Pseudomonas fluorescens | | Streptomyces avermitilis | | Homo sapiens | | Rattus norvegicus | | Most common |
|---|---|---|---|---|---|---|---|---|---|---|
| Pos. | Amino Acid | Pos. | Amino Acid | Pos. | Amino Acid | Pos. | Amino Acid | Pos. | Amino Acid | Amino Acid |
| 228 | V | 164 | T | 189 | V | 185 | V | 185 | V | VTCA |
| 248 | H | 186 | R | 211 | T | 207 | H | 207 | H | HRQTKEYA |
| 250 | F | 188 | A | 213 | M | 209 | F | 209 | F | FYILVAEDTM |
| 251 | A | 189 | R | 214 | K | 210 | W | 210 | W | AWILRKHDEPY |
| 252 | E | 190 | Y | 215 | E | 211 | S | 211 | S | ESTRY |
| 253 | F | 191 | F | 216 | F | 212 | V | 212 | V | FVIALWY |
| 265 | L | 200 | L | 228 | L | 224 | L | 224 | L | LMVA |
| 268 | A | 203 | K | 231 | K | 227 | I | 227 | I | AVLMIKRQY |
| 269 | V | 204 | A | 232 | V | 228 | V | 228 | V | VA |
| 270 | L | 205 | M | 233 | V | 229 | V | 229 | V | LVMI |
| 271 | A | 206 | S | 234 | A | 230 | A | 230 | A | ASTVREGK |
| 280 | P | 215 | P | 243 | P | 239 | P | 239 | P | PAVT |
| 293 | Q | 226 | Q | 255 | Q | 251 | Q | 251 | Q | QLA |
| 294 | I | 227 | I | 256 | I | 252 | I | 252 | I | IMVA |
| 307 | Q | 240 | Q | 269 | Q | 265 | Q | 265 | Q | QHN |
| 335 | M | 264 | M | 293 | L | 289 | L | 289 | L | MLI |
| 368 | L | 295 | L | 323 | L | 323 | L | 323 | L | LM |
| 379 | Q | 310 | Q | 334 | Q | 334 | Q | 334 | Q | QH |
| 381 | F | 312 | F | 336 | F | 336 | F | 336 | F | FY |
| 392 | F | 321 | F | 347 | F | 347 | F | 347 | F | F |
| 419 | F | 333 | F | 359 | F | 359 | F | 359 | F | FY |
| 421 | K | 335 | E | 361 | K | 361 | A | 361 | A | KQAIVLDE |
| 422 | G | 336 | G | 362 | G | 362 | G | 362 | G | GA |
| 424 | F | 338 | F | 364 | F | 364 | F | 364 | F | FAVL |
| 425 | S | 339 | K | 365 | K | 365 | N | 365 | N | SNKRAIQ |
| 426 | E | 340 | A | 366 | A | 366 | S | 366 | S | ESAIVF |
| 427 | L | 341 | L | 367 | L | 367 | L | 367 | L | LR |
| 431 | I | 345 | I | 371 | I | 371 | F | 371 | F | IFVMQRL |

Not all amino acids occurring at the variable positions have the same likelihood to be present in an active protein. In some cases most sequences have the same amino acids at a particular position while other amino acids are present at that position in only few HPPD sequences. An example is position 392. Most sequences have at a corresponding position a phenylalanine while few i.e. the burkholderia sequences have a serine. Whereas in some rare cases, the rare amino acids may be the result of a sequencing error, in most other cases the resulting protein is active. Table 5c shows a list with the most common amino acids at the variable positions.

The influence of the amino acids at the variable positions is different. Some of these positions are crucial for catalysis and/or the interaction of HPPD with an inhibitor while others may have less impact. For instances changes at positions 269 and 280 which are in direct contact with the inhibitor and the substrate will very likely have a great impact upon catalysis and inhibitor binding. Also modifications at positions involved in the helix movement induced by inhibitor or substrate binding such as positions 252, 421 and 422 are likely to have high impact on inhibitor binding. In contrast modifications at positions such as 293 are less likely to influence inhibitor binding because this position is quite distant from the active site. Table 6a shows the position with very high impact and Table 6b with high impact on activity and inhibitor binding.

From these observations, the present inventors drew the conclusion that amino acids are preferred in the binding sites which naturally occur at the corresponding positions. This means, that those modifications which exchange a naturally occurring amino acid into another are likely providing a catalytically active HPPD protein which may exert a modified or even increased tolerance to HPPD herbicide inhibitors. Even more promising are those mutant proteins which have the amino acid most often found in nature at the variable position chosen from Table 5c.

**Table 6a: Amino acid positions with high priority shown for the x-ray structures**

| Arabidopsis thaliana | | Pseudomonas fluorescens | | Streptomyces avermitilis | | Homo sapiens | | Rattus norvegicus | |
|---|---|---|---|---|---|---|---|---|---|
| Pos. | Amino Acid | Pos. | Amino Acid | Pos. | Amino Acid | Pos. | Amino Acid | Pos. | Amino Acid |
| 228 | V | 164 | T | 189 | V | 185 | V | 185 | V |
| 250 | F | 188 | A | 213 | M | 209 | F | 209 | F |
| 251 | A | 189 | R | 214 | K | 210 | W | 210 | W |
| 252 | E | 190 | Y | 215 | E | 211 | S | 211 | S |
| 253 | F | 191 | F | 216 | F | 212 | V | 212 | V |
| 265 | L | 200 | L | 228 | L | 224 | L | 224 | L |
| 268 | A | 203 | K | 231 | K | 227 | I | 227 | I |
| 269 | V | 204 | A | 232 | V | 228 | V | 228 | V |
| 270 | L | 205 | M | 233 | V | 229 | V | 229 | V |
| 271 | A | 206 | S | 234 | A | 230 | A | 230 | A |
| 280 | P | 215 | P | 243 | P | 239 | P | 239 | P |
| 307 | Q | 240 | Q | 269 | Q | 265 | Q | 265 | Q |
| 335 | M | 264 | M | 293 | L | 289 | L | 289 | L |
| 368 | L | 295 | L | 323 | L | 323 | L | 323 | L |
| 379 | Q | 310 | Q | 334 | Q | 334 | Q | 334 | Q |
| 392 | F | 321 | F | 347 | F | 347 | F | 347 | F |
| 421 | K | 335 | E | 361 | K | 361 | A | 361 | A |
| 422 | G | 336 | G | 362 | G | 362 | G | 362 | G |
| 426 | E | 340 | A | 366 | A | 366 | S | 366 | S |
| 427 | L | 341 | L | 367 | L | 367 | L | 367 | L |

**Table 6b: Amino acid positions with very high priority shown for the x-ray structures**

| Arabidopsis thaliana | | Pseudomonas fluorescens | | Streptomyces avermitilis | | Homo sapiens | | Rattus norvegicus | |
|---|---|---|---|---|---|---|---|---|---|
| Pos. | Amino Acid | Pos. | Amino Acid | Pos. | Amino Acid | Pos. | Amino Acid | Pos. | Amino Acid |
| 252 | E | 190 | Y | 215 | E | 211 | S | 211 | S |
| 269 | V | 204 | A | 232 | V | 228 | V | 228 | V |
| 280 | P | 215 | P | 243 | P | 239 | P | 239 | P |
| 335 | M | 264 | M | 293 | L | 289 | L | 289 | L |
| 368 | L | 295 | L | 323 | L | 323 | L | 323 | L |
| 421 | K | 335 | E | 361 | K | 361 | A | 361 | A |
| 422 | G | 336 | G | 362 | G | 362 | G | 362 | G |

In another embodiment, in the isolated nucleic acid of the invention as defined above, said at least on amino acid is selected from
a) Ala, Asp, Glu, Phe, Thr, Val or Tyr at a position in an HPPD protein, said position corresponding to position 250 of the amino acid sequence of SEQ ID No. 2;
b) Ala, Asp, Glu, Ile, Lys, Leu, Pro or Arg at a position in an HPPD protein, said position corresponding to position 251 of the amino acid sequence of SEQ ID No. 2;
c) Glu, Thr, Arg, Ser or Tyr at a position in an HPPD protein, said position corresponding to position 252 of the amino acid sequence of SEQ ID No. 2;
d) Ala, Phe, Val or Trp at a position in an HPPD protein, said position corresponding to position 253 of the amino acid sequence of SEQ ID No. 2;
e) Leu or Val at a position in an HPPD protein, said position corresponding to position 265 of the amino acid sequence of SEQ ID No. 2;
f) Leu, Gln, Arg, Val or Tyr at a position in an HPPD protein, said position corresponding to position 268 of the amino acid sequence of SEQ ID NO: 2;
g) Ala or Val at a position in an HPPD protein, said position corresponding to position 269 of the amino acid sequence of SEQ ID No. 2;
h) Ala, Pro, Thr or Val at a position in an HPPD protein, said position corresponding to position 280 of the amino acid sequence of SEQ ID No. 2;
i) Gln at a position in an HPPD protein, said position corresponding to position 307 of the amino acid sequence of SEQ ID No. 2;
j) Ile or Met at a position in an HPPD protein, said position corresponding to position 335 of the amino acid sequence of SEQ ID No. 2;
k) Leu at a position in an HPPD protein, said position corresponding to position 368 of the amino acid sequence of SEQ ID No. 2;
l) Phe at a position in an HPPD protein, said position corresponding to position 392 of the amino acid sequence of SEQ ID No. 2;
m) Asp, Lys, or Asn at a position in an HPPD protein, said position corresponding to position 421 of the amino acid sequence of SEQ ID No. 2;
n) Ala or Gly at a position in an HPPD protein, said position corresponding to position 422 of the amino acid sequence of SEQ ID No. 2;
o) Ile, Arg or Ser at a position in an HPPD protein, said position corresponding to position 425 of the amino acid sequence of SEQ ID No. 2
p) Glu, Phe or Val at a position in an HPPD protein, said position corresponding to position 426 of the amino acid sequence of SEQ ID No. 2;
q) Ile, Met, Gln or Val or Phe at a position in an HPPD protein, said position corresponding to position 431 of the amino acid sequence of SEQ ID No. 2;
r) Ala, Cys, Thr or Val at a position in an HPPD protein, said position corresponding to position 228 of the amino acid sequence of SEQ ID No. 2;
s) Ala, Glu, His, Lys, Gln, Arg, Thr or Tyr at a position in an HPPD protein, said position corresponding to position 248 of the amino acid sequence of SEQ ID No. 2;
t) Ile, Leu, Met or Val at a position in an HPPD protein, said position corresponding to position 270 of the amino acid sequence of SEQ ID No. 2;
u) Ala, Glu, Lys, Arg, Ser, Thr or Val at a position in an HPPD protein, said position corresponding to position 271 of the amino acid sequence of SEQ ID No. 2;
v) His or Gln at a position in an HPPD protein, said position corresponding to position 379 of the amino acid sequence of SEQ ID No. 2;
w) Leu or Arg at a position in an HPPD protein, said position corresponding to
   position 427 of the amino acid sequence of SEQ ID No. 2.

In an alternative embodiment of the nucleic acid of the invention, said at least one amino acid is selected from
a) Glu, Thr, Arg, Ser or Tyr at a position in an HPPD protein, said position corresponding to position 252 of the amino acid sequence of SEQ ID No. 2;
b) Ala or Val at a position in an HPPD protein, said position corresponding to position 269 of the amino acid sequence of SEQ ID No. 2;
c) Ala, Pro, Thr or Val at a position in an HPPD protein, said position corresponding to position 280 of the amino acid sequence of SEQ ID No. 2;
d) Ile or Met at a position in an HPPD protein, said position corresponding to position 335 of the amino acid sequence of SEQ ID No. 2;
e) Leu at a position in an HPPD protein, said position corresponding to position 368 of the amino acid sequence of SEQ ID No. 2;
f) Asp" Asn or Lys at a position in an HPPD protein, said position corresponding to position 421 of the amino acid sequence of SEQ ID No. 2;
g) Ala or Gly at a position in an HPPD protein, said position corresponding to position 422 of the amino acid sequence of SEQ ID No. 2;
h) Ala, Cys, Thr or Val at a position in an HPPD protein, said position corresponding to position 228 of the amino acid sequence of SEQ ID No. 2;
i) Ala, Glu, His, Lys, Gln, Arg, Thr or Tyr at a position in an HPPD protein, said position corresponding to position 248 of the amino acid sequence of SEQ ID No. 2;
j) Ile, Leu, Met or Val at a position in an HPPD protein, said position corresponding to position 270 of the amino acid sequence of SEQ ID No. 2;
k) Ala, Glu, Lys, Arg, Ser, Thr or Val at a position in an HPPD protein, said position corresponding to position 271 of the amino acid sequence of SEQ ID No. 2;
l) His or Gln at a position in an HPPD protein, said position corresponding to position 379 of the amino acid sequence of SEQ ID No. 2;
m) Leu or Arg at a position in an HPPD protein, said position corresponding to position 427 of the amino acid sequence of SEQ ID No. 2.

Various sequences of HPPD proteins or predicted HPPD proteins are known in the art. These include the HPPD sequences of Streptomyces avermitilis (Genebank SAV11864), Daucus carota (Genebank DCU 87257), Arabidopsis thaliana (Genebank AF047834), Mycosphaerella graminicola (Genebank AF038152), oryza sativa / rice [BAD26248], Zea mays / corn [ACN36372], avena sativa [ABZ23427], Pseudomonas fluorescens [ABF50055], Synechococcus sp. [YP_473959], Blepharisma japonicum [BAF91881], Rhodococcus RHA1 sp. ro0240 [YP_702005], Rhodococcus RHA1 sp. ro0341 [YP_703002], Picrophilus torridus [YP_024147], Kordia algicida [ZP_02161490], Sorghum bicolor [XP_002453359], Triticum aestivum / wheat [AAZ67144], or Hordeum vulgare / barley [O48604].

The sequence of the HPPD protein taken as a starting point may be any amino acid sequence encoding a catalytically active HPPD protein. In one embodiment of the isolated nucleic acid of the invention, said HPPD protein comprises the amino acid sequence of SEQ ID No. 4 [Oryza sativa], wherein the resulting amino acid sequence comprises at least one amino acid selected from
a) Ala, Asp, Glu, Gly, Lys, Gln, Arg, Ser, Thr, Val, Tyr, Ile, Leu or Met at position 247 of the amino acid sequence of SEQ ID No. 4 (corresponding to position 250 of SEQ ID No. 2);
b) Asp, Glu, Ile, Lys, Leu, Asn, Pro, Arg, Ser, Gly, His or Tyr at position 248 of the amino acid sequence of SEQ ID No. 4 (corresponding to position 251 of SEQ ID No. 2);
c) Phe, Gly, His, Ile, Leu, Met, Asn, Gln, Arg, Ser, Val, Tyr or Thr at position 249 of the amino acid sequence of SEQ ID No. 4 (corresponding to position 252 of SEQ ID No. 2) ;
d) Ala, His, Gln, Val, Trp, Ile, Leu or Met at position 250 of the amino acid sequence of SEQ ID No. 4 (corresponding to position 253 of SEQ ID No. 2);
e) Val or Met at position 262 of the amino acid sequence of SEQ ID No. 4 (corresponding to position 265 of SEQ ID No. 2);
f) Leu, Gln, Arg, Tyr, Ala, Ile, Lys or Met at position 265 of the amino acid sequence of SEQ ID No. 4 (corresponding to position 268 of SEQ ID No. 2);
g) Ala or Thr at position 266 of the amino acid sequence of SEQ ID No. 4 (corresponding to position 269 of SEQ ID No. 2);
h) Ala, Ile, Asn, Thr or Val at position 277 of the amino acid sequence of SEQ ID No. 4 (corresponding to position 280 of SEQ ID No. 2);
i) Leu, Val, Ala, Phe, Gly, Met, Arg or Ser at position 290 of the amino acid sequence of SEQ ID No. 4 (corresponding to position 293 of SEQ ID No. 2);
j) Met, Ala, Pro, Ser, Thr or Val at position 291 of the amino acid sequence of SEQ ID No. 4 (corresponding to position 294 of SEQ ID No. 2);
k) Ile, Met or Asn at position 332 of the amino acid sequence of SEQ ID No. 4 (corresponding to position 335 of SEQ ID No. 2);
l) Met at position 365 of the amino acid sequence of SEQ ID No. 4 (corresponding to position 368 of SEQ ID No. 2);
m) Tyr at position 378 of the amino acid sequence of SEQ ID No. 4 (corresponding to position 381 of SEQ ID No. 2);
n) Ser at position 389 of the amino acid sequence of SEQ ID No. 4 (corresponding to position 392 of SEQ ID No. 2);
o) Tyr at position 416 of the amino acid sequence of SEQ ID No. 4 (corresponding to position 419 of SEQ ID No. 2);
p) Asp, Phe, Asn, Ala, Cys, Glu, Gly, His, Ile, Leu, Met, Gln, Ser, Thr or Val at position 418 of the amino acid sequence of SEQ ID No. 4 (corresponding to position 421 of SEQ ID No. 2);
q) Ala, Met, Pro or Thr at position 419 of the amino acid sequence of SEQ ID No. 4 (corresponding to position 422 of SEQ ID No. 2);
r) Ala, Ile or Val at position 421 of the amino acid sequence of SEQ ID No. 4 (corresponding to position 424 of SEQ ID No. 2);
s) Ile, Pro, Arg, Ala, Gly, Lys, Asn or Gln at position 422 of the amino acid sequence of SEQ ID No. 4 (corresponding to position 425 of SEQ ID No. 2);
t) Phe, Thr, Val, Ala, Gln or Ser at position 423 of the amino acid sequence of SEQ ID No. 4 (corresponding to position 426 of SEQ ID No. 2);
u) Met, Gln, Val, Phe or Leu at position 428 of the amino acid sequence of SEQ ID No. 4 (corresponding to position 431 of SEQ ID No. 2);
v) Phe at position 431 of the amino acid sequence of SEQ ID No. 4 (corresponding to position 434 of SEQ ID No. 2);
w) Ala, Cys, Gly or Thr at position 225 of the amino acid sequence of SEQ ID No. 4 (corresponding to position 228 of SEQ ID No. 2);
x) Ala, Glu, Gly, Lys, Leu, Asn, Gln, Arg, Ser, Thr or Tyr at position 245 of the amino acid sequence of SEQ ID No. 4 (corresponding to position 248 of SEQ ID No. 2);
y) Ala, Ile, Met or Val at position 267 of the amino acid sequence of SEQ ID No. 4 (corresponding to position 270 of SEQ ID No. 2);
z) Glu, His, Ile, Lys, Leu, Met, Arg, Ser, Thr or Val at position 268 of the amino acid sequence of SEQ ID No. 4 (corresponding to position 271 of SEQ ID No. 2);

   aa)His at position 376 of the amino acid sequence of SEQ ID No. 4 (corresponding to position 379 of SEQ ID No. 2);
   bb)Arg at a position in an HPPD protein, said position corresponding to position 424 of the amino acid sequence of SEQ ID No. 4 (corresponding to position 427 of SEQ ID No. 2).

In another embodiment of the isolated nucleic acid of the invention, said HPPD protein comprises the amino acid sequence of SEQ ID No. 6 [Zea mays], wherein the resulting amino acid sequence comprises at least one amino acid selected from
a) Ala, Asp, Glu, Gly, Lys, Gln, Arg, Ser, Thr, Val, Tyr, Ile, Leu or Met at position 224 of the amino acid sequence of SEQ ID No. 6 (corresponding to position 250 of SEQ ID No. 2);
b) Asp, Glu, Ile, Lys, Leu, Asn, Pro, Arg, Ser, Gly, His or Tyr at position 225 of the amino acid sequence of SEQ ID No. 6 (corresponding to position 251 of SEQ ID No. 2);
c) Phe, Gly, His, Ile, Leu, Met, Asn, Gln, Arg, Ser, Val, Tyr or Thr at position 226 of the amino acid sequence of SEQ ID No. 6 (corresponding to position 252 of SEQ ID No. 2);
d) Ala, His, Gln, Val, Trp, Ile, Leu or Met at position 227 of the amino acid sequence of SEQ ID No. 6 (corresponding to position 253 of SEQ ID No. 2);
e) Val or Met at position 239 of the amino acid sequence of SEQ ID No. 6 (corresponding to position 265 of SEQ ID No. 2);
f) Leu, Gln, Arg, Val, Tyr, Ala, Ile or Lys at position 242 of the amino acid sequence of SEQ ID No. 6 (corresponding to position 268 of SEQ ID No. 2);
g) Ala or Thr at position 243 of the amino acid sequence of SEQ ID No. 6 (corresponding to position 269 of SEQ ID No. 2);
h) Ala, Ile, Asn, Thr or Val at position 254 of the amino acid sequence of SEQ ID No. 6 (corresponding to position 280 of SEQ ID No. 2);
i) Leu, Val, Ala, Phe, Gly, Met, Arg or Ser at position 267 of the amino acid sequence of SEQ ID No. 6 (corresponding to position 293 of SEQ ID No. 2);
j) Met at position 268 of the amino acid sequence of SEQ ID No. 6 (corresponding to position 294 of SEQ ID No. 2);
k) His or Asn at position 281 of the amino acid sequence of SEQ ID No. 6 (corresponding to position 307 of SEQ ID No. 2);
l) Ile, Asn or Leu at position 309 of the amino acid sequence of SEQ ID No. 6 (corresponding to position 335 of SEQ ID No. 2);
m) Met at position 342 of the amino acid sequence of SEQ ID No. 6 (corresponding to position 368 of SEQ ID No. 2);
n) Tyr at position 355 of the amino acid sequence of SEQ ID No. 6 (corresponding to position 381 of SEQ ID No. 2);
o) Ser at position 366 of the amino acid sequence of SEQ ID No. 6 (corresponding to position 392 of SEQ ID No. 2);
p) Tyr at position 393 of the amino acid sequence of SEQ ID No. 6 (corresponding to position 419 of SEQ ID No. 2);
q) Asp, Phe, Asn, Ala, Cys, Glu, Gly, His, Ile, Leu, Met, Gln, Ser, Thr or Val at position 395 of the amino acid sequence of SEQ ID No. 6 (corresponding to position 421 of SEQ ID No. 2);
r) Ala, Met, Pro or Thr at position 396 of the amino acid sequence of SEQ ID No. 6 (corresponding to position 422 of SEQ ID No. 2);
s) Ala, Ile or Val at position 398 of the amino acid sequence of SEQ ID No. 6 (corresponding to position 424 of SEQ ID No. 2);
t) Ile, Pro, Arg, Ala, Gly, Lys, Asn or Gln at position 399 of the amino acid sequence of SEQ ID No. 6 (corresponding to position 425 of SEQ ID No. 2);
u) Glu, Phe, Thr, Val, Ala or Ser at position 400 of the amino acid sequence of SEQ ID No. 6 (corresponding to position 426 of SEQ ID No. 2);
v) Met, Gln, Val, Phe or Leu at position 405 of the amino acid sequence of SEQ ID No. 6 (corresponding to position 431 of SEQ ID No. 2);
w) Ala, Cys, Gly or Thr at a position in an HPPD protein, said position corresponding to position 202 of the amino acid sequence of SEQ ID No. 6 (corresponding to position 228 of SEQ ID No. 2);
x) Ala, Glu, Gly, Lys, Leu, Asn, Gln, Arg, Ser, Thr or Tyr at position 222 of the amino acid sequence of SEQ ID No. 6 (corresponding to position 248 of SEQ ID No. 2);
y) Ala, Ile, Met or Val at position 244 of the amino acid sequence of SEQ ID No. 6 (corresponding to position 270 of SEQ ID No. 2);
z) Glu, His, Ile, Lys, Leu, Met, Arg, Ser, Thr or Val at position 245 of the amino acid sequence of SEQ ID No. 6 (corresponding to position 271 of SEQ ID No. 2);
   aa) Gln at position 353 of the amino acid sequence of SEQ ID No. 6 (corresponding to position 379 of SEQ ID No. 2);
   ab) Arg at position 401 of the amino acid sequence of SEQ ID No. 6 (corresponding to position 427 of SEQ ID No. 2).

In another embodiment of the isolated nucleic acid of the invention, said HPPD protein comprises the amino acid sequence of SEQ ID No. 8 [Avena sativa], wherein the resulting amino acid sequence comprises at least one amino acid selected from
a) Ala, Asp, Glu, Gly, Lys, Gln, Arg, Ser, Thr, Val, Tyr, Ile, Leu or Met at position 241 of the amino acid sequence of SEQ ID No. 8 (corresponding to position 250 of SEQ ID No. 2), preferably Ala ;
b) Asp, Glu, Ile, Lys, Leu, Asn, Pro, Arg, Ser, Gly, His or Tyr at position 242 of the amino acid sequence of SEQ ID No. 8 (corresponding to position 251 of SEQ ID No. 2), preferably Arg or Lys;
c) Phe, Gly, His, Ile, Leu, Met, Asn, Gln, Arg, Ser, Val, Tyr or Thr at position 243 of the amino acid sequence of SEQ ID No. 8 (corresponding to position 252 of SEQ ID No. 2), preferably Tyr;
d) Ala, His, Gln, Val, Trp, Ile, Leu or Met at position 244 of the amino acid sequence of SEQ ID No. 8 (corresponding to position 253 of SEQ ID No. 2), preferably Val;
e) Val or Met at position 256 of the amino acid sequence of SEQ ID No. 8 (corresponding to position 265 of SEQ ID No. 2);
f) Leu, Gln, Arg, Tyr, Ala, Ile, Lys or Met at position 259 of the amino acid sequence of SEQ ID No. 8 (corresponding to position 268 of SEQ ID No. 2);
g) Ala or Thr at position 260 of the amino acid sequence of SEQ ID No. 8 (corresponding to position 269 of SEQ ID No. 2), preferably Ala;
h) Ala, Ile, Asn, Thr or Val at position 271 of the amino acid sequence of SEQ ID No. 8 (corresponding to position 280 of SEQ ID No. 2);
i) Leu, Val, Ala, Phe, Gly, Met, Arg or Ser at position 284 of the amino acid sequence of SEQ ID No. 8 (corresponding to position 293 of SEQ ID No. 2);
j) Met, Ala, Pro, Ser, Thr or Val at position 285 of the amino acid sequence of SEQ ID No. 8 (corresponding to position 294 of SEQ ID No. 2);
k) His or Asn at position 265 of the amino acid sequence of SEQ ID No. 8 (corresponding to position 307 of SEQ ID No. 2);
l) Ile or Asn or Leu at position 326 of the amino acid sequence of SEQ ID No. 8 (corresponding to position 335 of SEQ ID No. 2);
m) Met at position 359 of the amino acid sequence of SEQ ID No. 8 (corresponding to position 368 of SEQ ID No. 2);
n) Tyr at position 372 of the amino acid sequence of SEQ ID No. 8 (corresponding to position 381 of SEQ ID No. 2);
o) Ser at position 383 of the amino acid sequence of SEQ ID No. 8 (corresponding to position 392 of SEQ ID No. 2);
p) Tyr at position 410 of the amino acid sequence of SEQ ID No. 8 (corresponding to position 419 of SEQ ID No. 2);
q) Asp, Phe, Asn, Ala, Cys, Glu, Gly, His, Ile, Leu, Met, Gln, Ser, Thr or Val at position 412 of the amino acid sequence of SEQ ID No. 8 (corresponding to position 421 of SEQ ID No. 2), preferably Lys;
r) Ala, Met, Pro or Thr at position 413 of the amino acid sequence of SEQ ID No. 8 (corresponding to position 422 of SEQ ID No. 2);
s) Ala, Ile or Val at position 415 of the amino acid sequence of SEQ ID No. 8 (corresponding to position 424 of SEQ ID No. 2);
t) Ile, Pro, Arg, Ala, Gly, Lys, Asn or Gln at position 416 of the amino acid sequence of SEQ ID No. 8 (corresponding to position 425 of SEQ ID No. 2);
u) Phe, Thr, Val, Ala, Gln or Ser at position 417 of the amino acid sequence of SEQ ID No. 8 (corresponding to position 426 of SEQ ID No. 2);
v) Met, Gln, Val, Phe or Leu at position 422 of the amino acid sequence of SEQ ID No. 8 (corresponding to position 431 of SEQ ID No. 2);
w) Ala, Cys, Gly or Thr at position 219 of the amino acid sequence of SEQ ID No. 8 (corresponding to position 228 of SEQ ID No. 2);
x) Ala, Glu, Gly, Lys, Leu, Asn, Gln, Arg, Ser, Thr or Tyr at position 239 of the amino acid sequence of SEQ ID No. 8 (corresponding to position 248 of SEQ ID No. 2);
y) Ala, Ile, Met or Val at position 261 of the amino acid sequence of SEQ ID No. 8 (corresponding to position 270 of SEQ ID No. 2);
z) Glu, His, Ile, Lys, Leu, Met, Arg, Ser, Thr or Val at position 262 of the amino acid sequence of SEQ ID No. 8 (corresponding to position 271 of SEQ ID No. 2);
   aa) His at position 370 of the amino acid sequence of SEQ ID No. 8 (corresponding to position 379 of SEQ ID No. 2);
   bb) Arg at position 418 of the amino acid sequence of SEQ ID No. 8 (corresponding to position 427 of SEQ ID No. 2).

In another embodiment, of the isolated nucleic acid of the invention, said HPPD protein comprises the amino acid sequence of SEQ ID No. 10 [Pseudomonas fluorescens], wherein the resulting amino acid sequence comprises at least one amino acid selected from
a) Phe, Asp, Glu, Gly, Lys, Gln, Arg, Ser, Thr, Val, Tyr, Ile, Leu or Met at position 188 of the amino acid sequence of SEQ ID No. 10 (corresponding to position 250 of SEQ ID No. 2), preferably Ala;
b) Ala, Asp, Glu, Ile, Lys, Leu, Asn, Pro, Ser, Gly, His or Tyr at position 189 of the amino acid sequence of SEQ ID No. 10 (corresponding to position 251 of SEQ ID No. 2), preferably Ala or Lys;
c) Phe, Gly, His, Ile, Leu, Met, Asn, Gln, Arg, Ser, Val, Glu or Thr at position 190 of the amino acid sequence of SEQ ID No. 10 (corresponding to position 252 of SEQ ID No. 2), preferably Ser or Tyr;
d) Ala, His, Gln, Val, Trp, Ile, Leu or Met at position 191 of the amino acid sequence of SEQ ID No. 10 (corresponding to position 253 of SEQ ID No. 2), preferably Phe or Val;
e) Val or Met at position 200 of the amino acid sequence of SEQ ID No. 10 (corresponding to position 265 of SEQ ID No. 2);
f) Leu, Gln, Arg, Val Tyr, Ala, Ile or Met at position 203 of the amino acid sequence of SEQ ID No. 10 (corresponding to position 268 of SEQ ID No. 2);
g) Thr or Val at position 204 of the amino acid sequence of SEQ ID No. 10 (corresponding to position 269 of SEQ ID No. 2), preferably Val;
h) Ala, Ile, Asn, Thr or Val at position 215 of the amino acid sequence of SEQ ID No. 10 (corresponding to position 280 of SEQ ID No. 2);
i) Leu, Val, Ala, Phe, Gly, Met, Arg or Ser at position 226 of the amino acid sequence of SEQ ID NO:SEQ ID No. 10 (corresponding to position 293 of SEQ ID No. 2);
j) Met, Thr, Ala, Pro, Ser or Val at position 227 of the amino acid sequence of SEQ ID No. 10 (corresponding to position 294 of SEQ ID No. 2);
k) His or Asn at position 240 of the amino acid sequence of SEQ ID No. 10 (corresponding to position 307 of SEQ ID No. 2);
l) Ile, Asn or Leu at position 264 of the amino acid sequence of SEQ ID No. 10 (corresponding to position 335 of SEQ ID No. 2);
m) Met at position 295 of the amino acid sequence of SEQ ID No. 10 (corresponding to position 368 of SEQ ID No. 2);
n) Tyr at position 312 of the amino acid sequence of SEQ ID No. 10 (corresponding to position 381 of SEQ ID No. 2);
o) Ser at position 321 of the amino acid sequence of SEQ ID No. 10 (corresponding to position 392 of SEQ ID No. 2);
p) Tyr at position 333 of the amino acid sequence of SEQ ID No. 10 (corresponding to position 419 of SEQ ID No. 2);
q) Asp, Lys, Asn, Ala, Cys, Gly, His, Ile, Leu, Met, Gln, Ser, Thr or Val at position 335 of the amino acid sequence of SEQ ID No. 10 (corresponding to position 421 of SEQ ID No. 2), preferably Lys;
r) Ala, Met, Pro or Thr at position 336 of the amino acid sequence of SEQ ID No. 10 (corresponding to position 422 of SEQ ID No. 2);
s) Ala, Ile or Val at position 338 of the amino acid sequence of SEQ ID No. 10 (corresponding to position 424 of SEQ ID No. 2);
t) Ile, Pro, Ala, Asn, Gly or Gln at position 339 of the amino acid sequence of SEQ ID No. 10 (corresponding to position 425 of SEQ ID No. 2);
u) Phe, Thr or Val at position 340 of the amino acid sequence of SEQ ID No. 10 (corresponding to position 426 of SEQ ID No. 2);
v) Met, Phe, Leu, Gln or Val at position 345 of the amino acid sequence of SEQ ID No. 10 (corresponding to position 431 of SEQ ID No. 2);
w) Ala, Cys, Gly or Val at position 164 of the amino acid sequence of SEQ ID No. 10 (corresponding to position 228 of SEQ ID No. 2);
x) Ala, Glu, Gly, His, Lys, Leu, Asn, Gln, Ser, Thr or Tyr at position 186 of the amino acid sequence of SEQ ID No. 10 (corresponding to position 248 of SEQ ID No. 2);
y) Ala, Ile, Leu or Val at position 205 of the amino acid sequence of SEQ ID No. 10 (corresponding to position 270 of SEQ ID No. 2);
z) Ala, Glu, His, Ile, Lys, Leu, Met, Arg, Thr or Val at position 206 of the amino acid sequence of SEQ ID No. 10 (corresponding to position 271 of SEQ ID No. 2);
   aa) His at position 310 of the amino acid sequence of SEQ ID No. 10
   (corresponding to position 379 of SEQ ID No. 2);
   bb) Arg at position 341 of the amino acid sequence of SEQ ID No. 10
   (corresponding to position 427 of SEQ ID No. 2).

In another embodiment of the isolated nucleic acid of the invention, said HPPD protein comprises the amino acid sequence of SEQ ID No. 14 [Synechococcus sp.], wherein the resulting amino acid sequence comprises at least one amino acid selected from
a) Ala, Asp, Glu, Phe, Gly, Lys, Gln, Arg, Ser, Thr, Val, Tyr, Ile or Met at position 169 of the amino acid sequence of SEQ ID No. 14 (corresponding to position 250 of SEQ ID No. 2), preferably Ala or Phe;
b) Ala, Asp, Glu, Ile, Lys, Leu, Asn, Pro, Arg or Ser, Gly or His at position 170 of the amino acid sequence of SEQ ID No. 14 (corresponding to position 251 of SEQ ID No. 2), preferably Ala, Arg or Lys;
c) Glu, Phe, Gly, His, Ile, Leu, Met, Asn, Gln, Ser, Val, Tyr or Thr at position 171 of the amino acid sequence of SEQ ID No. 14 (corresponding to position 252 of SEQ ID No. 2), preferably Glu, Ser or Tyr;
d) Ala, Phe, His, Gln, Val or Ile, Leu or Met at position 172 of the amino acid sequence of SEQ ID No. 14 (corresponding to position 253 of SEQ ID No. 2), preferably Phe or Val;
e) Val or Met at position 181 of the amino acid sequence of SEQ ID No. 14 (corresponding to position 265 of SEQ ID No. 2);
f) Leu, Gln, Arg, Tyr, Ala, Ile, Lys or Met at position 184 of the amino acid sequence of SEQ ID No. 14 (corresponding to position 268 of SEQ ID No. 2);
g) Ala or Thr at position 185 of the amino acid sequence of SEQ ID No. 14 (corresponding to position 269 of SEQ ID No. 2), preferably Ala ;
h) Ile, Asn, Pro, Thr or Val at position 196 of the amino acid sequence of SEQ ID No. 14 (corresponding to position 280 of SEQ ID No. 2), preferably Pro;
i) Leu, Val, Ala, Phe, Gly, Met, Arg or Ser at position 206 of the amino acid sequence of SEQ ID No. 14 (corresponding to position 293 of SEQ ID No. 2);
j) Met , Ala, Pro, Ser, Thr or Val at position 207 of the amino acid sequence of SEQ ID No. 14 (corresponding to position 294 of SEQ ID No. 2);
k) His or Asn at position 220 of the amino acid sequence of SEQ ID No. 14 (corresponding to position 307 of SEQ ID No. 2);
l) Ile, Met or Asn at position 244 of the amino acid sequence of SEQ ID No. 14 (corresponding to position 335 of SEQ ID No. 2), preferably Met;
m) Met at position 286 of the amino acid sequence of SEQ ID No. 14 (corresponding to position 368 of SEQ ID No. 2);
n) Phe or Tyr at position 301 of the amino acid sequence of SEQ ID No. 14 (corresponding to position 381 of SEQ ID No. 2);
o) Ser at position 312 of the amino acid sequence of SEQ ID No. 14 (corresponding to position 392 of SEQ ID No. 2);
p) Tyr at position 325 of the amino acid sequence of SEQ ID No. 14 (corresponding to position 419 of SEQ ID No. 2);
q) Asp, Phe, Lys, Asn, Ala, Cys, Gly, His, Ile, Leu, Met, Gln, Ser, Thr or Val at position 327 of the amino acid sequence of SEQ ID No. 14 (corresponding to position 421 of SEQ ID No. 2), preferably Lys;
r) Gly, Met, Pro or Thr at position 328 of the amino acid sequence of SEQ ID No. 14 (corresponding to position 422 of SEQ ID No. 2);
s) Ala, Ile or Val at position 330 of the amino acid sequence of SEQ ID No. 14 (corresponding to position 424 of SEQ ID No. 2);
t) Ile, Pro, Arg, Ser, Ala, Gly, Lys or Asn at position 331 of the amino acid sequence of SEQ ID No. 14 (corresponding to position 425 of SEQ ID No. 2);
u) Glu, Phe, Thr, Val, Gln or Ser at position 332 of the amino acid sequence of SEQ ID No. 14 (corresponding to position 426 of SEQ ID No. 2), preferably Glu;
v) Ile, Met, Gln, Val or Phe at position 337 of the amino acid sequence of SEQ ID No. 14 (corresponding to position 431 of SEQ ID No. 2);
w) Ala, Cys, Gly or Thr at position 145 of the amino acid sequence of SEQ ID No. 14 (corresponding to position 228 of SEQ ID No. 2);
x) Ala, Glu, Gly, His, Lys, Leu, Asn, Gln, Ser, Thr or Tyr at position 167 of the amino acid sequence of SEQ ID No. 14 (corresponding to position 248 of SEQ ID No. 2);
y) Ala, Ile, Leu or Met at position 186 of the amino acid sequence of SEQ ID No. 14 (corresponding to position 270 of SEQ ID No. 2);
z) Ala, Glu, His, Ile, Lys, Leu, Met, Arg, Ser, Thr or Val at position 187 of the amino acid sequence of SEQ ID No. 14 (corresponding to position 271 of SEQ ID No. 2);
   aa) His at a position at position 299 of the amino acid sequence of SEQ ID No. 14 (corresponding to position 379 of SEQ ID No. 2);
   bb) Arg at a position at position 333 of the amino acid sequence of SEQ ID No. 14 (corresponding to position 427 of SEQ ID No. 2).

In another embodiment of the isolated nucleic acid of the invention, said HPPD protein comprises the amino acid sequence of SEQ ID No. 16 [Blepharisma japonicum], wherein the resulting amino acid sequence comprises at least one amino acid selected from
a) Ala, Asp, Glu, Phe, Gly, Lys, Gln, Arg, Ser, Thr, Val, Ile, Leu or Met at position 209 of the amino acid sequence of SEQ ID No. 16 (corresponding to position 250 of SEQ ID No. 2), preferably Ala or Phe;
b) Ala, Asp, Glu, Ile, Lys, Leu, Asn, Pro, Arg, Ser, Gly, His or Tyr at position 210 of the amino acid sequence of SEQ ID No. 16 (corresponding to position 251 of SEQ ID No. 2), preferably Ala, Arg, Lys;
c) Glu, Phe, Gly, His, Ile, Leu, Met, Asn, Gln, Arg, Val, Tyr or Thr at position 211 of the amino acid sequence of SEQ ID No. 16 (corresponding to position 252 of SEQ ID No. 2), preferably Glu or Tyr;
d) Phe, His, Gln, Val, Trp, Ile, Leu or Met at position 212 of the amino acid sequence of SEQ ID No. 16 (corresponding to position 253 of SEQ ID No. 2), preferably Phe or Val;
e) Val or Met at position 224 of the amino acid sequence of SEQ ID No. 16 (corresponding to position 265 of SEQ ID No. 2);
f) Leu, Gln, Arg, Tyr, Ala, Ile, Lys or Met at position 227 of the amino acid sequence of SEQ ID No. 16 (corresponding to position 2268 of SEQ ID No. 2);
g) Ala or Thr at position 228 of the amino acid sequence of SEQ ID No. 16 (corresponding to position 269 of SEQ ID No. 2), preferably Ala;
h) Ala, Ile, Asn, Thr or Val at position 239 of the amino acid sequence of SEQ ID No. 16 (corresponding to position 280 of SEQ ID No. 2);
i) Leu, Val, Ala, Phe, Gly, Met, Arg or Ser at position 251 of the amino acid sequence of SEQ ID No. 16 (corresponding to position 293 of SEQ ID No. 2);
j) Met, Ala, Pro, Ser, Thr or Val at position 252 of the amino acid sequence of SEQ ID No. 16 (corresponding to position 294 of SEQ ID No. 2);
k) His or Asn at position 265 of the amino acid sequence of SEQ ID No. 16 (corresponding to position 307 of SEQ ID No. 2);
l) Ile, Met or Asn at position 289 of the amino acid sequence of SEQ ID No. 16 (corresponding to position 335 of SEQ ID No. 2), preferably Met;
m) Met at position 323 of the amino acid sequence of SEQ ID No. 16 (corresponding to position 368 of SEQ ID No. 2);
n) Tyr at position 336 of the amino acid sequence of SEQ ID No. 16 (corresponding to position 381 of SEQ ID No. 2);
o) Ser at position 347 of the amino acid sequence of SEQ ID No. 16 (corresponding to position 292 of SEQ ID No. 2);
p) Tyr at position 359 of the amino acid sequence of SEQ ID No. 16 (corresponding to position 419 of SEQ ID No. 2);
q) Asp, Phe, Lys, Asn, Ala, Cys, Glu, Gly, His, Leu, Met, Gln, Ser, Thr or Val at position 361 of the amino acid sequence of SEQ ID No. 16 (corresponding to position 421 of SEQ ID No. 2), preferably Lys;
r) Ala, Met, Pro or Thr at position 362 of the amino acid sequence of SEQ ID No. 16 (corresponding to position 422 of SEQ ID No. 2);
s) Ala, Ile or Val at position 364 of the amino acid sequence of SEQ ID No. 16 (corresponding to position 424 of SEQ ID No. 2);
t) Ile, Pro, Arg, Ser, Ala, Gly, Asn or Gln at position 365 of the amino acid sequence of SEQ ID No. 16 (corresponding to position 425 of SEQ ID No. 2);
u) Glu, Phe, Thr, Val, Gln or Ser at position 366 of the amino acid sequence of SEQ ID No. 16 (corresponding to position 426 of SEQ ID No. 2), preferably Glu;
v) Ile, Met, Gln, Val or Phe at position 371 of the amino acid sequence of SEQ ID No. 16 (corresponding to position 431 of SEQ ID No. 2);
w) Ala, Cys, Gly or Thr at a position in an HPPD protein, said position corresponding to position 185 of the amino acid sequence of SEQ ID No. 16 (corresponding to position 228 of SEQ ID No. 2);
x) Ala, Glu, Gly, Lys, Leu, Asn, Gln, Arg, Ser, Thr or Tyr at a position in an HPPD protein, said position corresponding to position 207 of the amino acid sequence of SEQ ID No. 16 (corresponding to position 248 of SEQ ID No. 2);
y) Ala, Ile, Leu or Met at a position in an HPPD protein, said position corresponding to position 229 of the amino acid sequence of SEQ ID No. 16 (corresponding to position 270 of SEQ ID No. 2);
z) Glu, His, Ile, Lys, Leu, Met, Arg, Ser, Thr or Val at a position in an HPPD protein, said position corresponding to position 230 of the amino acid sequence of SEQ ID No. 16 (corresponding to position 271 of SEQ ID No. 2);
   aa) His at a position in an HPPD protein, said position corresponding to position 334 of the amino acid sequence of SEQ ID No. 16 (corresponding to position 379 of SEQ ID No. 2);
   bb) Arg at a position in an HPPD protein, said position corresponding to position 367 of the amino acid sequence of SEQ ID No. 16 (corresponding to position 427 of SEQ ID No. 2).

In another embodiment, of the isolated nucleic acid of the invention, said HPPD protein comprises the amino acid sequence of SEQ ID No. 18 [Rhodococcus RHA1 sp. ro0240], wherein the resulting amino acid sequence comprises at least one amino acid selected from
a) Ala, Asp, Glu, Phe, Gly, Lys, Gln, Arg, Ser, Thr, Val, Tyr, Ile or Leu at position 231 of the amino acid sequence of SEQ ID No. 18 (corresponding to position 250 of SEQ ID No. 2), preferably Phe or Ala;
b) Asp, Glu, Ile, Lys, Leu, Asn, Pro, Arg, Ser, Gly, His or Tyr at position 232 of the amino acid sequence of SEQ ID No. 18 (corresponding to position 251 of SEQ ID No. 2), preferably Arg or Lys;
c) Phe, Gly, His, Ile, Leu, Met, Asn, Gln, Arg, Ser, Val, Tyr or Thr at position 233 of the amino acid sequence of SEQ ID No. 18 (corresponding to position 252 of SEQ ID No. 2), preferably Ser or Tyr;
d) Ala, His, Gln, Val, Trp, Ile, Leu or Met at position 234 of the amino acid sequence of SEQ ID No. 18 (corresponding to position 253 of SEQ ID No. 2), preferably Val;
e) Val or Met at position 246 of the amino acid sequence of SEQ ID No. 18 (corresponding to position 265 of SEQ ID No. 2);
f) Leu, Gln, Arg, Val, Tyr, Ala, Ile or Met at position 249 of the amino acid sequence of SEQ ID No. 18 (corresponding to position 268 of SEQ ID No. 2);
g) Ala or Thr at position 250 of the amino acid sequence of SEQ ID No. 18 (corresponding to position 269 of SEQ ID No. 2), preferably Ala;
h) Ala, Ile, Asn, Thr or Val at position 261 of the amino acid sequence of SEQ ID No. 18 (corresponding to position 280 of SEQ ID No. 2);
i) Leu, Val, Ala, Phe, Gly, Met, Arg or Ser at position 273 of the amino acid sequence of SEQ ID No. 18 (corresponding to position 293 of SEQ ID No. 2);
j) Met, Ala, Pro, Ser, Thr or Val at position 274 of the amino acid sequence of SEQ ID No. 18 (corresponding to position 294 of SEQ ID No. 2);
k) His or Asn at position 287 of the amino acid sequence of SEQ ID No. 18 (corresponding to position 307 of SEQ ID No. 2);
l) Ile, Met or Asn at position 311 of the amino acid sequence of SEQ ID No. 18 (corresponding to position 335 of SEQ ID No. 2), preferably Met;
m) Met at position 343 of the amino acid sequence of SEQ ID No. 18 (corresponding to position 368 of SEQ ID No. 2);
n) Tyr at position 356 of the amino acid sequence of SEQ ID No. 18 (corresponding to position 381 of SEQ ID No. 2);
o) Ser at position 367 of the amino acid sequence of SEQ ID No. 18 (corresponding to position 392 of SEQ ID No. 2);
p) Tyr at position 379 of the amino acid sequence of SEQ ID No. 18 (corresponding to position 419 of SEQ ID No. 2);
q) Asp, Lys, Asn, Ala, Cys, Glu, Gly, His, Leu, Met, Gln, Ser, Thr or Val at position 381 of the amino acid sequence of SEQ ID No. 18 (corresponding to position 421 of SEQ ID No. 2), preferably Lys;
r) Ala, Met, Pro or Thr at position 382 of the amino acid sequence of SEQ ID No. 18 (corresponding to position 422 of SEQ ID No. 2);
s) Ala, Ile or Val at position 384 of the amino acid sequence of SEQ ID No. 18 (corresponding to position 424 of SEQ ID No. 2);
t) Ile, Pro, Arg, Ser, Ala, Gly, Asn or Gln at position 385 of the amino acid sequence of SEQ ID No. 18 (corresponding to position 425 of SEQ ID No. 2);
u) Glu, Phe, Thr, Val, Gln or Ser at position 386 of the amino acid sequence of SEQ ID No. 18 (corresponding to position 426 of SEQ ID No. 2), preferably Glu;
v) Met, Gln, Phe, Val, Phe or Leu at position 391 of the amino acid sequence of SEQ ID No. 18 (corresponding to position 431 of SEQ ID No. 2);
w) Ala, Cys, Gly or Thr at a position in an HPPD protein, said position corresponding to position 207 of the amino acid sequence of SEQ ID No. 1 (corresponding to position 228 of SEQ ID No. 2)8;
x) Ala, Glu, Gly, His, Lys, Leu, Asn, Gln, Arg, Ser or Tyr at a position in an HPPD protein, said position corresponding to position 229 of the amino acid sequence of SEQ ID No. 18 (corresponding to position 248 of SEQ ID No. 2);
y) Ala, Ile, Leu or Met at a position in an HPPD protein, said position corresponding to position 251 of the amino acid sequence of SEQ ID No. 18 (corresponding to position 270 of SEQ ID No. 2);
z) Ala, Glu, His, Ile, Lys, Leu, Met, Arg, Thr or Val at a position in an HPPD protein, said position corresponding to position 252 of the amino acid sequence of SEQ ID No. 18 (corresponding to position 271 of SEQ ID No. 2);
   aa) His at a position in an HPPD protein, said position corresponding to position 354 of the amino acid sequence of SEQ ID No. 18 (corresponding to position 379 of SEQ ID No. 2);
   bb) Arg at a position in an HPPD protein, said position corresponding to position 387 of the amino acid sequence of SEQ ID No. 18 (corresponding to position 427 of SEQ ID No. 2).

In another embodiment, of the isolated nucleic acid of the invention, said HPPD protein comprises the amino acid sequence of SEQ ID No. 30 [Rhodococcus RHA1 sp. 0341], wherein the resulting amino acid sequence comprises at least one amino acid selected from
a) Ala, Asp, Glu, Phe, Gly, Lys, Gln, Arg, Ser, Thr, Val, Tyr, Ile or Leu at position 232 of the amino acid sequence of SEQ ID No. 30 (corresponding to position 250 of SEQ ID No. 2), preferably Phe or Ala;
b) Asp, Glu, Ile, Lys, Leu, Asn, Pro, Arg, Ser, Gly, His or Tyr at position 233 of the amino acid sequence of SEQ ID No. 30 (corresponding to position 251 of SEQ ID No. 2), preferably Arg or Lys;
c) Phe, Gly, His, Ile, Leu, Met, Asn, Gln, Arg, Ser, Val, Tyr or Thr at position 234 of the amino acid sequence of SEQ ID No. 30 (corresponding to position 252 of SEQ ID No. 2), preferably Ser or Tyr;
d) Ala, His, Gln, Val, Trp, Ile, Leu or Met at position 235 of the amino acid sequence of SEQ ID No. 30 (corresponding to position 253 of SEQ ID No. 2), preferably Val;
e) Val or Met at position 247 of the amino acid sequence of SEQ ID No. 30 (corresponding to position 265 of SEQ ID No. 2);
f) Leu, Gln, Arg, Val, Tyr, Ala, Ile or Met at position 250 of the amino acid sequence of SEQ ID No. 30 (corresponding to position 268 of SEQ ID No. 2);
g) Ala or Thr at position 251 of the amino acid sequence of SEQ ID No. 30 (corresponding to position 269 of SEQ ID No. 2), preferably Ala;
h) Ala, Ile, Asn, Thr or Val at position 262 of the amino acid sequence of SEQ ID No. 30 (corresponding to position 280 of SEQ ID No. 2);
i) Leu, Val, Ala, Phe, Gly, Met, Arg or Ser at position 274 of the amino acid sequence of SEQ ID No. 30 (corresponding to position 293 of SEQ ID No. 2);
j) Met, Ala, Pro, Ser, Thr or Val at position 275 of the amino acid sequence of SEQ ID No. 30 (corresponding to position 294 of SEQ ID No. 2);
k) His or Asn at position 288 of the amino acid sequence of SEQ ID No. 30 (corresponding to position 307 of SEQ ID No. 2);
l) Ile, Met or Asn at position 312 of the amino acid sequence of SEQ ID No. 30 (corresponding to position 335 of SEQ ID No. 2), preferably Met;
m) Met at position 344 of the amino acid sequence of SEQ ID No. 30 (corresponding to position 368 of SEQ ID No. 2);
n) Tyr at position 357 of the amino acid sequence of SEQ ID No. 30 (corresponding to position 381 of SEQ ID No. 2);
o) Ser at position 368 of the amino acid sequence of SEQ ID No. 30 (corresponding to position 392 of SEQ ID No. 2);
p) Tyr at position 380 of the amino acid sequence of SEQ ID No. 30 (corresponding to position 419 of SEQ ID No. 2);
q) Asp, Phe, Lys, Asn, Ala, Cys, Glu, Gly, His, Leu, Met, Gln, Ser, Thr or Val at position 382 of the amino acid sequence of SEQ ID No. 30 (corresponding to position 421 of SEQ ID No. 2), preferably Lys;
r) Ala, Met, Pro or Thr at position 383 of the amino acid sequence of SEQ ID No. 30 (corresponding to position 422 of SEQ ID No. 2);
s) Ala, Ile or Val at position 385 of the amino acid sequence of SEQ ID No. 30 (corresponding to position 424 of SEQ ID No. 2);
t) Ile, Pro, Arg, Ser, Ala, Gly, Asn or Gln at position 386 of the amino acid sequence of SEQ ID No. 30 (corresponding to position 425 of SEQ ID No. 2);
u) Glu, Phe, Thr, Val, Gln or Ser at position 387 of the amino acid sequence of SEQ ID No. 30 (corresponding to position 426 of SEQ ID No. 2), preferably Glu;
v) Met, Gln, Phe, Val, Phe or Leu at position 392 of the amino acid sequence of SEQ ID No. 30 (corresponding to position 431 of SEQ ID No. 2);
w) Ala, Cys, Gly or Thr at a position in an HPPD protein, said position corresponding to position 208 of the amino acid sequence of SEQ ID No. 30 (corresponding to position 228 of SEQ ID No. 2);
x) Ala, Glu, Gly, His, Lys, Leu, Asn, Gln, Arg, Ser or Tyr at a position in an HPPD protein, said position corresponding to position 230 of the amino acid sequence of SEQ ID No. 30 (corresponding to position 248 of SEQ ID No. 2);
y) Ala, Ile, Leu or Met at a position in an HPPD protein, said position corresponding to position 252 of the amino acid sequence of SEQ ID No. 30 (corresponding to position 270 of SEQ ID No. 2);
z) Glu, His, Ile, Lys, Leu, Met, Arg, Thr or Val at a position in an HPPD protein, said position corresponding to position 253 of the amino acid sequence of SEQ ID No. 30 (corresponding to position 271 of SEQ ID No. 2);
   aa)His at a position in an HPPD protein, said position corresponding to position 355 of the amino acid sequence of SEQ ID No. 30 (corresponding to position 379 of SEQ ID No. 2);
   bb)Arg at a position in an HPPD protein, said position corresponding to position 388 of the amino acid sequence of SEQ ID No. 30 (corresponding to position 427 of SEQ ID No. 2).

In another embodiment, of the isolated nucleic acid of the invention, said HPPD protein comprises the amino acid sequence of SEQ ID No. 20 [Picrophilus torridus], wherein the resulting amino acid sequence comprises at least one amino acid selected from
a) Ala, Asp, Glu, Phe, Gly, Lys, Gln, Arg, Ser, Thr, Val, Tyr, Ile or Met at position 201 of the amino acid sequence of SEQ ID No. 20 (corresponding to position 250 of SEQ ID No. 2), preferably Phe or Ala;
b) Ala, Asp, Glu, Lys, Leu, Asn, Pro, Arg or Ser, Gly, His or Trp at position 202 of the amino acid sequence of SEQ ID No. 20 (corresponding to position 251 of SEQ ID No. 2), preferably Ala, Arg or Lys;
c) Glu, Phe, Gly, His, Ile, Leu, Met, Asn, Gln, Arg, Ser, Val or Tyr at position 203 of the amino acid sequence of SEQ ID No. 20 (corresponding to position 252 of SEQ ID No. 2), preferably Glu, Ser or Tyr;
d) Ala, His, Gln, Val, Trp, Ile, Leu or Met at position 204 of the amino acid sequence of SEQ ID No. 20 (corresponding to position 253 of SEQ ID No. 2), preferably Val;
e) Val or Met at position 216 of the amino acid sequence of SEQ ID No. 20 (corresponding to position 265 of SEQ ID No. 2);
f) Leu, Gln, Arg, Val, Tyr, Ala, Ile or Met at position 219 of the amino acid sequence of SEQ ID No. 20 (corresponding to position 268 of SEQ ID No. 2);
g) Ala or Thr at position 220 of the amino acid sequence of SEQ ID No. 20 (corresponding to position 269 of SEQ ID No. 2), preferably Ala;
h) Ala, Ile, Asn, Thr or Val at position 230 of the amino acid sequence of SEQ ID No. 20 (corresponding to position 280 of SEQ ID No. 2);
i) Leu, Val, Ala, Phe, Gly, Met, Arg or Ser at position 242 of the amino acid sequence of SEQ ID No. 20 (corresponding to position 293 of SEQ ID No. 2);
j) Met, Ala, Pro, Ser, Thr or Val at position 243 of the amino acid sequence of SEQ ID No. 20 (corresponding to position 294 of SEQ ID No. 2);
k) His or Asn at position 256 of the amino acid sequence of SEQ ID No. 20 (corresponding to position 307 of SEQ ID No. 2);
l) Ile, Met or Asn at position 280 of the amino acid sequence of SEQ ID No. 20 (corresponding to position 335 of SEQ ID No. 2), preferably Met;
m) Met at position 310 of the amino acid sequence of SEQ ID No. 20 (corresponding to position 368 of SEQ ID No. 2);
n) Phe or Tyr at position 323 of the amino acid sequence of SEQ ID No. 20 (corresponding to position 381 of SEQ ID No. 2);
o) Ser at position 334 of the amino acid sequence of SEQ ID No. 20 (corresponding to position 392 of SEQ ID No. 2);
p) Tyr at position 346 of the amino acid sequence of SEQ ID No. 20 (corresponding to position 419 of SEQ ID No. 2);
q) Asp, Phe, Lys, Ala, Cys, Glu, Gly, His, Ile, Leu, Met, Gln, Ser, Thr or Val at position 348 of the amino acid sequence of SEQ ID No. 20 (corresponding to position 421 of SEQ ID No. 2), preferably Lys;
r) Ala, Met, Pro or Thr at position 349 of the amino acid sequence of SEQ ID No. 20 (corresponding to position 422 of SEQ ID No. 2);
s) Ala, Ile or Val at position 351 of the amino acid sequence of SEQ ID No. 20 (corresponding to position 424 of SEQ ID No. 2);
t) Ile, Pro, Arg, Ser, Ala, Gly, Lys or Gln at position 352 of the amino acid sequence of SEQ ID No. 20 (corresponding to position 425 of SEQ ID No. 2);
u) Glu, Phe, Thr, Val, Ala or Gln at position 353 of the amino acid sequence of SEQ ID No. 20 (corresponding to position 426 of SEQ ID No. 2), preferably Glu;
v) Met, Gln, Phe, Val, Phe or Leu at position 358 of the amino acid sequence of SEQ ID No. 20 (corresponding to position 431 of SEQ ID No. 2);
w) Ala, Cys, Gly or Thr at position 177 of the amino acid sequence of SEQ ID No. 20 (corresponding to position 228 of SEQ ID No. 2);
x) Ala, Glu, Gly, His, Lys, Leu, Asn, Gln, Arg, Ser, Thr or Tyr at position 199 of the amino acid sequence of SEQ ID No. 20 (corresponding to position 248 of SEQ ID No. 2);
y) Ala, Ile, Leu or Met at a position at position 221 of the amino acid sequence of SEQ ID No. 20 (corresponding to position 270 of SEQ ID No. 2);
z) Ala, Glu, His, Ile, Leu, Met, Arg, Ser, Thr or Val at position 222 of the amino acid sequence of SEQ ID No. 20 (corresponding to position 271 of SEQ ID No. 2);
   aa) His at position 321 of the amino acid sequence of SEQ ID No. 20
   (corresponding to position 379 of SEQ ID No. 2);
   bb) Arg at position 354 of the amino acid sequence of SEQ ID No. 20
   (corresponding to position 427 of SEQ ID No. 2).

In another embodiment, of the isolated nucleic acid of the invention, said HPPD protein comprises the amino acid sequence of SEQ ID No. 22 [Kordia algicida], wherein the resulting amino acid sequence comprises at least one amino acid selected from
a) Ala, Asp, Glu, Phe, Gly, Lys, Gln, Arg, Ser, Thr, Val, Tyr, Leu or Met at position 219 of the amino acid sequence of SEQ ID No. 22 (corresponding to position 250 of SEQ ID No. 2), preferably Phe or Ala;
b) Ala, Asp, Glu, Lys, Leu, Asn, Pro, Arg or Ser, Gly, His or Trp at position 220 of the amino acid sequence of SEQ ID No. 22 (corresponding to position 251 of SEQ ID No. 2), preferably Ala, Arg or Lys;
c) Glu, Phe, Gly, His, Ile, Leu, Met, Asn, Gln, Arg, Val, Tyr or Thr at position 221 of the amino acid sequence of SEQ ID No. 22 (corresponding to position 252 of SEQ ID No. 2), preferably Glu or Tyr;
d) Ala, His, Gln, Val Trp, Ile, Leu or Met at position 222 of the amino acid sequence of SEQ ID No. 22 (corresponding to position 253 of SEQ ID No. 2), preferably Val;
e) Val or Met at position 234 of the amino acid sequence of SEQ ID No. 22 (corresponding to position 265 of SEQ ID No. 2);
f) Leu, Gln, Arg, Val, Tyr, Ala, Ile or Met at position 237 of the amino acid sequence of SEQ ID No. 22 (corresponding to position 268 of SEQ ID No. 2);
g) Ala or Thr at position 238 of the amino acid sequence of SEQ ID No. 22 (corresponding to position 269 of SEQ ID No. 2), preferably Ala;
h) Ala, Ile, Asn, Thr or Val at position 249 of the amino acid sequence of SEQ ID No. 22 (corresponding to position 280 of SEQ ID No. 2);
i) Leu, Val, Ala, Phe, Gly, Met, Arg or Ser at position 261 of the amino acid sequence of SEQ ID No. 22 (corresponding to position 293 of SEQ ID No. 2);
j) Met, Ala, Pro, Ser, Thr or Val at position 262 of the amino acid sequence of SEQ ID No. 22 (corresponding to position 294 of SEQ ID No. 2);
k) His or Asn at position 275 of the amino acid sequence of SEQ ID No. 22 (corresponding to position 307 of SEQ ID No. 2);
l) Ile, Met or Asn at position 299 of the amino acid sequence of SEQ ID No. 22 (corresponding to position 335 of SEQ ID No. 2), preferably Met;
m) Met at position 329 of the amino acid sequence of SEQ ID No. 22 (corresponding to position 368 of SEQ ID No. 2);
n) Tyr at position 342 of the amino acid sequence of SEQ ID No. 22 (corresponding to position 381 of SEQ ID No. 2);
o) Ser at position 353 of the amino acid sequence of SEQ ID No. 22 (corresponding to position 392 of SEQ ID No. 2);
p) Tyr at position 365 of the amino acid sequence of SEQ ID No. 22 (corresponding to position 419 of SEQ ID No. 2);
q) Asp, Phe, Lys, Asn, Ala, Cys, Glu, Gly, His, Ile, Leu, Met, Gln, Ser or Thr at position 367 of the amino acid sequence of SEQ ID No. 22 (corresponding to position 421 of SEQ ID No. 2), preferably Lys;
r) Ala, Met, Pro or Thr at position 368 of the amino acid sequence of SEQ ID No. 22 (corresponding to position 422 of SEQ ID No. 2);
s) Ala, Ile or Val at position 370 of the amino acid sequence of SEQ ID No. 22 (corresponding to position 424 of SEQ ID No. 2);
t) Ile, Pro, Arg, Ser, Ala, Gly, Asn or Gln at position 371 of the amino acid sequence of SEQ ID No. 22 (corresponding to position 425 of SEQ ID No. 2);
u) Glu, Phe, Thr, Val, Gln or Ser at position 372 of the amino acid sequence of SEQ ID No. 22 (corresponding to position 426 of SEQ ID No. 2), preferably Glu;
v) Met, Gln, Phe, Val, Phe or Leu at position 377 of the amino acid sequence of SEQ ID No. 22 (corresponding to position 431 of SEQ ID No. 2);
w) Ala, Cys, Gly or Thr at position 195 of the amino acid sequence of SEQ ID No. 22 (corresponding to position 228 of SEQ ID No. 2);
x) Glu, Gly, His, Lys, Leu, Asn, Gln, Arg, Ser, Thr or Tyr at position 217 of the amino acid sequence of SEQ ID No. 22 (corresponding to position 248 of SEQ ID No. 2);
y) Ala, Ile, Leu or Val at position 239 of the amino acid sequence of SEQ ID No. 22 (corresponding to position 270 of SEQ ID No. 2);
z) Ala, Glu, His, Ile, Lys, Leu, Met, Arg, Thr or Val at position 240 of the amino acid sequence of SEQ ID No. 22 (corresponding to position 271 of SEQ ID No. 2);
   aa) His at a position at position 340 of the amino acid sequence of SEQ ID No. 22
   (corresponding to position 379 of SEQ ID No. 2);
   bb) Arg at a position at position 373 of the amino acid sequence of SEQ ID No. 22
   (corresponding to position 427 of SEQ ID No. 2).

In another embodiment, of the isolated nucleic acid of the invention, said HPPD protein comprises the amino acid sequence of SEQ ID No. 24 [Sorghum bicolor], wherein the resulting amino acid sequence comprises at least one amino acid selected from
a) Ala, Asp, Glu, Gly, Lys,Gln, Arg, Ser, Thr, Val Tyr, Ile, Leu or Met at position 241 of the amino acid sequence of SEQ ID No. 24 (corresponding to position 250 of SEQ ID No. 2), preferably Ala;
b) Asp, Glu, Lys, Leu, Asn, Pro, Arg, Ser, Gly, His or Trp at position 242 of the amino acid sequence of SEQ ID No. 24 (corresponding to position 251 of SEQ ID No. 2), preferably Arg or Lys;
c) Phe, Gly, His, Ile, Leu, Met, Asn, Gln, Arg, Val, Tyr or Thr at position 243 of the amino acid sequence of SEQ ID No. 24 (corresponding to position 252 of SEQ ID No. 2), preferably Tyr;
d) Ala, His, Gln, Val, Trp, Ile, Leu or Met at position 244 of the amino acid sequence of SEQ ID No. 24 (corresponding to position 253 of SEQ ID No. 2), preferably Val;
e) Val or Met at position 256 of the amino acid sequence of SEQ ID No. 24 (corresponding to position 265 of SEQ ID No. 2);
f) Leu, Gln, Arg, Val, Tyr, Ala, Ile or Lys at position 259 of the amino acid sequence of SEQ ID No. 24 (corresponding to position 268 of SEQ ID No. 2);
g) Ala or Thr at position 260 of the amino acid sequence of SEQ ID No. 24 (corresponding to position 269 of SEQ ID No. 2), preferably Ala;
h) Ala, Ile, Asn, Thr or Val at position 271 of the amino acid sequence of SEQ ID No. 24 (corresponding to position 280 of SEQ ID No. 2);
i) Leu or Val, Ala, Phe, Gly, Met, Arg or Ser at position 284 of the amino acid sequence of SEQ ID No. 24 (corresponding to position 293 of SEQ ID No. 2);
j) Met, Ala, Pro, Ser, Thr or Val at position 285 of the amino acid sequence of SEQ ID No. 24 (corresponding to position 294 of SEQ ID No. 2);
k) His or Asn at position 298 of the amino acid sequence of SEQ ID No. 24 (corresponding to position 307 of SEQ ID No. 2);
l) Ile, Asn or Leu at position 326 of the amino acid sequence of SEQ ID No. 24 (corresponding to position 335 of SEQ ID No. 2);
m) Met at position 359 of the amino acid sequence of SEQ ID No. 24 (corresponding to position 368 of SEQ ID No. 2);
n) Tyr at position 372 of the amino acid sequence of SEQ ID No. 24 (corresponding to position 381 of SEQ ID No. 2);
o) Ser at position 383 of the amino acid sequence of SEQ ID No. 24 (corresponding to position 392 of SEQ ID No. 2);
p) Tyr at position 410 of the amino acid sequence of SEQ ID No. 24 (corresponding to position 419 of SEQ ID No. 2);
q) Asp, Phe, Asn, Ala, Cys, Glu, Gly, His, Ile, Leu, Met, Gln, Ser, Thr or Val at position 412 of the amino acid sequence of SEQ ID No. 24 (corresponding to position 421 of SEQ ID No. 2);
r) Ala, Met, Pro or Thr at position 413 of the amino acid sequence of SEQ ID No. 24 (corresponding to position 422 of SEQ ID No. 2);
s) Ala, Ile or Val at position 415 of the amino acid sequence of SEQ ID No. 24 (corresponding to position 424 of SEQ ID No. 2);
t) Ile, Pro, Arg, Als, Gly, Lys, Asn or Gln at position 416 of the amino acid sequence of SEQ ID No. 24 (corresponding to position 425 of SEQ ID No. 2);
u) Phe, Thr, Val, Ala, Gln or Ser at position 417 of the amino acid sequence of SEQ ID No. 24 (corresponding to position 426 of SEQ ID No. 2);
v) Met, Gln, Phe, Val, Phe or Leu at position 422 of the amino acid sequence of SEQ ID No. 24 (corresponding to position 431 of SEQ ID No. 2);
w) Ala, Cys, Gly or Thr at position 219 of the amino acid sequence of SEQ ID No. 24 (corresponding to position 228 of SEQ ID No. 2);
x) Ala, Glu, Gly, Lys, Leu, Asn, Gln, Arg, Ser, Thr or Tyr at position 239 of the amino acid sequence of SEQ ID No. 24 (corresponding to position 248 of SEQ ID No. 2);
y) Ala, Ile, Met or Val at position 261 of the amino acid sequence of SEQ ID No. 24 (corresponding to position 270 of SEQ ID No. 2);
z) Glu, His, Ile, Lys, Leu, Met, Arg, Ser, Thr or Val at position 262 of the amino acid sequence of SEQ ID No. 24 (corresponding to position 271 of SEQ ID No. 2);
   aa) His at position 370 of the amino acid sequence of SEQ ID No. 24 (corresponding to position 379 of SEQ ID No. 2);
   bb) Arg at position 418 of the amino acid sequence of SEQ ID No. 24 (corresponding to position 427 of SEQ ID No. 2).

In another embodiment, of the isolated nucleic acid of the invention, said HPPD protein comprises the amino acid sequence of SEQ ID No. 26 [Triticum aestivum / wheat], wherein the resulting amino acid sequence comprises at least one amino acid selected from
a) Ala, Asp, Glu, Gly, Lys,Gln, Arg, Ser, Thr, Val, Tyr, Ile, Leu or Met at position 237 of the amino acid sequence of SEQ ID No. 26 (corresponding to position 250 of SEQ ID No. 2), preferably Ala;
b) Asp, Glu, Lys, Leu, Asn, Pro, Arg or Ser, Gly, His or Trp at position 238 of the amino acid sequence of SEQ ID No. 26 (corresponding to position 251 of SEQ ID No. 2), preferably Arg or Lys;
c) Phe, Gly, His, Ile, Leu, Met, Asn, Gln, Arg, Val or Tyr or Thr at position 239 of the amino acid sequence of SEQ ID No. 26 (corresponding to position 252 of SEQ ID No. 2), preferably Tyr;
d) Ala, His, Gln, Val Trp, Ile, Leu or Met at position 240 of the amino acid sequence of SEQ ID No. 26 (corresponding to position 253 of SEQ ID No. 2), preferably Val;
e) Val or Met at position 252 of the amino acid sequence of SEQ ID No. 26 (corresponding to position 265 of SEQ ID No. 2);
f) Leu, Gln, Arg, Val, Tyr, Ala, Ile or Lys at position 255 of the amino acid sequence of SEQ ID No. 26 (corresponding to position 268 of SEQ ID No. 2);
g) Ala or Thr at position 256 of the amino acid sequence of SEQ ID No. 26 (corresponding to position 269 of SEQ ID No. 2), preferably Ala;
h) Ala, Ile, Asn, Thr or Val at position 267 of the amino acid sequence of SEQ ID No. 26 (corresponding to position 280 of SEQ ID No. 2);
i) Leu, Val, Ala, Phe, Gly, Met, Arg or Ser at position 280 of the amino acid sequence of SEQ ID No. 26 (corresponding to position 293 of SEQ ID No. 2);
j) Met, Ala, Pro, Ser, Thr or Val at position 281 of the amino acid sequence of SEQ ID No. 26 (corresponding to position 294 of SEQ ID No. 2);
k) His or Asn at position 294 of the amino acid sequence of SEQ ID No. 26 (corresponding to position 307 of SEQ ID No. 2);
l) Ile, Met or Asn at position 322 of the amino acid sequence of SEQ ID No. 26 (corresponding to position 335 of SEQ ID No. 2), preferably Met;
m) Met at position 355 of the amino acid sequence of SEQ ID No. 26 (corresponding to position 368 of SEQ ID No. 2);
n) Tyr at position 368 of the amino acid sequence of SEQ ID No. 26 (corresponding to position 381 of SEQ ID No. 2);
o) Ser at position 379 of the amino acid sequence of SEQ ID No. 26 (corresponding to position 392 of SEQ ID No. 2);
p) Tyr at position 406 of the amino acid sequence of SEQ ID No. 26 (corresponding to position 419 of SEQ ID No. 2);
q) Asp, Phe, Asn, Ala, Cys, Glu, Gly, His, Ile, Leu, Met, Gln, Ser, Thr or Val at position 408 of the amino acid sequence of SEQ ID No. 26 (corresponding to position 421 of SEQ ID No. 2);
r) Ala, Met, Pro or Thr at position 409 of the amino acid sequence of SEQ ID No. 26 (corresponding to position 422 of SEQ ID No. 2);
s) Ala, Ile or Val at position 411 of the amino acid sequence of SEQ ID No. 26 (corresponding to position 424 of SEQ ID No. 2);
t) Ile, Pro, Arg, Ala, Gly, Lys, Asn or Gln at position 412 of the amino acid sequence of SEQ ID No. 26 (corresponding to position 425 of SEQ ID No. 2);
u) Phe, Thr or Val, Ala, Gln or Ser at position 413 of the amino acid sequence of SEQ ID No. 26 (corresponding to position 426 of SEQ ID No. 2);
v) Met, Gln, Phe, Leu or Val at position 418 of the amino acid sequence of SEQ ID No. 26 (corresponding to position 431 of SEQ ID No. 2);
w) Ala, Cys, Gly or Thr at a position in an HPPD protein, said position corresponding to position 215 of the amino acid sequence of SEQ ID No. 26 (corresponding to position 228 of SEQ ID No. 2);
x) Ala, Glu, Gly, Lys, Leu, Asn, Gln, Arg, Ser, Thr or Tyr at position 235 of the amino acid sequence of SEQ ID No. 26 (corresponding to position 248 of SEQ ID No. 2);
y) Ala, Ile, Met or Val at position 257 of the amino acid sequence of SEQ ID No. 26 (corresponding to position 270 of SEQ ID No. 2);
z) Glu, His, Ile, Lys, Leu, Met, Arg, Ser, Thr or Val at position 258 of the amino acid sequence of SEQ ID No. 26 (corresponding to position 271 of SEQ ID No. 2);
   aa) His at a position at position 366 of the amino acid sequence of SEQ ID No. 26
   (corresponding to position 379 of SEQ ID No. 2);
   bb) Arg at position 414 of the amino acid sequence of SEQ ID No. 26
   (corresponding to position 427 of SEQ ID No. 2).

In another embodiment, of the isolated nucleic acid of the invention, said HPPD protein comprises the amino acid sequence of SEQ ID No. 2 [Arabidopsis thaliana], wherein the resulting amino acid sequence comprises at least one amino acid selected from
(a) Ala, Asp, Glu, Gly, Lys, Gln, Arg, Ser, Thr, Val, Tyr, Ile, Leu or Met at position 250 of the amino acid sequence of SEQ ID No. 2;
(b) Asp, Glu, Ile, Lys, Leu, Asn, Pro, Arg, Ser, Gly, His or Trp at position 251 of the amino acid sequence of SEQ ID No. 2;
(c) Phe, Gly, His, Ile, Leu, Met, Asn, Gln, Arg, Ser, Val, Tyr or Thr at position 252 of the amino acid sequence of SEQ ID No. 2;
(d) Ala, His, Gln, Val, Trp, Ile, Leu or Met at position 253 of the amino acid sequence of SEQ ID No. 2;
(e) Val or Met at a position at position 265 of the amino acid sequence of SEQ ID No. 2;
(f) Leu, Gln, Arg, Val, Tyr, Ile, Lys or Met at position 268 of the amino acid sequence of SEQ ID No. 2;
(g) Ala or Thr at position 269 of the amino acid sequence of SEQ ID No. 2;
(h) Ala, Ile, Asn, Thr or Val at position 280 of the amino acid sequence of SEQ ID No. 2;
(i) Gln, Val, Ala, Phe, Gly, Met, Arg or Ser at a in an HPPD protein, said position corresponding to position 293 of the amino acid sequence of SEQ ID No. 2;
(j) Met, Ala, Pro, Ser, Thr or Val at position 294 of the amino acid sequence of SEQ ID No. 2;
(k) His or Asn at position 307 of the amino acid sequence of SEQ ID No. 2;
(l) Ile, Asn or Leu at position 335 of the amino acid sequence of SEQ ID No. 2;
(m) Met at position 368 of the amino acid sequence of SEQ ID No. 2;
(n) Tyr at position 381 of the amino acid sequence of SEQ ID No. 2;
(o) Ser at position 392 of the amino acid sequence of SEQ ID No. 2;
(p) Tyr at position 419 of the amino acid sequence of SEQ ID No. 2;
(q) Asp, Phe, Asn, Ala, Cys, Glu, Gly, His, Ile, Leu, Met, Gln, Ser, Thr or Val at position 421 of the amino acid sequence of SEQ ID No. 2 ;
(r) Ala, Met, Pro or Thr at position 422 of the amino acid sequence of SEQ ID No. 2;
(s) Ala, Ile or Val at position 424 of the amino acid sequence of SEQ ID No. 2;
(t) Ile, Pro, Arg, Ala, Gly, Lys, Asn or Gln at position 425 of the amino acid sequence of SEQ ID No. 2;
(u) Phe, Thr, Val, Ala, Gln or Ser at position 426 of the amino acid sequence of SEQ ID No. 2;
(v) Met, Gln, Val, Phe or Leu position 431 of the amino acid sequence of SEQ ID No. 2;
(w) Ala, Cys, Gly or Thr at position 228 of the amino acid sequence of SEQ ID No. 2;
(x) Ala, Glu, Gly, Lys, Leu, Asn, Gln, Arg, Ser, Thr or Tyr at position 248 of the amino acid sequence of SEQ ID No. 2;
(y) Ala, Ile, Met or Val at position 270 of the amino acid sequence of SEQ ID No. 2;
(z) Glu, His, Ile, Lys, Leu, Met, Arg, Ser, Thr or Val at position 271 of the amino acid sequence of SEQ ID No. 2;
   (aa) His at position 379 of the amino acid sequence of SEQ ID No. 2; or
   (bb) Arg at position 427 of the amino acid sequence of SEQ ID No. 2.

In a further embodiment of the nucleic acid of the invention, in said mutated HPPD protein at least one amino acid has been replaced so that the resulting amino acid sequence comprises (a) Glu, Ser or Tyr at position 252; (b) Val or Ala at position 269; (c) Pro, Val or Ala at position 280; (d) Asp, Lys or Asn at position 421; and (e) Gly or Ala at postion 422. These positions correspond to amino acid sequence of SEQ ID No 2.

In a further embodiment of the nucleic acid of the invention, in said mutated HPPD protein at least two amino acids have been replaced.

An isolated nucleic acid comprising a nucleotide sequence encoding a mutated HPPD protein,
wherein said mutated HPPD protein has HPPD activity,
wherein in said mutated HPPD protein at least one amino acid at position 228, 248, 270, 271, 379 and/or 427 has been replaced by another amino acid.

In an alternative embodiment of the nucleic acid of the invention having at least one amino acid at position 228, 248, 270, 271, 379 and/or 427 deleted or replaced by another amino acid as defined above, said mutated HPPD protein comprises
a) a His at a position in an HPPD protein, said position corresponding to position 226 of the amino acid sequence of SEQ ID No. 2;
b) a Ser at a position in an HPPD protein, said position corresponding to position 267 comprising the amino acid sequence of SEQ ID No. 2 or at a position corresponding thereto in a different HPPD enzyme;
c) an Asn at a position in an HPPD protein, said position corresponding to position 282 of the amino acid sequence of SEQ ID No. 2;
d) a His at a position in an HPPD protein, said position corresponding to position 308 of the amino acid sequence of SEQ ID No. 2;
e) a Tyr at a position in an HPPD protein, said position corresponding to position 342 of the amino acid sequence of SEQ ID No. 2;
f) a Glu at a position in an HPPD protein, said position corresponding to position 394 of the amino acid sequence of SEQ ID No. 2;
g) a Gly at a position in an HPPD protein, said position corresponding to position 420 of the amino acid sequence of SEQ ID No. 2; and
h) an Asn at a position in an HPPD protein, said position corresponding to position 423 of the amino acid sequence of SEQ ID No. 2.

In another embodiment of the isolated nucleic acid as defined above, in said mutated HPPD protein at least one amino acid has been replaced so that the resulting amino acid sequence has at least one selected from
a. Ala, Cys, Gly, Thr or Val at a position in an HPPD protein, said position corresponding to position 228 of the amino acid sequence of SEQ ID No. 2;
b. Ala, Glu, Gly, His, Lys, Leu, Asn, Gln, Arg, Ser, Thr or Tyr at a position in an HPPD protein, said position corresponding to position 248 of the amino acid sequence of SEQ ID No. 2;
c. Ala, Ile, Leu, Met or Val at a position in an HPPD protein, said position corresponding to position 270 of the amino acid sequence of SEQ ID No. 2;
d. Ala, Glu, Hils, Ile, Lys, Leu, Met, Arg, Ser, Thr or Val at a position in an HPPD protein, said position corresponding to position 271 of the amino acid sequence of SEQ ID No. 2;
e. His or Gln at a position in an HPPD protein, said position corresponding to position 379 of the amino acid sequence of SEQ ID No. 2; and
f. Leu or Arg at a position in an HPPD protein, said position corresponding to position 427 of the amino acid sequence of SEQ ID No. 2.

In another embodiment of the isolated nucleic acid as defined above, in said mutated HPPD protein at least one amino acid has been replaced so that the resulting amino acid sequence has at least one selected from
a. Val or Thr at a position in an HPPD protein, said position corresponding to position 228 of the amino acid sequence of SEQ ID No. 2;
b. Leu, Met or Val at a position in an HPPD protein, said position corresponding to position 270 of the amino acid sequence of SEQ ID No. 2;
c. Ala or Ser at a position in an HPPD protein, said position corresponding to position 271 of the amino acid sequence of SEQ ID No. 2,
d. Gln at a position in an HPPD protein, said position corresponding to position 379 of the amino acid sequence of SEQ ID No. 2; and
e. Leu at a position in an HPPD protein, said position corresponding to position 427 of the amino acid sequence of SEQ ID No. 2.

In another embodiment of the nucleic acid as defined above, said mutated HPPD protein is capable of increasing the tolerance of a plant to at least one herbicide acting on HPPD (also called HPPD inhibitor herbicide).

In another embodiment, the present invention relates to a protein encoded by the isolated nucleic acid of the invention.

In a further embodiment, the present invention relates to a chimeric gene comprising a coding sequence comprising the nucleic acid of the invention operably linked to a plant-expressible promoter and optionally a transcription termination and polyadenylation region.

As a regulatory sequence which functions as a promoter in plant cells and plants, use may be made of any promoter sequence of a gene which is naturally expressed in plants, in particular a promoter which is expressed especially in the leaves of plants, such as for example "constitutive" promoters of bacterial, viral or plant origin, or "light-dependent" promoters, such as that of a plant ribulose- biscarboxylase/oxygenase (RuBisCO) small subunit gene, or any suitable known promoter-expressible which may be used. Among the promoters of plant origin, mention will be made of the histone promoters as described in EP 0 507 698 A1, the rice actin promoter (US 5,641,876), or a plant ubiquitin promoter (US 5,510,474). Among the promoters of a plant virus gene, mention will be made of that of the cauliflower mosaic virus (CaMV 19S or 35S, Sanders et al. (1987), Nucleic Acids Res. 15(4):1543-58.), the circovirus (AU 689 311) or the Cassava vein mosaic virus (CsVMV, US 7,053,205).

In one embodiment of this invention, a promoter sequence specific for particular regions or tissues of plants can be used to express the HPPD proteins of the invention, such as promoters specific for seeds (Datla, R. et al., 1997, Biotechnology Ann. Rev. 3, 269-296), especially the napin promoter (EP 255 378 A1), the phaseolin promoter, the glutenin promoter, the helianthinin promoter (WO 92/17580), the albumin promoter (WO 98/45460), the oleosin promoter (WO 98/45461), the SAT1 promoter or the SAT3 promoter (PCT/US98/06978).

Use may also be made of an inducible promoter advantageously chosen from the phenylalanine ammonia lyase (PAL), HMG-CoA reductase (HMG), chitinase, glucanase, proteinase inhibitor (PI), PR1 family gene, nopaline synthase (nos) and vspB promoters (US 5 670 349, Table 3), the HMG2 promoter (US 5 670 349), the apple beta-galactosidase (ABG1) promoter and the apple aminocyclopropane carboxylate synthase (ACC synthase) promoter (WO 98/45445).

According to the invention, use may also be made, in combination with the promoter, of other regulatory sequences, which are located between the promoter and the coding sequence, such as transcription activators ("enhancers"), for instance the translation activator of the tobacco mosaic virus (TMV) described in Application WO 87/07644, or of the tobacco etch virus (TEV) described by Carrington & Freed 1990, J. Virol. 64: 1590-1597, for example, or introns such as the adh1 intron of maize or intron 1 of rice actin.

As a regulatory terminator or polyadenylation sequence, use may be made of any corresponding sequence of bacterial origin, such as for example the nos terminator of Agrobacterium tumefaciens, of viral origin, such as for example the CaMV 35S terminator, or of plant origin, such as for example a histone terminator as described in published Patent Application EP 0 633 317 A1.

A method of obtaining a mutated HPPD protein capable of modulating the tolerance of a plant to at least one herbicide acting on HPPD, wherein said mutated HPPD protein has HPPD activity, the method comprising
i. providing an HPPD protein, said HPPD optionally comprising an amino acid sequence, wherein
   a) a His is present at a position in an HPPD protein, said position corresponding to position 226 of the amino acid sequence of SEQ ID No. 2;
   b) a Ser is present at a position in an HPPD protein, said position corresponding to position 267 of the amino acid sequence of SEQ ID No. 2;
   c) an Asn is present at a position in an HPPD protein, said position corresponding to position 282 of the amino acid sequence of SEQ ID No. 2;
   d) a His is present at a position in an HPPD protein, said position corresponding to position 308 of the amino acid sequence of SEQ ID No. 2;
   e) a Tyr is present at a position in an HPPD protein, said position corresponding to position 342 of the amino acid sequence of SEQ ID No. 2;
   f) a Glu is present at a position in an HPPD protein, said position corresponding to position 394 of the amino acid sequence of SEQ ID No. 2;
   g) a Gly is present at a position in an HPPD protein, said position corresponding to position 420 of the amino acid sequence of SEQ ID No. 2; and
   h) an Asn is present at a position in an HPPD protein, said position corresponding to position 423 of the amino acid sequence of SEQ ID No. 2
ii) replacing at least one amino acid in said HPPD enzyme so that the resulting amino acid sequence has at least one selected from
   a. Ala, Asp, Glu, Phe, Gly, Lys,Gln, Arg, Ser, Thr, Val, Ile, Leu, Met or Tyr at a position in an HPPD protein, said position corresponding to position 250 of the amino acid sequence of SEQ ID No. 2;
   b. Ala, Asp, Glu, Ile, Lys, Leu, Asn, Pro, Arg, Gly, His, Tyr or Ser at a position in an HPPD protein, said position corresponding to position 251 of the amino acid sequence of SEQ ID No. 2;
   c. Glu, Phe, Gly, His, Ile, Leu, Met, Asn, Gln, Arg, Ser, Val, Thr or Tyr at a position in an HPPD protein, said position corresponding to position 252 of the amino acid sequence of SEQ ID No. 2;
   d. Ala, Phe, His, Gln, Val, Ile, Leu, Met or Trp at a position in an HPPD protein, said position corresponding to position 253 of the amino acid sequence of SEQ ID No. 2;
   e. Leu, Met or Val at a position in an HPPD protein, said position corresponding to position 265 of the amino acid sequence of SEQ ID No. 2;
   f. Leu, Gln, Arg, Val, Ala, Ile, Lys, Met or Tyr at a position in an HPPD protein, said position corresponding to position 268 of the amino acid sequence of SEQ ID No. 2;
   g. Ala, Thr or Val at a position in an HPPD protein, said position corresponding to position 269 of the amino acid sequence of SEQ ID No. 2;
   h. Ala, Ile, Asn, Pro, Thr or Val at a position in an HPPD protein, said position corresponding to position 280 of the amino acid sequence of SEQ ID No. 2;
   i. Leu, Gln, Ala, Phe, Gly, Met, Arg, Ser or Val at a position in an HPPD protein, said position corresponding to position 293 of the amino acid sequence of SEQ ID No. 2;
   j. Ile, ala, Pro, Ser, Thr, Val or Met at a position in an HPPD protein, said position corresponding to position 294 of the amino acid sequence of SEQ ID No. 2;
   k. Gln, His or Asn at a position in an HPPD protein, said position corresponding to position 307 of the amino acid sequence of SEQ ID No. 2;
   l. Ile, Met, Leu or Asn at a position in an HPPD protein, said position corresponding to position 335 of the amino acid sequence of SEQ ID No. 2;
   m. Leu or Met at a position in an HPPD protein, said position corresponding to position 368 of the amino acid sequence of SEQ ID No. 2;
   n. Phe or Tyr at a position in an HPPD protein, said position corresponding to position 381 of the amino acid sequence of SEQ ID No. 2;
   o. Phe or Ser at a position in an HPPD protein, said position corresponding to position 392 of the amino acid sequence of SEQ ID No. 2;
   p. Phe or Tyr at a position in an HPPD protein, said position corresponding to position 419 of the amino acid sequence of SEQ ID No. 2;
   q. Asp, Phe, Lys, Asn, Ala, Cys, Glu, Gly, His, Ile, Leu, Met, Gln, Ser, Thr or Val at a position in an HPPD protein, said position corresponding to position 421 of the amino acid sequence of SEQ ID No. 2;
   r. Ala, Gly, Met, Pro or Thr at a position in an HPPD protein, said position corresponding to position 422 of the amino acid sequence of SEQ ID No. 2;
   s. Ala, Phe, Ile or Val at a position in an HPPD protein, said position corresponding to position 424 of the amino acid sequence of SEQ ID No. 2 or;
   t. Ile, Pro, Arg, Ala, Gly, Lys, Asn, Gln or Ser at a position in an HPPD protein, said position corresponding to position 425 of the amino acid sequence of SEQ ID No. 2;
   u. Glu, Phe, Thr, Ala, Gln, Ser or Val at a position in an HPPD protein, said position corresponding to position 426 of the amino acid sequence of SEQ ID No. 2;
   v. Ile, Met, Gln, Phe, Leu or Val at a position in an HPPD protein, said position corresponding to position 431 of the amino acid sequence of SEQ ID No. 2;
   w. an amino acid deletion or replacement at least one position in an HPPD protein, said position corresponding to at least one of positions 228, 248, 270, 271, 379 and 427 of the amino acid sequence of SEQ ID No. 2;
iii) determining the inhibition of the resulting HPPD protein by at least one herbicide acting on HPPD;
   wherein an inhibition of the resulting protein of less or more than that observed with a reference HPPD protein is indicative that the resulting protein is capable of modulating the tolerance of a plant to said herbicide.

It is to be understood that also the (more specific) amino acids and positions listed above for other embodiments, such as the nucleic acid of the invention, may be applied to the method of obtaining a mutated HPPD protein as described above.

In an alternative embodiment of the method of obtaining a mutated HPPD protein as described above, said mutated HPPD protein is capable of increasing the tolerance of a plant to at least one herbicide acting on HPPD.

Within the above method of obtaining in mutated HPPD protein, different herbicides acting on HPPD may be chosen. Accordingly, in another embodiment of the method of obtaining a mutated HPPD protein as described above, wherein said mutated HPPD protein is capable of increasing the tolerance of a plant to at least one herbicide acting on HPPD, the herbicide acting on HPPD is selected from triketones, or pyrazolinates, preferably tembotrione, mesotrione, topramezone or sulcotrione, bicyclopyrone, pyrasulfotole, pyrazolate, benzofenap and tefuryltrione, particularly tembotrione and such plants containing the HPPD of the invention have an agronomically acceptable tolerance to an HPPD inhibitor herbicide particularly to triketones, or pyrazolinates, preferably tembotrione, mesotrione, topramezone or sulcotrione, bicyclopyrone, pyrasulfotole, pyrazolate, benzofenap and tefuryltrione, particularly tembotrione.

In another embodiment, the present invention relates to a method of producing a transgenic plant comprising introducing into a said plant genome the nucleic acid of the present invention operably linked to a plant expressible promoter, the chimeric gene of the invention or a nucleic acid encoding the HPPD enzyme identified by the method of of claim 27 or 28.

In an alternative embodiment of the method of producing a transgenic plant as described above, the nucleic acid of the invention, wherein said mutated HPPD protein is capable of increasing the tolerance of a plant to at least one herbicide acting on HPPD, or a nucleic acid identified by the method of obtaining a mutated HPPD protein, wherein said mutated HPPD protein is capable of increasing the tolerance of a plant to at least one herbicie acting on HPPD, both operably linked to a plant expressible promoter, or the chimeric gene of the invention comprising a nucleic acid, wherein said mutated HPPD protein is capable of increasing the tolerance of a plant to at least one herbicie acting on HPPD, is introduced into said plant.

In another embodiment, the present invention relates to a plant cell comprising the isolated nucleic acid of the invention or the chimeric gene of the invention in its genetic information.

The present invention also relates to a plant, a part of a plant or plant tissue consisting essentially of the plant cells of the invention.

Furthermore, the present invention relates to a plant obtainable from the method of obtaining a mutated HPPD protein capable of modulating or increasing the tolerance of a plant to at least one herbicide acting on HPPD in all alternative aspects described above.

The plant of the present invention can be any plant. Non-limiting examples of plants of the invention include wheat, cotton, canola, rice, corn, soy bean, sorghum,canola, sunflower, tobacco, sugarbeet, cotton, maize, wheat, barley, rice, sorghum, tomato, mango, peach, apple, pear, strawberry, banana, melon, potato, carrot, lettuce, cabbage, onion, soya spp, sugar cane, pea, field beans, poplar, grape, citrus, alfalfa, rye, oats, turf and forage grasses, flax and oilseed rape, and nut producing plants. The present invention also relates to a seed of the plant of the invention. Furthermore, the invention relates to progeny of the plant of the invention or the seed of the invention.

In a further embodiment, the present invention relates to a method of modulating a plant's tolerance to at least one herbicide acting on HPPD comprising introducing the isolated nucleic acid of the invention operably linked to a plant expressible promoter or the chimeric gene of the invention into a plant's genome

In an alternative embodiment, the present invention relates to a method of increasing a plant's tolerance to at least one herbicide acting on HPPD or of obtaining a plant tolerant to an HPPD inhibitor herbicide comprising introducing the isolated nucleic acid of the invention, wherein said nucleic acid encodes a mutated HPPD protein which is capable of increasing the tolerance of a plant to at least one herbicide acting on HPPD, operably linked to a plant expressible promoter or the chimeric gene of the invention comprising a nucleic acid of the invention, wherein said nucleic acid encodes a mutated HPPD protein which is capable of increasing the tolerance of a plant to at least one herbicide acting on HPPD, into a plant's genome.

Furthermore, the present invention relates to a method for controlling weeds comprising spraying at least one herbicide acting on HPPD on or around a crop plant, wherein said crop plant comprises the nucleic acid of the present invention, wherein said mutated HPPD protein is capable of increasing the tolerance of a plant to at least one herbicide acting on HPPD, operably linked to a plant expressible promoter or the chimeric gene of the invention comprising the nucleic acid of the invention, wherein said mutated HPPD protein is capable of increasing the tolerance of a plant to at least one herbicide acting on HPPD. In alternative embodiment of the method of controlling weeds, the tolerance of said plant to at least one herbicie acting on HPPD is increased.

In addition, the present invention relates to the use of a chimeric gene of the invention or the nucleic acid of the invention operably linked to a plant expressible promoter for modulating the tolerance of a plant to at least one herbicide acting on HPPD.

In an alternative embodiment, the present invention relates to the use of a chimeric gene of the invention or the nucleic acid of the invention operably linked to a plant expressible promoter for increasing the tolerance of a plant to at least one herbicide acting on HPPD. In this embodiment of the invention, the chimeric gene used comprises the nucleic acid of the invention, wherein the mutated HPPD protein encoded thereby is capable of increasing the tolerance of a plant to at least one herbicide acting on HPPD. Alternatively, if a nucleic acid operably linked to a plant expressible promoter is used, said nucleic is chose that the mutated HPPD protein encoded thereby is capable of increasing the tolerance of a plant to at least one herbicide acting on HPPD.

The present invention also relates to the plant cell of the invention and the plant of the invention which may comprise a further useful trait as described further below.

While a number of herbicide-tolerant crop plants are presently commercially available, one issue that has arisen for many commercial herbicides and herbicide/crop combinations is that individual herbicides typically have incomplete spectrum of activity against common weed species. For most individual herbicides which have been in use for some time, populations of herbicide resistant weed species and biotypes have become more prevalent (see, e.g., Tranel and Wright (2002) Weed Science 50: 700-712; Owen and Zelaya (2005) Pest Manag. Sci. 61: 301-311). Transgenic plants which are resistant to more than one herbicide have been described (see, e.g., W02005/012515). However, improvements in every aspect of crop production, weed control options, extension of residual weed control, and improvement in crop yield are continuously in demand.

The HPPD protein or gene of the invention is advantageously combined in plants with other genes which encode proteins or RNAs that confer useful agronomic properties to such plants. Among the genes which encode proteins or RNAs that confer useful agronomic properties on the transformed plants, mention can be made of the DNA sequences encoding proteins which confer tolerance to one or more herbicides that, according to their chemical structure, differ from HPPD inhibitor herbicides, and others which confer tolerance to certain insects, those which confer tolerance to certain diseases, DNAs that encodes RNAs that provide nematode or insect control, etc...

Such genes are in particular described in published PCT Patent Applications WO 91/02071 and WO95/06128.

Among the DNA sequences encoding proteins which confer tolerance to certain herbicides on the transformed plant cells and plants, mention can be made of a bar or PAT gene or the Streptomyces coelicolor gene described in WO2009/152359 which confers tolerance to glufosinate herbicides, a gene encoding a suitable EPSPS which confers tolerance to herbicides having EPSPS as a target, such as glyphosate and its salts (US 4,535,060, US 4,769,061, US 5,094,945, US 4,940,835, US 5,188,642, US 4,971,908, US 5,145,783, US 5,310,667, US 5,312,910, US 5,627,061, US 5,633,435), or a gene encoding glyphosate oxydoreductase (US 5,463,175).

Among the DNA sequences encoding a suitable EPSPS which confer tolerance to the herbicides which have EPSPS as a target, mention will more particularly be made of the gene which encodes a plant EPSPS, in particular maize EPSPS, particularly a maize EPSPS which comprises two mutations, particularly a mutation at amino acid position 102 and a mutation at amino acid position 106 (WO 2004/074443), and which is described in Patent Application US 6566587, hereinafter named double mutant maize EPSPS or 2mEPSPS, or the gene which encodes an EPSPS isolated from Agrobacterium and which is described by sequence ID No. 2 and sequence ID No. 3 of US Patent 5,633,435, also named CP4.

Among the DNA sequences encoding a suitable EPSPS which confer tolerance to the herbicides which have EPSPS as a target, mention will more particularly be made of the gene which encodes an EPSPS GRG23 from Arthrobacter globiformis, but also the mutants GRG23 ACE1, GRG23 ACE2, or GRG23 ACE3, particularly the mutants or variants of GRG23 as described in WO2008/100353, such as GRG23(ace3)R173K of SEQ ID No. 29 in WO2008/100353.

In the case of the DNA sequences encoding EPSPS, and more particularly encoding the above genes, the sequence encoding these enzymes is advantageously preceded by a sequence encoding a transit peptide, in particular the "optimized transit peptide" described in US Patent 5,510,471 or 5,633,448.

In WO 2007/024782, plants being tolerant to glyphosate and at least one ALS (acetolactate synthase) inhibitor are disclosed. More specifically plants containing genes encoding a GAT (Glyphosate-N-Acetyltransferase) polypeptide and a polypeptide conferring resistance to ALS inhibitors are disclosed. In US 6855533, transgenic tobacco plants containing mutated Arabidopsis ALS/AHAS genes were disclosed.

In US 6,153,401, plants containing genes encoding 2,4-D-monooxygenases conferring tolerance to 2,4-D (2,4-dichlorophenoxyacetic acid) by metabolisation are disclosed.

In US 2008/0119361 and US 2008/0120739, plants containing genes encoding Dicamba monooxygenases conferring tolerance to dicamba (3,6-dichloro-2-methoxybenzoic acid) by metabolisation are disclosed.

All the above mentioned herbicide tolerance traits can be combined with those performing HPPD tolerance which are subject matter of this invention.

Among the DNA sequences encoding proteins concerning properties of tolerance to insects, mention will more particularly be made of the Bt proteins widely described in the literature and well known to those skilled in the art. Mention will also be made of proteins extracted from bacteria such as Photorhabdus (WO 97/17432 & WO 98/08932).

Among such DNA sequences encoding proteins of interest which confer novel properties of tolerance to insects, mention will more particularly be made of the Bt Cry or VIP proteins widely described in the literature and well known to those skilled in the art. These include the Cry1 F protein or hybrids derived from a Cry1 F protein (e.g., the hybrid Cry1A-Cry1F proteins described in US 6,326,169; US 6,281,016; US 6,218,188, or toxic fragments thereof), the Cry1A-type proteins or toxic fragments thereof, preferably the Cry1Ac protein or hybrids derived from the Cry1Ac protein (e.g., the hybrid Cry1Ab-Cry1Ac protein described in US 5,880,275) or the Cry1Ab or Bt2 protein or insecticidal fragments thereof as described in EP451878, the Cry2Ae, Cry2Af or Cry2Ag proteins as described in WO02/057664 or toxic fragments thereof, the Cry1A.105 protein described in WO 2007/140256 (SEQ ID No. 7) or a toxic fragment thereof, the VIP3Aa19 protein of NCBI accession ABG20428, the VIP3Aa20 protein of NCBI accession ABG20429 (SEQ ID No. 2 in WO 2007/142840), the VIP3A proteins produced in the COT202 or COT203 cotton events (WO 2005/054479 and WO 2005/054480, respectively), the Cry proteins as described in WO01/47952, the VIP3Aa protein or a toxic fragment thereof as described in Estruch et al. (1996), Proc Natl Acad Sci USA. 28;93(11):5389-94 and US 6,291,156, the insecticidal proteins from Xenorhabdus (as described in WO98/50427), Serratia (particularly from S. entomophila) or Photorhabdus species strains, such as Tc-proteins from Photorhabdus as described in WO98/08932 (e.g., Waterfield et al., 2001, Appl Environ Microbiol. 67(11):5017-24; Ffrench-Constant and Bowen, 2000, Cell Mol Life Sci.; 57(5):828-33). Also any variants or mutants of any one of these proteins differing in some (1-10, preferably 1-5) amino acids from any of the above sequences, particularly the sequence of their toxic fragment, or which are fused to a transit peptide, such as a plastid transit peptide, or another protein or peptide, is included herein.

The FIGURES show:
- FIGURE1:: Superpositon of the x-ray structure of HPPD from Arabidopsis thaliana (dark grey) and (a) Pseudomonas fluorescens (light grey), (b) Streptomyces avermitilis, (c) Homo sapiens, (d) Rattus norvegicus. The structures are shown as ribbon plot.
- FIGURE2:: Amino acids forming the binding site of (a) Arabidopsis thaliana, (b) Pseudomonas fluorescens (c) Streptomyces avermitilis (d) Homo sapiens, and (e) Rattus norvegicus (represented by bold lines). The core of the HPPD protein is shown as Cₐₗₚₕₐ trace and the iron is marked.
- FIGURE3:: Brown test color in Escherichia coli

### SEQUENCES LISTING

- SEQ ID No. 1:: Nucleic acid sequence encoding Arabidopsis thaliana HPPD
- SEQ ID No. 2:: Protein encoded by SEQ ID No. 1
- SEQ ID No. 3:: Nucleic acid sequence encoding Oryza sativa HPPD
- SEQ ID No. 4:: Protein encoded by SEQ ID No. 3
- SEQ ID No. 5:: Nucleic acid sequence encoding Zea mays HPPD
- SEQ ID No. 6:: Protein encoded by SEQ ID No. 5
- SEQ ID No. 7:: Nucleic acid sequence encoding Avena sativa HPPD
- SEQ ID No. 8:: Protein encoded by SEQ ID No. 7
- SEQ ID No. 9:: Nucleic acid sequence encoding Pseudomonas fluorescens HPPD
- SEQ ID No. 10:: Protein encoded by SEQ ID No. 9
- SEQ ID No. 13:: Nucleic acid sequence encoding Synechococcus sp. HPPD
- SEQ ID No. 14:: Protein encoded by SEQ ID No. 13
- SEQ ID No. 15:: Nucleic acid sequence encoding Blepharsima japonicum HPPD
- SEQ ID No. 16:: Protein encoded by SEQ ID No. 15
- SEQ ID No. 17:: Nucleic acid sequence encoding Rhodococcus RHA1 isolate ro0341 HPPD
- SEQ ID No. 18:: Protein encoded by SEQ ID No. 17
- SEQ ID No. 19:: Nucleic acid sequence encoding Picrophilus torridus HPPD
- SEQ ID No. 20:: Protein encoded by SEQ ID No. 19
- SEQ ID No. 21:: Nucleic acid sequence encoding Kordia algicida HPPD
- SEQ ID No. 22:: Protein encoded by SEQ ID No. 21
- SEQ ID No. 23:: Nucleic acid sequence encoding Sorghum bicolor HPPD
- SEQ ID No. 24:: Protein encoded by SEQ ID No. 23
- SEQ ID No. 25:: Nucleic acid sequence encoding Triticum aestivum HPPD
- SEQ ID No. 26:: Protein encoded by SEQ ID No. 25
- SEQ ID No. 27:: Nucleic acid sequence encoding Hordeum vulgare HPPD
- SEQ ID No. 28:: Protein encoded by SEQ ID No. 27
- SEQ ID No. 29:: Nucleic acid sequence encoding Rhodococcus RHA1 isolate ro0240 HPPD
- SEQ ID No. 30:: Protein encoded by SEQ ID No. 29
- SEQ ID No. 31:: Nucleic acid sequence encoding Arabidopsis thaliana wild type HPPD polypeptide, plus containing at the 5' end a nucleic acid encoding an alanine and 6 histidine amino acids
- SEQ ID No. 32:: Protein encoded by SEQ ID No. 31
- SEQ ID No. 33:: Nucleic acid sequence encoding mutant HPPD polypeptide, plus containing at the 5' end a nucleic acid encoding an alanine and 6 histidine amino acids
- SEQ ID No. 34:: Protein encoded by SEQ ID No. 33,
- SEQ ID No. 35:: Nucleic acid sequence encoding mutant HPPD polypeptide, plus containing at the 5' end a nucleic acid encoding an alanine and 6 histidine amino acids
- SEQ ID No. 36:: Protein encoded by SEQ ID No. 35,
- SEQ ID No. 37:: Nucleic acid sequence encoding mutant HPPD polypeptide, plus containing at the 5' end a nucleic acid encoding an alanine and 6 histidine amino acids
- SEQ ID No. 38:: Protein encoded by SEQ ID No. 37,
- SEQ ID No. 39:: Nucleic acid sequence encoding mutant HPPD polypeptide, plus containing at the 5' end a nucleic acid encoding an alanine and 6 histidine amino acids
- SEQ ID No. 40:: Protein encoded by SEQ ID No. 39,
- SEQ ID No. 41:: Nucleic acid sequence encoding mutant HPPD polypeptide, plus containing at the 5' end a nucleic acid encoding an alanine and 6 histidine amino acids
- SEQ ID No. 42:: Protein encoded by SEQ ID No. 41,
- SEQ ID No. 43:: Nucleic acid sequence encoding mutant HPPD polypeptide, plus containing at the 5' end a nucleic acid encoding an alanine and 6 histidine amino acids
- SEQ ID No. 44:: Protein encoded by SEQ ID No. 43,
- SEQ ID No. 45:: Nucleic acid sequence encoding mutant HPPD polypeptide, plus containing at the 5' end a nucleic acid encoding an alanine and 6 histidine amino acids
- SEQ ID No. 46:: Protein encoded by SEQ ID No. 45
- SEQ ID No. 47:: Nucleic acid sequence encoding mutant HPPD polypeptide, plus containing at the 5' end a nucleic acid encoding an alanine and 6 histidine amino acids
- SEQ ID No. 48:: Protein encoded by SEQ ID No. 47
- SEQ ID No. 49:: Nucleic acid sequence encoding mutant HPPD polypeptide, plus containing at the 5' end a nucleic acid encoding an alanine and 6 histidine amino acids
- SEQ ID No. 50:: Protein encoded by SEQ ID No. 49,

The Examples illustrate the invention

### Example1:

A mutation of strictly conserved amino acids leads to an inactive protein. The point mutants S267A (SEQ ID No. 34) in A.thaliana and N282A (SEQ ID No. 36) in A.thaliana are inactive. The amino acid position given are referring to the position of SEQ ID No. 2.

Mutant HPPD polypeptides of the presenting invention have amino acids changes at one or more positions relative to the starting wild type sequence from which they are derived.

The DNA sequence SEQ ID No. 2 encoding the wild HPPD protein from Arabidopsis thaliana (1335 bp; Genebank AF047834; WO 96/38567) was cloned into the vector pSE420(RI)NX (modified from the the cloning and expression vector pSE420(RI)NX (5261 bp) is based on the plasmid pSE420 by Invitrogen (Karlsruhe, Germany)). At the 5' end, directly downstream to the ATG was inserted a nucleic acid sequence coding for an alanine amino acid and a nucleic acid sequence encoding a N-terminal HIS6-Tag (6x HIS, encoded by: cat cat cat cac cat cat). The resulting sequence is presented as SEQ ID No. 31. Upstream to the ATG, two additional cysteine base pairs were added in order to obtain a sequence corresponding to the recognition site of the restriction enzyme Ncol and downstream to the stop codon the sequences corresponding to the recognition site of the restriction enzyme Xbal were added. The resulting plasmid was then used to transform E. Coli cells BL21 (DE3) with 50µg/ml kanamycin or 100µg/ml carbenicillin selection as described in European patent applications "EP09015984.9"; EP09015985.6"; EP 09015986.4" EP 09015987.2"; and EP09015988.0" all filed on 23. December 2009.

Quick test of the activity of HPPD protein: Production of brown color

### Colorimetric Screening Test for Active HPPD Enzymes:

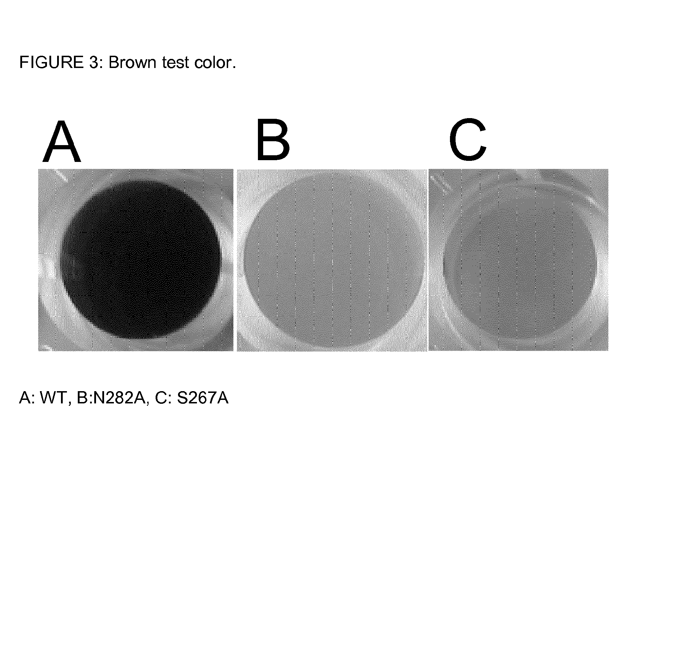

A YT-broth-type culture medium with 1% agarose, 5mM L-Tyrosine and 42mM Succinate, which contains the selection agent for the vector pSE420 is poured into deep well plates. E.coli culture in the exponentional growth phase which contains the vector pSE420-HPPDx (any gene coding for a putative HPPD enzyme/protein) is applied to each well. After 16 hours at 37°C, the wells which do not contain the culture medium, those which have been seeded with an E. coil culture containing the empty vector pSE420 are transparent, or those which have been seeded with an E. coli culture containing a vector pSE420 containing a gene coding for an inactive HPPD are transparent, while the wells seeded with an E. coli culture containing the vector pSE420-HPPD coding for active HPPD are brown. It has been previously demonstrated that this test refelects the HPPD activity, whatever the orgin of this activity, and allows the identification of HPPD activities (US 6,768,044). As it can be seen on Figure 3, the culture from bacteria containing the gene coding for the mutant HPPD did not develop a brown color while the one containing the gene coding of the wild type HPPD developed a strong brown color reflecting the activity of the HPPD enzymes. It can be concluded that the two HPPD mutants are not able to convert HPP into homogentisate. The two HPPD mutants are inactive. One can conclude that the amino acid positions 267 and 282 (referring to position in SEQ ID No. 2) are essential for HPPD activity.

The expression of the HPPD protein was then done as following.

Overnight cultures grown at 37°C were used to inoculate LB media at a ratio 1:100. Cells were allowed to grow until OD reached 0.5, then expression was initiated from the trp-lac (trc) promoter by induction with 1 mM IPTG which binds to the lac repressor and causes its dissociation from the lac operon. Expression was carried out over 15 h at 28 °C.

To prepare the pre-starter culture, 2 mL of TB medium (100 µg*mL⁻¹ carbenicillin) were inoculated with 50 µL of an E. coli K-12 BL21 glycerol stock. The pre-starter culture was incubated at 37 °C with shaking at 140 rpm for 15 h. 200µl of the pre-starter culture was used to initiate the starter culture (5mL TB supplement with 100 µg*L⁻¹), which was incubated 3 h at 37°C.

To prepare the main culture, 400 mL of TB medium (100 µg*mL⁻¹ carbenicillin) were inoculated with 4 mL of the starter culture. This starter culture was incubated at 37 °C with shaking at 140 rpm until OD₆₀₀ 0.5 was reached. Then recombinant protein expression was induced with 400 µl of 1 M IPTG solution. The cells were allowed to grow for an additional hour under these conditions, then the temperature was lowered to 28°C and the culture was shaken at 140 rpm for 15 h. Cells were harvested by centrifugation at 6000 x g for 15 min at 4 °C. Then cell pellets were stored at -80 °C.

Isolation and purification of His₆-AtHPPD in native form

### Lysis of cells

Cells were lysed using Lysozyme, an enzyme that cleaves the 1,4-β-linkages between N-acetylmuramic acid and N-acetyl-D-glucosamine residues in peptidoglycan which forms the bacterial cell wall. Cell membranes were then disrupted by the internal pressure of the bacterial cell. In addition, the lysis buffer contained Benzonase^{®} Nuclease, an endonuclease that hydrolyzes all forms of DNA and RNA without damaging proteins and thereby largely reduces viscosity of the cell lysate. Lysis under native conditions was carried out on ice.

For purification of His₆-tagged proteins the QlAexpress^{®} Ni-NTA Fast Start Kit was used following the user manual instruction.

### Purification of His₆-tagged proteins by immobilized metal ion affinity chromatography (IMAC)

The cleared cell lysate (10 mL) obtained after centrifugation of the lysis reaction was loaded onto a Ni-NTA Fast Start Column from the QlAexpress^{®} Ni-NTA Fast Start Kit (Qiagen, Hilden, Germany) and purification was carried out according to the instruction manual. The His₆-tagged protein was eluted with 2.5 mL of elution buffer.

### Desalting of HPPD solutions by gel filtration

HPPD solutions eluted from a Ni-NTA Fast Start Column with 2.5 mL of elution buffer were applied to a Sephadex G-25 PD-10 column (GE Healthcare, Freiburg, Germany) following the user manual instruction. After the whole sample had entered the gel bed, elution was performed with 3.5 mL of storage buffer. The HPPD solutions eluted from the desalting column were frozen at -80 °C in 1 mL aliquots.

Determination of HPPD protein concentration using the Bradford protein assay Protein concentration was determined using the standard Bradford assay (Bradford, (1976), Anal Biochem 72: 248-254).

### Determination of purity of HPPD solutions using SDS-PAGE

The integrity of the eluted protein was checked by SDS-PAGE protein gel electrophoresis using the gel NuPAGE^{®} Novex 4-12 % Bis-Tris Gels (Invitrogen, Karlsruhe, Germany), approximately 10 µg of protein were loaded. 10 µL of Laemmli Sample Buffer was added to 1-10 µL of protein solution and the mixture was incubated at 90 °C for 10 min. After short centrifugation step, the whole mixture was loaded into a slot of an SDS gel previously fixed in a XCell SureLock^{™} Novex Mini-Cell gel chamber filled with NuPAGE^{®} MOPS SDS Running Buffer (diluted from the 20 x-solution with ddH₂O). A voltage of 150 was then applied to the gel chamber for 1 h. For staining of protein bands, the gel was immersed in Coomassie Brilliant Blue R-250 Staining Solution. For destaining of the polyacrylamide gel, it was immersed in Coomassie Brilliant Blue R-250 Destaining Solution until protein bands appear blue on a white gel.

The HPPD activity was checked by the standard spectrophotmetric assay (method extensively described in WO 2009/144079)

In this content, pl₅₀-value means the log value of the concentration of inhibitor necessary to inhibit 50% of the enzyme activity in molar concentration.

pl₅₀-values for HPPD inhibitors were determined from dose-response plots of HPPD activity versus inhibitor concentration using the assay extensively described in WO 2009/144079 at 2 mM fixed HPP concentration and 3 minutes fixed incubation time using the ID Business Solutions Ltd. XLfit software suite.

Table 7: Determination of pl₅₀ HPPD enzymes (Arabidopsis thaliana wild type HPPD "SEQ ID No. 32", the mutants S267A (SEQ ID No. 34) and N282A (SEQ ID No. 36)" and the tolerance to the several listed below HPPD inhibitors tembotrione, diketonitrile, mesotrione, bicyclopyrone, pyrasulfotole, sulcotrione, pyrazolate, tefuryltrione, and benzofenap. The symbol ">" means that the value was far higher than the one indicated but could not be precisely calculated within in the range of concentration of inhibitor tested (2.5x10⁻⁶, 5.0x10⁻⁶, 1.0x10⁻⁵, 2.5x10⁻⁵ 6.3x10⁻⁵, and 2.5x10⁻⁴M).

| | Bicyclopyrone | Benzofenap | Diketonitrile | Mesotrione |
|---|---|---|---|---|
| WT (SEQ ID No. 32) | 5.2 | >5.6 | >5.6 | >5.6 |
| S267A (SEQ ID No. 34) | nd - la (P) | nd - la (P) | nd - la (P) | nd - la (P) |
| N282A (SEQ ID No. 36) | nd - la (P) | nd - la (P) | nd - la (P) | nd - la (P) |

| | Pyrasulfotole | Pyrazolate | Sulcotrione | Tefuryltrione | Tembotrione |
|---|---|---|---|---|---|
| WT (SEQ ID No. 32) | 5.4 | 5.4 | >5.6 | >5.6 | >5.6 |
| S267A (SEQ ID No. 34) | nd - la (P) | nd - la (P) | nd - la (P) | nd - la (P) | nd - la (P) |
| N282A (SEQ ID No. 36) | nd - la (P) | nd - la (P) | nd - la (P) | nd - la (P) | nd - la (P) |

As previously demonstrated, the 2 protein mutants are not able to convert HPP into Homogentisate, confirming that the 2 mutant proteins are inactive. It confirms the hypothesis that the position 267 and 282 (referring to position in SEQ ID No. 2) are absolutely essential for obtaining an active HPPD

### Example 2: Single point mutants displayed increased tolerance to HPPD inhibitor herbicides:

Kinetic characterization and evaluation of tolerance to HPPD inhibitors of HPPD enzyme "SEQ ID No. 32".

The HPPD activity was checked by the standard spectrophotmetric assay (method extensively described in WO 2009/144079)

### Determination of HPPD in vitro kinetic properties

Kₘ, Vₘₐₓ, and k_{cat} values for different HPPD enzyme preparations and Kᵢ, K₁=Kₒₙ, and K₋₁=K_{off} for different HPPD inhibitors were determined using a HPLC assay for measurements of HPPD activity. The assay mixtures contained in a volume of 1 ml 150 mM Tris-HCl buffer at pH 7.8, 10 mM sodium ascorbate, 650 units of bovine catalase (Sigma C30 (Sigma-Aldrich, Munich,Germany), 34 mg protein/ml, 23,000 units/mg), and appropriate amounts of HPP, purified HPPD enzyme and HPPD inhibitors. For Kₘ, Vₘₐₓ, and k_{cat} value determination HPP concentrations in the assay mixture were varied between 10 and 400 µM. For Kᵢ, K₁=Kₒₙ, and K₋₁=K_{off} value determination 2 mM HPP was used. All assays were started by the addition of HPPD enzyme to the assay mixture and stopped at a series of times between 0 and 240 s by addition of 200 µl of the reaction mixture to reaction assay tubes containing 20 µl 10% perchloric acid. Precipitated protein was pelleted by a 5 minute centrifugation at 10,000 g. 100 µl of the supernatant were loaded onto a 250 x 4mm Knauer (Berlin, Germany) Eurospher 100-5 C18-column equilibrated with 10% methanol, 0.1% trifluoroacetic acid (buffer A). The column was eluted, also at 1.5 ml/min, using a 4 minute wash with buffer A, followed by a 3 min wash with 95% methanol and by a further 2 minute wash with buffer A. The elution of HGA (homogentisic acid) and HPP (hydroxyphenylpyruvate) was monitored at 292 nm. HGA elutes at around 5 minutes and HPP elutes later. A standard set of concentrations of HGA were used to provide a standard curve in order to calibrate the 292 nm absorbance of the HGA peak versus HGA concentration. For Kₘ and Vₘₐₓ value determinations the initial rates of the HPPD reaction at different substrate concentrations were determined from plots of HGA formed versus time and fitted to the Michaelis-Menten equation for unireactant enzymes using the ID Business Solutions Ltd. (www.idbs.com) XLfit software suite. For the determination of Kᵢ, K₁=Kₒₙ, and K₋₁=K_{off} values the time-courses of the HPPD reaction at different inhibitor concentrations were fitted to the equations for Mechanism A, competitive inhibition, for tight-binding inhibitors (Cha, S. (1975) Tight-binding inhibitors - I. Kinetic behaviour. Biochemical Pharmacology 24, 2177-2185) using the ID Business Solutions Ltd. XLfit software suite

### Table 8

Protein SEQ ID No. 32 consists of protein SEQ ID No. 2 with an insertion of one amino acid Ala and six amino acids His directly behind the first methionine.

Kinetic characterization of HPPD enzymes (Arabidopsis thaliana "SEQ ID No. 32" and the respective tolerance to the HPPD inhibitors tembotrione and diketonitrile. In below given table1, "Kₘ" (Michaelis-Menten constant) means the kinetic parameter that is used to characterise an enzyme, and it is defined as the concentration of substrate that permits half maximal rate of the reaction. Kₘ is further defined as the substrate concentration at which the reaction rate reaches half of its maximum value (Vₘₐₓ/2) where Vmax has the meaning of being the maximum velocity of the reaction. Kₒₙ=K₁ equals the association rate constant of the enzyme-substrate binding and K_{off}=K₋₁ equals the rate constant of the enzyme-inhibitor complex dissociation. Ki defines the inhibition constant.

| | HPP | | Tembotrione | | | Diketonitrile | | |
|---|---|---|---|---|---|---|---|---|
| | Kₘ (µM) | Vₘₐₓ (µM) | k₁ (M⁻¹ s⁻¹) | k₋₁ (s⁻¹) | Kᵢ (µm) | k₁ (M⁻¹ s⁻¹) | k₋₁ (s⁻¹) | Kᵢ (µm) |
| SEQ ID No. 32 | 6.3 | 1.2 | 2.3E+05 | 3.5E-03 | 0.015 | 6.1E+05 | 1.1E-02 | 0.018 |

The kinetic parameters Kₘ and Vₘₐₓ of the plant HPPD "SEQ ID No. 32" summarized in Table 8 are well in the range of the specific activities measured from purified plant HPPD as for example for the carrot HPPD (Garcia et al. 2000, Biochemistry, 39, 7501-7507). The Arabidopsis thaliana HPPD is also sensitive in the smimilar range as the carrot HPPD to diketonitrile. It is the first time, that the kinetic parameter of Arabidopsis thaliana HPPD in presence of tembotrione are measured and being in a comparable range as the one measured in presence of the HPPD inhibitor diketonitrile.

### Determination of HPPD activity in presence of several HPPD inhibitors

In this content, pl₅₀-value means the log value of the concentration of inhibitor necessary to inhibit 50% of the enzyme activity in molar concentration.

pl₅₀-values for HPPD inhibitors were determined from dose-response plots of HPPD activity versus inhibitor concentration using the assay extensively described in WO 2009/144079 at 2 mM fixed HPP concentration and 3 minutes fixed incubation time using the ID Business Solutions Ltd. XLfit software suite.

Table 9: Determination of pl₅₀ HPPD enzymes (Arabidopsis thaliana "SEQ ID No. 32" and some Arabidopsis HPPD single point mutants "SEQ ID No. 35, SEQ ID No. 40, SEQ ID No. 42, SEQ ID No. 44, SEQ ID No. 46, SEQ ID No. 48, and SEQ ID No. 50") and their respective tolerance to the several listed below HPPD inhibitors tembotrione, diketonitrile, mesotrione, bicyclopyrone, pyrasulfotole, sulcotrione, pyrazolate, tefuryltrione, and benzofenap. The symbol ">" means that the value was far higher than the one indicated but could not be precisely calculated within in the range of concentration of inhibitor tested (2.5x10⁻⁶, 5.0x10⁻⁶, 1.0x10⁻⁵, 2.5x10⁻⁵, 6.3x10⁻⁵, and 2.5x10⁻⁴M).

| | Bicyclopyrone | Benzofenap | Diketonitrile | Mesotrione | Pyrasulfotole |
|---|---|---|---|---|---|
| SEQ ID No. 32 | 5.2 | >5.6 | >5.6 | >5.6 | 5.4 |
| E252S (SEQ ID No. 36) | 4.3 | 5.2 | 4.9 | 5.3 | 4.3 |
| V269A (SEQ ID No. 40) | 4.3 | 5.2 | 5.2 | 5.2 | 4.4 |
| P280A SEQ ID No. 42 | 4.3 | 5.2 | 5.2 | 5.3 | 4.4 |
| L368M SEQ ID No. 44 | 4.5 | >5.6 | 5.6 | 5.4 | 5.0 |
| K421I SEQ ID No. 46 | 4.2 | 5.1 | 5.0 | 5.2 | 4.3 |
| K421 Q SEQ ID No. 48 | 4.7 | >5.6 | >5.6 | >5.6 | 4.8 |
| K421V SEQ ID No. 50 | 4.2 | 5.2 | 5.1 | 5.3 | 4.4 |

| | Pyrazolat | Sulcotrione | Tefuryltrione | Tembotrione |
|---|---|---|---|---|
| SEQ ID No. 32 | 5.4 | >5.6 | >5.6 | >5.6 |
| E252S (SEQ ID No. 35) | 4.7 | 5.1 | 5.2 | 5.5 |
| V269A (SEQ ID No. 40) | 4.4 | 5.0 | 5.4 | >5.6 |
| P280A SEQ ID No. 42 | 4.7 | 5.0 | 5.5 | >5.6 |
| L368M SEQ ID No. 44 | 4.9 | 5.2 | >5.6 | >5,6 |
| K421I SEQ ID No. 46 | 4.5 | 5.0 | 5.2 | 5.6 |
| K421 Q SEQ ID No. 48 | 5.0 | >5.6 | >5.6 | >5.6 |
| K421V SEQ ID No. 50 | 4.5 | 5.1 | 5.4 | >5.6 |

Measurements show that the pl₅₀ in case of the point mutants is reduced compared to the wild-type protein.

In Table 9, it can be clearly seen that the HPPD mutants are all affected in their tolerance to at least one HPPD inhibitors and some to several HPPD inhibitors. All the tested mutants are more active (do show an increased tolerance concerning the various applyied HPPD inhibitors) in the presence of at least one of the selected HPPD inhibitors, therefore the mutant enzyme-inhibitor complex should be less stable the wild-type enzyme-inhibitor complex. It can be concluded that the positions 252, 269, 280, 368 and 421 (reference taken in SEQ ID No. 2) are all important in the modulation of tolerance of HPPD enzyme to HPPD inhibitors.

## Claims

1. An isolated nucleic acid comprising a nucleotide sequence encoding a mutated HPPD protein, wherein said mutated HPPD protein has HPPD activity, and wherein in said mutated HPPD protein at least one amino acid has been replaced so that the resulting amino acid sequence comprises anamino acid selected from Ile, Met or Asn at a position in an HPPD protein, said position corresponding to position 335 of the amino acid sequence of SEQ ID No. 2.

2. The isolated nucleic acid of claim 1, wherein said HPPD protein is derived from Streptomyces avermitilis (Genebank SAV11864), Daucus carota (Genebank DCU 87257), Arabidopsis thaliana (Genebank AF047834), Mycosphaerella graminicola (Genebank AF038152), oryza sativa / rice [BAD26248], Zea mays / corn [ACN36372], avena sativa [ABZ23427], Pseudomonas fluorescens [ABF50055], Synechococcus sp. [YP_473959], Blepharisma japonicum [BAF91881], Rhodococcus RHA1 sp. ro0240 [YP_702005], Rhodococcus RHA1 sp. ro0341 [YP_703002], Picrophilus torridus [YP_024147], Kordia algicida [ZP_02161490], Sorghum bicolor [XP_002453359], Triticum aestivum / wheat [AAZ67144],
or Hordeum vulgare / barley [O48604].

3. The isolated nucleic acid of claim 1, wherein said HPPD protein comprises the amino acid sequence of SEQ ID No. 4 and wherein the resulting amino acid sequence comprises an amino acid selected from Ile, Met or Asn at position 332 of the amino acid sequence of SEQ ID No. 4 (corresponding to position 335 of SEQ ID No. 2).

4. The isolated nucleic acid of claim 1, wherein said HPPD protein comprises the amino acid sequence of SEQ ID No. 6 and wherein the resulting amino acid sequence comprises an amino acid selected from Ile or Asn at position 309 of the amino acid sequence of SEQ ID No. 6 (corresponding to position 335 of SEQ ID No. 2).

5. The isolated nucleic acid of claim 1, wherein said HPPD protein comprises the amino acid sequence of SEQ ID No. 8 and wherein the resulting amino acid sequence comprises an amino acid selected from Ile at position 326 of the amino acid sequence of SEQ ID No. 8 (corresponding to position 335 of SEQ ID No. 2).

6. The isolated nucleic acid of claim 1, wherein said HPPD protein comprises the amino acid sequence of SEQ ID No. 10 and wherein the resulting amino acid sequence comprises an amino acid selected from Ile or Asn at position 264 of the amino acid sequence of SEQ ID No. 10 (corresponding to position 335 of SEQ ID No. 2).

7. The isolated nucleic acid of claim 1, wherein said HPPD protein comprises the amino acid sequence of SEQ ID No. 14 and wherein the resulting amino acid sequence comprises an amino acid selected from Ile, Met or Asn at position 244 of the amino acid sequence of SEQ ID No. 14 (corresponding to position 335 of SEQ ID No. 2).

8. The isolated nucleic acid of claims 1, wherein said HPPD protein comprises the amino acid sequence of SEQ ID No. 16 and wherein the resulting amino acid sequence comprises an amino acid selected from Ile, Met or Asn at position 289 of the amino acid sequence of SEQ ID No. 16 (corresponding to position 335 of SEQ ID No. 2).

9. The isolated nucleic acid of claim 1, wherein said HPPD protein comprises the amino acid sequence of SEQ ID No. 18 and wherein the resulting amino acid sequence comprises an amino acid selected from k) Ile, Met or Asn at position 311 of the amino acid sequence of SEQ ID No. 18 (corresponding to position 335 of SEQ ID No. 2).

10. The isolated nucleic acid of claim 1, wherein said HPPD protein comprises the amino acid sequence of SEQ ID No. 30 and wherein the resulting amino acid sequence comprises an amino acid selected from Ile, Met or Asn at position 312 of the amino acid sequence of SEQ ID No. 30 (corresponding to position 335 of SEQ ID No. 2).

11. The isolated nucleic acid of claims 1, wherein said HPPD protein comprises the amino acid sequence of SEQ ID No. 20 and wherein the resulting amino acid sequence comprises an amino acid selected from Ile, Met or Asn at position 280 of the amino acid sequence of SEQ ID No. 20 (corresponding to position 335 of SEQ ID No. 2).

12. The isolated nucleic acid of claim 1, wherein said HPPD protein comprises the amino acid sequence of SEQ ID No. 22 and wherein the resulting amino acid sequence comprises an amino acid selected from Ile, Met or Asn at position 299 of the amino acid sequence of SEQ ID No. 22 (corresponding to position 335 of SEQ ID No. 2).

13. The isolated nucleic acid of claim 1, wherein said HPPD protein comprises the amino acid sequence of SEQ ID No. 24 and wherein the resulting amino acid sequence comprises an amino acid selected from Ile or Asn at position 326 of the amino acid sequence of SEQ ID No. 24 (corresponding to position 335 of SEQ ID No. 2).

14. The isolated nucleic acid of claim 1, wherein said HPPD protein comprises the amino acid sequence of SEQ ID No. 26 and wherein the resulting amino acid sequence comprises an amino acid selected from Ile, Met or Asn at position 322 of the amino acid sequence of SEQ ID No. 26 (corresponding to position 335 of SEQ ID No. 2).

15. The isolated nucleic acid of claim 1, wherein said HPPD protein comprises the amino acid sequence of SEQ ID No. 2 and wherein the resulting amino acid sequence comprises an amino acid selected from Ile or Asn at position 335 of the amino acid sequence of SEQ ID No. 2.

16. The nucleic acid of any one of claims 1 to 15, wherein said mutated HPPD protein is capable of increasing the tolerance of a plant to at least one herbicide acting on HPPD.

17. A protein encoded by the isolated nucleic acid of any one of claims 1 to 15.

18. A chimeric gene comprising a coding sequence comprising the nucleic acid of any one of claims 1 to 15 operably linked to a plant-expressible promoter and optionally a transcription termination and polyadenylation region.

19. A method of obtaining a mutated HPPD protein capable of modulating the tolerance of a plant to at least one herbicide acting on HPPD, wherein said mutated HPPD protein has HPPD activity, the method comprising
i) providing an HPPD protein;
ii) replacing one amino acid in said HPPD protein so that the resulting amino acid sequence comprises one selected from Ile, Met or Asn at a position in an HPPD protein, said position corresponding to position 335 of the amino acid sequence of SEQ ID No. 2
iii) determining the inhibition of the resulting HPPD protein by at least one
herbicide acting on HPPD,
wherein an inhibition of the resulting protein of less or more than that observed with a reference HPPD protein is indicative that the resulting protein is capable of modulating the tolerance of a plant to said herbicide.

20. The method of claim 19, wherein said mutated HPPD protein is capable of increasing the tolerance of a plant to at least one HPPD inhibitor herbicide.

21. The nucleic acid of claim 16 or the method of claim 19, wherein the herbicide acting on HPPD is tembotrione.

22. A method of producing a transgenic plant comprising introducing into a said plant the nucleic acid of any one of claims 1 to 16 or a nucleic acid encoding the HPPD enzyme identified by the method of any one of claims 19 to 20, both operably linked to a plant expressible promoter or the chimeric gene of claim 18, and obtaining progeny thereof comprising said operably linked nucleic acid or said chimeric gene.

23. The method of claim 22, wherein the nucleic acid of claim 16 or a nucleic acid identified by the method of claim 19 or 20, both operably linked to a plant expressible promoter, or the chimeric gene of claim 18 comprising the nucleic acid of any one of claims 1 to 16 is introduced into said plant.

24. The nucleic acid of any one of claims 1 to 15 or the method of any one of claims 22 to 23, wherein said HPPD protein comprises
a) a His at a position in an HPPD protein, said position corresponding to position 226 of the amino acid sequence of SEQ ID No. 2;
b) a Ser at a position in an HPPD protein, said position corresponding to position 267 of the amino acid sequence of SEQ ID No. 2;
c) an Asn at a position in an HPPD protein, said position corresponding to position 282 of the amino acid sequence of SEQ ID No. 2;
d) a His at a position in an HPPD protein, said position corresponding to position 308 of the amino acid sequence of SEQ ID No. 2;
e) a Tyr at a position in an HPPD protein, said position corresponding to position 342 of the amino acid sequence of SEQ ID No. 2;
f) a Glu at a position in an HPPD protein, said position corresponding to position 394 of the amino acid sequence of SEQ ID No. 2;
g) a Gly at a position in an HPPD protein, said position corresponding to position 420 of the amino acid sequence of SEQ ID No. 2; and
h) an Asn at a position in an HPPD protein, said position corresponding to position 423 of the amino acid sequence of SEQ ID No. 2

25. A plant cell comprising the isolated nucleic acid of any one of claims 1 to 15 or the chimeric gene of claim 18 in its genetic information.

26. A plant, part thereof or plant tissue consisting essentially of the plant cells of claim 25.

27. A plant obtainable from the method of any one of claims 25 or 26.

28. The plant of claim 26 or 27 which is selected from wheat, cotton, canola, rice, corn, soy bean, and sorghum.

29. A seed of the plant of any one of claims 26 to 28.

30. Progeny of the plant of any one of claims 26 to 28 or the seed of claim 29.

31. A method of modulating a plant's tolerance to at least one herbicide acting on HPPD or of obtaining a plant tolerant to an HPPD inhibitor herbicide comprising introducing the isolated nucleic acid of any one of claims 1 to 15 operably linked to a plant expressible promoter or the chimeric gene of claim 18 into a plant's genome.

32. A method for controlling weeds comprising spraying at least one HPPD inhibitor herbicide on or around a crop plant, wherein said crop plant contains the nucleic acid according to any one of claims 1 to 15 operably linked to a plant expressible promoter or the chimeric gene of claim 18 comprising the nucleic acid of any one of claims 1 to 15.

33. Use of a chimeric gene of claim 18 or the nucleic acid of any one of claims 1 to 15 operably linked to a plant expressible promoter for modulating the tolerance of a plant to at least one HPPD inhibitor herbicide applied for controlling weeds.

34. The method of claim 19, 22, 31 or 32 or the use of claim 33, wherein the tolerance of a plant to at least one HPPD inhibitor herbicide is increased.
